(19) Europäisches Patentamt
European Patent Office
Office européen des brevets

(11) EP 2 599 872 A2

(12) EUROPEAN PATENT APPLICATION

(43) Date of publication:
**05.06.2013 Bulletin 2013/23**

(21) Application number: **13157387.5**

(22) Date of filing: **23.01.2009**

(51) Int Cl.:
***C12N 15/82*** *(2006.01)* ***C07K 14/415*** *(2006.01)*
***A01H 5/00*** *(2006.01)*

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority:
**25.01.2008 EP 08150637**
**31.01.2008 EP 08150897**
**31.01.2008 EP 08150913**
**31.01.2008 EP 08150893**
**31.01.2008 EP 08150912**
**26.02.2008 US 31546 P**
**26.02.2008 US 31444 P**
**27.02.2008 US 31736 P**
**27.02.2008 US 31713 P**
**27.02.2008 US 31716 P**
**27.02.2008 US 31723 P**

(62) Document number(s) of the earlier application(s) in accordance with Art. 76 EPC:
**09703383.1 / 2 245 168**

(71) Applicant: **BASF Plant Science GmbH**
**67056 Ludwigshafen (DE)**

(72) Inventor: **Sanz Molinero, Ana Isabel**
**28035 Madrid (ES)**

(74) Representative: **Hennin, Caroline Marie Odile**
**BASF SE**
**Global Intellectual Property**
**Carl-Bosch-Strasse 38**
**67056 Ludwigshafen (DE)**

Remarks:
This application was filed on 01-03-2013 as a divisional application to the application mentioned under INID code 62.

# (54) Plants having enhanced yield-related traits and a method for making the same

(57) The present invention relates generally to the field of molecular biology and concerns a method for enhancing various yield-related traits by modulating expression in a plant of a nucleic acid encoding an SCE1 (SUMO Conjugating Enzyme 1). The present invention also concerns plants having modulated expression of a nucleic acid encoding an SCE1, which plants have enhanced yield-related traits relative to corresponding wild type plants or other control plants. The invention also provides hitherto unknown SCE1-encoding nucleic acids, and constructs comprising the same, useful in performing the methods of the invention.

EP 2 599 872 A2

SEQUENCE LISTING

<110> BASF Plant Science GmbH

<120> Plants having enhanced yield-related traits and a method for making the same

<130> PF60503

<160> 450

<170> PatentIn version 3.3

<210> 1
<211> 279
<212> DNA
<213> Triticum aestivum

<400> 1
```
atgtcgagcc gtaggtcaag gtcaaggcag tccggctcgt cgaggatcac tgacgagcaa     60

atcagcgacc ttgtctccaa gttgcaggac ctccttcccg aggcgcgtct ccggggcaat    120

gatagagtgc catcttcaag ggtgctgcag gagacgtgca cctacatcag gagcctgcac    180

cgggaggtgg acgacctgag cgagaggctg tcggagctgc tggcgacctc ggacatgagc    240

agcgcgcaag cggccatcat ccgcagcttg ctgatgtag                           279
```

<210> 2
<211> 92
<212> PRT
<213> Triticum aestivum

<400> 2
```
Met Ser Ser Arg Arg Ser Arg Ser Arg Gln Ser Gly Ser Ser Arg Ile
1               5                   10                  15

Thr Asp Glu Gln Ile Ser Asp Leu Val Ser Lys Leu Gln Asp Leu Leu
            20                  25                  30

Pro Glu Ala Arg Leu Arg Gly Asn Asp Arg Val Pro Ser Ser Arg Val
        35                  40                  45

Leu Gln Glu Thr Cys Thr Tyr Ile Arg Ser Leu His Arg Glu Val Asp
    50                  55                  60

Asp Leu Ser Glu Arg Leu Ser Glu Leu Leu Ala Thr Ser Asp Met Ser
65                  70                  75                  80

Ser Ala Gln Ala Ala Ile Ile Arg Ser Leu Leu Met
                85                  90
```

<210> 3
<211> 54

<212> DNA
<213> Artificial sequence

<220>
<223> primer: prm09663

<400> 3
ggggacaagt ttgtacaaaa aagcaggctt aaacaatgtc gagccgtagg tcaa 54

<210> 4
<211> 48
<212> DNA
<213> Artificial sequence

<220>
<223> primer: prm09664

<400> 4
ggggaccact ttgtacaaga aagctgggtc cggctctaca tcagcaag 48

<210> 5
<211> 2194
<212> DNA
<213> Oryza sativa

<400> 5
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag atttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctccttctc ccatctataa attcctcccc ccttttcccc tctctatata 1020

ggaggcatcc aagccaagaa gagggagagc accaaggaca cgcgactagc agaagccgag 1080

cgaccgcctt ctcgatccat atcttccggt cgagttcttg gtcgatctct tccctcctcc 1140

acctcctcct cacagggtat gtgcctccct tcggttgttc ttggatttat tgttctaggt 1200

tgtgtagtac gggcgttgat gttaggaaag gggatctgta tctgtgatga ttcctgttct 1260

tggatttggg atagaggggt tcttgatgtt gcatgttatc ggttcggttt gattagtagt 1320

atggttttca atcgtctgga gagctctatg gaaatgaaat ggtttaggga tcggaatctt 1380

gcgattttgt gagtaccttt tgtttgaggt aaaatcagag caccggtgat tttgcttggt 1440

gtaataaagt acggttgttt ggtcctcgat tctggtagtg atgcttctcg atttgacgaa 1500

gctatccttt gtttattccc tattgaacaa aaataatcca actttgaaga cggtcccgtt 1560

gatgagattg aatgattgat tcttaagcct gtccaaaatt tcgcagctgg cttgtttaga 1620

tacagtagtc cccatcacga aattcatgga aacagttata atcctcagga acaggggatt 1680

ccctgttctt ccgatttgct ttagtcccag aatttttttt cccaaatatc ttaaaaagtc 1740

actttctggt tcagttcaat gaattgattg ctacaaataa tgcttttata gcgttatcct 1800

agctgtagtt cagttaatag gtaatacccc tatagtttag tcaggagaag aacttatccg 1860

atttctgatc tccattttta attatatgaa atgaactgta gcataagcag tattcatttg 1920

gattattttt tttattagct ctcacccctt cattattctg agctgaaagt ctggcatgaa 1980

ctgtcctcaa ttttgttttc aaattcacat cgattatcta tgcattatcc tcttgtatct 2040

acctgtagaa gtttcttttt ggttattcct tgactgcttg attacagaaa gaaatttatg 2100

aagctgtaat cgggatagtt atactgcttg ttcttatgat tcatttcctt tgtgcagttc 2160

ttggtgtagc ttgccacttt caccagcaaa gttc 2194

<210> 6
<211> 2527
<212> DNA
<213> Artificial sequence

<220>
<223> expression cassette

<400> 6
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag atttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480
ttagtaatta aagacaattg acttattttt attatttatc ttttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctcctcctc ccatctataa attcctcccc ccttttcccc tctctatata 1020
ggaggcatcc aagccaagaa gagggagagc accaaggaca cgcgactagc agaagccgag 1080
cgaccgcctt cttcgatcca tatcttccgg tcgagttctt ggtcgatctc ttccctcctc 1140
cacctcctcc tcacagggta tgtgcccttc ggttgttctt ggatttattg ttctaggttg 1200
tgtagtacgg gcgttgatgt taggaaaggg gatctgtatc tgtgatgatt cctgttcttg 1260
gatttgggat agaggggttc ttgatgttgc atgttatcgg ttcggtttga ttagtagtat 1320
ggttttcaat cgtctggaga gctctatgga aatgaaatgg tttagggtac ggaatcttgc 1380
gattttgtga gtaccttttg tttgaggtaa aatcagagca ccggtgattt tgcttggtgt 1440
aataaaagta cggttgtttg gtcctcgatt ctggtagtga tgcttctcga tttgacgaag 1500
ctatcctttg tttattccct attgaacaaa aataatccaa ctttgaagac ggtcccgttg 1560
atgagattga atgattgatt cttaagcctg tccaaaattt cgcagctggc ttgtttagat 1620
acagtagtcc ccatcacgaa attcatggaa acagttataa tcctcaggaa caggggattc 1680
cctgttcttc cgatttgctt tagtcccaga attttttttc ccaaatatct taaaaagtca 1740
ctttctggtt cagttcaatg aattgattgc tacaaataat gcttttatag cgttatccta 1800
gctgtagttc agttaatagg taatacccct atagtttagt caggagaaga acttatccga 1860
tttctgatct ccatttttaa ttatatgaaa tgaactgtag cataagcagt attcatttgg 1920
attatttttt ttattagctc tcaccccttc attattctga gctgaaagtc tggcatgaac 1980
tgtcctcaat tttgttttca aattcacatc gattatctat gcattatcct cttgtatcta 2040
cctgtagaag tttctttttg gttattcctt gactgcttga ttacagaaag aaatttatga 2100
agctgtaatc gggatagtta tactgcttgt tcttatgatt catttccttt gtgcagttct 2160
tggtgtagct tgccactttc accagcaaag ttcatttaaa tcaactaggg atatcacaag 2220
tttgtacaaa aaagcaggct taaacaatgt cgagccgtag gtcaaggtca aggcagtccg 2280
gctcgtcgag gatcactgac gagcaaatca gcgaccttgt ctccaagttg caggacctcc 2340

ttcccgaggc gcgtctccgg ggcaatgata gagtgccatc ttcaagggtg ctgcaggaga 2400

cgtgcaccta catcaggagc ctgcaccggg aggtggacga cctgagcgag aggctgtcgg 2460

agctgctggc gacctcggac atgagcagcg cgcaagcggc catcatccgc agcttgctga 2520

tgtagag 2527

<210> 7
<211> 16
<212> PRT
<213> Artificial sequence

<220>
<223> motif 1

<220>
<221> VARIANT
<222> (1)..(1)
<223> / replace = "Asp" / replace = "Asn"

<220>
<221> UNSURE
<222> (2)..(2)
<223> Xaa can be any naturally occurring amino acid

<220>
<221> VARIANT
<222> (3)..(3)
<223> / replace = "Gln"

<220>
<221> VARIANT
<222> (4)..(4)
<223> / replace = "Val" / replace = "Met"

<220>
<221> UNSURE
<222> (5)..(5)
<223> Xaa can be any naturally occurring amino acid

<220>
<221> VARIANT
<222> (6)..(6)
<223> / replace = "Asp" / replace = "Gln" / replace = "Ala" / replace = "Asn"

<220>
<221> VARIANT
<222> (7)..(7)
<223> / replace = "Phe" / replace = "Ile"

<220>
<221> VARIANT
<222> (8)..(8)
<223> / replace = "Val" / replace = "Leu" / replace = "Met"

<220>
<221> VARIANT
<222> (9)..(9)

<223> / replace = "Ile" / replace = "Thr" / replace = "Leu" / replace = "Tyr"

<220>
<221> UNSURE
<222> (10)..(10)
<223> Xaa can be any naturally occurring amino acid

<220>
<221> VARIANT
<222> (12)..(12)
<223> / replace = "Arg" / replace = "His"

<220>
<221> UNSURE
<222> (13)..(13)
<223> Xaa can be any naturally occurring amino acid

<220>
<221> VARIANT
<222> (14)..(14)
<223> / replace = "Phe" / replace = "Ile" / replace = "Ser"

<220>
<221> VARIANT
<222> (15)..(15)
<223> / replace = "Val" / replace = "Ile"

<220>
<221> VARIANT
<222> (16)..(16)
<223> / replace = "Ala"

<400> 7

Glu Xaa Glu Ile Xaa Glu Leu Ile Ser Xaa Leu Gln Xaa Leu Leu Pro
1 5 10 15

<210> 8
<211> 16
<212> PRT
<213> Artificial sequence

<220>
<223> motif 2

<220>
<221> VARIANT
<222> (1)..(1)
<223> / replace = "Thr" / replace = "Ser"

<220>
<221> UNSURE
<222> (2)..(2)
<223> Xaa can be any naturally occurring amino acid

<220>
<221> VARIANT
<222> (3)..(3)
<223> / replace = "Arg" / replace = "Asn" / replace = "Ser"

```
<220>
<221>  VARIANT
<222>  (4)..(4)
<223>  / replace = "Leu" / replace = "Ile" / replace = "Met" / replace =
       "Ala"

<220>
<221>  VARIANT
<222>  (6)..(6)
<223>  / replace = "Lys" / replace = "Arg" / replace = "Glu" / replace =
       "His"

<220>
<221>  VARIANT
<222>  (7)..(7)
<223>  / replace = "Asp" / replace = "Tyr" / replace = "Gln"

<220>
<221>  VARIANT
<222>  (10)..(10)
<223>  / replace = "Ser" / replace = "Thr" / replace = "Ile" / replace =
       "Ala"

<220>
<221>  VARIANT
<222>  (11)..(11)
<223>  / replace = "Ser" / replace = "Cys"

<220>
<221>  VARIANT
<222>  (12)..(12)
<223>  / replace = "Phe" / replace = "Val"

<220>
<221>  VARIANT
<222>  (13)..(13)
<223>  / replace = "Lys" / replace = "Gly"

<220>
<221>  VARIANT
<222>  (14)..(14)
<223>  / replace = "Asn" / replace = "Asp" / replace = "Thr" / replace =
       "Arg"

<220>
<221>  VARIANT
<222>  (15)..(15)
<223>  / replace = "Ser"

<220>
<221>  VARIANT
<222>  (16)..(16)
<223>  / replace = "Gln" / replace = "Asn" / replace = "Ser"

<400>  8

Ala Xaa Lys Val Leu Gln Glu Thr Cys Asn Tyr Ile Arg Ser Leu His
1               5                   10                  15


<210>  9
<211>  8
<212>  PRT
```

<213> Artificial sequence

<220>
<223> motif 3

<220>
<221> VARIANT
<222> (1)..(1)
<223> / replace = "Gln"

<220>
<221> VARIANT
<222> (3)..(3)
<223> / replace = "Glu"

<400> 9

Glu Ala Ala Ile Ile Arg Ser Leu
1               5

<210> 10
<211> 11
<212> PRT
<213> Artificial sequence

<220>
<223> motif 4

<220>
<221> VARIANT
<222> (3)..(3)
<223> / replace = "Gly"

<220>
<221> VARIANT
<222> (5)..(5)
<223> / replace = "Lys"

<220>
<221> VARIANT
<222> (11)..(11)
<223> / replace = "Thr"

<400> 10

Met Ser Ser Arg Arg Ser Arg Ser Arg Gln Ser
1               5                   10

<210> 11
<211> 8
<212> PRT
<213> Artificial sequence

<220>
<223> motif 5

<220>
<221> VARIANT

<222> (1)..(1)
<223> / replace = "Gln"

<220>
<221> VARIANT
<222> (3)..(3)
<223> / replace = "His"

<220>
<221> VARIANT
<222> (4)..(4)
<223> / replace = "Gln" / replace = "Arg"

<400> 11

Lys Leu Gln Asp Leu Leu Pro Glu
1 5

<210> 12
<211> 8
<212> PRT
<213> Artificial sequence

<220>
<223> motif 6

<220>
<221> VARIANT
<222> (3)..(3)
<223> / replace = "Asp"

<220>
<221> VARIANT
<222> (6)..(6)
<223> / replace = "Asn" / replace = "Ser"

<400> 12

Leu Gln Glu Thr Cys Thr Tyr Ile
1 5

<210> 13
<211> 13
<212> PRT
<213> Artificial sequence

<220>
<223> motif 7

<220>
<221> VARIANT
<222> (3)..(3)
<223> / replace = "Gly"

<220>
<221> VARIANT
<222> (11)..(11)
<223> / replace = "Gln"

<400> 13

Glu Val Asp Asp Leu Ser Glu Arg Leu Ser Glu Leu Leu
1 5 10

<210> 14
<211> 9
<212> PRT
<213> Artificial sequence

<220>
<223> motif 8

<220>
<221> VARIANT
<222> (4)..(4)
<223> / replace = "Val" / replace = "Leu"

<220>
<221> VARIANT
<222> (7)..(7)
<223> / replace = "Asn" / replace = "Arg"

<400> 14

Gln Ala Ala Ile Ile Arg Ser Leu Leu
1 5

<210> 15
<211> 90
<212> PRT
<213> Populus trichocarpa

<400> 15

Met Ser Ser Arg Arg Pro Arg Gln Ser Ser Val Pro Arg Ile Thr Asp
1 5 10 15

Asp Gln Ile Ile Asp Leu Val Ser Lys Leu Arg Gln Leu Leu Pro Glu
20 25 30

Ile Ser Gln Arg Arg Ser Asp Lys Val Ser Ala Ser Lys Val Leu Gln
35 40 45

Glu Thr Cys Asn Tyr Ile Arg Asn Leu His Arg Glu Val Asp Asp Leu
50 55 60

Ser Glu Arg Leu Ser Gln Leu Leu Ala Thr Ile Asp Ala Asp Ser Pro
65 70 75 80

Glu Ala Ala Ile Ile Arg Ser Leu Ile Met
85 90

<210> 16

<211> 383
<212> DNA
<213> Populus trichocarpa

<400> 16
```
cagacgcgta acaaaaatcc gtgtgtaggc atgtctagca gaaggccaag gcaatctagc      60

gttccaagga tcactgatga tcagatcatc gaccttgtct ccaaattacg ccagcttctc     120

cctgagatta gtcaaaggcg ctccgataag gtatcagctt ccaaggtcct acaagagact     180

tgcaattata tcaggaactt gcacagggag gttgatgact taagtgagcg attgtctcag     240

cttttggcaa caattgatgc tgatagtcct gaagcagcga taataaggag tttaattatg     300

taatatcaat taattagatg atcaggcacc ggcccttaaa ccgatttata tctattttca     360

gtttaataat ttgttagtag gct                                             383
```

<210> 17
<211> 86
<212> PRT
<213> Allium cepa

<400> 17

```
Met Ser Ser Arg Arg Ser Arg Ile Ser Glu Glu Glu Ile Gly Glu Leu
1               5                   10                  15

Ile Ser Lys Leu Gln Ser Leu Leu Pro Asp Ser Arg Arg Arg Gly Ser
            20                  25                  30

Asn Arg Ala Ser Ala Ser Lys Leu Leu Lys Glu Thr Cys Asn Tyr Ile
        35                  40                  45

Lys Ser Leu His Arg Glu Val Asp Asp Leu Ser Glu Arg Leu Ser Glu
    50                  55                  60

Leu Ile Ser Thr Met Asp Asn Gly Ser Glu Gln Ala Glu Ile Ile Arg
65                  70                  75                  80

Ser Leu Leu Arg Ser Asn
                85
```

<210> 18
<211> 522
<212> DNA
<213> Allium cepa

<400> 18
```
aattcttcct ctctctcatt tcacactttg cattttccac aatgtcgagt cgaaggtcca      60

gaattagcga ggaagagatc ggagagctca tttcaaagct gcagtctctc cttcccgatt     120

cacgtaggcg cggttcaaac cgggcatcgg cgtccaagtt gctaaaggag acgtgcaact     180
```

acattaagag cttgcacaga gaagtcgacg acttgagtga gaggctttct gaactgatct 240

ctaccatgga caatggaagc gagcaagctg agatcattcg aagcttgctt cgttctaact 300

aaagtatggt catgactgat ttgaattgaa ttactcttaa atatgatata ttttagcttt 360

tgaaagttta gctactagtg gcagttgtag tagaaatgtt ggtgtttttt ttttcccttt 420

ctttttcatc tttatattaa ttgtgtacat ttattttaat ggtttggatc gagtttgttg 480

cttctataaa tgacaaaccg accaacatct ctccaaaaaa aa 522

<210> 19
<211> 91
<212> PRT
<213> Antirrhinum majus

<400> 19

Met Ser Gly Arg Arg Ser Arg Gln Ser Thr Gly Ser Ser Arg Ile Ser
1 5 10 15

Asn Asp Gln Ile Ile Asp Leu Val Ser Lys Leu His Gln Leu Leu Pro
20 25 30

Glu Ile Gly Asn Arg Arg Arg Ser Asn Lys Thr Ser Ala Asn Lys Val
35 40 45

Leu Gln Glu Thr Cys Asn Tyr Ile Lys Asn Leu His Lys Glu Val Asp
50 55 60

Asp Leu Ser Glu Arg Leu Ser Gln Leu Leu Ser Thr Ile Asp Ala Asp
65 70 75 80

Ser Pro Glu Ala Ala Ile Ile Arg Ser Leu Ile
85 90

<210> 20
<211> 676
<212> DNA
<213> Antirrhinum majus

<400> 20
ccctctgtac aactaaactt ttatctcaag tcttcttttc acttttctgc gccctgtttc 60

ttatattaat ctactaccta tttaattatt aactagttta attagacttt ttataaaaaa 120

gaaaagaaga gaatattttt aaggatgtct ggaagaagat caaggcagtc aacggggagt 180

tcaaggattt caaatgatca aatcattgac cttgtgtcca aactccacca gctccttcct 240

gaaattggca acagaaggcg ttcaaacaag acatcagcca ataaagttct tcaggagact 300

tgcaactaca tcaagaactt gcacaaagaa gtggatgatt tgagcgagag gctttcccag 360

ctactgtcta ctatagatgc ggatagccca gaggccgcaa taatcaggag tttaatttag 420

ttaattagtg taataatgaa gttattattg gaagaagcca attttatgta ttaattagct 480

agatttttat ctaggctgtg acttctgcat gggtttaatc aggcattaag acctaattac 540

tagtaggttt ccctagccat taattgttgg gtgcaactat atatgcagca attaagtttg 600

tagtttaatt cgtactgtgt aataagggag ctgtactttg cgatagttcc tatattgatt 660

gtgttgtatt taaatt 676

<210> 21
<211> 94
<212> PRT
<213> Arabidopsis thaliana

<400> 21

Met Ser Ser Arg Arg Ser Ser Arg Ser Arg Gln Ser Gly Ser Ser Arg
1 5 10 15

Ile Ser Asp Asp Gln Ile Ser Asp Leu Val Ser Lys Leu Gln His Leu
20 25 30

Ile Pro Glu Leu Arg Arg Arg Arg Ser Asp Lys Val Ser Ala Ser Lys
35 40 45

Val Leu Gln Glu Thr Cys Asn Tyr Ile Arg Asn Leu His Arg Glu Val
50 55 60

Asp Asp Leu Ser Asp Arg Leu Ser Glu Leu Leu Ala Ser Thr Asp Asp
65 70 75 80

Asn Ser Ala Glu Ala Ala Ile Ile Arg Ser Leu Leu Asn Tyr
85 90

<210> 22
<211> 689
<212> DNA
<213> Arabidopsis thaliana

<400> 22
aacaccttct tctccactct cattctctct ttctgacaca ttaactactt atccttcttg 60

cattcttctc tctctctaca cccaaacaaa cacacttata atatatcaag aaagaagatg 120

tctagcagaa gatcatcacg ttcaagacag tcaggaagct caagaatctc tgacgatcag 180

atttccgatc ttgtttctaa gctccaacac ctcatccctg aacttcgccg ccgccgttct 240

gacaaggtgt cagcatctaa ggtactacaa gagacttgca actacatcag gaacttacac 300

agagaggttg atgacctcag tgaccgtttg tcggaactct tggcttcgac ggacgacaac 360

agcgccgaag cagccatcat taggagcttg cttaattatt aaatccgcat tacttaatct 420

gagagctatt aatcatccgt ttccggccac caaatttatc ttattatggg tatcgtctgt 480

ttacttctac atcatatatt atgagatata gctagggttt cgggtcattg ttaggccaac 540

tcatatattt atatttaata tatggttatg tatgtatgta tgcatgttaa ttgtatctga 600

gggtccagac ctggcgtata gtagcctgtg tatcatgaga tcctctaata tttatgatta 660

atgacacggt ccgtttcctt ttttactat 689

<210> 23
<211> 93
<212> PRT
<213> Arabidopsis thaliana

<400> 23

Met Ser Gly Arg Arg Ser Arg Ser Arg Gln Ser Ser Gly Thr Ser Arg
1 5 10 15

Ile Ser Glu Asp Gln Ile Asn Asp Leu Ile Ile Lys Leu Gln Gln Leu
20 25 30

Leu Pro Glu Leu Arg Asp Ser Arg Arg Ser Asp Lys Val Ser Ala Ala
35 40 45

Arg Val Leu Gln Asp Thr Cys Asn Tyr Ile Arg Asn Leu His Arg Glu
50 55 60

Val Asp Asp Leu Ser Glu Arg Leu Ser Glu Leu Leu Ala Asn Ser Asp
65 70 75 80

Thr Ala Gln Ala Ala Leu Ile Arg Ser Leu Leu Thr Gln
85 90

<210> 24
<211> 558
<212> DNA
<213> Arabidopsis thaliana

<400> 24
atactatcaa cttttctcta tctatctctc tctcttcttt ttccggcata acttctgtgt 60

taccctaaac tccataacct gtttcaccga taaagtgcct ttgcttctat ctctgtcact 120

cttactactt gttgaacaat attctacaaa aaaatgtcgg gaagaagatc acgttcgagg 180

caatcatcag gaacttcaag gatctcagaa gatcaaatca atgatctgat tatcaagttg 240

caacagcttc ttcctgagct cagggacagt cgtcgttccg acaaggtttc agcagcgagg 300

gtgttacaag atacgtgcaa ctacatacgg aatctgcata gagaggttga tgatctaagt 360

gagaggctat ctgagttact agcaaactca gacactgcac aagctgcttt aatcagaagc 420

ttacttaccc aataattcct atctatcttt ttcttcttct tcttttttt gtttactata 480

ataataataa tagtttgcgg gttttttttt ctatagatgt tgatgacctt ataaacgttt 540

aatgatacga gttcgtca 558

<210> 25
<211> 92
<212> PRT
<213> Arabidopsis thaliana

<400> 25

Met Ser Ser Arg Lys Ser Arg Ser Arg Gln Thr Gly Ala Ser Met Ile
1 5 10 15

Thr Asp Glu Gln Ile Asn Asp Leu Val Leu Gln Leu His Arg Leu Leu
20 25 30

Pro Glu Leu Ala Asn Asn Arg Arg Ser Gly Lys Val Ser Ala Ser Arg
35 40 45

Val Leu Gln Glu Thr Cys Ser Tyr Ile Arg Asn Leu Ser Lys Glu Val
50 55 60

Asp Asp Leu Ser Glu Arg Leu Ser Gln Leu Leu Glu Ser Thr Asp Ser
65 70 75 80

Ala Gln Ala Ala Leu Ile Arg Ser Leu Leu Met Gln
85 90

<210> 26
<211> 279
<212> DNA
<213> Arabidopsis thaliana

<400> 26
atgtctagca gaaaatcacg ttcaagacaa actggagctt ccatgatcac ggatgaacaa 60

atcaacgatc ttgtcctcca gcttcatcgg cttctccccg aacttgctaa caacagacgc 120

tctggaaagg tttcagcatc aagggtatta caagagacat gcagttacat aaggaacttg 180

agcaaagaag tggatgatct tagtgaaaga ttgtctcaac ttttggaatc aactgattca 240

gctcaagctg cactaatccg aagtttgctt atgcagtag 279

<210> 27
<211> 92
<212> PRT
<213> Arabidopsis thaliana

<400> 27

Met Ser Asn Arg Arg Ser Arg Gln Thr Ser Asn Ala Ser Arg Ile Ser
1 5 10 15

Asp Asp Gln Met Ile Asp Leu Val Ser Lys Leu Arg Gln Phe Leu Pro
20 25 30

Glu Ile His Glu Arg Arg Arg Ser Asp Lys Val Ser Ala Ser Lys Val
35 40 45

Leu Gln Glu Thr Cys Asn Tyr Ile Arg Lys Leu His Arg Glu Val Asp
50 55 60

Asn Leu Ser Asp Arg Leu Ser Gln Leu Leu Asp Ser Val Asp Glu Asp
65 70 75 80

Ser Pro Glu Ala Ala Val Ile Arg Ser Leu Leu Met
85 90

<210> 28
<211> 739
<212> DNA
<213> Arabidopsis thaliana

<400> 28
tatatctcga agtgtctcta ttacccgaaa cactttctta caattttctc ttctcttctc 60

ttttcgttgc tcttcttttt ctttctttca cacctcttca acacaaatat aaaacctgta 120

gaataaacac aaaccttcta cataacttct ctcacttttt ttttttaaa actctcttct 180

taataacaaa cccttctctc tcaatctctt ctctattatc taatctagaa aagagagaaa 240

gcatacaaca taaaggttat tttcttgcgg cattgtagtg ttacacctaa tcacaaagta 300

aaaacaagaa aatgtctaac agaagatcaa gacaaacttc gaatgcttcg aggatctccg 360

atgaccagat gatcgacctc gttagtaagc tccgtcagtt tttgccggag attcacgaac 420

ggcgtcgttc tgataaggtg tcagcatcaa aggtactaca agagacatgc aactacataa 480

gaaaattgca tagagaagtt gacaatctca gtgatcgttt gtcgcagctt cttgactctg 540

ttgatgaaga tagccctgaa gctgccgtga ttagaagctt actcatgtaa ccttccaata 600

ttttttatta taacttctta atatagtatt tattaattta tctatatatg taatctttat 660

cgtcctttat atatcaagcg acgtgctttt atcttttatg aactttggaa ttttggtaca 720

gaaatttaca ttaatttct 739

<210> 29
<211> 92
<212> PRT
<213> Arabidopsis thaliana

<400> 29

Met Ser Asn Arg Arg Ser Arg Gln Ser Ser Ser Ala Pro Arg Ile Ser

1 5 10 15

Asp Asn Gln Met Ile Asp Leu Val Ser Lys Leu Arg Gln Ile Leu Pro
20 25 30

Glu Ile Gly Gln Arg Arg Arg Ser Asp Lys Ala Ser Ala Ser Lys Val
35 40 45

Leu Gln Glu Thr Cys Asn Tyr Ile Arg Asn Leu Asn Arg Glu Val Asp
50 55 60

Asn Leu Ser Glu Arg Leu Ser Gln Leu Leu Glu Ser Val Asp Glu Asp
65 70 75 80

Ser Pro Glu Ala Ala Val Ile Arg Ser Leu Leu Met
85 90

<210> 30
<211> 703
<212> DNA
<213> Arabidopsis thaliana

<400> 30
gtgtattcaa aaccccaaaa cacttttctc attctcttct ctattttctt cttgctctct 60

agtttttctt tcttcttggt cgtttccttt cagcataaaa accttataaa atcataaaag 120

cttacaccta cttgccacat agacatagcc gatctcatta tatctctatt tctatttctc 180

aatagaactt gtttgagcta gtgtgagaga agtaaagaaa gagagaagaa tccacaactt 240

agttagggtc ttttcttgcc acattgttga acatgtcgaa cagaagatca aggcaatctt 300

caagtgctcc aaggatctcc gataatcaaa tgattgacct cgtatctaag ctccgtcaaa 360

ttttgccgga gattggtcaa cgacgtcgtt ctgataaggc atcagcctcg aaagtattgc 420

aagagacatg caattacata cgaaatttga acagagaagt tgacaatctg agcgagcgtt 480

tgtctcagct tctcgaatct gtcgatgaag atagccctga agccgccgtt attagaagcc 540

tactcatgta atcttttttg ttcttttgtt tgtttttgac aagcctatcc atgtaatctt 600

aaatgatcgc tctataataa ttatattttt aacataatcg tcttattatg taaaattcaa 660

agagatgggc ttgatcttta atgacatacg aatttcatag ggt 703

<210> 31
<211> 94
<212> PRT
<213> Arabidopsis thaliana

<400> 31

Met Ser Ser Ser Arg Arg Ser Arg Gln Ala Ser Ser Ser Ser Arg Ile
1 5 10 15

Ser Asp Asp Gln Ile Thr Asp Leu Ile Ser Lys Leu Arg Gln Ser Ile
            20                  25                  30

Pro Glu Ile Arg Gln Asn Arg Arg Ser Asn Thr Val Ser Ala Ser Lys
        35                  40                  45

Val Leu Gln Glu Thr Cys Asn Tyr Ile Arg Asn Leu Asn Lys Glu Ala
    50                  55                  60

Asp Asp Leu Ser Asp Arg Leu Thr Gln Leu Leu Glu Ser Ile Asp Pro
65                  70                  75                  80

Asn Ser Pro Gln Ala Ala Val Ile Arg Ser Leu Ile Asn Gly
                85                  90

<210> 32
<211> 601
<212> DNA
<213> Arabidopsis thaliana

<400> 32
ctccctttct ttcgacaagc acaaacaaag ccatcaagag aagaaagcct tttcttggat 60

tcacatatat ataagaatat tttttcaaat caaacatgtc ttctagcaga aggtcgagac 120

aagcaagctc atcatcaaga attagcgatg accagatcac tgatctcatc tcaaagctcc 180

gacagtccat tccggagatt cgccagaacc gtcgttccaa cacggtatca gcgtcgaaag 240

tgttacaaga gacttgcaac tacataagaa acttgaacaa ggaagccgat gacctcagtg 300

atcgattgac tcagcttctg gaatccattg atcctaatag cccacaagcc gcagttatta 360

ggagcttgat taatggataa ttaagatata aattgattag ttgtgcttta tatatataag 420

cttaaaatct cgttgggagg ttgatccatc agggtgttgc ataattatat atctatttta 480

tgtttcttat atattattta caatcctatc tagttagggt tcatattttg acccttttttt 540

ggtttaacgt catgcatgca attccattaa gcttaaaaat tataataaat aagatttcga 600

g 601

<210> 33
<211> 90
<212> PRT
<213> Brachypodium distachyon

<400> 33

Met Ser Gly Arg Arg Ser Ser Ser Arg Gly Asn Ser Val Ser Glu Glu
1               5                   10                  15

Glu Ile Asn Glu Leu Ile Ser Lys Leu Gln Ser Leu Leu Pro Ala Ser

20 25 30

Ala Arg Arg Arg Gly Ser Ser Gln Ala Ser Thr Thr Lys Leu Leu Lys
35 40 45

Glu Thr Cys Ser Tyr Ile Lys Ser Leu His Arg Glu Val Asp Asp Leu
50 55 60

Ser Asp Arg Leu Ser Asp Leu Met Ala Thr Met Asp His Asn Ser Pro
65 70 75 80

Gly Ala Glu Ile Ile Arg Ser Leu Leu Arg
85 90

<210> 34
<211> 603
<212> DNA
<213> Brachypodium distachyon

<400> 34
ccacgcgtcc gcaaaaacaa acttcagcta accggccact cgatctactt ttgggatcac 60

acgcgcctag cttctcgtcg atcgtcttca agctcagttc agtcctcttt ctgccgggct 120

aggcgcgggc tgcattattc agagacgtag tacgacgatg tcgggcagga ggtcgtcgtc 180

ccgcggtaac tccgtgtcgg aggaggagat caacgagctc atctccaagc tccagtcttt 240

gctcccggcc agcgcgcgcc gccgcggcag cagccaggcg tcgacgacga agctgctcaa 300

ggagacgtgc agctacatca agagcctgca ccgggaagtg gacgacctga gcgaccggct 360

ctccgacctc atggccacca tggaccacaa cagccccggc gccgagatca tccgcagcct 420

tctccgctag cttaattctc tcatgcatgc atggtcgacc acgcccggcc tcctgataga 480

tcgatgtgat gtcctaatta attaagctag ctcctcacct atataaatat atatgtatac 540

atatacacat gatatatctg tgtccatcga tcgatctctg catatacatg ccgatcgatc 600

gat 603

<210> 35
<211> 92
<212> PRT
<213> Cathamus tinctorius

<400> 35

Met Ser Ser Arg Arg Ser Arg Gln Ser Ser Ser Gly Gly Pro Arg Ile
1 5 10 15

Thr Asp Asp Gln Ile Ile Gln Leu Val Ser Lys Leu Gln Gln Leu Leu
20 25 30

```
Pro Gly Thr Arg Ile Gln Arg Ser Asn Lys Ala Ser Ala Ser Lys Val
        35                  40                  45

Leu Gln Glu Thr Cys Asn Tyr Val Arg Ser Leu His Arg Glu Val Asp
    50                  55                  60

Asp Leu Ser Asp Arg Leu Ser Gln Leu Leu Ser Thr Ile Asp Ala Asp
65                  70                  75                  80

Ser Pro Glu Ala Ser Ile Ile Arg Ser Leu Ile Met
                85                  90


<210>  36
<211>  585
<212>  DNA
<213>  Cathamus tinctorius


<220>
<221>  misc_feature
<222>  (487)..(487)
<223>  n is a, c, g, or t

<400>  36
tcgggcacca ccactttgcg ctccttaatg tcgagtagaa gatcaagaca atcgtcatca     60

ggggtccga ggatcacaga tgaccaaatc atacaactcg tctccaagtt acaacaactt    120

cttcctggaa ctcgcatcca acgatctaac aaggcatcgg cttcaaaggt gttacaagag    180

acttgcaact acgtcagaag cttgcatagg gaggttgatg atctcagtga ccgactatcg    240

cagttattat ccaccattga cgctgatagc cccgaagctt cgattattcg aagcttaatt    300

atgtaatatg caaatctcta catataaatt attcgttagc ttattgatta agcataatta    360

tggtttctta atcttatagt taattatctc catagggttt aatttaatta atagcccatg    420

ttacatgtag acttgtccca gtacttgtcg aaatgataat aacaataata attacgttga    480

gaaactnaga aaaaaaaaaa aaaagagggg tagaaggcaa ctagaaaaaa acattatgaa    540

tgttaaaaaa gggcggctaa gaatttattt tttccaatta agaaa                    585


<210>  37
<211>  91
<212>  PRT
<213>  Camellia sinensis

<400>  37

Met Ser Ser Arg Arg Ser Arg Ser Arg Gln Ser Gly Ser Ser Arg Ile
1               5                   10                  15

Thr Asp Asp Gln Ile Asn Asp Leu Val Ser Lys Leu Gln Gln Leu Leu
            20                  25                  30
```

```
Pro Glu Leu Arg Asn Asn Arg Ser Asp Lys Val Ser Ala Gly Lys Val
        35                  40                  45

Leu Gln Glu Thr Cys Asn Tyr Ile Arg Ser Leu His Arg Glu Val Asp
    50                  55                  60

Asp Leu Ser Glu Arg Leu Ser Glu Leu Leu Ala Thr Thr Asp Thr Ala
65                  70                  75                  80

Gln Ala Ala Ile Ile Arg Ser Leu Leu Met Gln
                85                  90
```

<210> 38
<211> 527
<212> DNA
<213> Camellia sinensis

<400> 38

```
caaattaaaa atattatata agatgtccag cagaagatca agatcaaggc aatcaggaag      60

ctcaaggatc actgatgatc agatcaatga ccttgtctcc aaattgcaac agcttcttcc     120

tgagcttcgc aataaccgct ctgacaaggt ttcggcgggg aaggtcttac aagagacctg     180

caactacatt agaagcttgc acagagaggt agatgatctt agcgagagac tgtctgagct     240

actggcaact actgacactg cacaagctgc aataatccgg agcttactca tgcaatagac     300

ctgaatccat actagttcat tttgtttatg caattaatag acagccagtc ctctatcttc     360

ttcatttctg tgcgtctcca ggtcttcgtc aagagagtga tattttgaac ttatgtagtt     420

gcagttgatc gcttagagag aatctttttc tttgcaaagt tgtgttttga gtaacgaata     480

taataaaaag tacttctggc ctcagacaca atgtttctca aaaaaaa                  527
```

<210> 39
<211> 91
<212> PRT
<213> Camellia sinensis

<400> 39

```
Met Ser Ser Arg Arg Ser Arg Gln Ala Ala Gly Val Ser Arg Ile Ser
1               5                   10                  15

Asp Asp Gln Ile Ile Glu Leu Val Ser Lys Leu Arg Gln Leu Leu Pro
            20                  25                  30

Glu Ile Arg Asp Arg Arg Pro Gln Lys Val Ser Ala Ser Lys Val Leu
        35                  40                  45

Gln Glu Thr Cys Asn Tyr Ile Arg Ser Leu His Arg Glu Val Asp Asp
    50                  55                  60
```

Leu Ser Glu Arg Leu Ser Gln Leu Leu Ser Thr Ile Asp Ala Asp Ser
65 70 75 80

Pro Glu Ala Ala Ile Ile Arg Ser Leu Ile Met
85 90

<210> 40
<211> 746
<212> DNA
<213> Camellia sinensis

<220>
<221> misc_feature
<222> (55)..(57)
<223> n is a, c, g, or t

<400> 40
gaggaatgca cttgtcttct tcatccaaca tcactgtctt tgttgtggtc caatnnntct 60

ttataactga tctcttatca cattctccat atagctcttt aagtccatct tgcttctctt 120

gccctctctt gaacttcatt tcagagttca ttctgcgcaa ccccttcggc cttcagtatc 180

tttctttttt atttttccca agtgaatatt gcaagtgcct tttaattagc tcatttacat 240

taatatatac aaagcaagtc agcagctagc tccaaggatc atcatgtcta gcagaaggtc 300

gaggcaagct gccggtgtat cgaggatcag tgatgatcag atcattgaac ttgtctcaaa 360

gctacgccaa ctcctccctg agattcgcga tagacgccca caaaaggttt cagcttctaa 420

ggttctacag gaaacttgca attatattag aagcttgcac agggaagttg atgacctaag 480

tgagcgacta tcccagcttt tatctactat agatgctgat agtcccgaag ctgcgataat 540

taggagttta attatgtaat tctgtagcct taattactta attatctttc tagttcttct 600

ctactttaat cttactaatt aagttctggt cactacatag atcaaacaag aactagaatg 660

tattgtaact ataattaagt ttgtataata aaaggacttg cactagcaaa gcccaagtta 720

taatcaatat tataatatat ttttac 746

<210> 41
<211> 93
<212> PRT
<213> Coffea canephora

<400> 41

Met Ser Ser Arg Arg Ser Arg Gln Ser Ser Gly Ser Ser Arg Ile Thr
1 5 10 15

Asp Asp Gln Ile Ile Glu Leu Val Ser Lys Leu Gln Gln Leu Leu Pro
20 25 30

```
Glu Ile Arg Thr Arg Arg Ser Asn Lys Ala Ser Ala Ser Lys Val Leu
        35                  40                  45

Gln Asp Thr Cys Asn Tyr Ile Arg Ser Leu His Lys Glu Val Asp Asp
    50                  55                  60

Leu Ser Asp Arg Leu Ser Gln Leu Leu Ser Thr Ile Asp Ala Asp Ser
65                  70                  75                  80

Pro Glu Ala Ala Ile Ile Arg Ser Leu Leu Ala Glu Ser
                85                  90


<210>  42
<211>  764
<212>  DNA
<213>  Coffea canephora

<400>  42
catttgcgct gcttaattaa taaccattag tgatcgacag cacttgaagt tcccgtagga      60

gtcaaaacaa ccagcttaaa gaatcaagtt tggagccctc ttatcttata ctaacataag     120

catgtctagc agaaggtcaa ggcaatcatc gggttcttca aggatcacgg atgatcagat     180

aattgagctt gtctccaagt tacaacaact tcttcccgag attcgtacta ggcgctcgaa     240

caaggcatcg gcgtctaagg ttctccagga tacttgcaac tacattcgaa gcctgcacaa     300

agaggtggat gacctcagtg accgtctctc tcagctactg tcgacaattg atgctgatag     360

cccagaggct gccatcatta ggagcttatt agctgaatct tgaccatctc ctacgatcga     420

tctcgatctc tctataatca tcatcgtcgt catcatcatc attccagtac gtagcctgct     480

cttgctatgc cgcctgcttc cttatcaaga gctagagctg aaaagaatac atatagatat     540

atatatatat gcattactta tgtatggtac ttgcgttatg aaacttagac gttggattac     600

gactccaagt cctagtcctg gctctctggt tagctagttc ttgcaatcta agctctgtaa     660

aaataaaaga tgttgctgta cttgtacatg gcaacacctt gcactcgcat gtatgtatgt     720

acctagtaat taagctatat tatatatgaa gttttttttt tttt                      764


<210>  43
<211>  94
<212>  PRT
<213>  Fragaria vesca

<400>  43

Met Ser Ser Arg Arg Ser Ser Arg Gln Ser Ser Gly Ser Thr Pro Ser
1               5                   10                  15

Ile Lys Asp Asp Gln Ile Ile Glu Leu Val Ser Lys Leu Arg Gln Leu
            20                  25                  30
```

Val Pro Glu Ile Arg Asp Arg Arg Ser Asp Lys Val Ser Ala Ser Lys
35 40 45

Val Leu Gln Glu Thr Cys Ser Tyr Ile Arg Asn Leu His Arg Glu Val
50 55 60

Asp Asp Leu Ser Glu Arg Leu Ser Gln Leu Leu Ala Thr Ile Asp Ala
65 70 75 80

Asp Ser Ala Glu Ala Ala Ile Ile Arg Ser Leu Ile Met Gln
85 90

<210> 44
<211> 827
<212> DNA
<213> Fragaria vesca

<400> 44
catgctcata tatatcttac tcccatctta catttttcta agacaaacta ccactgctac 60

tactcctact tcctctgctt cttcttctgc tctttcttcc tcggcccctt ctcttctatc 120

tcagaacttg cttctctagg tttttctcct cctccggtac cggtactact ctactacgta 180

ctatataatc tactctaggt tgctcataag ctttggcaaa cgctaggttg atatataaac 240

taagtagcta tatctagctg ctgagctgat acatatagaa ggaatcagtt tgtctgggaa 300

acacagtccg atcgatcatg tctagcagaa ggtcatcaag gcagtcatcg ggaagtactc 360

catcaatcaa agatgaccag atcatcgagc tcgtctccaa gttgcgccag ctggttcctg 420

agattcgcga caggcgctcc gataaggtat cagcatccaa ggtcctacaa gagacctgca 480

gctacatcag aaacttacac agagaagttg acgacttgag cgagaggctg tcccaactgc 540

tcgctacaat tgacgctgat agcgctgagg ccgccattat taggagcttg attatgcagt 600

agatcgacgt gtactctata actctataaa tatcgttatt ttagttgatt tataaatatc 660

tatatagttg cactacatct ttatattact taatttctag gtttcgatca ccatgatcat 720

caagcacggt taattgagca ctactacgta ctcatgtacg tacccatcta gacaagagct 780

catgtatgga tcagtttgtt gatataaaag actgcactag ctagcaa 827

<210> 45
<211> 93
<212> PRT
<213> Gerbera hybrid

<400> 45

Met Ser Ser Arg Arg Ser Arg Ser Arg Gln Ser Gly Val Ser Arg Ile
1 5 10 15

```
Ser Asp Asp Gln Ile Ala Asn Leu Val Ser Lys Leu Gln Gln Leu Ile
            20                  25                  30

Pro His Asn Leu His Thr Ser Pro Ser Asp Lys Val Ser Ala Ser Lys
        35                  40                  45

Val Leu Gln Glu Thr Cys Asn Tyr Ile Arg Ser Leu His Lys Glu Val
    50                  55                  60

Asp Asp Leu Ser Glu Arg Leu Ser Glu Leu Leu Gln Leu Thr Asp Thr
65                  70                  75                  80

Asn Ser Ala Glu Ala Ala Ile Ile Arg Ser Leu Phe Met
                85                  90
```

```
<210>  46
<211>  541
<212>  DNA
<213>  Gerbera hybrid

<400>  46
atttgtaaag cttctctgac aaaaatagaa agaaaaataa aagaatccgt cttactatct      60

caattgtctc tgataatgtc tagcagaaga tcgcgttcac gtcaatctgg agtgtcaagg     120

atcagcgacg accagatcgc caatctcgtg tccaagttac aacaactcat tccacacaac     180

cttcacacca gcccttctga caaggtttca gcttcaaaag ttctgcaaga gacttgcaat     240

tatatcagaa gcttacacaa agaagtggat gatttaagtg agagattatc agagctttta     300

caactcacag acaccaacag tgctgaagca gccattatta ggagcttatt tatgtaacca     360

tttcttatat tatatactaa ttaattaagc aatcatgagt ttttgtgctt tttaatcaat     420

tatgtcctaa ccatgtttta gctaaatatt tatatgcata tattaattat taaaataaaa     480

tgtaatttaa gtttcataat tattttttgt gcactgttat ttattatttc tatattgcgt     540

t                                                                     541
```

```
<210>  47
<211>  92
<212>  PRT
<213>  Gerbera hybrid

<400>  47

Met Ser Ser Arg Arg Ser Arg Gln Ser Ser Ser Gly Ala Ser Arg Ile
1               5                   10                  15

Thr Asp Asp Gln Ile Ile Gln Leu Leu Ser Lys Leu Gln Gln Leu Leu
            20                  25                  30
```

Pro Glu Ile Arg Asn Arg Arg Ser Asn Lys Ala Ser Ala Ser Lys Val
35 40 45

Leu Gln Glu Thr Cys Asn Tyr Val Arg Ser Leu His Lys Glu Val Asp
50 55 60

Asp Leu Ser Asp Arg Leu Ser Gly Leu Leu Ser Thr Ile Asp Ala Asp
65 70 75 80

Ser Pro Glu Ala Ser Ile Ile Arg Ser Leu Phe Met
85 90

<210> 48
<211> 476
<212> DNA
<213> Gerbera hybrid

<400> 48
gctcctctct ctttcatttc attcttccaa aacaccaaac ttgatcatcg gtctatataa 60

accctctcac ttaacaaaca cataattatt gacaacatac agatttgatc ataatgtcga 120

gtagaaggtc gagacaatcg tcaagtggag cttcgaggat cactgatgat caaatcatac 180

aactactatc gaagttgcaa caacttcttc ctgaaattcg taatcgtcgt tccaacaagg 240

catcggcttc gaaggtgtta caagaaacct gcaattatgt gagaagctta cacaaagagg 300

ttgatgatct tagcgaccga ttgtcggggt tattatccac cattgatgct gatagccccg 360

aagcttcaat tattcgaagt ctatttatgt aaattttatt aactaattag ctttttttatt 420

atatataaat tattaataca gtgtttaagt taactgtttt cctgaatgtt tctcta 476

<210> 49
<211> 93
<212> PRT
<213> Glycine max

<400> 49

Met Ser Ser Arg Arg Ser Arg Ser Arg Gln Ser Gly Val Ser Thr Glu
1 5 10 15

Ile Thr Asp Ala Gln Ile Thr Asp Leu Ile Ser Lys Leu Gln Gln Leu
20 25 30

Ile Pro Glu Leu Arg Ala Arg Arg Ser Asp Lys Val Ser Ala Ser Lys
35 40 45

Val Leu Gln Glu Thr Cys Asn Tyr Ile Lys Ser Leu His Arg Glu Val
50 55 60

Asp Asp Leu Ser Asp Arg Leu Ser Gln Leu Leu Ala Thr Thr Asp Ser

65 70 75 80

Asn Ser Ala Gln Ala Ala Ile Ile Arg Ser Leu Leu Met
85 90

<210> 50
<211> 997
<212> DNA
<213> Glycine max

<400> 50
cgggcacgag gccaacaccc actagactgg cacattctct caactctctc aataagcttt 60

ctctcatgct catggcctct accactacct ttatctctct ctcttcctag ttcattcatt 120

ctcctctctc aaaaacataa acatcagcac ttctctcttt tgaatattcc tcaattttat 180

agctacctag ctacctagct acctagctaa gctatacttg gttttcttta atttctctga 240

caaatattcc aacttctttt ctatataggc tctagtagct tagtagttat ctttcagtta 300

ccttgaacaa atcaacagca aaatatattt ctgacacact catcagagac cattttaaat 360

ttaattaaca acaaacaatg tctagcagaa gatctcgttc gagacaatcg ggtgtttcca 420

ctgagatcac tgatgctcag atcactgatc tcatctcaaa gttacaacaa ctgatccctg 480

agctacgcgc aagacgttct gacaaggttt cagcttccaa ggtgttgcaa gagacttgca 540

actacatcaa aagcttgcac agagaggttg atgatctaag tgaccggttg tcacaacttt 600

tggccaccac cgactccaac agtgcccaag cagccattat taggagctta cttatgtaat 660

atataataat attctaataa ttactattaa ttatagagct ttaattttat gtgtcgtttg 720

catgtccatg ttaatttttt ttattgtcat gatatcctct attctgggta gggtttggtt 780

tttaagacca aagcaaaaag gccaccgtgg gctccatgtt ctcccttact tagttttatc 840

agacacttta tattattgac tatcatcaaa ttgtattact aaaataaaat gaccttgcat 900

cttgatgatc gagtctttag tttgaatgta aagtcatata tattaatgtg tataaatata 960

tatacatgaa tctgtacccg agtagaacat atatgac 997

<210> 51
<211> 93
<212> PRT
<213> Glycine max

<400> 51

Met Ser Ser Arg Arg Ser Arg Ser Arg Gln Ser Gly Ala Ser Ala Glu
1 5 10 15

Ile Thr Asp Ala Gln Ile Thr Asp Leu Val Ser Lys Leu Gln Gln Leu
20 25 30

```
Ile Pro Glu Leu Arg Ala Arg Arg Ser Asp Lys Val Ser Ala Ala Lys
        35                  40                  45


Val Leu Gln Glu Thr Cys Asn Tyr Ile Lys Asn Leu His Arg Glu Val
    50                  55                  60


Asp Asp Leu Ser Asp Arg Leu Ser Glu Leu Leu Ala Asn Thr Asp Ser
65                  70                  75                  80


Asn Ser Ala Gln Ala Ala Ile Ile Arg Ser Leu Leu Met
                85                  90


<210>  52
<211>  827
<212>  DNA
<213>  Glycine max

<400>  52
ctctcttttt gagataagtt tgtttagttt cattttctgt gattctcgtc tgacaagccc      60

cccccccccc ccccccaatt tcctgcttct tcttggccct ccaatttccc ctcagacctt     120

gttctctcat cactcactca ctcacaacaa caacaacaac aacactctct ttcctctctc     180

atttttaatt attctcttca attccttaag tcattaagag gtagctagaa gtagtagctc     240

gcaacagcaa atatttctga cacaaacatc atcacaaaag ggtagtagtg gacgttgttg     300

ttaataaatt gttcctctca ttaattaatt gacaatgtct agcagaagat ctcgttcaag     360

acaatccggt gcttccgctg agatcactga tgctcaaatc accgatctcg tttccaagtt     420

acaacaactt atccctgagc ttcgtgctag gcgctccgac aaggtttcag ctgctaaggt     480

attgcaggag acatgcaact acataaagaa cttgcacaga gaggttgatg atctaagtga     540

ccgattatcg gagcttttgg ctaacacaga ctccaacagt gctcaagcag ccattattag     600

gagcttactt atgtaatagt ctagtctagt gcattaattt gtgtcgtgtg catgtccatg     660

tctctctcac tttttttttt ttttttatca ctctgggtag ggtttggttt gtactttgtt     720

tatcaacgca aaggactacc atcggatcca tgtgaattag ttcttaatta gttaatttta     780

attatcatgt ggcactttgt agtaattaat atcaacagct tctacgg                   827


<210>  53
<211>  92
<212>  PRT
<213>  Glycine max

<400>  53

Met Ser Ser Arg Arg Ser Arg Ser Arg Gln Thr Ser Ser Ser Arg Asn
1               5                   10                  15


Ile Thr Asp Asp Gln Ile Asn Asp Leu Val Ser Lys Leu Gln Gln Leu
```

20 25 30

Leu Pro Glu Ile Arg Asp Arg Arg Ser Asp Lys Val Ser Ala Ser Lys
35 40 45

Val Leu Gln Glu Thr Cys Asn Tyr Ile Arg Ser Leu His Arg Glu Val
50 55 60

Gly Asp Leu Ser Glu Arg Leu Ser Glu Leu Leu Asp Thr Thr Asp Thr
65 70 75 80

Ala Gln Ala Ala Ile Ile Arg Asn Leu Leu Met Gln
85 90

<210> 54
<211> 821
<212> DNA
<213> Glycine max

<400> 54
gatttctttc tcgatcccca acatcactag ctagctcctt gtacacactc tacaacccca 60

cctagctaca tcacttaatt agttttacca atttcaaatt ctcacctgtc actagctata 120

tttcataact gatcattacc aactcatcac tacatattat tggctaggat tcaccattag 180

acttaagatt agttgattta ttacatatat aagatgtcta gcaggaggtc acggtcaagg 240

caaacaagta gttcaaggaa tatcaccgat gatcagatca atgatcttgt ctctaagttg 300

caacagcttc ttccagagat tcgcgatagg cgctctgaca aggtttcagc ttccaaggtg 360

ttgcaagaga catgcaacta tattagaagc ttacacaggg aagtgggtga cctaagcgag 420

cgtttatctg agctcctgga tacaactgac acggctcaag ctgcaataat tagaaattta 480

ctgatgcaat agatcggtgc agttgttaat ttatcgtata attcatagtt aacacttcag 540

tacttgtgaa ccgatccagt cactggtcgt gtatttctta ttctcttttc gtttcacttt 600

tttttttttt ttttgtgctg gttcttgtcc actaatatga atgattactg cttttgcaaa 660

gcccaatttc cttatatatt aaataaaagt ttcagagttc gtgctttgct aaattaaata 720

tacattttct ctttctaagc acacttaata ttagatggac atttttttaa aaaatatttg 780

tctgaaattt gaccctatcg tttttaatta tttaccaggg g 821

<210> 55
<211> 93
<212> PRT
<213> Glycine max

<400> 55

Met Ser Ser Arg Arg Ser Arg Ser Ser Gln Ser Asp Val Ser Thr Glu
1 5 10 15

```
Ile Thr Asp Ala Gln Ile Thr Asp Ile Ile Ser Lys Leu Gln Gln Leu
            20                  25                  30

Ile Pro Glu Leu Asp Ala Arg Arg Ser Asp Lys Val Ser Ala Ser Lys
        35                  40                  45

Val Leu Gln Glu Thr Cys Asn Tyr Ile Lys Ser Leu His Arg Glu Val
    50                  55                  60

Asp Asp Leu Ser Asp Arg Leu Ser Gln Leu Leu Ala Thr Thr Asp Ser
65                  70                  75                  80

Asn Ser Ala Gln Ala Ala Ile Ile Arg Ser Leu Leu Leu
                85                  90


<210>  56
<211>  642
<212>  DNA
<213>  Glycine max

<400>  56
gctacctcta taggctctag ctagcttagt agtcagaagt agttagtact tatctttcag      60

ttaccttgaa caaatcaagt gtacacaaca gcaaaatatt tctgtcacac aaacacactc     120

gtcacaaacc cttttaaatt taaaattaac aacaatgtct agcagaagat ctcgttcgag     180

tcaatcggat gtttccactg agatcactga tgcccagatc actgatatca tctcaaagtt     240

acaacaacta atccctgaac tagatgcaag gcgttcagac aaggtttcag cttccaaggt     300

gttgcaggag acttgcaact acatcaaaag cttgcacaga gaggttgatg atctaagtga     360

ccggttgtca caacttttgg ccaccacaga ttccaacagt gcccaagcag ccataattag     420

aagcttactt ttgtaataat aatattattc taatacttat tacttataga gctttaattt     480

atgtgacgtg tgcatgttat ttttggttat aagaccaaat tgtattacta taaataaaat     540

gagctggcat cttgatgatc gagttccagt tagttcgaat agttatatta atgtgtataa     600

atatattata catgaatctg tacccgtgta gaaatatatg ac                        642


<210>  57
<211>  91
<212>  PRT
<213>  Glycine max

<400>  57

Met Ser Ser Arg Arg Ser Arg Gln Gln Ser Ala Ser Thr Arg Ile Ser
1               5                   10                  15

Asp Asp Gln Ile Ile Asp Leu Val Ser Lys Leu Arg Gln Leu Val Pro
```

20 25 30

Glu Ile Arg Asp Arg Arg Ser Asp Lys Val Ser Ala Ser Lys Val Leu
35 40 45

Gln Glu Thr Cys Asn Tyr Ile Arg Ser Leu His Arg Glu Val Asp Asp
50 55 60

Leu Ser Glu Arg Leu Ser Gln Leu Leu Ala Thr Ile Asp Ala Asp Ser
65 70 75 80

Pro Glu Ala Ala Ile Ile Arg Ser Leu Ile Asn
85 90

<210> 58
<211> 835
<212> DNA
<213> Glycine max

<400> 58
gcaacactac tatatcttag ccttttctct ccctcccttc tcccatatta tatagcttgt 60

cttttatttc ttagactcca tccattcttc tccccaattg aatcttcttt attttgtttc 120

ttcactgtct cgttatggct atagttttgc atagtaaata aactgaactg aagctatcta 180

tatagcagca agtgttgatt taattactta ctttagacaa taattatatt aattacacca 240

atttataagc tctttatcta tctatctagc tagggaaaat taaaatgtct agcagaaggt 300

ccaggcagca atctgcatcc acaaggatct ccgatgacca aatcatcgac ctcgtttcaa 360

agttgcgtca acttgttcct gagattcgcg ataggcgctc tgacaaggta tcagcatcta 420

aggtcctaca agagacctgc aactacatca gaagcttaca cagagaagtg gatgacttaa 480

gcgaacgact gtctcagttg ttggccacaa tcgatgctga tagccctgaa gctgccatca 540

ttaggagcct aattaactaa taatatatat taagcgcaag taatcatcta attttcctat 600

attcaaggag atatattata agagtgtatt aatttcttct tctaaattag gtggcataga 660

gtgcagtttg aggtgcgtac gtacgtcctt ccaatatatt atagtacatg gcaggaatgg 720

tgcacttgtg taagttaaag gtttttgcaa taagaactaa ggactctctg tattatggct 780

atagtgctat ataataatat atgcatgcca catttataga tggcctccaa aaaaa 835

<210> 59
<211> 92
<212> PRT
<213> Glycine soja

<400> 59

Met Ser Ser Arg Arg Ser Arg Ser Arg Gln Thr Ser Ser Ser Arg Asn
1 5 10 15

```
Ile Thr Asp Asp Gln Ile Asn Asp Leu Val Ser Lys Leu Gln Gln Leu
            20                  25                  30

Leu Pro Glu Ile Arg Asp Arg Arg Ser Asp Lys Val Ser Ala Ser Lys
        35                  40                  45

Val Leu Gln Glu Thr Cys Asn Tyr Ile Arg Ser Leu His Arg Glu Val
    50                  55                  60

Asp Asp Leu Ser Glu Arg Leu Ser Glu Leu Leu Ala Thr Thr Asp Thr
65                  70                  75                  80

Ala Gln Ala Ala Ile Ile Arg Asn Leu Leu Met Gln
                85                  90


<210>  60
<211>  704
<212>  DNA
<213>  Glycine soja

<400>  60
ctcgatcccc aacatcacta gctagctcct tttgtacaca ctctacaacc ccacctagct      60

acatcactta attagttttc ccatatctat aaccaatttc aaattctcac ccttaactag     120

ctagctatat ttcataactg attattacca actcactaca tattattggc taggattcac     180

cattagactt aaaagtagtt gatttattat atatataaga tgtctagcag gaggtcacgg     240

tcaaggcaaa caagtagttc aaggaatatc accgatgatc agatcaatga tcttgtctcc     300

aagttgcaac agcttcttcc agagattcgc gataggcgct ctgacaaggt ttcagcttcc     360

aaggtgttgc aagagacatg caactatatt agaagcttac acagggaagt ggatgaccta     420

agcgagcgtt tatctgagct cttggctaca actgacacag cacaagctgc aataattaga     480

aatctactaa tgcaatagat cggtgcagta gttaatttat cgcataattc atagttagca     540

cttcagtact tgtgaaccga tccagtcagt agtcgcgtat ttcttattct ctttttgttt     600

cacttttttt ttctggtttt tgtccactaa tatgcatgat tactgctttt gcaaagccca     660

ttttcctaag atattaaata aaagtctgag tttgcgcttt gcta                      704


<210>  61
<211>  91
<212>  PRT
<213>  Gossypium arboreum

<400>  61

Met Ser Ser Arg Arg Trp Arg Glu Ser Ser Arg Thr Asn Ile Trp Glu
1               5                   10                  15
```

Glu Gln Ile Thr Gln Leu Leu Ser Thr Leu Arg Gln Leu Leu Pro Glu
20 25 30

Ile Pro His Ser His Ser His Lys Ala Ser Ser Ala Ala Lys Val Leu
35 40 45

Glu Gln Thr Cys Asn Tyr Ile Lys Thr Leu His Arg Glu Val Asp Asp
50 55 60

Leu Ser Asp Arg Leu Ser Gln Leu Leu Ala Thr Ile Asp Ala Asp Ser
65 70 75 80

Ala Glu Ala Ala Ile Ile Arg Ser Leu Phe Asn
85 90

<210> 62
<211> 487
<212> DNA
<213> Gossypium arboreum

<220>
<221> misc_feature
<222> (486)..(486)
<223> n is a, c, g, or t

<400> 62
gttataggca gtgataagat gtcaagcaga aggtggaggg agtcgtccag gaccaatatt 60

tgggaggagc agatcactca acttctctct accttacgcc aacttcttcc cgagattcct 120

cattcccatt ctcacaaggc atcatcagcg gccaaggttt tagaacagac atgcaattac 180

ataaaaacct tgcatcgtga agttgatgat ctgagtgacc ggctgtccca gctcctagcc 240

accatcgatg ccgacagtgc cgaagccgcc atcatccgaa gcttatttaa ttaatacaaa 300

aaaaaaaaaa cctccttctg tattccttca attctctctg ctttgtctat acttttagtt 360

tttactagct aggctctaat gaatcattac atcgtacaca catgactata tattttgaga 420

cactatgctt ccctttcgtg agactgtaaa taaaagatat ttgcactagc aaacgccttt 480

ttgctnt 487

<210> 63
<211> 92
<212> PRT
<213> Gossypium hirsutum

<400> 63

Met Ser Ser Arg Arg Ser Arg Ser Arg Gln Ser Gly Ala Ser Arg Ile
1 5 10 15

```
Thr Asp Asp Gln Ile Ile Asp Leu Val Ser Lys Leu Gln Gln Leu Ile
            20                  25                  30

Pro Glu Leu Arg Gly Arg Arg Pro Asp Lys Val Ser Ala Ser Lys Val
        35                  40                  45

Leu Gln Glu Thr Cys Asn Tyr Ile Arg Ser Leu His Arg Glu Val Asp
    50                  55                  60

Gly Leu Ser Asp Arg Leu Ser Gln Leu Leu Ala Ser Thr Asp Thr Asp
65                  70                  75                  80

Ser Asp Gln Ala Ala Ile Ile Arg Ser Leu Leu Met
                85                  90
```

<210> 64
<211> 663
<212> DNA
<213> Gossypium hirsutum

<400> 64

```
atgcatttgt cccttataat tctactaccc aagcatatct cttctctacc tttcttgctc      60
ttgtttttta cttcgtttct aatttcctcc tctcatccca ttttcccctt aaacttatat     120
ttataatatc cttaaacttt cactttgttg attactttcc tgagccaaat atcagtacaa     180
tgtcaagcag aagatcacgt tctaggcaat caggtgcttc aaggatcact gatgatcaga     240
tcatcgatct tgtttccaag ttgcaacagc ttatccctga gcttcgtgga agacgccccg     300
acaaggtatc agcttctaag gtcttacagg aaacctgcaa ctatatcaga agcttacaca     360
gagaagttga cggcttaagc gatcggttat ctcagctatt agcttccaca gacaccgata     420
gcgaccaagc agccattatc aggagtttac ttatgtaatg atcagaccta gattaagtat     480
tactcacttt ttccaggtct agggtttgtt tatcatcgct tgtattgtag taatgcagaa     540
caagggtgga atagtggcta cagtgaacca tgtttcgtaa tccttgtcat caagagactt     600
gtataccgtt cgagtttatc ctcgtatatt tataaaataa aaataaatta tgagttgcgg     660
ggg                                                                   663
```

<210> 65
<211> 92
<212> PRT
<213> Gossypium hirsutum

<400> 65

```
Met Ser Ser Arg Arg Ser Arg Gln Ser Thr Ala Gly Val Ser Arg Ile
1               5                   10                  15

Ser Asp Asp Gln Ile Ile Glu Leu Val Ser Lys Leu Arg Gln Leu Leu
```

20 25 30

Pro Glu Ile Arg Asp Arg Arg Ser Asp Lys Val Ser Ala Ser Lys Val
35 40 45

Leu Gln Glu Thr Cys Asn Tyr Ile Arg Ser Leu His Arg Glu Val Asp
50 55 60

Asp Leu Ser Glu Arg Leu Ser Gln Leu Leu Ala Thr Ile Asp Ala Asp
65 70 75 80

Ser Ala Glu Ala Ala Ile Ile Arg Ser Leu Ile Met
85 90

<210> 66
<211> 602
<212> DNA
<213> Gossypium hirsutum

<400> 66
ctactctcta ctctttcttt ctttcttctt cttcttcttc ttcttcttgc cttttccaaa 60

ccataaccat ttttctacac catatatact tatttttcct tctgcatttc catatcttgg 120

gggtattatt ttggaactta ctgaatattt tagaacaatt cttggttttt ggtcattagg 180

gtgttattat tactttaaat cccccacaag atgtctagca gaaggtcgag acagtcaaca 240

gcaggtgttt cgaggatttc agatgatcaa atcattgaac ttgtatcaaa gttacgacag 300

cttctgcctg agattcgtga taggcgatct gataaggtat cagcatccaa ggtcttacaa 360

gagacttgca attacattag aagcttgcat agggaggtgg acgacctaag tgaacggctc 420

tcacagttgt tggccaccat tgatgctgat agtgccgagg ctgctattat taggagttta 480

attatgtaat aatatttatt ataaaccaat gtttttattt attactaggg ttatatatat 540

atatatgata ttatatattt ggatttatat atagtttaag atgcttcctc catgtggaag 600

gg 602

<210> 67
<211> 93
<212> PRT
<213> Gossypium hirsutum

<400> 67

Met Ser Gly Arg Arg Ser Arg Ser Lys Gln Ser Ser Val Ser Ser Ile
1 5 10 15

Thr Asp Asn Gln Ile Thr Asp Leu Val Ser Lys Leu Gln His Leu Ile
20 25 30

```
Pro Glu Leu Arg Arg Arg Arg Phe Asp Lys Val Ser Thr Ser Lys Val
        35                  40                  45

Leu Gln Glu Thr Cys Asn Tyr Ile Arg Ser Leu His Arg Glu Val Glu
    50                  55                  60

Asp Leu Ser Asp Arg Leu Ser Gln Leu Leu Ala Ser Thr Asp Gly Gly
65                  70                  75                  80

Ser Asp Gln Ala Ala Ile Ile Arg Ser Leu Leu Met Gln
                85                  90
```

<210>  68
<211>  717
<212>  DNA
<213>  Gossypium hirsutum

<400>  68

```
aaagcaaagc atcataacct ctcttctctt tgttcttggt taactcatct ctttgctctt      60

ttcccagctt aagtttccag gtcctttatg catatattat cgtattccct tttttcttag     120

gttttcttcg gtgacaaatc cacatcccat ttgtcaattt aatcaagagt ttcagatatc     180

ctcactttcc ttaagccttc attcatttag tttcttgaca caaaaaatat agtagaagtt     240

attttaaaac acaatgtcag gcagaagatc acgttccaag cagtcaagtg tttccagtat     300

cactgacaat cagatcaccg atcttgtttc caagctgcaa caccttatcc ctgaacttcg     360

tcgaaggcga ttcgacaagg tatcaacttc caaggtgtta caggagactt gcaactatat     420

cagaagctta catagagaag tggaggacct aagcgaccgg ttatcccagc tattagcttc     480

cacagacggc ggtagcgacc aagcagccat tataaggagt ttacttatgc aataaacacc     540

cattttcatt cacctttggt tttactatat taagtactac acacctttc catatcttag     600

gatttccggt agtaatgcag aagagatagg aaaagggtga aacatggagt accatgcatg     660

ttttatgatt cttgtcatca acagactctg taaatcattc aagtctatca ttatata        717
```

<210>  69
<211>  94
<212>  PRT
<213>  Gossypium hirsutum

<400>  69

```
Met Ser Ser Arg Arg Pro Ser Ser Arg Gln Ser Ser Gly Gly Val Ser
1               5                   10                  15

Arg Ile Ser Asp Asp Gln Ile Ile Ala Leu Val Ser Lys Leu Arg His
            20                  25                  30

Leu Leu Pro Glu Ile Arg Asp Asn Arg Ser Asp Lys Val Ser Ala Ser
```

35 40 45

Lys Val Leu Gln Glu Thr Cys Asn Tyr Ile Arg Ser Leu His Lys Glu
50 55 60

Val Glu Asp Leu Ser Asp Arg Leu Ser Gln Leu Leu Ala Thr Ile Asp
65 70 75 80

Ala Glu Ser Ala Glu Ala Ala Ile Ile Arg Ser Leu Leu Met
85 90

<210> 70
<211> 638
<212> DNA
<213> Gossypium hirsutum

<400> 70
atctcttaca tctaacactg ctcattcacg ttgttgtcaa aaccactaca tgttccattt 60

atacacttgc tagcttggct tttgttggtt ttaattgaat atataaaccc ctcgccaacc 120

ctcctaccaa cagatacaag aaacttggtc ttaatttccc aaagatgtcg agccgaaggc 180

cgtcgtcgag gcagtccagc ggcggcgttt cccggatttc agatgatcag ataattgcac 240

ttgtctccaa gttacgccac cttcttcctg agattcgtga caaccgatct gacaaggtat 300

cagcatcgaa ggttttacaa gagacgtgca attacattag aagcttgcat aaagaggtgg 360

aagatctaag tgaccgactc tctcagcttt tggctaccat tgatgccgaa agcgccgagg 420

ctgctattat taggagttta cttatgtaat aataatccaa ctttactatt attatttatt 480

aggttcggtc gctaggtcca acataaacta gattattgtt taagatgctt acccccatgt 540

gcattgtaat tacttagtat aataaaaaga cttgcaatag caaagtcttt taattgtaat 600

gacaatatgg atgacttata taattatgtt tagctttt 638

<210> 71
<211> 92
<212> PRT
<213> Hedyotis terminalis

<400> 71

Met Ser Ser Arg Arg Ser Arg Gln Ser Ser Ser Gly Ser Thr Arg Ile
1 5 10 15

Thr Asp Asp Gln Ile Ile Glu Leu Val Ser Lys Leu Gln Gln Leu Leu
20 25 30

Pro Glu Ile Arg Thr Arg Arg Ser Asn Lys Ala Ser Ala Ser Lys Val
35 40 45

```
Leu Gln Glu Thr Cys Asn Tyr Ile Arg Asn Leu His Arg Glu Val Asp
    50                  55                  60

Asp Leu Ser Asp Arg Leu Ser Gln Leu Leu Ser Thr Ile Asp Ala Glu
65                  70                  75                  80

Ser Pro Glu Ala Ala Ile Ile Arg Ser Leu Leu Ser
                85                  90
```

```
<210>  72
<211>  605
<212>  DNA
<213>  Hedyotis terminalis

<400>  72
gcagaaacga acagagtatc ccagtacttc cttcttaatt ggtttcattc cgactggatt      60

ttgtaattcc actgtatcat cattcattag aaaacatttt aatatgctta tgatttatga     120

aacatacatg ttttaccgtt ctgtaattta tctgagtatt tctttacgct agatacaaag     180

aaaaacttat aataaaaaaa tttcaaactg catccagcag ctctgtagta ttctatcagg     240

tgaaaagaaa aaaaaacggt agcaataaaa taatgtctag cagaaggtcc aggcaatcat     300

catcagggtc tactaggatt acagatgatc agattatcga gctggtctca aagttgcagc     360

aacttcttcc tgagattcgt actaggcgtt ccaacaaggc atcggcatct aaggtgctgc     420

aagagacttg caattacatc cgaaacttgc acagagaagt tgatgacctt agcgaccgtc     480

tttcacaact cctgtccacc attgatgctg aaagcccaga ggctgccatt attaggagct     540

tactatctta atccttccat gtagtaataa ctaagtatta gcaagctgcg ttgtaatcag     600

ccagc                                                                 605
```

```
<210>  73
<211>  91
<212>  PRT
<213>  Helianthus paradoxus

<400>  73

Met Ser Ser Arg Arg Ser Arg Gln Ser Ser Ser Gly Ala Arg Ile Ser
1               5                   10                  15

Asp Asp Gln Ile Ile Gln Leu Ile Ser Lys Leu Gln Gln Leu Leu Pro
            20                  25                  30

Gly Thr Arg Ile Gln Arg Ser Asn Lys Ala Ser Ala Ser Lys Val Leu
        35                  40                  45

Gln Glu Thr Cys Thr Tyr Val Arg Ser Leu His Arg Glu Val Asp Asp
    50                  55                  60
```

```
Leu Ser Asp Arg Leu Ser Gln Leu Leu Ser Thr Ile Asp Ala Asp Ser
65                  70                  75                  80

Pro Glu Ala Ser Ile Ile Arg Ser Leu Ile Met
                85                  90

<210>  74
<211>  541
<212>  DNA
<213>  Helianthus paradoxus

<400>  74
tatctacata atctaattat aaagatacga tcgataatac tacgatgtca agcagaagat      60

cgaggcaatc atcatcgggg gctaggatct cagatgatca aatcatacag ctcatctcca     120

agctacaaca acttcttccc gggactcgta tacaaagatc taacaaggcg tcggcttcga     180

aggtgctaca agagacttgc acatatgtta gaagcttgca tagggaggtt gatgacctta     240

gtgatcgact atcgcagtta ttatccacca ttgatgccga tagccctgaa gcttctatta     300

ttcgaagttt aattatgtag aatactcatt tctacttata attatgttat ttgttagctt     360

cttgattaag gataaatgtg gtacgttaat ctctaaatta atcatatgtg tttctagggt     420

ttaatctaat taataaccct agtttcatgt agtgatatta ttttagatat atgtcaatag     480

ttttgatgat gatgataata ataagagcta caccggtagt gtattaaaag ttttctcata     540

a                                                                     541

<210>  75
<211>  97
<212>  PRT
<213>  Helianthus paradoxus

<400>  75

Met Ser Thr Ser Arg Ser Arg Tyr Arg Gln Thr Arg Pro Ser Arg Ile
1               5                   10                  15

Thr Asp Asp Gln Ile Ala Asp Leu Ile Tyr Glu Leu Gln Gln Leu Ile
            20                  25                  30

Pro Asn Asp His Arg Arg Lys Gly Ser Ser Ala Lys Val Leu Glu Glu
        35                  40                  45

Thr Cys Ser Tyr Val Gly Ser Ser Gln Arg Glu Val Glu Asp Leu Ser
    50                  55                  60

Gln Arg Leu Ser Lys Leu Leu Gln Pro Met Asp Thr Asn Ser Pro Gln
65                  70                  75                  80
```

Ala Ala Ile Ile Arg Thr Leu Leu Met Ser Pro Lys Leu Ile Tyr Asp
85 90 95

Tyr

<210> 76
<211> 715
<212> DNA
<213> Helianthus paradoxus

<400> 76
gtttgtttga tcacctccaa gtgtctttat ttttctcatc ttctccatta tttttctccc 60

cataacttgt cggcatcatt caattaacac cttcatatat accactttca cactaatacc 120

actatatcaa accatctact atcttagtta tcacctcatc tctttgctaa ctcatccatg 180

tctacctcaa gatcacgcta ccgacaaacc cggccatcga ggatcaccga cgaccaaatc 240

gcggatctca tatacgagct acaacaactc attcctaacg atcatcgtcg caaggggtca 300

agtgcaaagg tattggagga gacatgtagt tacgttggaa gttcacagag agaagttgaa 360

gacttgagtc aaaggctatc aaagcttttg cagcccatgg acaccaacag tccacaagca 420

gccatcatta gaaccttact tatgtcaccc aagctaatat atgattatta atgatcttga 480

tatatgtgat ttgatcaatg tcattttgat ttcttctgca tgtacgtgca ttttccttga 540

gtatgttcgc ttgctgagaa tttctctggc agcgatgagc acaagacttc agcccctgcc 600

accataggcg gtggtgaata tggatataag gaacgagagg aaggacatga gaagaaagga 660

ctgatggata agattaagga aaggctacct ggcggcgatc atggcaggga tgagc 715

<210> 77
<211> 91
<212> PRT
<213> Helianthus tuberosus

<400> 77

Met Ser Thr Ser Arg Pro Arg Tyr Arg Gln Thr Arg Pro Ser Arg Ile
1 5 10 15

Thr Asp Asp Gln Ile Ala Asp Leu Ile Tyr Lys Leu Gln Gln Leu Ile
20 25 30

Pro Asn Asp His His Arg Lys Gly Ser Ser Ala Lys Val Leu Glu Glu
35 40 45

Thr Cys Ser Tyr Val Arg Ser Leu Gln Arg Glu Val Glu Asp Leu Ser
50 55 60

Gln Arg Leu Ser Glu Leu Leu Gln Ser Met Asp Thr Asn Ser Pro Gln

65 70 75 80

Ala Ala Ile Ile Arg Ser Leu Leu Met Ser Pro
85 90

<210> 78
<211> 494
<212> DNA
<213> Helianthus tuberosus

<400> 78
cgggatgccc ttgagttaat caactccaat gtctttattt ttctcatctt ctccattatt 60

tttctcccca taacttgtcg gaatcattca acaccttcat atatatcact ttctcacgaa 120

taccattata tcaaaccatc ttatcacctc atctctttgc taactcatct atgtctacct 180

caagaccacg ctaccgacaa accagaccat cgaggatcac cgacgaccaa atcgcggatc 240

tcatatacaa gttacaacaa ctcattccta acgatcatca tcgcaagggt tcaagtgcaa 300

aggtattgga ggagacatgt agttacgtta gaagtttaca gagagaagtt gaagacttga 360

gtcaaaggct atcagagctt ttgcagtcca tggacaccaa cagtccacaa gcagccatca 420

ttagaagctt acttatgtca ccctagcttg ctaatatatt tatttatctt tatatatgtt 480

atttgatcaa tgtc 494

<210> 79
<211> 88
<212> PRT
<213> Hordeum vulgare

<400> 79

Met Ser Ser Arg Arg Ser Ser Arg Gly Ala Ile Ser Asp Glu Glu Ile
1 5 10 15

Asn Glu Leu Ile Ser Lys Leu Gln Ser Leu Leu Pro Asn Ser Arg Arg
20 25 30

Arg Gly Ser Ser Gln Ala Ser Thr Thr Lys Leu Leu Lys Glu Thr Cys
35 40 45

Ser Tyr Ile Lys Ser Leu His Arg Glu Val Asp Asp Leu Ser Asp Arg
50 55 60

Leu Ser Asp Leu Met Ser Thr Met Asp His Asn Ser Ala Gly Ala Glu
65 70 75 80

Ile Ile Arg Ser Ile Leu Arg Ser
85

<210> 80
<211> 1139
<212> DNA
<213> Hordeum vulgare

<220>
<221> misc_feature
<222> (3)..(3)
<223> n is a, c, g, or t

<220>
<221> misc_feature
<222> (11)..(11)
<223> n is a, c, g, or t

<220>
<221> misc_feature
<222> (1105)..(1105)
<223> n is a, c, g, or t

<220>
<221> misc_feature
<222> (1123)..(1123)
<223> n is a, c, g, or t

<400> 80
ggnctgcagg naattcggca cgaggagcga ttgcacaact acaagttata tccagcagca 60

aagtatcctt gagttgctcg acgtcagcga gggcctccct tcgctcactg gccaactgcc 120

cgccgctcct tgagcataca cactgtgtgg ctttacttgc cggcggcagt ctgcagtctc 180

tgttagctcc ggccggcggt agctagcttg tcatcccggc cggcgacgca gcggcggcta 240

ggaaggaaga cgatgtcgag cagaaggtcg tcgcgcggcg ccatctccga cgaggagatc 300

aacgagctca tctccaagct ccagtctctg ctccccaact ctcgccgccg cggctccagc 360

caggcgtcga cgacgaagct gctcaaggag acgtgcagct acatcaagag cctccaccgg 420

gaggtggacg acctcagcga ccggctgtca gacctcatgt cgaccatgga ccacaatagc 480

gccggagcag agatcatccg cagcatcctc cgctcgtgat cgtacgtact gaagtgccgg 540

caggtcggcg aggactaaac cgccgggacg attaagcggc ggcggcgcca tgggtttctc 600

cggccagccg gacacgtacg cacgagagct ttgcttagct agggtatata tatttgtcct 660

ccacatattt aaatatgtat ctctttcctg ctccctttct gcctagatcg atctgatcgt 720

gtagatcgaa aaatgtactc cgtgtcccta agcttcactc cttctgctgt actgcgtagg 780

gcattagctt agctagcgtc cctaccttgg gccaaagctt atcctcgcgc gctggctgcc 840

gcttgagtta atctctcgat cgtctcctcc gtgtgcgtgt ttccctcgct agctcggagc 900

tggatagatg gctccgttcc tcctcctgtc tgcctcttcc cctcttttgt tctccctttc 960

tcgatctact actcgatatg taaatttagt tggtggcatt ggatcgagtt gtgtcctcta 1020

tagacaaccg accgaccact actacggtac tactcctcct actattacct agagcaaact 1080

aatatcaacg ccatgttgta ccatnccagg tttaactttt gtngaatacg tactacgag 1139

<210> 81
<211> 90
<212> PRT
<213> Hordeum vulgare

<400> 81

Met Ser Gly Arg Arg Ser Arg Gly Ser Val Ser Glu Glu Glu Ile Asn
1               5                   10                  15

Glu Leu Ile Ser Arg Leu Gln Thr Leu Leu Pro Thr Thr Arg Arg Arg
            20                  25                  30

Gly Ser Ser Ser Ser Ser Ser Gln Ala Ser Thr Thr Lys Met Leu Lys
        35                  40                  45

Glu Thr Cys Ser Tyr Ile Lys Ser Leu His Arg Glu Val Asp Asp Leu
    50                  55                  60

Ser Asp Arg Leu Ser Asp Leu Met Ser Thr Met Asp Asn Asn Ser Pro
65                  70                  75                  80

Ala Ala Glu Ile Ile Arg Ser Leu Leu Arg
                85                  90

<210> 82
<211> 901
<212> DNA
<213> Hordeum vulgare

<220>
<221> misc_feature
<222> (862)..(862)
<223> n is a, c, g, or t

<400> 82
cagctagccg cccactctac tccttccgat cacacaggac acagaagcct ctccgtcgac 60

ctcggcctta acttgctcgc gcctcattat tatcatcgga cgacggcgat gtcgggcagg 120

aggtcgcgcg gctccgtgtc ggaggaggag atcaacgagc tcatctccag gctccagacc 180

ctgctcccca ccacgcgccg ccgcggcagc agcagcagca gcagccaggc gtcgacgacg 240

aaaatgctca aggagacgtg cagctacatc aagagcctgc acagggaggt ggacgacctg 300

agcgaccgcc tctccgacct catgtccacc atggacaaca acagccccgc cgccgagatc 360

atccgcagcc tcctccgcta gctagctaga gctacctgca gctcaactgc tcatcatcat 420

catcatcatc gatcacgtcc agctgattgt cctaagctag ctcatcatct acttacagct 480

cgtgcatgcc tagctaagcc cgcgcatata tctacatata catacaagta tggtatatat 540

gtcgtccgtc gatctctgca aggcatatat atgcagatcg atcgacacct aaggactgca 600

tgcatatgca tccatgctaa aggctggtct aatttacttt ggtagggcat cattagctag 660

ggccgcctaa ttaagttcac tatagctggt taattagctc atcccgctag cttcttgcat 720

gcatgtggtt tcctcccagc ttccctctct cttgttttca tttgtttttc cctgtgtaaa 780

cttacttatt tgcagtctgg atcgagttgt ttccctagag acaccgaccg accgctagct 840

acccatccgt ggtactgcat gnctatatat atatagcact agtaccatca cacatgcatg 900

a 901

<210> 83
<211> 96
<212> PRT
<213> Lactuca sativa

<400> 83

Met Val Met Ser Ser Arg Met Ser Arg Gln Ser Thr Thr Gly Ala Ser
1 5 10 15

Lys Ile Thr Asp Asp Glu Ile Met Gln Leu Leu Ser Gln Leu Gln Gln
20 25 30

Leu Leu Pro Glu Ile Thr Asn Arg Arg Ser Asp Thr Ala Ser Ala Ser
35 40 45

Lys Val Leu Gln Glu Thr Cys Ser Tyr Val Arg Ser Leu His Arg Glu
50 55 60

Val Asp Asp Leu Ser Asp Arg Leu Ser Gln Leu Leu Ser Thr Ile Asp
65 70 75 80

Ala Arg Gln Pro Pro Ser Phe Asn Tyr Arg Lys Phe Asn Asp Leu Ile
85 90 95

<210> 84
<211> 681
<212> DNA
<213> Lactuca sativa

<400> 84
ttcttatatt cacatcgttg ggcatgcttc caagaatcat atcgtactct ttctctttct 60

tagagtaaat tgttgcatca ctgaccttct gtctaaaaat actgcgatat attgacgtga 120

aactgaaact attttgacta aacattgtat ttatacatca ttgccacctc tctcaaaaca 180

cctctatttc tttctcacct acttgtcata cacatacaaa ttttatggta atgtcaagca 240

gaatgtcaag gcaatcgaca accggagctt ccaagatcac cgatgatgag atcatgcaac 300

ttctctcaca gttgcagcaa cttcttcccg agataacaaa tcggcgttcc gacacggcgt 360

cggcttcaaa ggtgctacaa gagacttgca gctatgtgag aagcttgcat agggaagttg 420

acgatcttag cgaccgattg tcgcaactat tgtccaccat tgacgctcgg cagcccccaa 480

gcttcaatta tcgaaagttt aatgatttga tttgaatgga ctttttteta attacatgaa 540

ataatattgt atttcagtta attaacaatc tgagtgattt ctgggggtac gaaataccct 600

tgtggggatc atttatgact accgcttgta attatatatg accaccgatt gtactttcta 660

tatctaacat tcaccttcgt c 681

<210> 85
<211> 93
<212> PRT
<213> Lactuca virosa

<400> 85

Met Ser Gly Arg Arg Ser Arg Ser Arg Gln Thr Gly Val Ser Arg Ile
1 5 10 15

Ser Asp Asp Gln Ile Ala Asp Leu Val Ser Lys Leu Gln Gln Ile Ile
20 25 30

Pro His Asn Ile His Ala Thr Arg Ser Asp Lys Val Ser Ala Ser Arg
35 40 45

Val Leu Gln Glu Thr Cys Asn Tyr Ile Arg Ser Leu His Arg Glu Val
50 55 60

Asp Asp Leu Ser Glu Arg Leu Ser Glu Leu Leu Gln Ser Thr Asp Ala
65 70 75 80

Asn Ser Ala Glu Ala Ala Ile Ile Arg Ser Leu Phe Met
85 90

<210> 86
<211> 459
<212> DNA
<213> Lactuca virosa

<400> 86
gcggggatct tgtgatcatt aatttgtaag acttctctga caaattaata aggaaagaaa 60

ccccttctta taataccatc tcagttgtcc ctctagatct caatgtctgg cagaagatct 120

cgatcacggc aaactggagt ttccaggatc agcgacgatc agattgccga cctcgtctcc 180

aagttacaac aaattatacc tcataatatc cacgcaaccc gttctgacaa ggtttcagct 240

tcaagagtgt tgcaggagac atgcaattat atcagaagct tacacagaga agtggatgat 300

ttaagcgaaa gactttcaga gcttttgcaa tctacggacg ccaacagtgc tgaagcagcc 360

attattagga gcttatttat gtaactatat tgttaacaaa ttaagcagtt aatgtaaggc 420

ttttctgtgt taataaatca gcataaatat gttttcttt 459

<210> 87
<211> 96
<212> PRT
<213> Malus x domestica

<400> 87

Met Ser Ser Arg Arg Ser Ser Arg Ser Arg Gln Ser Ser Ser Arg Asn
1               5                   10                  15

Asn Asn Ser Ile Ser Asp Asp Gln Ile Thr Asp Leu Val Ser Lys Leu
            20                  25                  30

Gln Gln Leu Leu Pro Glu Ile Arg Pro Arg Arg Ser Asn Lys Ala Ser
        35                  40                  45

Ala Ser Lys Val Leu Gln Glu Thr Cys Asn Tyr Ile Arg Asn Leu His
    50                  55                  60

Arg Glu Val Asp Asp Leu Ser Glu Arg Leu Ser Glu Leu Leu Ala Thr
65                  70                  75                  80

Thr Asp Met Asp Asn Asp Gln Ala Ala Ile Thr Arg Ser Leu Leu Leu
                85                  90                  95

<210> 88
<211> 455
<212> DNA
<213> Malus x domestica

<400> 88
tcccgctcat ttgtctctca tagtttttc tctagaagaa cttcaaactc taatattatt 60

atttcttctg agttttctgt cagaacttca aactctaata ttattatttc ttctgagttt 120

tctgtcagaa cttcaaactc tccaaatatt tgacaatgtc gagcagaaga tcctctcggt 180

cgaggcagtc gtcgagtagg aacaacaaca gtatcagtga tgaccagatc actgatctcg 240

tatccaagtt acagcagctt cttcctgaga ttcgccctag gcgttccaac aaggcgtcgg 300

cgtcgaaggt tttgcaagag acttgcaatt atattagaaa cttacacaga gaggtggatg 360

acctaagtga gcgcttatca gagcttttgg ccacgacgga catggataac gaccaagcag 420

ccattactag gagtttactt ttgtgatggt gtctg 455

<210> 89
<211> 91
<212> PRT

<213> Malus x domestica

<400> 89

Met Ser Ser Arg Arg Ser Arg Gln Ser Gly Thr Pro Ala Ile Lys Asp
1 5 10 15

Asp Gln Ile Ile Glu Leu Val Ser Lys Leu Arg Gln Leu Val Pro Glu
20 25 30

Ile Arg Asp Arg Arg Ser Asn Lys Ala Pro Ala Ser Lys Val Leu Gln
35 40 45

Glu Thr Cys Ser Tyr Ile Arg Asn Leu His Arg Glu Val Asp Asp Leu
50 55 60

Ser Glu Arg Leu Ser Gln Leu Leu Ser Thr Ile Asp Ala Asp Ser Pro
65 70 75 80

Glu Ala Ala Ile Ile Arg Ser Leu Ile Met Gln
85 90

<210> 90
<211> 586
<212> DNA
<213> Malus x domestica

<400> 90
aggcaggctg atcatacata cgaacatata tatatgttct gagaacgata tataaaacta 60

gagctacttg tctagctagc tagctaggtg aaggggatca tgtctagcag aaggtcgagg 120

cagtcaggta ctccagcgat caaagatgac caaatcatcg aactggtgtc caaattacgt 180

caactggttc ctgagattcg agataggcgc tccaacaagg caccagcatc taaggtccta 240

caagagactt gcagctacat cagaaactta cacagagagg ttgacgacct aagcgagcga 300

ctctcccaac tgctctctac aattgatgct gatagtccgg aggccgctat aattaggagc 360

ttgattatgc agtagatgga tcatcaaatc acgagcaacc ctaattatat atattggcgt 420

tttaattata tagttaattg tccttttatt tgtttccagg ttatagtact tcgtattgta 480

tgtatttaga gatgtcgtgt gtggttaatt agagtgttta ataaattgaa ggatttgcac 540

tgctactagc aagtcctatt ataaagaaac cctatttact ttctcc 586

<210> 91
<211> 91
<212> PRT
<213> Malus x domestica

<400> 91

Met Ser Ser Arg Gly Ser Arg Gln Ser Gly Thr Pro Ala Ile Lys Asp

1 5 10 15

Asp Gln Ile Ile Glu Leu Val Ser Lys Leu Arg Gln Leu Val Pro Glu
20 25 30

Ile Arg Asp Arg Arg Ser Asn Lys Val Pro Ala Ser Lys Val Leu Gln
35 40 45

Glu Thr Cys Asn Tyr Ile Arg Asn Leu His Thr Glu Val Asp Asp Leu
50 55 60

Ser Glu Arg Leu Ser Gln Leu Leu Ser Thr Ile Asp Ala Asp Ser Pro
65 70 75 80

Glu Ala Ala Ile Ile Arg Ser Leu Ile Thr Gln
85 90

<210> 92
<211> 587
<212> DNA
<213> Malus x domestica

<400> 92
ttttaggcta atattctcgt tcctaaaccc tatatatata aagctccatt gccaggctga 60

tcattcatac atatatatat tttgaaaacg ataatataaa attagctact tgtctagcta 120

ggtaagtaag gatcatgtct agcagagggt caaggcagtc aggtactcca gcgatcaaag 180

atgaccaaat catcgaactg gtgtccaaat tacgtcaact ggttcctgag attcgagata 240

ggcgctccaa caaggtgcca gcatcgaagg tcctgcaaga gacttgcaac tatatcagaa 300

acttacacac agaggttgac gacctaagcg agcgactctc ccaactgctc tctacaattg 360

atgctgatag tccggaggcc gctataatta ggagcttgat tacgcagtag atggatcatc 420

agatcatgat gagaaaccct aatattatat atatatatat gtatgtatgt atatgtatat 480

gtgtgtgtat gtgtgtctaa gagcatttta atttatatag ttaagtgtcc ttttactttc 540

tttccaggtt agagtactta gtactgtatg tatttaaaga tgtcatg 587

<210> 93
<211> 92
<212> PRT
<213> Malus x domestica

<400> 93

Met Ser Ser Arg Arg Ser Ser Ser Ser Ser Arg Thr Ser Lys Pro Ser
1 5 10 15

Asp Asp Glu Ile Lys Glu Leu Ile Ser Lys Leu Gln Pro Leu Leu Pro
20 25 30

Gln Leu His His Thr Arg Asn Ala Pro Val Ser Ala Ser Ser Ile Leu
        35                  40                  45

Glu Glu Thr Cys Ser Tyr Ile Lys Arg Leu His Arg Glu Val Glu Asp
    50                  55                  60

Leu Ser Gln Arg Ile Ser Gln Leu Leu Asp Ser Ala Gly Ile Ser Asp
65                  70                  75                  80

Val Asp Glu Glu Leu Ile Arg Arg Leu Leu Gln His
                85                  90

<210> 94
<211> 547
<212> DNA
<213> Malus x domestica

<400> 94
atttgatagt gtaaagaagc taaaagctat aagcagaaaa taagcaaaag tgcttcttca 60

accagccatg tcaagcagaa gatcatcatc atcatcaaga acttctaaac cctcagatga 120

tgagattaag gagctcatct caaaattaca acctcttctt cctcagcttc atcatacgcg 180

taatgctccg gtatcggcgt cgagcatttt ggaagaaact tgcagttaca taaagaggct 240

gcatagggag gtggaagatc tgagccaaag aatatctcaa ctcctggatt ctgcaggcat 300

ctctgatgtt gatgaagagc ttattagaag acttttgcag cattaataat cgctctctct 360

ctctctaggc tagcaatttt aattacatga gttaagtttt ggttggacta tccaaccagt 420

gagagattat atatatgagg aatgcatata tatatatata tatctgtgta tatatgtttg 480

taatcttcag tgtaccttct gatatcttat gtgttgttaa aatggtatct agtcattgat 540

ctagctc 547

<210> 95
<211> 92
<212> PRT
<213> Medicago truncatula

<400> 95

Met Ser Ser Arg Arg Ser Arg Ser Arg Gln Thr Ser Ser Ser Arg Asn
1               5                   10                  15

Ile Thr Asp Asp Gln Ile His Asp Leu Val Ser Lys Leu Gln Gln Leu
            20                  25                  30

Leu Pro Glu Ile Arg Asn Arg Ser Ser Asp Lys Val Ser Ala Ser Arg
        35                  40                  45

Val Leu Gln Glu Thr Cys Asn Tyr Ile Arg Asn Leu Asn Arg Glu Val
50 55 60

Asp Asp Leu Ser Glu Arg Leu Ser Glu Leu Leu Ala Thr Thr Asp Thr
65 70 75 80

Ala Gln Ala Ala Ile Ile Arg Asn Leu Leu Met Gln
85 90

<210> 96
<211> 647
<212> DNA
<213> Medicago truncatula

<400> 96
ctccaacatc actgtccctt ccccattcct tgttggaagc tagtcactcc ttagtgcacc 60

tatctccata tcctatttca atttgtcttt gaaatttcca tacattacat tttctatacc 120

aaatcactaa gattaaggtc acatatatat ccaaaactaa agtagtatag tatatttgtt 180

ttctaggatt cacctaggct tgaaattatt gaattttata aagatgtcta gtaggaggtc 240

gcggtcacgg caaacaagta gctcgaggaa tatcaccgac gatcagatcc atgatcttgt 300

ctccaagttg cagcaacttc ttcctgagat tcgcaatagg agctctgaca aggtttcagc 360

ttcgagggta ttgcaggaga cttgcaacta tattagaaac ttgaacaggg aagtcgacga 420

cctaagcgag cgtttgtctg agctattggc tacaacagac acagcacaag cagcaataat 480

tagaaattta cttatgcaat agatttttct tgtttcatta tttttaatta gcactcaagg 540

actttgtgac ctgatgtatt tcttcttcat tttcgtactt cagttttaaa tttgtatttc 600

ctaatttcat ccacttaatg caagttttct agtttatgtt ttttttt 647

<210> 97
<211> 93
<212> PRT
<213> Medicago truncatula

<400> 97

Met Ser Ser Arg Arg Ser Arg Ser Arg Gln Ser Gly Gly Ser Ser Glu
1 5 10 15

Ile Thr Asp Ala Gln Ile Thr Asp Leu Ile Ser Lys Leu Gln Gln Leu
20 25 30

Ile Pro Glu Leu His Ala Ser Arg Ser Asn Lys Val Ser Ala Thr Lys
35 40 45

Val Leu Gln Glu Thr Cys Asn Tyr Ile Lys Asn Leu His Arg Glu Val
50 55 60

Asp Asp Leu Ser Asp Arg Leu Ser Gln Leu Leu Ala Ser Thr Asp Ser
65                  70                  75                  80

Asn Ser Ala Gln Ala Ala Ile Ile Lys Ser Leu Leu Met
                85                  90

<210>  98
<211>  723
<212>  DNA
<213>  Medicago truncatula

<400>  98
cttcaaccca ctcctcttca ctctcttcgc caaatttaat tttattatca taccttagca      60

acattacaaa aaacatataa ccacttttat ttatttctct cttaataaat actattccct     120

tactctccat actcatttca cataacttct cttcactttc ttcccttctt tctctgacaa     180

ccaaccaacc tcatttcatc tctttctctt tatttatttt cttgtatcac aaattaatat     240

ttctgacata tagttacttt acattaccac tacctcctta attataacaa tgtctagcag     300

aagatctcgt tccagacaat ccggtggttc ctctgagatc actgatgctc aaatcactga     360

tctcatttcc aagttacaac aacttatccc cgaacttcac gctagccgct ccaacaaggt     420

ttcagctact aaggtattgc aagagacttg caactacata aaaaacttgc atagagaagt     480

tgatgattta agtgacagat tgtcacaact tttggcttct acagattcca acagtgctca     540

agcagctatt attaagagct tacttatgta atagtgtcta gttatatata tatttataac     600

tactactact cttgcatttg tttgtcgtgt gcatgtcctt ggctcacttt attggtattc     660

tcttatctgg ggaagggtta atttgggttg gaaaccaagg caaaagggtt actatatacc     720

ctt                                                                   723

<210>  99
<211>  92
<212>  PRT
<213>  Medicago truncatula

<400>  99

Met Ser Ser Arg Arg Ser Arg Gln Gln Ser Ser Ser Ser Arg Ile Ser
1               5                   10                  15

Asp Asp Gln Ile Ile Glu Leu Val Ser Lys Leu Arg Gln Leu Val Pro
            20                  25                  30

Glu Ile Arg His Arg Arg Ser Asp Lys Val Ser Ala Ser Lys Val Leu
        35                  40                  45

Gln Glu Thr Cys Asn Tyr Ile Arg Asn Leu His Arg Glu Val Asp Asp

```
    50                  55                  60

Leu Ser Glu Arg Leu Ser Gln Leu Leu Val Thr Ile Asp Ala Asp Ser
65                  70                  75                  80

Pro Glu Ala Asn Ile Ile Arg Ser Leu Ile Asn Gln
                85                  90
```

<210> 100
<211> 795
<212> DNA
<213> Medicago truncatula

<400> 100

```
tttaggctca tctctctcca ttttaattag caacatccta cgccactgct ttcccaatgc      60

aattgtttgc ttcaaatcta acatcattgt ctatctaatt gtaccctctt ccttcattta     120

tggtcccttt ctctcccttt atatctctct catcactttc tccatttctt cattcatacc     180

tatagctttt aactctacca ctcctaccat tcctcctctc cttttctcca ctacatagct     240

tgtcttttgt ttgttaattt ccaacaaaat aaaccaatta attcttgtta ctttcttact     300

ttatcctccc tcatttgtat cttcttggtt atattataaa ctatatatat aaatagaaaa     360

atacaccaat tctaggtata tattatgtct agcagaaggt caaggcaaca atcttcttct     420

tcaagaattt ctgatgatca aatcatcgaa cttgtttcca agttacgtca acttgttcct     480

gagattcgtc ataggcgttc agacaaggta tcagcatcta aggtcctaca agagacttgt     540

aactacataa gaaacttaca cagagaagtt gatgatttaa gtgaaagact gtctcagtta     600

ttggtcacaa ttgatgctga tagtccagag gctaatatta tcagaagcct aattaatcaa     660

taagtttaga aattaatcta attttcttaa tttccattat gatgagaaat atatatttat     720

atatctataa gagtgtattt tattttaaat gggctagcta gagtgtagtt tgaggtgtgc     780

aattttcata tggcg                                                      795
```

<210> 101
<211> 92
<212> PRT
<213> Nicotiana benthamiana

<400> 101

```
Met Ser Ser Arg Arg Ser Arg Gln Ser Ser Ala Gly Ser Ser Arg Ile
1               5                   10                  15

Ser Asp Asp Gln Ile Ile Asp Leu Val Ser Lys Leu Gln Gln Leu Leu
            20                  25                  30

Pro Glu Ile Arg Thr Arg Arg Ser Asn Lys Ala Ser Ala Ser Lys Val
        35                  40                  45
```

Leu Gln Glu Thr Cys Asn Tyr Ile Arg Asn Leu Asn Arg Glu Val Asp
    50                  55                  60

Asp Leu Ser Asp Arg Leu Ser Gln Leu Leu Ser Thr Ile Asp Ala Asp
65                  70                  75                  80

Ser Pro Glu Ala Ala Ile Ile Arg Ser Leu Leu Met
                85                  90

<210> 102
<211> 587
<212> DNA
<213> Nicotiana benthamiana

<400> 102
ctcgttctaa acaaataaaa ggagacaaag attagttgta gacaagaaca aaaaagacaa 60

ttaactttaa tttaaggatg tcaagcagaa ggtcaagaca gtcatcggca ggttcctcaa 120

gaatttcaga tgatcagata attgacctcg tatccaagct gcaacaactt cttccggaaa 180

ttcgaacccg tcgttccaac aaggcatcgg catcaaaggt gctacaagaa acttgcaact 240

atataagaaa tttgaataga gaagtggatg atcttagtga tcgtctttct caattactct 300

caaccattga tgctgatagt ccagaagctg caatcattcg gagtttatta atgtagctat 360

taattaatgt attatgagtt ttaaatattg gagttatatt ttactgcgta tcaattaagt 420

tcttgttttt tcttcttaat tgctatagac tcagctggtc actagctagg ccaatcatga 480

gttagaattt agaactgaaa tattagtaat atcccccttc ctatggcaca cgcttgtaat 540

tatatatttc ccttagtata attaataaga tggagtactt gtgtttt 587

<210> 103
<211> 93
<212> PRT
<213> Nicotiana tabacum

<400> 103

Met Ser Gly Arg Arg Ser Arg Gln Ser Ser Glu Glu Gly Thr Ser Arg
1               5                   10                  15

Ile Ser Asp Asp Gln Ile Ile Glu Leu Met Ser Lys Leu Gln Gln Leu
            20                  25                  30

Leu Pro Glu Ile Pro Thr Arg Arg Thr Asn Lys Ala Ser Ala Ser Lys
        35                  40                  45

Val Leu Gln Glu Thr Cys Asn Tyr Ile Arg Ser Leu His Lys Glu Val
    50                  55                  60

Asp Asp Leu Ser Asp Arg Leu Ser Gln Leu Leu Ser Thr Ile Asp Ala
65 70 75 80

Asp Ser Pro Glu Ala Ala Ile Ile Arg Ser Leu Leu Met
85 90

<210> 104
<211> 650
<212> DNA
<213> Nicotiana tabacum

<400> 104
ggttgcaggt ctattttcta actaccagta cgccctcctc tttactgctt tactactact 60

actactacca cttcccattg tgtaagctgc cattcaaaca agttttgagc cattaatttg 120

taccccaaaa agaaaaggag aaaaattact agtagttgaa gtatgtcagg gagaaggtcg 180

aggcaatcat cagaggaggg gacgtcgagg atttcagatg atcagatcat tgaactgatg 240

tccaagttgc aacaacttct tcctgaaatt cccactcgtc gcaccaacaa ggcatcagca 300

tctaaagtgc ttcaggaaac atgcaactac ataagaagct tgcataaaga ggtggatgat 360

ctcagtgatc gactttctca gttgttgtcc accattgatg ctgacagtcc tgaagctgca 420

atcattcgta gtttattaat gtaatcttca atttgttcat catatattga atagacgtat 480

actactacct agttcttcgt ttcttcttcc tctctaggct ttgctggtca ctactattag 540

ctaggccaat ctcattagtt agcatttata acttcccctt gcttattata tgtacacgcg 600

cttgtaagga tgtttcccta gattaaataa taataagatg tacttgtgct 650

<210> 105
<211> 104
<212> PRT
<213> Oryza sativa

<400> 105

Met Ser Ser Ser Arg Arg Ser Arg Ser Arg Arg Ala Gly Ser Ser Val
1 5 10 15

Pro Ser Ser Ser Ser Ser Ser Arg Thr Ser Ile Ser Glu Asp Gln Ile
20 25 30

Ala Glu Leu Leu Ser Lys Leu Gln Ala Leu Leu Pro Glu Ser Gln Ala
35 40 45

Arg Asn Gly Ala His Arg Gly Ser Ala Ala Arg Val Leu Gln Glu Thr
50 55 60

Cys Ser Tyr Ile Arg Ser Leu His Gln Glu Val Asp Asn Leu Ser Glu
65 70 75 80

Thr Leu Ala Gln Leu Leu Ala Ser Pro Asp Val Thr Ser Asp Gln Ala
85 90 95

Ala Val Ile Arg Ser Leu Leu Met
100

<210> 106
<211> 315
<212> DNA
<213> Oryza sativa

<400> 106
atgtcgagca gccggaggtc gcgctcacgg cgagccggga gctcggtgcc gtcgtcgtcg 60

tcgtcgtcga ggacgtcgat ctcggaggac cagatcgccg agcttctctc caagcttcag 120

gccctgctcc cggagtctca ggctcgcaat ggcgcccata ggggctcggc ggcgagggtt 180

ttgcaggaga cgtgcagcta catcaggagc ctgcaccagg aggtggacaa cctcagcgag 240

acgctcgctc agctgctcgc ctccccgac gtcaccagcg accaggcggc cgtcatcagg 300

agcctcctca tgtga 315

<210> 107
<211> 91
<212> PRT
<213> Oryza sativa

<400> 107

Met Ser Ser Arg Arg Gly Gly Gly Gly Gly Gly Gly Arg Ile Thr Asp
1 5 10 15

Glu Glu Ile Asn Glu Leu Ile Ser Lys Leu Gln Ala Leu Leu Pro Glu
20 25 30

Ser Ser Arg Ser Arg Gly Ala Ser Arg Ser Ser Ala Ser Lys Leu Leu
35 40 45

Lys Glu Thr Cys Ser Tyr Ile Lys Ser Leu His Arg Glu Val Asp Asp
50 55 60

Leu Ser Asp Arg Leu Ser Glu Leu Met Ser Thr Met Asp Asn Asn Ser
65 70 75 80

Pro Gln Ala Glu Ile Ile Arg Ser Leu Leu Arg
85 90

<210> 108
<211> 878
<212> DNA

<213> Oryza sativa

<400> 108
atacaaccac tccctggctc cctgcttcta ctgaaaccta taggctacta gctagtgctc 60

tcactctcac tcactcacac tgtcacttcc acattttctt tgctctctca ctgtccgtac 120

gtcgtcggct tgatcaccaa ccttgctgac ggtggtcggt cgccggagtt cgaggagaag 180

aaggcagtag aggtgtggtg gtgatattgc aggcggcgac catgtcgagc cgccgtggtg 240

gtggtggtgg aggagggagg atcaccgacg aggagatcaa cgagctcatc tccaagcttc 300

aggccctcct cccggagtcc tcccgcagcc gcggcgcaag ccggtcgtcg gcgtcgaagc 360

tgctcaagga gacgtgcagc tacatcaaga gcctgcaccg ggaggtggac gacctgtccg 420

accggctgtc ggagctcatg tcgacgatgg acaacaacag cccgcaggcc gagatcatcc 480

ggagcctcct ccggtgatcg atcctagctc tatcagtagc tgcagcgacg acgacgattg 540

atggaggacg agcttgctag ctagcgattg aggagggaga cgacaagatt tttttgctct 600

tctttgtttc tttcttcgat ctctcctgaa aagggaaaag tgtttgtttc tcaggtaatc 660

accgaaggcc cctgcattgt ttaggagaag aaaaagggtt gtcttgtttg gtatgtactg 720

taccagggct aatagagatc gatatatgtg gatttctatt agctgctagt agagttatta 780

gtagctagat tagctactta atttagcttg tagacgtact actactgtac ttaagctgct 840

ttgataagct tcagagatgc atctagaggt gtttgttt 878

<210> 109
<211> 88
<212> PRT
<213> Oryza sativa

<400> 109

Met Ser Ser Arg Arg Ser Ser Arg Gly Ser Ile Ser Glu Glu Glu Ile
1 5 10 15

Asn Glu Leu Ile Ser Lys Leu Gln Ser Leu Leu Pro Asn Ser Arg Arg
20 25 30

Arg Gly Ser Ser Gln Ala Ser Thr Thr Lys Leu Leu Lys Glu Thr Cys
35 40 45

Asn Tyr Ile Lys Ser Leu His Arg Glu Val Asp Asp Leu Ser Asp Arg
50 55 60

Leu Ser Asp Leu Met Ala Thr Met Asp His Asn Ser Pro Gly Ala Glu
65 70 75 80

Ile Ile Arg Ser Ile Leu Arg Ser
85

<210> 110
<211> 1071
<212> DNA
<213> Oryza sativa

<400> 110
cacacacact tctcccatcc agctagcttt tgcagatcga gcgaaccttc aacacaaacc 60

ggccagcttg ttcctctcct ctctctctct ctctctctct ctctcggtcg cagccagcta 120

acttcgagtg tgaagaacac tcacgctagc tagctttctc aaggccacat acgctagctc 180

cgcctccacg cgcggcgtac gtctagtttg attgaggctg aagagagtgc gtgtttggta 240

gaagaggcta gctgttgacg atgtcgagca gaaggtcgtc gcgtggctcc atctcggagg 300

aggaaatcaa cgagctcatc tccaagctcc agtcgctgct ccccaactca cgccgacgcg 360

gctccagcca ggcgtcgacg acgaagctgc tgaaggagac gtgcaactac atcaagagcc 420

ttcaccggga ggtggatgac ctcagcgacc ggctgtccga cctcatggcc accatggatc 480

acaacagccc cggcgcggag atcatccgca gcatcctccg ctcctgatcg gcaagcagca 540

gcagcaagcg gcaccggccg gccggccggc ctatgtcgac gacgagatag gccgggccgg 600

ccgggcaacg cccgcggcgc caattaatca agcgtacgtc ctgccggcgc cattctccgg 660

ccaccagaga gcatttagct agggtatata tatctacata tataaaatat ttatgtcgta 720

tgtccctccc caaatgtacg agccacgctt acacgcgcgt gtatcgatct ctcgatctct 780

ctctctaagc tccactcgaa gtagggcatt agcactaggg gcctaagcag aggcttatcc 840

tcgctttagc tcatattttc atcgcttgat gtgtcctctc tgaaccccca catcttgtct 900

tgtttggttt ttccctctcc cttccaatct atgtaaattt agctggtgtg gtttggatcg 960

agttgtgtcc tctacagaca accgaccgac cactactacc tagaggaaat taatatcatc 1020

atgggtgtac tgctccagct ttaacttcaa ttcagttggc tacgtactac g 1071

<210> 111
<211> 87
<212> PRT
<213> Oryza sativa

<400> 111

Met Ser Ser Arg Arg Ser Ser Arg Ser Ser Val Ser Glu Glu Glu Ile
1 5 10 15

Asn Glu Leu Ile Ser Lys Leu Gln Ser Leu Leu Pro Ser Ser Arg Arg
20 25 30

Arg Gly Ala Asn Gln Ala Ser Thr Thr Lys Leu Leu Lys Glu Thr Cys
35 40 45

```
Ser Tyr Ile Lys Ser Leu His Arg Glu Val Asp Asp Leu Ser Asp Arg
    50                  55                  60

Leu Ser Asp Leu Met Ala Gly Met Asp His Asn Ser Pro Gly Ala Glu
65                  70                  75                  80

Ile Ile Arg Ser Leu Leu Arg
                85

<210>  112
<211>  877
<212>  DNA
<213>  Oryza sativa

<400>  112
ctgcttcaat tcctacctca cacttctgca acaagcttta gctccagcca ccggaacgag      60

agatcgatac agctcgatca gctagccgca ttcgccctcg ccgccgccga cgatgtcgag     120

ccggaggtcg tcgcgctcct ccgtgtcgga ggaggagatc aacgagctca tctccaagct     180

ccagtccctc ctccccagct cccgccgccg cggcgccaac caggcgtcga cgacgaagct     240

gctgaaggag acgtgcagct acatcaagag cctgcaccgg gaggtggacg acctcagcga     300

caggctctcc gacctcatgg ccggcatgga tcacaacagc ccaggcgccg agatcatccg     360

cagcctcctc cgctagctca tctcttctca tctgttcttc ctcctcgatg atcgatcgat     420

ctcgtgttat tctacgtaca tgaaggatga tatgaatgca gctcgtgccc tgtagctacg     480

tataaatata cacatatgta tacatgcata cagtacatga tatgcatatg cctagatctg     540

atctagctca gtagaaaatc tactcatctc ggtgtactac tgatgatgat gatgattaag     600

gcatgcagct ggcttgatga tcggtactac tactactact tcacttcagt tcagtagggg     660

cattagctag ctagctaggg ccggctgcct aattaagtaa ttaagtaatt tattatttgt     720

agtagccagc ttgatctcat ctctcctcat gagccatatg acatgtggtt aagttaatta     780

atccttgatg cagtagagtc gtcccagctg tttcatcgat gatcgtcgtc atatgtgtgt     840

atctcttgat ctagctttgc tgatctcctc ttgtttc                              877

<210>  113
<211>  77
<212>  PRT
<213>  Panicum virgatum

<400>  113

Met Ser Gly Arg Arg Gly Arg Ile Ser Asp Asp Glu Ile Asn Glu Leu
1               5                   10                  15

Ile Ser Lys Leu Gln Ala Leu Leu Pro Glu Ser Ser Arg Arg Arg Asn
            20                  25                  30
```

```
Ala Ser Arg Ser Ser Ala Ser Lys Leu Leu Lys Glu Thr Cys Thr Tyr
        35                  40                  45

Ile Lys Ser Leu His Arg Glu Val Asp Asp Leu Ser Glu Arg Leu Ser
    50                  55                  60

Gly Leu Met Ala Thr Met Asp Asn Asp Ser Pro Gln Ala
65                  70                  75
```

<210> 114
<211> 617
<212> DNA
<213> Panicum virgatum

<400> 114

```
ccacgcgtcc gctctggctc tggctcctcc cggttgcagt tgcaagctga cctcgctgct      60

gctgccacca ctcactgctc tgccagttct ttccctcgac ctcacacagt agcaagctat     120

taagagcact ccccggagtt caccagctag gtttaaggca ttgaaaccct atccttttca     180

ggtccctctc gccttttctt ttcctccctt ggctcctatt tccgtttgca gaccggagct     240

cgcacacacg tactactaca tatataccca agttctgcga gaggccgagg ccgaggcagg     300

gacgacgacg aagaagaata atccaccagg agctgtatag tagcatcatc caagcacgta     360

ctctctgagc taggactcgc cggagatgtc aggccgccgc ggcaggatca gcgacgacga     420

gatcaacgag ctaatctcca agctccaggc gctcctcccg gagtcctcac gccgccggaa     480

cgcgagccgg tcgtcggcgt cgaagctcct gaaggagacg tgcacctaca ttaagagcct     540

gcaccgggag gtggacgacc tctcggagcg gctgtcgggg ctcatggcga ccatggacaa     600

cgacagcccc caggccg                                                    617
```

<210> 115
<211> 92
<212> PRT
<213> Petunia x hybrida

<400> 115

```
Met Ser Ser Arg Arg Ser Arg Gln Ser Ser Val Gly Ser Ser Arg Ile
1               5                   10                  15

Ser Asp Asp Gln Ile Ile Glu Leu Val Ser Lys Leu Gln Gln Leu Leu
            20                  25                  30

Pro Glu Ile Arg Thr Arg Arg Ser Asn Lys Ala Ser Ala Ser Lys Val
        35                  40                  45

Leu Gln Glu Thr Cys Asn Tyr Ile Arg Asn Leu Asn Arg Glu Val Asp
```

```
    50                  55                  60

Asp Leu Ser Asp Arg Leu Ser Gln Leu Leu Ser Thr Ile Asp Ala Glu
65                  70                  75                  80

Ser Pro Glu Ala Ala Ile Ile Arg Ser Leu Leu Met
                85                  90
```

<210> 116
<211> 540
<212> DNA
<213> Petunia x hybrida

<400> 116

```
cagaaaactt ctcttttact agcttcttcc tcattccaat ttcttctact accagttaat     60

taattattgt tcttaaattc ctacgataaa ttcgaccatt tgttataaac acttaagagg    120

agacagacat tagtcgagta cgtaaattgt agaaattaac aataagacat ctttgtttaa    180

tttaaggatg tctagcagaa ggtcaaggca atcatcagta ggttcttcga ggatttcaga    240

tgatcaaatc attgaactcg tatccaaatt gcaacaactt cttcctgaga ttcgcactcg    300

tcgctccaac aaggcatcgg catcaaaggt gcttcaagaa acttgcaact acattaggaa    360

cttgaataga gaagtggatg atcttagtga tcgtctttct cagttactct ccaccattga    420

tgctgagagc ccagaggctg caatcatccg aagtttatta atgtgacaaa tttattttga    480

tttctaaata ttggagctat atattttaag taccaagttc ttgtttttc ttcttgctct    540
```

<210> 117
<211> 92
<212> PRT
<213> Petunia x hybrida

<400> 117

```
Met Ser Gly Arg Arg Ser Arg Gln Ala Ser Glu Gly Ser Ser Arg Ile
1               5                   10                  15

Ser Asp Asp Gln Ile Ile Glu Leu Met Ser Lys Leu Gln Gln Leu Leu
            20                  25                  30

Pro Glu Ile Arg Ser Arg Arg Thr Asn Lys Glu Pro Ala Ser Lys Val
        35                  40                  45

Leu Gln Glu Thr Cys Asn Tyr Ile Arg Asn Leu His Lys Gln Val Asp
    50                  55                  60

Asp Leu Ser Asp Arg Leu Ser Gln Leu Leu Ser Thr Ile Asp Ala Asp
65                  70                  75                  80
```

```
Ser Pro Glu Ala Ala Ile Ile Arg Ser Leu Ile Met
                85                  90


<210>  118
<211>  539
<212>  DNA
<213>  Petunia x hybrida

<400>  118
tagactagtg atccccgggc tgaggaatcg gcacggaggt atactaccgg taggcagtag      60

ttgctcttac tactactact actactacta cttcttgttc cagttttaag tcgtgtacca     120

tttaaattag ttgaagaatg tcaggaagaa ggtcgaggca ggcatcagag gggtcttcaa     180

gaatttcaga tgatcagatc atagaattaa tgtccaaatt gcagcaactt cttcctgaaa     240

ttcgcagtcg ccgcaccaac aaggaaccag catctaaggt tctccaagag acatgcaact     300

acattagaaa tttgcataaa caggtggatg atctcagtga ccgactttct cagttattgt     360

ccaccattga tgctgacagt ccagaagctg caatcattcg tagtttaata atgtagtagt     420

ataacttttt tctttattaa atgttggagg cattattcta ctccctgttt cttgtttaat     480

ttcttcttaa tacctctagg ctatgctgga ctggcgaggc aaatctgatc atgtgttag      539


<210>  119
<211>  85
<212>  PRT
<213>  Petunia x hybrida

<400>  119

Met Ser Ser Arg Arg Ser Ser Arg Ile Ser Asp Asp Gln Ile Ile Glu
1               5                   10                  15


Leu Val Ser Lys Leu Gln Gln Phe Leu Pro Glu Ile Arg Thr Arg Arg
            20                  25                  30


Ser Ser Lys Ala Ser Ala Ser Lys Val Leu Gln Glu Thr Cys Asn Tyr
        35                  40                  45


Ile Arg Asp Leu Asn Arg Glu Val Asp Asp Leu Ser Asp Arg Leu Ser
    50                  55                  60


Gln Leu Leu Ser Thr Ile Asp Ala Asp Ser Gln Glu Ala Ala Ile Ile
65                  70                  75                  80


Arg Ser Leu Ile Met
                85


<210>  120
<211>  553
<212>  DNA
```

<213> Petunia x hybrida

<400> 120
gggatcccgg gctgaggaat tcggcacgag ggttaccata tattttgaat tatgtcaagt 60

agaaggtctt caaggatttc agatgatcaa attattgaac ttgtgtccaa gttgcagcaa 120

tttcttcctg aaattcggac tcgtcgttcc agcaaggcat cagcatcaaa ggtgctccaa 180

gaaacttgca actacattag agacttgaac agagaagtgg atgaccttag tgatcgactt 240

tctcaattac tatccactat tgatgctgac agtcaagaag ctgcaataat tcgtagttta 300

ataatgtaat tatttttatt tatatactaa ttgcaatgta ctctaccacc taattgttgc 360

ttctttaact tcctctagtc tctaagtact ggtcattagc tataggccaa tcatgagtta 420

gaatttaact cgaaatatat gcaagtattg gatctgatgt ccccctagct aaggacacat 480

gcttctgatt atgttcccac ccataaatta aataagatgg tattgcattt taattaaaac 540

aaccccaaac aaa 553

<210> 121
<211> 92
<212> PRT
<213> Phaseolus vulgaris

<400> 121

Met Ser Ser Arg Arg Ser Arg Gln His Ser Gly Ser Thr Arg Ile Ser
1 5 10 15

Asp Asp Gln Ile Ile Glu Leu Val Ser Lys Leu Arg Gln Leu Val Pro
20 25 30

Glu Ile Arg Ser Arg Arg Ser Asp Lys Val Ser Ala Ser Lys Val Leu
35 40 45

Gln Glu Thr Cys Asn Tyr Ile Arg Ser Leu His Arg Glu Val Ser Asp
50 55 60

Leu Ser Glu Arg Leu Ser Gln Leu Leu Thr Thr Ile Asp Ala Asp Ser
65 70 75 80

Ala Glu Ala Gly Ile Ile Arg Ser Leu Leu Asn Gln
85 90

<210> 122
<211> 692
<212> DNA
<213> Phaseolus vulgaris

<400> 122
gtcctttctc ttccttcatt ataactctct catcactctc tcctctctct caaaacctcc 60

ctcgtatcca acactcttct cttcttcctc ctcctactcc tatatatccc cttccccttc 120

ttccactaac tccatgtgtt ttatttctca cttcccaacc caaaaccatt cattctttgc 180

ttccttctgc ttctatatca ctacctgcta aaacttatca tacatataaa tacgcacaca 240

catacctctg cttatagcac caaaactagg ctagcttagc ttgcactttc aacaattata 300

tacatacaca agtacaccaa gctctaccta gcaacctacc aacatgtcta gccgaagatc 360

cagacaacat tcagggtcta caaggatctc cgatgaccaa atcatcgagc ttgtttccaa 420

attgcgccaa cttgttcctg agattcgcag taggcgatct gacaaggttt cagcgtccaa 480

ggtcctacaa gaaacctgca actacatcag aagcttgcat agagaggtga gtgacttgag 540

tgagcgactg tctcagttgt tgaccacaat tgatgctgat agtgctgaag ctggaatcat 600

taggagccta cttaatcaat aagcaatgag tgttatgatt ttttcattca aagagtgcta 660

attattatga gagtgtactt cattctaggt gt 692

<210> 123
<211> 91
<212> PRT
<213> Populus trichocarpa

<400> 123

Met Ser Ser Arg Arg Ser Arg Ser Arg Gln Ser Ser Ser Ser Arg Ile
1 5 10 15

Ser Asp Asp Gln Ile Leu Asp Leu Val Thr Lys Leu Gln Gln Leu Leu
20 25 30

Pro Glu Ile Arg Asn Arg Arg Ser Asp Lys Val Ser Ala Ala Lys Ile
35 40 45

Leu Gln Glu Thr Cys Asn Tyr Ile Lys Ser Leu His Arg Glu Val Gly
50 55 60

Asp Leu Ser Glu Arg Leu Ser Glu Leu Leu Glu Thr Thr Asp Thr Ala
65 70 75 80

Gln Ala Ala Ile Ile Arg Asn Leu Leu Met Gln
85 90

<210> 124
<211> 386
<212> DNA
<213> Populus trichocarpa

<400> 124
cataattaag tgaatatcaa tttcaagaac atgtctagcc gaaggtcacg atcaaggcaa 60

tcaagtagtt caagaatcag tgatgatcag atccttgatc ttgttacaaa gttgcaacaa 120

cttcttcctg agattcgtaa caggcgttct gacaaggttt cggctgccaa gatcttgcag 180

gagacatgca actatattaa aagcttgcat agagaggttg gtgatcttag cgagcggctg 240

tctgagctat tggaaacaac tgatacagcc caagctgcaa taatcaggaa cttacttatg 300

caatagagct aattaaggat taatactact tgcttggctt atgcagtcgg atcatgtttc 360

ctctctcttc cttaattttg ttcttc 386

<210> 125
<211> 91
<212> PRT
<213> Populus trichocarpa

<400> 125

Met Ser Ser Arg Lys Ser Arg Ser Arg Gln Ser Gly Ser Ser Arg Ile
1 5 10 15

Asn Asp Asp Gln Ile Leu Asp Leu Val Thr Lys Leu Gln Gln Leu Leu
20 25 30

Pro Glu Thr Arg Asn Arg Arg Ser Glu Lys Val Ser Ala Ala Lys Ala
35 40 45

Leu Gln Glu Thr Cys Asn Tyr Ile Lys Ser Leu His Arg Glu Val Asp
50 55 60

Asp Leu Ser Glu Arg Leu Ser Glu Leu Leu Glu Thr Thr Asp Thr Thr
65 70 75 80

Gln Ala Ala Ile Ile Arg Asn Leu Leu Met Gln
85 90

<210> 126
<211> 386
<212> DNA
<213> Populus trichocarpa

<400> 126
attacaagtt aatataaatt tcaataagat atgtctagcc ggaagtcgcg atcaaggcaa 60

tcaggtagtt caagaatcaa tgatgatcag atccttgatc ttgttacaaa gttgcaacaa 120

cttcttcccg agactcgaaa tcggcgttct gaaaaggtct cagctgccaa ggccttgcag 180

gagacatgca actatattaa aagcttgcac agagaggttg atgatcttag cgagcggctt 240

tctgagctat tagagacaac tgacactact caagctgcga taattaggaa tttgcttatg 300

caatagggct agttaaggat tagtacttgt tagcctatgc agtaggatcg tgtgcttgtt 360

tccctctctt cctttgtttt tctcta 386

<210> 127
<211> 91
<212> PRT
<213> Prunus persica

<400> 127

```
Met Ser Ser Arg Arg Ser Arg Gln Ser Gly Thr Pro Thr Ile Lys Asp
1               5                   10                  15

Asp Gln Ile Ile Glu Leu Val Ser Lys Leu Arg Gln Leu Val Pro Glu
            20                  25                  30

Ile Arg Asp Arg Arg Ser Asp Lys Val Ser Ala Ser Lys Val Leu Gln
        35                  40                  45

Glu Thr Cys Ser Tyr Ile Arg Asn Leu His Arg Glu Val Asp Asp Leu
    50                  55                  60

Ser Glu Arg Leu Ser Gln Leu Leu Ser Thr Ile Asp Ala Asp Ser Pro
65                  70                  75                  80

Glu Ala Ala Ile Ile Arg Ser Leu Ile Thr Gln
                85                  90
```

<210> 128
<211> 742
<212> DNA
<213> Prunus persica

<400> 128

```
aaaaaacaaa aaaaaaaaaa aaaaaaaaaa aactcagacc tagttctctc cctcgtgccc      60

aaattggctc ttgcgtacgt ggccacttac ctcttttagg ttaatatttc tccttcccag     120

ctagctatat atgtatatat attaatatta atatataaag ccccgtagcc agtctgatca     180

tcgatctctt tatatatata tatataacca gctgtagcta acagtgtaga tatatagcta     240

gagatcatgt ctagcagaag gtcgaggcag tctggaactc caacgatcaa agatgaccaa     300

atcattgaac ttgtctccaa attgcgccaa ctggttcctg agattcgtga taggcgctcc     360

gacaaggtat cagcatctaa ggtcctacaa gagacttgca gctacatcag aaacttacac     420

agagaggttg acgacctaag tgagcggctc tctcaactac tctcgacaat tgatgctgat     480

agcccggagg ccgccataat taggagcttg attacgcagt agatgatcag ctagatgatg     540

atcatcagac ccttaattat atatttatat ataattgtgc tttgatgatg aatttatata     600

gttaaattgt gttcatctag gttatctggt cacccgatta ttaaacaagg ccagttagct     660

agggtactca gcagtattgt atgtatttaa agatgtcctt tcctgtcctg tcctgtgttg     720

ttaattagag cgaggcccta gt                                              742
```

<210> 129
<211> 91
<212> PRT
<213> Prunus persica

<400> 129

```
Met Ser Ser Arg Arg Ser Arg Gln Ser Gly Thr Pro Thr Ile Lys Asp
1               5                   10                  15

Asp Gln Ile Ile Glu Leu Val Ser Lys Leu Arg Gln Leu Val Pro Glu
            20                  25                  30

Ile Arg Asp Lys Arg Ser Asp Lys Val Ser Ala Ser Asn Val Leu Gln
        35                  40                  45

Glu Thr Cys Ser Tyr Ile Arg Asn Leu His Arg Glu Val Gly Asp Leu
    50                  55                  60

Ser Glu Arg Leu Ser Gln Leu Leu Ser Asp Ile Asp Ala Asp Ser Pro
65                  70                  75                  80

Glu Ala Ala Ile Ile Lys Ser Leu Ile Thr His
                85                  90
```

<210> 130
<211> 580
<212> DNA
<213> Prunus persica

<400> 130

```
tcttctcctc ctcctccttt tgatctactt cttctccttc tttttcctcc tctcgagtac      60

tttttcctca caaattggct cttgcgtacg tggccactta cctcttttag gttaatattt     120

ctccttccca gctagctata tatgtatata tattaatatt aatatataaa gccccgtagc     180

cagtctgatc atcgatctct ttatatatat atatataacc agctgtagct aacagtgtag     240

atatatagct agagatcatg tctagcagaa ggtcgaggca gtctggaact ccaacgatca     300

aagatgacca aatcattgaa cttgtctcca aattgcgcca actggttcct gagattcgtg     360

ataagcgctc cgacaaggta tcagcatcta atgtcctaca agagacttgc agctacatca     420

gaaacttaca cagagaggtt ggcgacctaa gtgagcggct ctctcaacta ctctcggaca     480

ttgatgctga tagcccggag gccgccataa ttaagagctt gatcacgcac tagatgatca     540

gctaaatgat gatcatcaga cccctgatta tatatctata                           580
```

<210> 131
<211> 93
<212> PRT

<213> Prunus persica

<400> 131

Met Ser Ser Lys Val Ser Arg Gln Ser Gly Thr Pro Thr Ile Lys Asp
1 5 10 15

Asp Gln Ile Ile Glu Leu Val Tyr Lys Leu Arg Gln Leu Val Pro Asp
20 25 30

Ile Arg Asp Lys Arg Ser Asp Lys Val Ser Ala Tyr Asn Val Leu Gln
35 40 45

Glu Thr Cys Ser Ser Ile Arg Asn Leu His Lys Glu Val Asp Asp Leu
50 55 60

Ser Glu Arg Phe Ser Gln Leu Leu Ser Thr Ile Asp Ala Asp Ser Thr
65 70 75 80

Glu Ala Ala Leu Ile Lys Ser Leu Met Ser Gln Glu Met
85 90

<210> 132
<211> 442
<212> DNA
<213> Prunus persica

<400> 132
cttacctctt ggatgttaat atttttcctt gccagcttgc tttatatgta tatatattaa 60

tattaatata taaagccccg tttctagtct gatcatcgat ctctttatgt atatatatat 120

aaccatctgt aactaacagt gtatgatata tagctagaca tcatgtctag caaagtgtca 180

aggcagtctg gaactccaac gatcaaagat gaccaaatca ttgaacttgt ctacaaattg 240

cgccaactgg tacctgatat tcgtgataag cgctccgaca aggtatcagc atataacgtc 300

ctacaagaga cttgcagctc catcagaaac ttacacaaag aggtagacga cctaagtgag 360

cggttctctc aactactctc gacaattgat gctgatagca cggaagccgc cttaattaag 420

agcttgatgt cgcaggaaat ga 442

<210> 133
<211> 91
<212> PRT
<213> Prunus persica

<400> 133

Met Ser Ser Lys Arg Trp Arg Gln Phe Gly Ile Pro Thr Ile Lys Asp
1 5 10 15

Asp Gln Ile Ile Glu Phe Val Ser Lys Leu Arg Gln Leu Val Pro Glu

20 25 30

Ile Cys Asp Arg Arg Ser Asp Lys Val Ser Ala Phe Lys Val Leu Gln
35 40 45

Glu Thr Cys Ser Tyr Phe Arg Asn Leu His Arg Glu Val Asp Asp Leu
50 55 60

Ser Glu Gly Leu Phe Gln Leu Leu Ser Thr Ile Asp Val Asp Ser Pro
65 70 75 80

Glu Ala Ala Ile Ile Arg Ser Leu Phe Thr Gln
85 90

<210> 134
<211> 612
<212> DNA
<213> Prunus persica

<400> 134
taaagcccgg tagccagtct gatcatggat ctctttataa aaaaatatat aaccagctgt 60

agctaacagt gtagatatat atttagagat catgtctagc aaaaggtgga ggcagtttgg 120

aattccaacg atcaaagatg accaaatcat tgaatttgtt tccaaattgc gccaactggt 180

tcctgagatt tgtgataggc gttccgacaa ggtatcagca tttaaggtcc tacaagagac 240

ttgcagttac ttcagaaact tacacagaga ggttgacgac ctaagtgagg ggctttttca 300

actactctcg acaattgatg ttgatagccc ggaggccgcc ataattagga gcttgtttac 360

gcagtagatg atcagctaga tgatgatcat cagaccctta attatatatt tatatataat 420

tgtgctttga tgatgaattt atatagttaa attgtgttca tttaggttat ttggtcaccc 480

gatttttaaa caaggccagt tagctagggt attcagcagt attgtatgta tttaaagatg 540

tcttttcttg tcttgttttg tgttgttaat tagagggagg ccttttttaaa gaatatttat 600

taaagttttt tt 612

<210> 135
<211> 83
<212> PRT
<213> Prunus persica

<400> 135

Met Ser Ile Lys Lys Ser Arg Gln Ser Gly Thr Pro Thr Ile Lys Asp
1 5 10 15

Asp Gln Ile Ile Glu Leu Val Ser Lys Leu Arg Gln Leu Val Pro Glu
20 25 30

```
Ile Arg Asp Thr Arg Ser Asn Glu Val Ser Ala Ser Lys Val Leu Gln
        35                  40                  45

Tyr Thr Cys Ser Cys Ile Thr Asn Leu His Arg Glu Val Asp Asp Leu
    50                  55                  60

Ser Glu Arg Leu Ser Gln Leu Leu Ser Lys Leu Met Leu Ile Ala Arg
65                  70                  75                  80

Arg Pro Pro
```

<210> 136
<211> 615
<212> DNA
<213> Prunus persica

<400> 136

```
cttcttcttc tcctcctcct cctttagatc tacttcttct ccttcttttt cctcactctc      60
aagtactttt tcctcacaaa ttggctcttg cgtacgtggc cacttacctc tgttaggtca     120
atatttctcc ttaccagcta gctatatatg tatatatatt aatattaata tataaagccc     180
cgtcagccag tctgatcatc gatctcttta tatatatata tataaccagc tgtacctaac     240
agtgtaaata tatacctaga gatcatgtct atcaaaaagt ctaggcaatc tggaactcca     300
acgatcaaag atgaccaaat cattgaactt gtctccaaat tgcgccaact ggttcctgag     360
attcgtgata cgcgctccaa cgaggtatca gcatctaagg tcctacaata tacttgcagc     420
tgcatcacaa acttacacag agaggttgac gacctaagtg agcggctctc tcaactactc     480
tcgaaattga tgctgatagc ccggaggccg ccctaattag gagctcgatt acgcagctga     540
tgatcagcta gatgatgatc atcagaccct ggattatata tgcatatata attgtgcttc     600
gatgatgaat ttata                                                      615
```

<210> 137
<211> 90
<212> PRT
<213> Ricinus communis

<400> 137

```
Met Ser Gly Arg Arg Ser Arg Gln Pro Ser Val Pro Arg Ile Thr Asp
1               5                   10                  15

Asp Gln Ile Ile Asp Leu Val Ser Lys Leu Arg Gln Leu Leu Pro Glu
            20                  25                  30

Ile Arg Gln Arg Arg Pro Asp Lys Val Ser Ala Ser Lys Val Leu Gln
        35                  40                  45
```

```
Glu Thr Cys Asn Tyr Ile Arg Asn Leu His Arg Glu Val Asp Asp Leu
    50                  55                  60

Ser Glu Arg Leu Ser Gln Leu Leu Ala Thr Ile Asp Ala Asp Ser Pro
65                  70                  75                  80

Glu Ala Ala Ile Ile Arg Ser Leu Ile Met
                85                  90


<210>  138
<211>  727
<212>  DNA
<213>  Ricinus communis

<400>  138
aattcggcac gaggcttgtc cttccctact cttgctctct tcttgctact tatatttttc      60

catttccctt tttgatttct ttcctccatt tttatgtttc ttgagttatc gattctaaca     120

tattattaat taacacatac acaaccttct tgaattactt aaaaagaaag gatcatgtct     180

ggaagaaggt cgaggcagcc aagtgttcct agaatcactg atgatcaaat catcgacctt     240

gtctccaagt tacgccagct tcttcctgag attcgccaaa ggcgtcccga taaggtatca     300

gcttctaagg tcctacaaga gacctgcaac tacatcagga acttgcacag ggaggtggat     360

gacttaagcg agcgattgtc tcagcttttg gctacaattg acgctgatag tcctgaagct     420

gctataatta ggagtttaat tatgtaacta gagcagagct ccctgtgtta attaattaat     480

taattaaaaa atatttttcc cattgtaact ttattagtag agagagtttg atcattatgt     540

agatcagaca aaaagggttt ttagaatgca ttttaagata tatgttatat atatatatat     600

atatatatac acacatatgc atgctatatg gataagcatg cagtgtaatt aggttgtata     660

ataaaaggac tttgcactta gcaatgccta atttatatgg ataatttatc ttggttttac     720

agttggt                                                               727


<210>  139
<211>  87
<212>  PRT
<213>  Saccharum officinarum

<400>  139

Met Ser Ser Ser Gly Arg Arg Gly Arg Ile Ser Asp Asp Glu Ile Asn
1               5                   10                  15

Glu Leu Ile Ser Lys Leu Gln Ala Leu Leu Pro Glu Ser Ser Arg Arg
            20                  25                  30

Arg Asn Ala Ser Arg Ser Ser Ala Ser Lys Leu Leu Lys Glu Thr Cys
        35                  40                  45
```

Ala Tyr Ile Lys Ser Leu His Arg Glu Val Asp Asp Leu Ser Glu Arg
    50                  55                  60

Leu Ser Gly Leu Met Ser Thr Met Asp Asn Asp Ser Pro Gln Ala Glu
65                  70                  75                  80

Ile Ile Arg Ser Leu Leu Arg
                85

<210> 140
<211> 675
<212> DNA
<213> Saccharum officinarum

<220>
<221> misc_feature
<222> (583)..(583)
<223> n is a, c, g, or t

<400> 140
cacgtacaca ctgcataaat aggcgagctg cgagaggcag agacgacgag gacgaagagg 60

aattaagcca acgaggtgct gtagcttccg agcgactggt gtctctcagc aagctacgac 120

cgtcgtcacc agccggagat atgtcgtcgt cgggccgacg tggcaggatc agcgacgacg 180

agatcaacga gctcatctcc aagctccagg cgctcctccc ggagtcctca cgccgccgga 240

acgcgagccg gtcgtcggcg tcgaagcttc tgaaggagac gtgtgcctac atcaagagct 300

tgcaccggga ggtggacgac ctctcggaac ggctgtcggg gctcatgtcg accatggaca 360

acgacagccc ccaggcggag atcatccgga gcctcctccg gtgacccggc cgcccgccc 420

cggcgcgcgc ggtccggcct cttctgcctg cgatctagct agctagctgc agaagacgac 480

gacttccggg cgagcttgcc ttgctcgttt gctacggcga cgaccttaat tatgttcctt 540

tgtcttttaa tttcttcttc ttcttcttcc ggtgtggtgt gtncgttccc gtgtttaatt 600

aattcaagag caagagctct ggctcccaag acaacgacca aaggttatgg atcttctcct 660

ggtacacggc aagca 675

<210> 141
<211> 92
<212> PRT
<213> Salvia miltiorrhiza

<400> 141

Met Ser Ser Arg Arg Ser Arg Ser Arg Ala Ser Gly Ser Ser Arg Ile
1               5                   10                  15

Thr Asp Asp Gln Ile Ala Asp Leu Val Ser Lys Leu Gln Gln Leu Ile

20 25 30

Pro Glu Ile Arg Ser Arg Arg Ser Asp Lys Ala Ser Ala Ser Lys Val
35 40 45

Leu Gln Glu Thr Cys Asn Tyr Ile Arg Asn Leu His Arg Glu Val Asp
50 55 60

Asp Leu Ser His Arg Leu Ser Gly Leu Leu Glu Ser Thr Asp Gly Asp
65 70 75 80

Ser Ala Gln Ala Ala Ile Ile Arg Ser Leu Leu Leu
85 90

<210> 142
<211> 509
<212> DNA
<213> Salvia miltiorrhiza

<400> 142
aggaattcgg cacgaggctc tttctctctc ttacacacac aacatccaac aaacacaaca 60

actaattaaa ctcccttcac tctcaccacc accaccacca ccacaacttc ttgtaactta 120

aaatgtctag cagaagatcg cgttcgaggg cgtcgggatc ctcgaggata accgacgatc 180

agatcgccga cctcgtctcg aaattgcagc aactcatccc cgagatccgc agccgccgtt 240

ccgacaaggc ttcggcttcg aaggtgttgc aggagacgtg caactacata aggaacttgc 300

acagagaggt ggatgatctg agccatcgat tgtcggggct gctggaatcg acggacggcg 360

acagcgctca agccgccatt attaggagct tgctattgta atgttgttac agcgcataga 420

tgtggttgta cgtttcgctt ctgtagtcgt acgtctaggg ttaattttgc cagtatatgt 480

atgtagctat atggtttggt atgctatcc 509

<210> 143
<211> 91
<212> PRT
<213> Salvia miltiorrhiza

<400> 143

Met Ser Gly Arg Arg Ser Arg Pro Ser Thr Asn Thr Ser Arg Ile Thr
1 5 10 15

Asp Asp Gln Ile Ile Asp Leu Val Ser Lys Leu His Gln Leu Leu Pro
20 25 30

Glu Ile Arg Asn Asn Arg Arg Ser Asn Lys Ala Ser Ala Asn Lys Val
35 40 45

Leu Gln Glu Thr Cys Asn Tyr Ile Arg Asn Leu His Lys Glu Val Asp
    50                  55                  60

Asp Leu Ser Glu Arg Leu Ser Arg Leu Leu Ser Ser Ile Asp Ala Asp
65                  70                  75                  80

Ser Pro Glu Ala Ala Ile Ile Arg Ser Leu Ile
                85                  90

<210> 144
<211> 425
<212> DNA
<213> Salvia miltiorrhiza

<400> 144
gcacgaggct tccttcaagc taactttata cacacacaca cacatatact ttaatttgaa 60

tttctggcat taaacataga tatttataat ctttaagaag gatgtctggg agaagatcac 120

ggccgtcaac caacacatca agaatcacag acgaccagat catcgatctc gtctccaaac 180

tgcatcaact cctccctgaa atccgcaaca accgccgctc caacaaggct tctgcaaata 240

aggtgcttca agaaacttgc aactacatca gaaatttgca caaagaggtg gatgatttga 300

gtgagaggct atcgcggcta ctgtcgtcga tagatgctga tagccctgag gcagccataa 360

ttaggagctt aatatgagtg attaattatg taataattat gattctatat ataggatatg 420

tggct 425

<210> 145
<211> 88
<212> PRT
<213> Secale cereale

<400> 145

Met Ser Ser Arg Arg Ser Ser Arg Gly Ala Ile Ser Asp Glu Glu Ile
1               5                   10                  15

Asn Glu Leu Met Ser Lys Leu Gln Ser Leu Leu Pro Asn Ser Arg Arg
            20                  25                  30

Arg Gly Ser Ser Gln Ala Ser Thr Thr Lys Leu Leu Lys Glu Thr Cys
        35                  40                  45

Thr Tyr Ile Lys Ser Leu His Arg Glu Val Asp Asp Leu Ser Asp Arg
    50                  55                  60

Leu Ser Glu Leu Met Ser Thr Met Asp His Asn Ser Ala Gly Ala Glu
65                  70                  75                  80

Ile Ile Arg Ser Ile Leu Arg Ser

85

<210> 146
<211> 643
<212> DNA
<213> Secale cereale

<400> 146
tcgcacgagc tcgtgccgcg ggagtctgct tgctccggca ccagcttgct catcccggtc 60

agccagtgac gtagcagcct ctaggaggac gatgtcgagc agaaggtcgt cgcggggcgc 120

catctccgac gaggagatca acgagctcat gtccaagctc cagtctctgc tccccaactc 180

acgccgccgc ggctccagcc aggcgtcgac gacgaagctg ctcaaggaga cgtgcaccta 240

catcaagagc ctccaccggg aggtggacga cctcagcgac cggctgtcgg aactgatgtc 300

gaccatggac cacaacagcg ccggagcgga gatcatccgc agcatcctcc gctcgtgatc 360

atactacagc gccggccggc cgatcggaga gagctcaacc gccaggacaa ttaagcggcg 420

gcggcgccat gggactctcc ggccagccgg acacgtacga gagctttgct tagctagggt 480

atatatatcg tcctccacat atttaaatat gtatctcttt tcgcctccct ttctgcctag 540

atctgatcgt gtagatcgaa aaatgtacta cgtgtctcca agcttcactc cgtctgtact 600

gcgtagggca ttagcttagc tagcgttgct accttgagcc aaa 643

<210> 147
<211> 92
<212> PRT
<213> Senecio squalidus

<400> 147

Met Ser Ser Arg Arg Ser Arg Gln Ser Ser Ser Gly Ser Ser Arg Ile
1               5                   10                  15

Thr Asp Asp Gln Ile Ile Gln Leu Ile Ser Lys Leu Gln Gln Leu Leu
            20                  25                  30

Pro Gly Asn Arg Ile Gln Arg Ser Asn Lys Ala Ser Ala Ser Lys Val
        35                  40                  45

Leu Gln Asp Thr Cys Asn Tyr Val Arg Ser Leu His Arg Glu Val Asp
    50                  55                  60

Asp Leu Ser Asp Arg Leu Ser Glu Leu Leu Ser Thr Ile Asp Pro Asn
65                  70                  75                  80

Ser Pro Glu Ala Ser Ile Ile Gln Ser Leu Ile Met
                85                  90

<210> 148
<211> 551
<212> DNA
<213> Senecio squalidus

<400> 148
cgtatcataa ataatcactc tacatctgcg accaactaac atcattaatt taatcacatc 60

atatagtttg atcgattcta ttatgtcgag cagaagatca agacaatcat catcagggtc 120

ttcgagaatc accgatgatc agatcataca actcatctcc aagttacaac aacttcttcc 180

tgggaatcgt atccaacgat ctaacaaggc gtcagcgtcg aaggtgctac aagatacttg 240

caactatgtt agaagcttgc atagagaggt tgatgacctt agcgatcgac tgtcagagtt 300

attatcgacc attgacccca atagtcccga agcgtccatc attcaaagtt taattatgta 360

aaatgcactt gtttactttt aaactattca ttagcttctt gattaagcat aattggagtt 420

ccttaatctc ttaattaact tcttaataat gtgtagggtt aaaaatctaa ttaatgaccc 480

atgttaatgt tacgtgtagt atcattatag ttctttgtcg aataataata aataattaaa 540

agttttctag t 551

<210> 149
<211> 88
<212> PRT
<213> Senecio vulgaris

<400> 149

Met Ser Ser Arg Arg Ser Gly Ala Pro Pro Arg Ile Thr Asp Glu Gln
1 5 10 15

Ile Ile Glu Leu Val Ser Lys Leu Gln Gln Leu Leu Pro Glu Leu Arg
20 25 30

Thr Arg Arg Ser Asn Lys Ala Ser Ala Ser Lys Val Leu Gln Glu Thr
35 40 45

Cys Asn Tyr Val Arg Asn Leu His Lys Glu Val Asp Asp Leu Ser Glu
50 55 60

Arg Leu Ser Arg Leu Leu Ser Thr Ile Asp Asp Asn Ser Pro Gln Ala
65 70 75 80

Ser Ile Ile Arg Ser Leu Ile Asp
85

<210> 150
<211> 369
<212> DNA
<213> Senecio vulgaris

<400> 150
cttaaaagga ttcaattcta tttgatcata atgtcgagca gaaggtctgg agcacctcct 60

aggatcacgg atgagcagat cattgaactt gtctccaagc tgcaacaact acttccagag 120

cttcgaactc gtcgttccaa caaggcatca gcttcaaagg tgttacaaga gacgtgcaac 180

tatgtgagaa acttgcacaa ggaggttgat gaccttagtg aacgtctctc ccggttattg 240

tccaccattg atgataacag ccctcaagct tccatcatta ggagtttaat cgattagtga 300

acgacaatta tatatagata agcatttact cttttcaatt aatcatatcg attgctagtg 360

ttgctcatc 369

<210> 151
<211> 86
<212> PRT
<213> Solanum lycopersicum

<400> 151

Met Ala Ser Arg Arg Ser Arg Ser Arg Ile Ser Asp Asp Gln Ile Ala
1 5 10 15

Asp Leu Val Ser Lys Leu Gln Gln Leu Ile Pro Glu Ile Arg Asn Arg
20 25 30

Arg Ser Asp Lys Val Ser Ala Ser Lys Val Leu Gln Glu Thr Cys Asn
35 40 45

Tyr Ile Arg Asn Leu His Arg Glu Val Asp Gly Leu Ser Glu Arg Leu
50 55 60

Ser Gln Leu Leu Glu Ser Thr Asp Ser Asp Ser Ala Gln Ala Ala Ile
65 70 75 80

Ile Arg Ser Leu Leu Met
85

<210> 152
<211> 532
<212> DNA
<213> Solanum lycopersicum

<220>
<221> misc_feature
<222> (514)..(514)
<223> n is a, c, g, or t

<400> 152
tgttctttct tgaatttcat tatatataat ggcaagcaga cgatcacgtt ccagaataag 60

cgatgatcaa atcgctgatc ttgtttccaa gttgcaacaa cttatccctg aaattcgtaa 120

tagacgttct gacaaggttt cagcttcaaa agtgcttcaa gaaacttgca actatataag 180

aaatttacac agagaagtgg atggattaag tgagagatta tcacaacttt tggaatcaac 240

tgatagtgat agtgctcaag ctgctattat tagaagctta cttatgtagt agctatttaa 300

ttaaatagct caaacttcat taaaatttct attattaaaa aaagatggag catttatatt 360

attaattagg gtttttttgg ccactatagg tcaaaattaa tttctatttt tctgttttcc 420

aattttgaat agttcttttt ttttctttta ctttgtgttg tattgttgta tccatctgtt 480

ttaagagctg atgtttcttt gatctcagcc tttnttaagt atataagtat tt 532

<210> 153
<211> 92
<212> PRT
<213> Solanum tuberosum

<400> 153

Met Ser Ser Arg Arg Ser Arg Gln Ser Ser Thr Gly Ser Ser Arg Ile
1 5 10 15

Ser Asp Asp Gln Ile Ile Glu Leu Val Ser Lys Leu Gln Gln Leu Leu
20 25 30

Pro Glu Ile Arg Asn Arg Arg Ser Ser Lys Ala Ser Ala Ser Lys Val
35 40 45

Leu Gln Glu Thr Cys Asn Tyr Ile Arg Asn Leu Asn Arg Gln Val Asp
50 55 60

Asp Leu Ser Asp Arg Leu Ser Gln Leu Leu Ser Thr Ile Asp Ala Asp
65 70 75 80

Ser Pro Glu Ala Ala Ile Ile Arg Ser Leu Leu Met
85 90

<210> 154
<211> 766
<212> DNA
<213> Solanum tuberosum

<400> 154
ccttaccaca acttcctctc cttaacaagt ttcatacaca cacacaaaaa aaaatatctt 60

ctttcttttc cttatccata tgtgttgtgt aagcaattaa ataaaagaaa actacgctag 120

cacagagcca tatttgtaac gaaaagagag acattttttt gttgaattta aggatgtcga 180

gcagaaggtc gaggcaatca tcaacaggat cctcgaggat ttcagatgat cagataattg 240

aacttgtctc aaaattgcaa cagcttctac cggagattcg caatcgtcgc tctagcaagg 300

catcggcatc gaaagtactg caagaaacat gcaactacat aagaaatttg aatagacaag 360

tggatgatct tagtgatcga ctttctcagt tactctcaac tattgatgct gatagtccag 420

aagcagcaat catcaggagt ttattaatgt agtagccata tactcctatg aattaattaa 480

tgtattttca tttctaaata ttggagctat atttatatat aaatcttact accaagttct 540

tgattttctg gttgttgtta agctctagta gactcagctg gtcactatag ctaggccaat 600

catgagttag aatttaatta gaacttcaac tatatagtag tgccgattga accaatgtga 660

tccccctagc taagggcaca cgtacgtacg tttgtatcta tatttccttt actccttagt 720

taaatataat taattaataa gatgtacaaa gacaagctaa aaaact 766

<210> 155
<211> 86
<212> PRT
<213> Solanum tuberosum

<400> 155

```
Met Ser Ser Arg Arg Ser Arg Ser Arg Ile Ser Asp Asp Gln Ile Ala
1               5                   10                  15

Asp Leu Val Ser Lys Leu Gln Gln Leu Ile Pro Glu Ile Arg Asn Arg
            20                  25                  30

Arg Ser Asp Lys Val Ser Ala Ser Lys Val Leu Gln Glu Thr Cys Asn
        35                  40                  45

Tyr Ile Arg Asn Leu His Arg Glu Val Asp Gly Leu Ser Glu Arg Leu
    50                  55                  60

Ser Gln Leu Leu Glu Ser Thr Asp Ser Asp Ser Ala Gln Ala Ala Ile
65                  70                  75                  80

Ile Arg Ser Leu Leu Met
                85
```

<210> 156
<211> 622
<212> DNA
<213> Solanum tuberosum

<400> 156
gcagcgagga ttctattcta tcgtgacaac tcactttaac aacaacaaca actctttctc 60

ccattgttta ttttctctgc cccttttgtt ctttcttcag tttcattata tataatgtca 120

agcagacgat cacgttccag aataagcgat gatcaaatcg ctgatcttgt ttccaagttg 180

caacaactaa ttcctgaaat tcgcaataga cgttctgaca aggtttcagc ttcaaaagtg 240

ctgcaagaga cttgcaacta tataagaaat ttacacagag aagtggatgg attaagtgag 300

agattatcac aacttttgga atcaactgat agtgatagtg ctcaagctgc tattattaga 360

agcttactta tgtagctatt taattaaata gctcaacttc attaaaattt ctattattaa 420

aaaagatgga gtatttatat tattgattag ggtttttgg ccactatagg tcaaaattaa 480

tttctatttt tctgttttcc aattttgaat agtttttta ctttatgttg tattgttgta 540

tccatctgtt ttaagagctg atgtttcttt gatctcagcc ttttttaag tatataagta 600

tttagagatg tttgttatcg tt 622

<210> 157
<211> 95
<212> PRT
<213> Solanum tuberosum

<400> 157

Met Ser Asn Arg Arg Thr Arg Gly Ser Arg Gln Ser Ser Gly Ala Ser
1 5 10 15

Arg Ile Ser Asp Asp Gln Ile Ala Asp Leu Val Ser Lys Leu Gln Leu
20 25 30

Leu Ile Pro Glu Ser Arg Ser Thr Arg Ser Ser Asp Lys Val Glu Ala
35 40 45

Ser Lys Val Leu Gln Glu Thr Cys Asn Tyr Ile Arg Ser Leu His Arg
50 55 60

Glu Val Glu Asp Leu Ser Asp Arg Leu Ser Val Leu Leu Glu Ser Thr
65 70 75 80

Glu Ser Asp Ser Ala Gln Ala Ala Ile Ile Arg Ser Leu Phe Met
85 90 95

<210> 158
<211> 693
<212> DNA
<213> Solanum tuberosum

<400> 158
catatggttc tcttctctct ttgcccataa cttgtctctc aaatagttat catcctcttc 60

tctttttaaa ctccccacaa cacacacaca actctcacac atattcattc aaacaacaca 120

ttctattact atatataact tctatcttga caccttaatt aacacaactt cttgcctact 180

taacacaata taatgtcaaa tcgaagaaca cgcggttcga gacaatcatc aggagcttct 240

agaataagtg atgatcaaat tgctgatctc gtatcaaagt tacaattact tatccctgaa 300

agccgcagta ctaggagttc cgataaggtt gaagcttcca aagtgttgca agaaacatgt 360

aattacataa gaagtttaca cagagaagtg gaagacttaa gtgatagatt atcagtcctt 420

ttggaatcta ctgagagtga cagtgctcaa gctgctatta ttagaagcct atttatgtga 480

caattattgc cattatttga agattactag ttatgtaaca ataatttcaa tttactaggt 540

tctattttca tttgtaattt actttaatta tcatcttgtt attattttc ttcttctaag 600

atgcaatagt acttatagtt aattaattaa ttaagacatt atatttgtat tgagaaattt 660

actaaatatt tataaattga ctatggattc agt 693

<210> 159
<211> 92
<212> PRT
<213> Sorghum bicolor

<400> 159

Met Ser Ser Arg Arg Ser Arg Ser Arg Gln Ser Gly Ser Ser Arg Ile
1 5 10 15

Thr Glu Glu Gln Ile Ser Asp Leu Val Ser Lys Leu Gln Asp Leu Leu
20 25 30

Pro Glu Ala Arg Leu Gln Ser Asn Ala Arg Val Pro Ser Ala Arg Val
35 40 45

Leu Gln Glu Thr Cys Asn Tyr Ile Arg Ser Leu His Gln Glu Val Asp
50 55 60

Asp Leu Ser Glu Arg Leu Ser Glu Leu Leu Ala Thr Ser Asp Met Ser
65 70 75 80

Ser Ala Gln Ala Ala Val Ile Arg Ser Leu Leu Met
85 90

<210> 160
<211> 648
<212> DNA
<213> Sorghum bicolor

<400> 160
cccatgcact tgtcttcctc ctccaataag cttctctcca gtccttgact accatttcat 60

catctgtgta tctcaaactt gcttgcctca ctcctaacat ctcagtttgt ttctcgatct 120

cttgcaaaac ttctttcccc aatctcccag acaaccacat caaccaagat gtcgagccgg 180

aggtcacggt ctaggcagtc tggttcgtcg aggatcactg aggagcaaat cagcgacctt 240

gtatcaaagc tgcaggacct cctccccgaa gctcgccttc agagcaatgc tagagtgcca 300

tctgcgaggg tgttgcagga gacatgcaac tacatcagga gcttgcacca ggaggtggac 360

gacctgagcg agaggctgtc ggagctgctg gctacgtccg acatgagcag cgcgcaggcg 420

gctgtcatcc gaagcctgct catgtagctg agacatgcat ctcgcagcag cgttcatagc 480

ctaagtagag tgatttttag tacttgttgg agaggcaggt caatcaccta attcgcccgt 540

gtacttcgcc tacgtgcatt acgcactgtt gtcgtctcgc tacctgagcc agaggccaga 600

gctatctttt ttgtgtactt attaatcaat cctatcgttg ttggcgcg 648

<210> 161
<211> 90
<212> PRT
<213> Sorghum bicolor

<400> 161

Met Ser Ser Arg Arg Ser Ser Ser Ser Arg Gly Asn Ile Ser Glu Asp
1 5 10 15

Glu Ile Asn Glu Leu Ile Ser Lys Leu Gln Ala Leu Leu Pro Ser Ser
20 25 30

Arg Arg Arg Gly Ser Gly Gln Ala Ser Thr Thr Lys Leu Leu Lys Glu
35 40 45

Thr Cys Ser Tyr Ile Lys Ser Leu His Arg Glu Val Asp Asp Leu Ser
50 55 60

Asp Arg Leu Ser Asp Leu Met Ser Thr Met Asp His Asn Ser Pro Gly
65 70 75 80

Ala Glu Ile Ile Arg Ser Ile Leu Arg Ser
85 90

<210> 162
<211> 861
<212> DNA
<213> Sorghum bicolor

<400> 162
aattcgcaga gcgaccccca gtccagcgcg cgcgggtagt ccacacacac acaccttcgt 60

tcccagttcc caccaataca cagcgattga gccgcgcgcc agtggacgca cacacgcggt 120

agctttagct tcgttctcaa gcttagcccg gccgattcta cgcgcgcagt gttcgtctcg 180

ttcgttccgg cagcaggcgg cgaagtacga cgacgacgac gagctagaga gcgaggatgt 240

cgagccgaag gtcgtcgtcg tcgcgtggca acatctccga ggacgagatc aacgagctca 300

tctccaagct ccaggccctg ctccccagct cccgccgccg cggctccggc caggcgtcga 360

cgacgaagct gctgaaggag acctgcagct acatcaagag cctccaccgg gaggtcgacg 420

acctgagcga ccggctgtcg gacctgatgt ccactatgga ccacaacagc cccggcgcgg 480

aaatcatccg cagcatcctc cgctcctgat cacgtacctc ctactgcggc gccggcgccg 540

ggccgggggc tgcgcgtgag agctagcgag gtcaacgccg gcctcgtcgc cgacgacgac 600

gaccgcaacc gttctccgcc tgatccggct ggccacgtgc gggagctgag ctcaattagc 660

tagggtatat atatatcttt ctctctacaa gtatgtgtat ctttctctgc cttttacctg 720

tctccctaga tctgagatca tgtggctagc tccatatcaa aatgtactag ctacacgcac 780

acgtatgatg gtctctgcta agcttcacac tgggtagggt actactagcg cactagggcc 840

taagcaagct tatccccccgg c 861

<210> 163
<211> 90
<212> PRT
<213> Spartina alterniflora

<400> 163

Met Ser Ser Arg Arg Ser Ser Arg Gly Gly Asn Ile Ser Asp Glu Glu
1 5 10 15

Ile Asn Glu Leu Ile Ser Lys Leu Gln Ala Leu Leu Pro Val Ser Ser
20 25 30

Arg Arg Arg Gly Ser Gly Gln Ala Ser Thr Thr Lys Leu Leu Lys Glu
35 40 45

Thr Cys Ser Tyr Ile Lys Ser Leu His Arg Glu Val Asp Asp Leu Ser
50 55 60

Asp Arg Leu Ser Asp Leu Met Ser Thr Met Asp Gln Asn Ser Pro Gly
65 70 75 80

Ala Glu Ile Ile Arg Ser Ile Leu Arg Ser
85 90

<210> 164
<211> 531
<212> DNA
<213> Spartina alterniflora

<400> 164
ccaagcttga tttgactaat agatcgttcc cagtcggcga cggcgaagcg aagcacgacg 60

agcggcgagc tagagagcat gtccagccgg aggtcgtcgc gtggcggcaa catctccgac 120

gaggagatca acgagctcat ctccaagctt caggccctgc tcccagtcag ctctcgcaga 180

cgcggctccg gccaggcgtc aacgacgaaa ctgctgaagg agacttgcag ctacatcaag 240

agcctccacc gggaggtgga cgacctcagc gaccggcttt cggacctcat gtccaccatg 300

gaccaaaaca gccccggcgc ggagatcatc cgcagcattc tccgctcatg atgacctgcg 360

gcaagcggtg tgtgtggctg tcaccgtcga ccaaccgcca acgacgaccg gcctccacgc 420

catgcattat tccccgggcc agattacggg agctccacat tagctagggt atatgcacat 480

atatatttct atctatccac aaatattgtg tactgtctca acaaaaaaaa a 531

<210> 165
<211> 91
<212> PRT
<213> Triphysaria versicolor

<400> 165

Met Ser Ser Arg Arg Ser Arg Ser Arg Gln Ser Gly Thr Ser Arg Ile
1 5 10 15

Ser Glu Asp Gln Ile Asn Glu Leu Val Ser Lys Leu Gln Gln Leu Leu
20 25 30

Pro Glu Leu His Asn Arg Arg Thr Asp Lys Arg Ser Ala Thr Asn Val
35 40 45

Leu Gln Glu Thr Cys Asn Tyr Ile Arg Ser Leu His Arg Glu Val Asp
50 55 60

Asp Leu Ser Glu Arg Leu Ser Glu Leu Leu Ala Thr Thr Asp Thr Thr
65 70 75 80

Gln Ala Ala Leu Ile Arg Arg Leu Leu Ser Gln
85 90

<210> 166
<211> 597
<212> DNA
<213> Triphysaria versicolor

<400> 166
tgtctcacca ctgtttctaa ctctcaactt cacttcaaac ttagttaata ctcttgcttt 60

ttaagttatt tattacataa ttgttaaaaa tgtcgagccg aagatcacgg tcaagacaat 120

ccggaacttc gaggatctcc gaagatcaaa tcaacgagct cgtttccaag ttacagcagc 180

ttcttcccga gttgcataac cgacgtaccg acaagagatc agcaacaaat gtgttgcaag 240

agacatgtaa ctacataaga agcttgcata gagaggtgga tgatttaagt gagagattgt 300

ctgaattgtt ggcaacaaca gataccactc aagctgctct aattagaaga ctgctgtcac 360

agtagtagac ttaattgatt ttgctttcct ttttaaaaat aaaaaataaa acatttttgt 420

ttttattatt tttcatttct caagtaattg caatattcga gtattattgt tactagctag 480

atctactttg tagacgagga tttaatgtac tttgatgggt ttttgagatg tttaatcatc 540

tttgctacct ttcttatttc attttcgata atactaaatg aaaactacaa tttcaaa 597

<210> 167
<211> 88
<212> PRT
<213> Triticum aestivum

<400> 167

```
Met Ser Ser Arg Arg Ser Ser Arg Gly Ala Ile Ser Asp Glu Glu Val
1               5                   10                  15

Asn Glu Leu Met Ser Lys Leu Gln Ser Leu Leu Pro Asn Ser Arg Arg
            20                  25                  30

Arg Gly Ser Ser Gln Ala Ser Thr Thr Lys Leu Leu Lys Glu Thr Cys
        35                  40                  45

Ser Tyr Ile Lys Ser Leu His Arg Glu Val Asp Asp Leu Ser Asp Arg
    50                  55                  60

Leu Ser Asp Leu Met Ser Thr Met Asp His Asn Ser Ala Glu Ala Glu
65                  70                  75                  80

Ile Ile Arg Gly Ile Leu Arg Ser
                85
```

<210> 168
<211> 1096
<212> DNA
<213> Triticum aestivum

<220>
<221> misc_feature
<222> (1068)..(1068)
<223> n is a, c, g, or t

<220>
<221> misc_feature
<222> (1083)..(1083)
<223> n is a, c, g, or t

<220>
<221> misc_feature
<222> (1086)..(1086)
<223> n is a, c, g, or t

<400> 168
```
gcacgaggca caactagaag ttatccagcg agtatcctga gcagctcgac gtcagcgagg      60

gccgcccttc gctcaccggc caaccgcccg gcacctttg agcgtacaca ctccgaagct      120

ttgttagccg gcgggagtct gcagtctgct tgctccggca ctagctagct agctcatccc     180

ggccggccag cgacgtagca gcggctagga agaagatgtc gagcagaagg tcgtcgcgcg     240
```

gcgccatctc cgacgaggag gtcaacgagc tcatgtccaa gctccagtct ctgctcccca 300

actctcgccg ccgcggctcc agccaggcgt cgacgacgaa gctgctgaag gagacgtgca 360

gctacatcaa gagcctccac cgggaggtgg acgacctcag cgaccggctg tcggacctca 420

tgtcgaccat ggaccacaat agcgccgaag cggagatcat ccgcggcatc ctccgctcgt 480

gatcgtacca cagcgccggc cggtcgatcg gcgagagctc aaccgccagg acaattaagc 540

ggcagcggcg ccatgggtct ctccgccggc cagccggaca cgtacgagag ctttgcttag 600

ctagggtata tatatcgtcc tccacatatt taaatatgta atgtctcttt tctgctccct 660

ttctgcctag atctgatcgt gtagatcaaa aaatgtactg cgtgtctcta agcttcactc 720

cgtctgtact acgtagggca ttagcttagc tagcgttcct accttgggcc aaagcttatc 780

ctcgcgcgct ggctgctgct tgagctaatc tttcgatcgt ctcctccgtg tgcttccctc 840

gctagctcgg agctggatag atagctcccc ccgtcctcct gtctgcctct tcccctcttt 900

tgttgtccct ttcttgatct actactcgat ctgtaaattt agttggtggc attggatcga 960

gttgtgtcct ctatagacaa ccgaccgacc actactacgg tactactact acctagagca 1020

aattaatatc atcgtcatgt tgtaccaccc cagctttaac ttattgtnga atacgtacta 1080

cgnagnatca aattaa 1096

<210> 169
<211> 85
<212> PRT
<213> Triticum aestivum

<400> 169

Met Ser Ser Arg Arg Gly Arg Ile Thr Asp Glu Glu Ile Asn Glu Leu
1 5 10 15

Ile Ser Lys Leu Gln Ala Leu Val Pro Glu Ser Ser Arg Arg Arg Ser
20 25 30

Ala Ser Arg Ser Ser Ala Ser Lys Leu Leu Lys Glu Thr Cys Gly Tyr
35 40 45

Ile Lys Ser Leu His Gln Glu Val Glu Asp Leu Ser Asp Arg Leu Ser
50 55 60

Glu Leu Met Ser Thr Leu Asp Glu Thr Ser Pro Gln Ala Glu Ile Ile
65 70 75 80

Arg Gly Leu Leu Arg
85

<210> 170

<211> 890
<212> DNA
<213> Triticum aestivum

<220>
<221> misc_feature
<222> (8)..(8)
<223> n is a, c, g, or t

<400> 170
cacgcagnac gcttcatcgg ccgaatcaca ggctaaggta aagcatcaac atatacacga 60

gcaagcaagg aaagccaagc gttagctgtc tccgatcaga gccggccggc cgcagagaga 120

gagagggagg gagatgtcga gccgccgtgg caggatcacc gacgaggaga tcaacgagct 180

catctccaag ctccaggcgc tggtcccgga atcatcccgc cgccgctccg cgagccggtc 240

gtcggcgtcg aagctgctga aggagacgtg cggatacatc aagagcctcc accaggaggt 300

cgaggacctc tccgacaggc tctcggagct aatgtcgacc ttggacgaga ccagccccca 360

ggccgagatc atccggggcc ttctccgcta gccggatttt atcctaccga ttgagccagt 420

aggcagtagc tacatatata ttagcttgaa ttcatcggcc gaaggaggga gacgaggagg 480

caagacaaga gaagagaaga caacaagaga ggcatagcat tttttagctg ctgcttgttt 540

cttttcctgc ctgctccccc gtctcctggt acgtcgtcgt ccgtgtgcgt gtgtgtcttt 600

gtgaggccct catttagctt ccggtatact ccactgtgtt tcatttatgc accaggttgg 660

tagaggttaa taatatatat ggtgcttcta ccgattagcc gagtgtatta ctactagctt 720

gtagacgtgt gtgtttgtgc tgttgtaggc ctgtagcttc tctcagactg agatgcatcc 780

tctggctata gagctgttgt tttgtactac tagtactatc tattgctagg tttgtccctg 840

ttttccaacg gacaagctag ctaggttaac gacgagcgtg gactacgggg 890

<210> 171
<211> 88
<212> PRT
<213> Triticum aestivum

<400> 171

Met Ser Gly Arg Arg Ser Arg Gly Ser Val Ser Glu Glu Glu Ile Asn
1 5 10 15

Glu Leu Ile Ser Arg Leu Gln Thr Leu Leu Pro Thr Ala Arg Arg Arg
20 25 30

Gly Ser Ser Ser Ser Gln Ala Ser Thr Thr Lys Met Leu Lys Glu Thr
35 40 45

Cys Ser Tyr Ile Lys Ser Leu His Arg Glu Val Asp Asp Leu Ser Asp
50 55 60

Arg Leu Ser Asp Leu Met Ser Thr Met Asp Asn Asn Ser Pro Ala Ala
65 70 75 80

Glu Ile Ile Arg Ser Leu Leu Arg
85

<210> 172
<211> 894
<212> DNA
<213> Triticum aestivum

<220>
<221> misc_feature
<222> (43)..(45)
<223> n is a, c, g, or t

<400> 172
gcacgagggc acttcgcagc tagccgccca ctctacttcc gannnacagc ctctccatcg 60

accgaccttc gttcgccctg cattactctg tgaagacgat gtctggcagg aggtcgcgcg 120

gctccgtgtc ggaggaggag atcaacgagc tcatctccag gctccagacc ctgctcccca 180

ccgcgcgccg ccgcggcagc agcagcagcc aggcgtcgac gacgaaaatg ctcaaggaga 240

cgtgcagcta catcaagagc ctgcacaggg aagtggacga cctcagcgac cgcctctccg 300

acctcatgtc caccatggac aacaacagcc ccgccgccga gatcatccgc agcctcctcc 360

gctagctacc tagctggctg actgctcatc atcatcatcg atcacctcct gctgattgtc 420

ctaagctagt tcatcatcta cgtacagctc gtgcagtcct agctaattaa gcgcatatga 480

tctacacata cagtacatgg tatatatgtc gtccgtcgat ctatgcaagc atatatatgc 540

agatcgatcg atatctaatt aaggaggact gcatgctaag gccggctggt ctaatttact 600

ttggtagggc atccatcatt agctagctag ggccgccagc taagttctat agctgcttca 660

ttagctcacc cttgcatgcg gtttcctcac agtttccatc ccccctcccc ctctctctta 720

ttttcatttg cttgtgtaaa cttggttatt tgcagtctgg atcgagttgt ttcccctaga 780

gacaaccggc cgaccgctag ctacccatcc ggtggtggta ctgctaatat actactactg 840

ctactcagta tcaatcaccc atgaatgtat gtactatgac tgtcgggctt cggc 894

<210> 173
<211> 91
<212> PRT
<213> Vitis vinifera

<400> 173

Met Ser Ser Arg Arg Ser Arg Ser Arg Gln Ser Gly Gly Ser Arg Ile
1 5 10 15

Thr Asp Asp Gln Ile Asn Asp Leu Val Ser Lys Leu Gln Gln Leu Leu
20 25 30

Pro Glu Ile Arg Gly Arg His Ser Asp Lys Val Ser Ala Ala Lys Val
35 40 45

Leu Gln Glu Thr Cys Asn Tyr Ile Arg Ser Leu Asn Arg Glu Val Asp
50 55 60

Asp Leu Ser Glu Arg Leu Ser Glu Leu Leu Ala Thr Thr Asp Ser Ala
65 70 75 80

Gln Ala Ala Ile Ile Arg Ser Leu Leu Thr Gln
85 90

<210> 174
<211> 276
<212> DNA
<213> Vitis vinifera

<400> 174
atgtctagca gaagatcacg ctcaaggcaa tccggaggtt ccaggatcac ggatgaccag 60

atcaatgatc tggtttccaa gttgcaacag cttcttcctg agattcgagg caggcactcg 120

gacaaggtct cggcagctaa ggtcttacag gagacatgca actatattag aagcctgaac 180

agagaggttg atgacctaag tgagcgattg tctgagttat tggcaacaac agactctgcc 240

caggcagcca ttattaggag tctacttacg caatag 276

<210> 175
<211> 92
<212> PRT
<213> Vitis vinifera

<400> 175

Met Ser Ser Arg Arg Ser Arg Ser Arg Gln Pro Gly Val Ser Arg Ile
1 5 10 15

Ser Asp Asp Gln Ile Ala Asp Leu Val Ser Lys Leu Gln Gln Leu Ile
20 25 30

Pro Glu Ile Arg Asn Arg Arg Ser Asp Lys Val Ser Ala Ser Lys Val
35 40 45

Leu Gln Glu Thr Cys Asn Tyr Ile Arg Asn Leu His Arg Glu Val Asp
50 55 60

Asp Leu Ser Asp Arg Leu Ser Ala Leu Leu Ala Ser Thr Asp Thr Asp
65 70 75 80

Ser Asp Gln Ala Ala Ile Ile Arg Ser Leu Leu Met
85 90

<210> 176
<211> 279
<212> DNA
<213> Vitis vinifera

<400> 176
atgtcaagca ggagatcgcg ttcaagacag ccaggagttt cgaggataag tgacgatcag 60

attgctgatc tcgtgtccaa gttacagcag cttattcctg agattcgcaa taggcgctcc 120

gacaaggtat cggcttctaa agtcttgcag gagacttgca actatattag aaatttgcat 180

agagaggtgg atgacctaag cgatcgattg tctgcgcttt tggcttccac cgacacggat 240

agcgatcagg ctgccataat taggagctta ctcatgtaa 279

<210> 177
<211> 90
<212> PRT
<213> Vitis vinifera

<400> 177

Met Ser Thr Gln Arg Ala Arg Ala Ser Arg Val Thr Asp Asp Glu Ile
1 5 10 15

Asn Asp Leu Ile Leu Lys Leu Gln Ala Leu Leu Pro His Ser Asn Gln
20 25 30

Arg Arg Thr Ser Thr Gly Ala Ser Ala Trp Arg Ile Leu Lys Glu Thr
35 40 45

Cys Ser Tyr Ile Lys Arg Leu His Arg Glu Val Gly Asp Leu Ser Glu
50 55 60

Arg Leu Ser Gln Leu Leu Asp Ser Leu Asp Asn Ile Asn Gly Val Glu
65 70 75 80

Val Glu Gln Leu Arg Ser Leu Leu Gln Arg
85 90

<210> 178
<211> 273
<212> DNA
<213> Vitis vinifera

<400> 178
atgtctaccc aaagagcaag agcttcacga gtcaccgacg atgagattaa cgacctcatc 60

ctcaaactgc aggcgttgct acctcattca aatcaaaggc gcacgtctac aggggcatcg 120

gcatggagga ttctgaaaga aacgtgcagt tacataaaga ggctacacag agaggtgggc 180

gacctgagtg agagactatc ccagcttctt gattctcttg ataatattaa tggtgttgag 240

gttgagcaac ttagaagttt attgcagcga tag 273

<210> 179
<211> 90
<212> PRT
<213> Vitis vinifera

<400> 179

Met Ser Ser Arg Arg Ser Arg Gln Ser Gly Ser Ser Arg Ile Ser Asp
1 5 10 15

Asp Gln Ile Ile Glu Leu Val Ser Lys Leu Gln Gln Leu Leu Pro Glu
20 25 30

Ile Arg Asn Arg Arg Ser Asp Lys Val Ser Ala Ser Lys Val Leu Gln
35 40 45

Glu Thr Cys Asn Tyr Ile Arg Ser Leu His Arg Glu Val Asp Asp Leu
50 55 60

Ser Glu Arg Leu Ser Arg Leu Leu Ala Thr Val Asp Ala Asp Ser Pro
65 70 75 80

Glu Ala Ala Ile Ile Arg Ser Leu Ile Met
85 90

<210> 180
<211> 273
<212> DNA
<213> Vitis vinifera

<400> 180
atgtctagca gaaggtcgag gcagtcaggg tcttcgagga tctcagatga tcagatcatt 60

gaacttgtgt ccaagttgca gcaacttctt cctgagattc gcaataggcg ttcagacaag 120

gtgtcagctt ccaaggtcct acaggagacc tgcaactaca ttagaagctt acacagagag 180

gtggatgacc taagcgaacg actgtccagg ttactggcta cagtcgatgc tgatagtcct 240

gaggctgcaa taatcaggag tttaattatg taa 273

<210> 181
<211> 103
<212> PRT
<213> Welwitschia mirabilis

<400> 181

```
Met Glu Gly Ser Ser Ser Ser Ser Arg Ser Arg Arg Ser Ser Gly Ser
1               5                   10                  15

Ser Ser Arg Gly Ala Ser Arg His Ser Cys Arg Val Ser Glu Gln Gln
            20                  25                  30

Ile Asn Asp Leu Leu Ser Lys Leu Gln Ser Leu Leu Pro Asp Val Cys
        35                  40                  45

Glu Ser Gly Asp Lys Met Pro Ala Ser Lys Val Leu Gln Glu Thr Cys
    50                  55                  60

Asn Tyr Ile Lys Ser Leu His Arg Glu Val Asp Asp Leu Ser Glu Arg
65                  70                  75                  80

Leu Ala Glu Ile Leu Ala Asn Val Glu Ser Asp Ser Val Gln Ala Ala
                85                  90                  95

Ile Ile Arg Ser Leu Leu Thr
            100


<210>  182
<211>  657
<212>  DNA
<213>  Welwitschia mirabilis

<400>  182
gtctggttta gtctcagttc agctcagttt actctccagc tgagtttgtc ggattgagtg      60

tgtgaaagga aagtcatagt gtgtggaatg gaaggatcgt cgagctccag tagaagcaga     120

aggtcttctg gttcttcgtc gcgcggtgcc agcaggcact cttgcagagt ttccgaacag     180

caaatcaatg atcttctctc gaagcttcag tcgcttctgc cggatgtttg tgaatccgga     240

gataagatgc ctgcgtccaa agttctacaa gaaacatgca actacatcaa gagtcttcac     300

agagaggtgg acgatttaag cgagcgctta gctgaaattc tcgcaaacgt agagagcgac     360

agcgtgcagg ctgcaatcat caggagcctt ctcacataaa tgctcctgtt tctttctaat     420

ttgtctacct caggcccctt tctacttctt tgctttctgc ttcttatttt gtaacagaca     480

agaagcacag ttaagcataa actttagagt atcggcgatc ttatgttgct catgtcatat     540

atatcataaa aaagaatttg ctttttttact ttgtttctca ctcttgatga acatcatctg     600

gtgagttgca gaatctaata attcacagac atacactgtg tatggatctg tattacg        657


<210>  183
<211>  87
<212>  PRT
<213>  Zea mays

<400>  183
```

```
Met Ser Ser Arg Arg Ser Arg Ala Ser Thr Val Ser Glu Glu Glu Ile
1               5                   10                  15

Asn Glu Leu Ile Ser Arg Leu Gln Thr Leu Leu Pro Ser Ala Arg Arg
            20                  25                  30

Arg Gly Gly Ser Gln Ala Ser Thr Thr Lys Leu Leu Lys Glu Thr Cys
        35                  40                  45

Ser Tyr Ile Lys Ser Leu His Arg Glu Val Asp Asp Leu Ser Asp Arg
    50                  55                  60

Leu Ser Asp Leu Met Ala Ser Met Asp His Asn Ser Pro Gly Ala Glu
65                  70                  75                  80

Ile Ile Arg Ser Leu Leu Arg
                85
```

```
<210>  184
<211>  954
<212>  DNA
<213>  Zea mays

<400>  184
ctcccctgag ctcgatcgga tcgtgcacag cccgaacagc cgctccaccc tcccgctcag      60

ctcgctcttc ggcggtgctg ctctcgatcg tcttctccgt caccggcggc cgcttagcct     120

ccgcgatccc ggccggtggt cttcttcgcc gcattatctc tctctctctc tctctctctc     180

tctaacacaa ggacgatgtc gagccggagg tcccgcgcgt cgacggtctc ggaggaggag     240

atcaacgagc ttatctcgag gctgcagacg ctgctcccca gcgcgcgccg ccgtggcggc     300

agccaggcgt cgacgacgaa gctgctcaag gagacctgca gctacatcaa gagcctgcac     360

cgggaggtgg acgatctgag cgaccgcctg tcggacctca tggccagcat ggaccacaac     420

agccccggcg ccgagatcat ccgcagcctc ctccgctagc ccaagtccgt ccgtccggcc     480

ggccggccac gcgcgccgca tatatgcagc atctgcgcgc gcgctgtctc tctccatcca     540

tggacgacgg ccggcctctc gccatcgcca gatctcagcg cattgccgag tgtgtgtgtg     600

tacccatgca tatagcagct gaatataccc aaggacgaca ggctaaggct ggtttattaa     660

ttggtagggc attattaagt actactccgt actattaact agggctgcct agcctaagta     720

cggtttctct ctctcttcca ctcttggttg tttgtttcct tgctatactc ctagtagctt     780

agctcctttt ccatttgttt gtactcttgg ctgcttcgtc acatgttctt cctcgtcgtc     840

gtcgtcgtcg tcgtaaacct atgtgtggtc tggatcgagt tgtttcctcc ttgacagaca     900

accgaccaac cgcgagtcga gctaccgatc gatctgtcaa cttgtactac gtac           954
```

<210> 185
<211> 89
<212> PRT
<213> Zea mays

<400> 185

```
Met Ser Ser Arg Arg Pro Ser Ser Arg Gly Asn Ile Ser Glu Asp Glu
1               5                   10                  15

Ile Asn Glu Leu Ile Ser Lys Leu Gln Ala Leu Leu Pro Ser Ser Arg
            20                  25                  30

Arg Arg Gly Ser Gly Gln Ala Ser Thr Thr Lys Leu Leu Lys Glu Thr
        35                  40                  45

Cys Ser Tyr Ile Lys Ser Leu His Arg Glu Val Asp Asp Leu Ser Asp
    50                  55                  60

Arg Leu Ser Asp Leu Met Ala Thr Met Asp His Asn Ser Pro Gly Ala
65                  70                  75                  80

Glu Ile Ile Arg Ser Ile Leu Arg Ser
                85
```

<210> 186
<211> 1489
<212> DNA
<213> Zea mays

<400> 186

```
cccccgcccc gagccagctc gctataaagg cacccctctc cctttctgtc tccctcgcag      60

ctcagttact cactccggcc tccagctttt cctttgcgcc ccagggccgg aagagcccac     120

acaccaccac caccactaga tagccagcga cgatcgagcc gcgcgccggt ggacgcgcac     180

atacacgcgg tagcttcctt ctcaagccta gctagcccca tcctgcgcgc aggcggcggc     240

agcgagaatt gacgtgcgac gacgagcttg tgcttgctag ctagagagcg aggatgtcga     300

gccggaggcc gtcgtcgcgt ggcaacatct ccgaggacga gatcaacgag ctcatctcca     360

agctgcaggc cctgctcccc agctcccgcc gccgcggctc cggccaggcg tcgacgacga     420

agctgctcaa ggagacctgc agctacatca agagcctgca ccgggaggtg gacgacctga     480

gcgaccggct gtccgacctc atggccacca tggaccacaa cagccccggc gcggagatca     540

tccgcagcat cctccgctcc tgatcgccca cgcgcctcct actgcggcgg cgccggccgg     600

cgcgcgagag agcgaggtcg gcgacgacct cgatcgccga caacgacgac cgcgcgcgcc     660

cgcgcccccc ggcccatctg ttctctccac cggctggccg ccgggagccg agctcaatta     720

gctagggtat atctcaacct ctctctctct ctctctctcg ctcgctctac atgtgtgtgt     780
```

```
acctttctct tcctttcacc tgcctgtctc ctccctagat ctgagatcat gtggctagct     840

ctcccatatc aaaatgtact agctacacgc gcacgtatgg tggtctctgc taagcctaag     900

cttcactggg tagtagggta ctagtagcac taggggctta agcaagctaa gcttatcctc     960

ctcatcagaa tcacattagc tcgcaccgca ctctctggct tctcgtcgat cgtcttcgtc    1020

gtcctcgctc ctccgaccca catgatggct agctctccat gtgccgcttc tcctctcgca    1080

cgtgccgctt ctcctcttgt gttcgatatg ttgtattgtt tccatgtaaa tttagtcggt    1140

ggcctggatc gagttggcta gctctctcta caggcaagag acaaccgacc gaccactact    1200

atcctagagc aaattgatta tcgtcatgat ggtgtactgt tcaagctttt attaacgact    1260

tttaatttac ttgctacgta cagcacgtac gtcctacgag aaatgctttt gtgttttttt    1320

tttctttcac tcagggtgcc atggaccatg gttaagagat gcaaccgctg tgtatgtact    1380

gtagtactag tatgcagcga aaacgttgcc ctgcctttca tattggctgc ttcgatcggt    1440

ccacttattt ggttccgtac ggtggaacgt gaaacgacgc gtttacttc                1489


<210>  187
<211>  89
<212>  PRT
<213>  Zea mays

<400>  187

Met Ser Ser Arg Arg Pro Ser Ser Arg Gly Asn Ile Ser Glu Asp Glu
1               5                   10                  15


Ile Asn Glu Leu Ile Ser Lys Leu Arg Ala Leu Leu Pro Ser Ser Arg
            20                  25                  30


Arg Arg Gly Ser Gly Gln Ala Ser Thr Thr Asn Leu Leu Arg Glu Thr
        35                  40                  45


Cys Ile Tyr Ile Lys Ser Leu His Arg Glu Val Asp Asp Leu Ser Asp
    50                  55                  60


Arg Val Ser Asp Leu Val Ala Thr Met Asp His Asn Ser Pro Gly Ala
65                  70                  75                  80


Glu Ile Ile Arg Ser Ile Leu Arg Ser
                85


<210>  188
<211>  606
<212>  DNA
<213>  Zea mays

<400>  188
```

```
cagttactca ctccggcctc cagtttttct tttgcgcccc agggccggag gagcccaccc      60

accaccacca ccactaaata gccagcgacg atcgagccgc gcgccggtgg acgcgcacat     120

acacgcggta gtttctttct caagcctagc tagccccatc ctgcgcgcag gggggggcag     180

cgaaaattga cgtgcgacga cgagcttgtg cttgctatct agagagcgag gatgtcgagc     240

cggaggccgt cgtcgcgtgg caacatctcc gaggacgaga tcaacgagct catctccaag     300

ctgcgggccc tgctccccag ctcccgccgc cgcggctccg gccaggcgtc gacgacgaat     360

ctgctcaggg agacctgcat ctacatcaag agcctgcacc gggaggtgga cgacctgagc     420

gaccgggtgt ccgacctcgt ggccaccatg gaccacaaca gccccggcgc ggagatcatc     480

cgcagcatcc tccgctcctg atcgcccacg cgcctcctac tgcggcggcc ccggccggcg     540

cgcgagagag cgaggtcgga cacgacctcg atcgccgaca aagacgaccg cgcgcgcccg     600

cgcccc                                                                606


<210>  189
<211>  90
<212>  PRT
<213>  Zea mays

<400>  189

Met Ser Ser Arg Arg Ser Ser Ser His Gly Asn Ile Ser Glu Asp Glu
1               5                   10                  15


Met Asn Glu Leu Val Ser Lys Leu Gln Ala Leu Leu Pro Ser Ser Arg
            20                  25                  30


Arg Arg Arg Gly Ser Gly Gln Ala Ser Thr Ala Lys Leu Leu Lys Glu
        35                  40                  45


Thr Cys Ser Tyr Ile Lys Ser Leu Gln Arg Glu Val Asp Asp Leu Ser
    50                  55                  60


Asp Arg Leu Ser Asp Leu Leu Ser Thr Met Asp His Asn Ser Pro Ala
65                  70                  75                  80


Ala Glu Ile Ile Arg Ser Ile Leu Arg Ser
                85                  90


<210>  190
<211>  999
<212>  DNA
<213>  Zea mays


<220>
<221>  misc_feature
<222>  (986)..(986)
```

<223> n is a, c, g, or t

<400> 190
gctacagctt taattttgca gagcgaccac cagtccaggt ccagcgcggg acgcatcaca 60

tacacgcggt accttcgttc gttctcaagc ttatagcgcc cgatcgaccc tgcgcggagc 120

tagttcgttc gttccggcag gcggcggcag cgaagcagtg cgacgacgac gacgaggtac 180

gtagagagcg agaggataga tgtcgagccg aaggtcgtcg tcgcacggca acatctccga 240

ggacgagatg aacgagctcg tctccaagct ccaggccctg ctccccagct cccgccgccg 300

ccgcggctcc ggccaggcgt cgacggcgaa gctgctgaag gagacctgca gctacatcaa 360

gagcctccag cgggaggtgg acgacctcag cgaccggctg tcggacctct tgtccaccat 420

ggaccacaac agccccgcgg cggagatcat ccgaagcatc ctccgctcct gagcgcgcgc 480

aagggcgagg tcaacgaacg ccggcctccg atcgatcgcc gacagcgcgc gctctccggc 540

cggctggtca cgtgcgggag ctgagctcaa ttaggtagct agggtatata tacatataat 600

atatatatct acatgtacgt gtatctacct tttcctttac cagtctccct agatctgaga 660

tcatgtggct agctccgtat aaaaatgtac tagccacacg ctgacacgca cacgtatgca 720

tgatggtctc tgcgctaagc ttcactgggt agtagggtat agcactagag cctaagcaag 780

cttatcctcc tcactttagt atagctcgca gcagcagcag tctctcgatt cctcggcgat 840

cttcggtggc ttaattggat cgagctggct agtgcgctct ctctctctct ctcatctcta 900

gcaggcagga aaagagacaa ccgaccgacc actactagcc tagcctagag caaattgact 960

gtcgttatga tgatgatgat gtactntaaa gcttttatt 999

<210> 191
<211> 105
<212> PRT
<213> Zea mays

<400> 191

Met Ser Ser Gly Arg Arg Pro Ser Arg Thr Arg Arg Ala Gly Ser Ser
1 5 10 15

Ser Leu Ser Ser Ser Ser Thr Ser Arg Ser Ile Ser Asp Asp Gln Ile
20 25 30

Ser Glu Leu Leu Ser Lys Leu His Ala Leu Leu Ala Glu Ser Gln Ala
35 40 45

Arg Asn Gly Gly Ala His Arg Gly Ser Ala Ala Arg Val Leu His Asp
50 55 60

Thr Cys Ser Tyr Ile Arg Ser Leu His His Glu Ala Asp Asn Leu Thr
65 70 75 80

```
Glu Thr Leu Ala Glu Leu Leu Thr Ser Ala Asp Val Thr Ser Asp Gln
                85                  90                  95


Pro Pro Val Ile Thr Ser Leu Phe Met
            100                 105
```

<210> 192
<211> 497
<212> DNA
<213> Zea mays

<400> 192

```
ctattcgttg gggaggaaaa gacttaaagc agtctatttg tctactcgcc gagagctctc     60

tttcccctct tctatagcta ctgcatgctc tgctagtcga tcagctaggc agctaggtag    120

ctagctccga tccacatata taaaatcaga tcgcacagct actagcggca accgacatgt    180

ccagcggccg gaggccgtca cgcacgcggc gtgcggggag cagctcgctg tcgtcgtcgt    240

cgacgtccag gtccatctcg gatgaccaga tctccgagct cctgtccaag cttcacgcgc    300

tgctcgcgga gtctcaagct cgcaatggcg gcgcacatag ggggtccgcg gcgagggtgc    360

tgcacgacac gtgcagctac atcaggagcc tgcaccacga ggcggacaac ctcaccgaga    420

cgctggccga gctgctcacc tccgccgatg tcaccagcga ccagcccccc gtcatcacga    480

gcctgttcat gtgacca                                                   497
```

<210> 193
<211> 85
<212> PRT
<213> Zingiber officinale

<400> 193

```
Met Ser Ser Arg Arg Asn Arg Val Ser Glu Glu Glu Ile Asn Glu Leu
1               5                   10                  15


Ile Ser Lys Leu Gln Ser Leu Leu Pro Glu Thr Arg Arg Arg Gly Ala
            20                  25                  30


Gly Arg Ala Ser Ala Ala Lys Leu Leu Lys Glu Thr Cys Ser Tyr Ile
        35                  40                  45


Arg Ser Leu Asn Arg Glu Val Asp Asp Leu Ser Asp Arg Leu Ser Gly
    50                  55                  60


Leu Met Ala Thr Leu Asp Ser Asn Ser Ala Glu Ala Glu Ile Ile Arg
65                  70                  75                  80


Ser Leu Leu Pro Ser
```

85

<210> 194
<211> 535
<212> DNA
<213> Zingiber officinale

<400> 194
ttctagattt aagatgtcga gccggaggaa cagggtctcg gaggaggaga tcaatgagct 60

catctccaaa cttcagtctc tcctcccgga aacccgccgc cggggcgctg gccgggcgtc 120

cgcggcgaag ttgctgaagg agacgtgcag ctacatcagg agcctgaaca gggaggtgga 180

cgacctcagc gacaggctct cggggctcat ggcgacgctg gacagcaaca gcgccgaggc 240

ggagatcatc cggagcctgc tcccctcctg attcaaattc ctgatcgtca gttaggactt 300

actccagacg taaagatata tgtagtgtac gtacctctgt tctgaaagct taggagttag 360

gacgacgaag ctagcagcga ttttccttta ctcgaagcct gattcgagtt gttttctctg 420

tagacaaccg accgacacat ctgcttaaaa aaatcagtaa tgcttcccat gtaatcgagg 480

ttcgagatgt agtgtcgtat tttactatcc actgtccgtc tcttgttctt acgtg 535

<210> 195
<211> 85
<212> PRT
<213> Zingiber officinale

<400> 195

Met Ser Ser Gln Arg Gly Arg Ile Thr Asp Lys Glu Ile His Glu Leu
1 5 10 15

Val Ser Ser Leu Gln Ala Leu Leu Pro Glu Ser Arg Arg Arg Ser Thr
20 25 30

Ser Arg Ala Ser Ser Ser Lys Leu Leu Lys Glu Thr Cys Ser Tyr Ile
35 40 45

Arg Ser Leu Gln Arg Glu Val Asp Asp Leu Ser Gly Arg Leu Ala Glu
50 55 60

Leu Met Ser Thr Met Asp Ser Asp Ser Pro Gln Ala Glu Ile Ile Arg
65 70 75 80

Ser Ile Phe Arg Ser
85

<210> 196
<211> 449
<212> DNA
<213> Zingiber officinale

<400> 196
gcacccttttt tcctctccgc aaacacatcc tcagattact gcgcgcgcta gaatgtcgag 60

ccagagagga aggattactg acaaggaaat ccacgagctc gtctcctcgc tgcaggctct 120

tctcccggag tctcgccgca ggagcacgag tagggcatca tcatccaagt tgctgaagga 180

gacatgcagc tacatcagga gcttgcagcg ggaggtggac gacctcagcg gccggctcgc 240

cgagttgatg tcgacgatgg actccgacag ccctcaggct gagatcatta ggagcatctt 300

ccggtcctaa ataataacta tatatagtca tctttgtacc atttcatcag cctattagct 360

agtgttatat ataagcatgc agaattaata ctgctgctgc tgcttcttct tcttgatgcc 420

atcgatgcga tctagcgagc aataaagtt 449

<210> 197
<211> 483
<212> DNA
<213> Arabidopsis thaliana

<400> 197
atggctagtg gaatcgctcg tggtcgttta gctgaagaga ggaaatcgtg gaggaagaat 60

catcctcatg gttttgtggc aaagccggag acggggcagg atggaactgt gaatctaatg 120

gtgtggcatt gcactatacc tggtaaagct gggactgatt gggaaggtgg attctttcca 180

ttaacgatgc acttcagtga ggattatccg agcaaacctc cgaaatgtaa atttccacaa 240

gggtttttcc accctaatgt ctatccatct ggaactgtct gtctctctat ccttaacgag 300

gattatggat ggagaccagc catcaccgtg aagcagattc ttgttggtat tcaggattta 360

cttgacacac cgaatcccgc tgaccctgca cagacagatg gttatcatct cttctgtcag 420

gatccagttg agtacaagaa aagggtgaag ctgcagtcca agcagtatcc tgctcttgtc 480

taa 483

<210> 198
<211> 160
<212> PRT
<213> Arabidopsis thaliana

<400> 198

Met Ala Ser Gly Ile Ala Arg Gly Arg Leu Ala Glu Glu Arg Lys Ser
1 5 10 15

Trp Arg Lys Asn His Pro His Gly Phe Val Ala Lys Pro Glu Thr Gly
20 25 30

Gln Asp Gly Thr Val Asn Leu Met Val Trp His Cys Thr Ile Pro Gly
35 40 45

```
Lys Ala Gly Thr Asp Trp Glu Gly Gly Phe Phe Pro Leu Thr Met His
    50                  55                  60

Phe Ser Glu Asp Tyr Pro Ser Lys Pro Pro Lys Cys Lys Phe Pro Gln
65                  70                  75                  80

Gly Phe Phe His Pro Asn Val Tyr Pro Ser Gly Thr Val Cys Leu Ser
                85                  90                  95

Ile Leu Asn Glu Asp Tyr Gly Trp Arg Pro Ala Ile Thr Val Lys Gln
            100                 105                 110

Ile Leu Val Gly Ile Gln Asp Leu Leu Asp Thr Pro Asn Pro Ala Asp
        115                 120                 125

Pro Ala Gln Thr Asp Gly Tyr His Leu Phe Cys Gln Asp Pro Val Glu
    130                 135                 140

Tyr Lys Lys Arg Val Lys Leu Gln Ser Lys Gln Tyr Pro Ala Leu Val
145                 150                 155                 160


<210>  199
<211>  483
<212>  DNA
<213>  Helianus annuus

<400>  199
atgtccggtg gaattgctcg cggccgtctc accgaggaac gcaaagcatg gcgcaagaat      60

catcctcatg gttttgtggc gaaaccggag actctacctg gcggtacggt taatttgatg     120

atttggagtt gtatcatccc tggtaagaat gggaccgact gggagggcgg tttttacccc     180

cttaccctcc acttcaccga ggattatccg agcaaaccac caaagtgtaa attccctcaa     240

ggcttcttcc accccaatgt ttacccttct gggaccgttt gtttgtccat ccttaacgaa     300

gatagtggtt ggaggccagc aataacagtt aaacaaattc tagttggcat ccaggacttg     360

ctggattccc ccaatcccgc tgatcccgcc cagactgatg gatatcatct ctttatccag     420

gacacggtgg agtacaagag acgggtccgc cagcaagcaa agcaataccc agcactcgtg     480

tag                                                                   483


<210>  200
<211>  160
<212>  PRT
<213>  Helianus annuus

<400>  200

Met Ser Gly Gly Ile Ala Arg Gly Arg Leu Thr Glu Glu Arg Lys Ala
1               5                   10                  15
```

```
Trp Arg Lys Asn His Pro His Gly Phe Val Ala Lys Pro Glu Thr Leu
            20                  25                  30

Pro Gly Gly Thr Val Asn Leu Met Ile Trp Ser Cys Ile Ile Pro Gly
        35                  40                  45

Lys Asn Gly Thr Asp Trp Glu Gly Gly Phe Tyr Pro Leu Thr Leu His
    50                  55                  60

Phe Thr Glu Asp Tyr Pro Ser Lys Pro Pro Lys Cys Lys Phe Pro Gln
65                  70                  75                  80

Gly Phe Phe His Pro Asn Val Tyr Pro Ser Gly Thr Val Cys Leu Ser
                85                  90                  95

Ile Leu Asn Glu Asp Ser Gly Trp Arg Pro Ala Ile Thr Val Lys Gln
            100                 105                 110

Ile Leu Val Gly Ile Gln Asp Leu Leu Asp Ser Pro Asn Pro Ala Asp
        115                 120                 125

Pro Ala Gln Thr Asp Gly Tyr His Leu Phe Ile Gln Asp Thr Val Glu
    130                 135                 140

Tyr Lys Arg Arg Val Arg Gln Gln Ala Lys Gln Tyr Pro Ala Leu Val
145                 150                 155                 160


<210>  201
<211>  1119
<212>  DNA
<213>  Triticum aestivum


<220>
<221>  misc_feature
<222>  (873)..(873)
<223>  n is a, c, g, or t

<400>  201
aaaaaaagtc gagaagacac aatcaaggag tcaagttcaa aagtatccac acatccatca      60

ttcaagcaga aggacctgat ttttgtttgg tcaacaccag accacacgtc acaaatgtcc     120

acggtcacaa tggaaagctc tccacaatac cccgctttcg gttatattta gtcatacttc     180

atattttcag gtgcggtaat ttatgatttt agactgctac atggatcctc aaaccagagc     240

agggtactgc ttggcctgca gccgaatgcg tctcttgtac tctgctggat cctggataaa     300

gaggtgataa ccatcagtct gggcaggatc agctggatta ggttgatcaa gcaaatcctg     360

tattccaact agaatctgct taacagtgat ggcaggtctc caaccactat cctcattaag     420
```

aatcgagagg cagaccgtcc ctgaaggata gacatttggg tggaaaaaac cctgcgggaa 480

cttgcacttg ggaggtttgc tagggtagtc ctcactgaaa tggagagtaa gtgggtagta 540

tccactttcc caatcagtcc cctgctttcc ggggatggtg cagttccaga tcatgagatt 600

caccgacccg tcggccaccg tctccggctt cgcgacgaat ccatgggggt ggttcttgcg 660

ccaggccttg cgctcctccg cgaggcggcc ccgcgcgatc cctcctcctg acatgggcgg 720

cggcggagga ggctgctggc tgctctctgt cgctgagtct ggggtttggg tttcggagtc 780

tgcgcagttt ctaatccttc agtccatgtc agtgtcctcg tgctccagtc gcggacgcgt 840

tgggtcaaga ttttccggga atttcgggac cgntaccagc ctggtttttt tgttacaaac 900

tggttctata ggtgtcacta aataggccta atggtcatac ctggttcctg tgtgaaaatg 960

ttatcggccc gggggctaag gtaaagttcg gaggttggat ggtcccgggg ttttctatgg 1020

aggtcaaaac agggtgattg ggtttccggg gaactgacgg ttactaaacg agcttgtttt 1080

tttaaactcc acgggtaacc cttccgccct atttgttaa 1119

<210> 202
<211> 161
<212> PRT
<213> Triticum aestivum

<400> 202

Met Ser Gly Gly Gly Ile Ala Arg Gly Arg Leu Ala Glu Glu Arg Lys
1 5 10 15

Ala Trp Arg Lys Asn His Pro His Gly Phe Val Ala Lys Pro Glu Thr
20 25 30

Val Ala Asp Gly Ser Val Asn Leu Met Ile Trp Asn Cys Thr Ile Pro
35 40 45

Gly Lys Gln Gly Thr Asp Trp Glu Ser Gly Tyr Tyr Pro Leu Thr Leu
50 55 60

His Phe Ser Glu Asp Tyr Pro Ser Lys Pro Pro Lys Cys Lys Phe Pro
65 70 75 80

Gln Gly Phe Phe His Pro Asn Val Tyr Pro Ser Gly Thr Val Cys Leu
85 90 95

Ser Ile Leu Asn Glu Asp Ser Gly Trp Arg Pro Ala Ile Thr Val Lys
100 105 110

Gln Ile Leu Val Gly Ile Gln Asp Leu Leu Asp Gln Pro Asn Pro Ala
115 120 125

Asp Pro Ala Gln Thr Asp Gly Tyr His Leu Phe Ile Gln Asp Pro Ala
    130                 135                 140

Glu Tyr Lys Arg Arg Ile Arg Leu Gln Ala Lys Gln Tyr Pro Ala Leu
145                 150                 155                 160

Val

<210> 203
<211> 486
<212> DNA
<213> Hordeum vulgare

<400> 203
atgtcaggag gagggatcgc ccgcggccgc ctcgcggagg agcgcaaggc ctggcgcaag 60

aaccaccccc atggattcgt cgcgaagccg gagacgatgg ccgacgggtc ggtgaatctc 120

atgatctgga actgcaccat ccccggaaag caggggactg attgggaaag tggatactac 180

ccacttaccc tccatttcag tgaggactac cctagtaaac ctcccaaatg caagttcccg 240

cagggttttt tccacccaaa tgtctatcct tcagggacgg tctgcctctc gattcttaat 300

gaggatagcg gttggagacc tgccatcact gttaagcaga tcctagttgg aatacaggat 360

ttgcttgatc aacctaatcc agctgatcct gcccagactg atggttatca cctctttatc 420

caggatccag cagagtatag gaggcgtatt cggctgcagg ctaagcagta ccctgctctg 480

gtttga 486

<210> 204
<211> 161
<212> PRT
<213> Hordeum vulgare

<400> 204

Met Ser Gly Gly Gly Ile Ala Arg Gly Arg Leu Ala Glu Glu Arg Lys
1               5                   10                  15

Ala Trp Arg Lys Asn His Pro His Gly Phe Val Ala Lys Pro Glu Thr
            20                  25                  30

Met Ala Asp Gly Ser Val Asn Leu Met Ile Trp Asn Cys Thr Ile Pro
        35                  40                  45

Gly Lys Gln Gly Thr Asp Trp Glu Ser Gly Tyr Tyr Pro Leu Thr Leu
    50                  55                  60

His Phe Ser Glu Asp Tyr Pro Ser Lys Pro Pro Lys Cys Lys Phe Pro
65                  70                  75                  80

Gln Gly Phe Phe His Pro Asn Val Tyr Pro Ser Gly Thr Val Cys Leu
85 90 95

Ser Ile Leu Asn Glu Asp Ser Gly Trp Arg Pro Ala Ile Thr Val Lys
100 105 110

Gln Ile Leu Val Gly Ile Gln Asp Leu Leu Asp Gln Pro Asn Pro Ala
115 120 125

Asp Pro Ala Gln Thr Asp Gly Tyr His Leu Phe Ile Gln Asp Pro Ala
130 135 140

Glu Tyr Arg Arg Arg Ile Arg Leu Gln Ala Lys Gln Tyr Pro Ala Leu
145 150 155 160

Val

<210> 205
<211> 480
<212> DNA
<213> Glycine max

<400> 205
atgtctggta tcgcccgtgg acgtcttgcc gaggagcgaa agtcatggcg caaaaaccac 60

cctcatggtt tcgttgccaa gcccgagact ctccctgatg ccaccgttaa tttgatggtc 120

tggcattgca ctattcctgg caaggctggg actgattggg agggtggata tttcccactg 180

acaatgcact tcagtgaaga ttaccctagc aagcctccca agtgcaaatt ccctcaaggt 240

ttctttcacc ccaatgtgta tccatctgga actgtttgct tgtctatact taatgaagat 300

agtggatgga gaccagctat aacagtgaag caaattcttg tgggcatcca ggacctgctt 360

gatcaaccaa atcctgctga ccctgcccag acggagggtt atcatctatt catccaggat 420

gcagctgagt acaagagaag agtccgacag cagtcaaagc aatatccacc tcttgtctag 480

<210> 206
<211> 159
<212> PRT
<213> Glycine max

<400> 206

Met Ser Gly Ile Ala Arg Gly Arg Leu Ala Glu Glu Arg Lys Ser Trp
1 5 10 15

Arg Lys Asn His Pro His Gly Phe Val Ala Lys Pro Glu Thr Leu Pro
20 25 30

```
Asp Ala Thr Val Asn Leu Met Val Trp His Cys Thr Ile Pro Gly Lys
        35                  40                  45

Ala Gly Thr Asp Trp Glu Gly Gly Tyr Phe Pro Leu Thr Met His Phe
    50                  55                  60

Ser Glu Asp Tyr Pro Ser Lys Pro Pro Lys Cys Lys Phe Pro Gln Gly
65                  70                  75                  80

Phe Phe His Pro Asn Val Tyr Pro Ser Gly Thr Val Cys Leu Ser Ile
                85                  90                  95

Leu Asn Glu Asp Ser Gly Trp Arg Pro Ala Ile Thr Val Lys Gln Ile
            100                 105                 110

Leu Val Gly Ile Gln Asp Leu Leu Asp Gln Pro Asn Pro Ala Asp Pro
        115                 120                 125

Ala Gln Thr Glu Gly Tyr His Leu Phe Ile Gln Asp Ala Ala Glu Tyr
    130                 135                 140

Lys Arg Arg Val Arg Gln Gln Ser Lys Gln Tyr Pro Pro Leu Val
145                 150                 155
```

<210> 207
<211> 483
<212> DNA
<213> Zea mays

<400> 207

```
atgtcgggag gaatcgcgcg cggccgcctc gccgaggagc gcaaggcctg gcgcaagaac     60

cacccccacg gtttcgtggc gaggccggaa acgctggccg acgggtcggc gaacctcatg    120

gtctggagct gtaccatccc cggcaagcag gggactgatt gggaaagtgg gtactaccca    180

cttacccttc atttcagtga agattaccca agcaagcctc ccaaatgcaa gttcccacag    240

ggtttttccc acccaaatgt ttatccttca ggaacagtct gcctctcaat tctcaatgag    300

gatagtggtt ggagacctgc tatcactgtt aagcagattc tagttggaat acaagacttg    360

cttgatcagc ccaatccagc tgatcctgcc caaactgatg gttatcacct attcatccag    420

gatccaacag aatataagcg acgtgttcgt ctgcaggcca agcaatatcc tgctctggtc    480

tga                                                                  483
```

<210> 208
<211> 160
<212> PRT
<213> Zea mays

<400> 208

Met Ser Gly Gly Ile Ala Arg Gly Arg Leu Ala Glu Glu Arg Lys Ala
1 5 10 15

Trp Arg Lys Asn His Pro His Gly Phe Val Ala Arg Pro Glu Thr Leu
20 25 30

Ala Asp Gly Ser Ala Asn Leu Met Val Trp Ser Cys Thr Ile Pro Gly
35 40 45

Lys Gln Gly Thr Asp Trp Glu Ser Gly Tyr Tyr Pro Leu Thr Leu His
50 55 60

Phe Ser Glu Asp Tyr Pro Ser Lys Pro Pro Lys Cys Lys Phe Pro Gln
65 70 75 80

Gly Phe Phe His Pro Asn Val Tyr Pro Ser Gly Thr Val Cys Leu Ser
85 90 95

Ile Leu Asn Glu Asp Ser Gly Trp Arg Pro Ala Ile Thr Val Lys Gln
100 105 110

Ile Leu Val Gly Ile Gln Asp Leu Leu Asp Gln Pro Asn Pro Ala Asp
115 120 125

Pro Ala Gln Thr Asp Gly Tyr His Leu Phe Ile Gln Asp Pro Thr Glu
130 135 140

Tyr Lys Arg Arg Val Arg Leu Gln Ala Lys Gln Tyr Pro Ala Leu Val
145 150 155 160

<210> 209
<211> 483
<212> DNA
<213> Zea mays

<400> 209
atgtctggag ggatcgcgcg cggccgcctc gccgaggagc gcaaggcctg gcgcaagaat 60

cacccccacg gtttcgtggc gaggccggag tcgctgaccg acgggtccgt gaacctcatg 120

gtctggaact gtaccatccc cggcaagcac gggaccgatt gggaaggtgg gtactaccca 180

cttacccttc atttcagtga agattaccca agcaaacctc ccaaatgcaa gtttccacag 240

ggtttttcc atccaaatgt ttatccatca ggaacagtct gcctctcaat tctgaacgag 300

gatagcgatt ggagacctgc tatcactgtt aagcagattc tagttggaat acaggacttg 360

cttgatcagc ccaatccagc tgatcctgct cagactgatg gttatcacct attcatccag 420

gatcctgcag aatataagcg acgtgttcgt ctgcaggcca agcaatatcc tgctctggtc 480

tga 483

<210> 210
<211> 160
<212> PRT
<213> Zea mays

<400> 210

Met Ser Gly Gly Ile Ala Arg Gly Arg Leu Ala Glu Glu Arg Lys Ala
1               5                   10                  15

Trp Arg Lys Asn His Pro His Gly Phe Val Ala Arg Pro Glu Ser Leu
            20                  25                  30

Thr Asp Gly Ser Val Asn Leu Met Val Trp Asn Cys Thr Ile Pro Gly
        35                  40                  45

Lys His Gly Thr Asp Trp Glu Gly Gly Tyr Tyr Pro Leu Thr Leu His
    50                  55                  60

Phe Ser Glu Asp Tyr Pro Ser Lys Pro Pro Lys Cys Lys Phe Pro Gln
65                  70                  75                  80

Gly Phe Phe His Pro Asn Val Tyr Pro Ser Gly Thr Val Cys Leu Ser
                85                  90                  95

Ile Leu Asn Glu Asp Ser Asp Trp Arg Pro Ala Ile Thr Val Lys Gln
            100                 105                 110

Ile Leu Val Gly Ile Gln Asp Leu Leu Asp Gln Pro Asn Pro Ala Asp
        115                 120                 125

Pro Ala Gln Thr Asp Gly Tyr His Leu Phe Ile Gln Asp Pro Ala Glu
    130                 135                 140

Tyr Lys Arg Arg Val Arg Leu Gln Ala Lys Gln Tyr Pro Ala Leu Val
145                 150                 155                 160

<210> 211
<211> 483
<212> DNA
<213> Zea mays

<400> 211
atgtctgggg gaatcgcccg cggccgcctc gccgaggagc gcaaggcctg gcgcaagaac 60

cacccgcacg gtttcgtcgc gaagccggag tcgctgcccg acgggacggt gaacctgatg 120

atctggcagt gcaccatccc cggcaagcaa gggactgact gggaaggtgg atatttccct 180

ctcacccttc attttagtga ggattaccct agcaagcctc ccaagtgcaa gttccctcag 240

ggtttcttcc acccaaatgt gtatccttct ggaacagtct gtctttcgat ccttaatgaa 300

gatagtggtt ggagaccagc tattactgtt aagcagattc tcgtcgggat ccaggacttg 360

ctagatcagc caaatcctgc tgatcctgct caaacggatg gctatcacct ttttatccag 420

gatcctacag aatataagag gcgtgttaaa ctgcaggcga agcagtatcc cgcgttggtc 480

tga 483

<210> 212
<211> 160
<212> PRT
<213> Zea mays

<400> 212

Met Ser Gly Gly Ile Ala Arg Gly Arg Leu Ala Glu Glu Arg Lys Ala
1 5 10 15

Trp Arg Lys Asn His Pro His Gly Phe Val Ala Lys Pro Glu Ser Leu
20 25 30

Pro Asp Gly Thr Val Asn Leu Met Ile Trp Gln Cys Thr Ile Pro Gly
35 40 45

Lys Gln Gly Thr Asp Trp Glu Gly Gly Tyr Phe Pro Leu Thr Leu His
50 55 60

Phe Ser Glu Asp Tyr Pro Ser Lys Pro Pro Lys Cys Lys Phe Pro Gln
65 70 75 80

Gly Phe Phe His Pro Asn Val Tyr Pro Ser Gly Thr Val Cys Leu Ser
85 90 95

Ile Leu Asn Glu Asp Ser Gly Trp Arg Pro Ala Ile Thr Val Lys Gln
100 105 110

Ile Leu Val Gly Ile Gln Asp Leu Leu Asp Gln Pro Asn Pro Ala Asp
115 120 125

Pro Ala Gln Thr Asp Gly Tyr His Leu Phe Ile Gln Asp Pro Thr Glu
130 135 140

Tyr Lys Arg Arg Val Lys Leu Gln Ala Lys Gln Tyr Pro Ala Leu Val
145 150 155 160

<210> 213
<211> 483
<212> DNA

<213>  Oryza sativa

<400>  213
atgtcggggg gaatcgcgcg cggccgcctc gcggaggagc ggaaggcgtg gcggaagaac    60

cacccacacg gtttcgtcgc caagccggag acgttggccg acgggacggt caacctcatg    120

atctggcact gcacaatccc cggcaagcaa gggactgatt gggaaggtgg atactttcct    180

ctcactcttc atttcagtga ggattaccct agcaaacctc ccaagtgcaa gttcccacag    240

ggtttcttcc acccaaatgt ctatccttca gggacagtct gcctttcaat tcttaatgaa    300

gacagcggtt ggagacctgc tattaccgtc aagcaaattc ttgttggaat ccaggacttg    360

cttgatcagc ctaatcctgc tgatcctgct cagaccgatg gttaccatct tttatccag    420

gatcctacgg aatacaagag gcgtgttcgg ctgcaggcca agcagtatcc tccgattgtc    480

tga    483

<210>  214
<211>  160
<212>  PRT
<213>  Oryza sativa

<400>  214

Met Ser Gly Gly Ile Ala Arg Gly Arg Leu Ala Glu Glu Arg Lys Ala
1               5                   10                  15

Trp Arg Lys Asn His Pro His Gly Phe Val Ala Lys Pro Glu Thr Leu
            20                  25                  30

Ala Asp Gly Thr Val Asn Leu Met Ile Trp His Cys Thr Ile Pro Gly
        35                  40                  45

Lys Gln Gly Thr Asp Trp Glu Gly Gly Tyr Phe Pro Leu Thr Leu His
    50                  55                  60

Phe Ser Glu Asp Tyr Pro Ser Lys Pro Pro Lys Cys Lys Phe Pro Gln
65                  70                  75                  80

Gly Phe Phe His Pro Asn Val Tyr Pro Ser Gly Thr Val Cys Leu Ser
                85                  90                  95

Ile Leu Asn Glu Asp Ser Gly Trp Arg Pro Ala Ile Thr Val Lys Gln
            100                 105                 110

Ile Leu Val Gly Ile Gln Asp Leu Leu Asp Gln Pro Asn Pro Ala Asp
        115                 120                 125

Pro Ala Gln Thr Asp Gly Tyr His Leu Phe Ile Gln Asp Pro Thr Glu
    130                 135                 140

```
Tyr Lys Arg Arg Val Arg Leu Gln Ala Lys Gln Tyr Pro Pro Ile Val
145                 150                 155                 160


<210>  215
<211>  483
<212>  DNA
<213>  Oryza sativa

<400>  215
atgtcgggag ggatcgcacg cggccgcctc gcggaggagc gcaaggcctg gcggaagaac      60

caccctcacg ggttcgtggc gaagccggag acgatggccg acgggtcggc gaacctcatg     120

atctggcact gcaccatccc cggcaagcag gggaccgatt gggaaggtgg gtactaccct     180

cttacccttc acttcagtga ggactatcct agcaaaccac ccaagtgcaa gttcccacag     240

ggctttttcc acccaaatgt ctatccttca ggaacagtgt gcctctcaat tcttaatgag     300

gatagtggct ggagacctgc tatcactgta aagcagatcc ttgttggaat acaggacttg     360

cttgatcagc caaatcctgc tgatcctgca cagactgacg gttatcacat tttatacag     420

gacaaaccag aatataagag gcgtgttcgt gttcaggcca agcagtaccc tgctttgctt     480

tga                                                                   483


<210>  216
<211>  160
<212>  PRT
<213>  Oryza sativa

<400>  216

Met Ser Gly Gly Ile Ala Arg Gly Arg Leu Ala Glu Glu Arg Lys Ala
1               5                   10                  15


Trp Arg Lys Asn His Pro His Gly Phe Val Ala Lys Pro Glu Thr Met
            20                  25                  30


Ala Asp Gly Ser Ala Asn Leu Met Ile Trp His Cys Thr Ile Pro Gly
        35                  40                  45


Lys Gln Gly Thr Asp Trp Glu Gly Gly Tyr Tyr Pro Leu Thr Leu His
    50                  55                  60


Phe Ser Glu Asp Tyr Pro Ser Lys Pro Pro Lys Cys Lys Phe Pro Gln
65                  70                  75                  80


Gly Phe Phe His Pro Asn Val Tyr Pro Ser Gly Thr Val Cys Leu Ser
                85                  90                  95


Ile Leu Asn Glu Asp Ser Gly Trp Arg Pro Ala Ile Thr Val Lys Gln
```

100 105 110

Ile Leu Val Gly Ile Gln Asp Leu Leu Asp Gln Pro Asn Pro Ala Asp
115 120 125

Pro Ala Gln Thr Asp Gly Tyr His Ile Phe Ile Gln Asp Lys Pro Glu
130 135 140

Tyr Lys Arg Arg Val Arg Val Gln Ala Lys Gln Tyr Pro Ala Leu Leu
145 150 155 160

<210> 217
<211> 402
<212> DNA
<213> Oryza sativa

<400> 217
atggtctggc gatgcatcat ccccggcaaa gaagggactg attgggaggg tggatatttc 60

ccacttacta tgcaattcac tgaagactat ccaaccaacg ctccttcttg caagttccca 120

tcgggtttct tccacatcaa tgtctatgac tctggggcag tatgcctatc aatcttgagt 180

accgcatgga aaccttcaat tacagtgagg caaattctta taggcatcca ggaattgttt 240

gatgatccaa accctaactc tgctgcacag aatataagct atgagcttta taggacatgg 300

aggagtacag gaaacgcgtt cgtcagcagg ctaagaagta tccttcagct ctgtagccgc 360

gcaatgcctg caggattctg gcagctaaaa cattttgatt ga 402

<210> 218
<211> 133
<212> PRT
<213> Oryza sativa

<400> 218

Met Val Trp Arg Cys Ile Ile Pro Gly Lys Glu Gly Thr Asp Trp Glu
1 5 10 15

Gly Gly Tyr Phe Pro Leu Thr Met Gln Phe Thr Glu Asp Tyr Pro Thr
20 25 30

Asn Ala Pro Ser Cys Lys Phe Pro Ser Gly Phe Phe His Ile Asn Val
35 40 45

Tyr Asp Ser Gly Ala Val Cys Leu Ser Ile Leu Ser Thr Ala Trp Lys
50 55 60

Pro Ser Ile Thr Val Arg Gln Ile Leu Ile Gly Ile Gln Glu Leu Phe
65 70 75 80

```
Asp Asp Pro Asn Pro Asn Ser Ala Ala Gln Asn Ile Ser Tyr Glu Leu
                85                  90                  95

Tyr Arg Thr Trp Arg Ser Thr Gly Asn Ala Phe Val Ser Arg Leu Arg
            100                 105                 110

Ser Ile Leu Gln Leu Cys Ser Arg Ala Met Pro Ala Gly Phe Trp Gln
        115                 120                 125

Leu Lys His Phe Asp
    130
```

<210> 219
<211> 483
<212> DNA
<213> Vitis vinifera

<400> 219

```
atgtcaggag gcatcgcgcg tggtcgtctc gccgaggagc gaaaagcctg gcgtaagaat      60

catccccatg gtttcgtggc taagccagag actggtccgg acggttctgt caatttgatg     120

gtgtggcatt gcaccatccc tggtaaggct gggactgatt gggaaggggg ctacttccca     180

cttactttgc acttcagtga ggactaccct agcaaacccc caaagtgcaa gttccctcaa     240

ggtttcttcc accctaatgt ctacccatct ggaactgtat gtctctcgat cctcaatgaa     300

gacagtggtt ggagacctgc cattacagtg aaacaaattc tagtgggcat tcaagacttg     360

ctggaccagc ccaatcctgc agatccagca caaactgatg ggtatcagct cttcatccag     420

gaacccgcag agtataaaag aagggtgcgg caacaggcca agcaatatcc acctcttgtc     480

taa                                                                   483
```

<210> 220
<211> 160
<212> PRT
<213> Vitis vinifera

<400> 220

```
Met Ser Gly Gly Ile Ala Arg Gly Arg Leu Ala Glu Glu Arg Lys Ala
1               5                   10                  15

Trp Arg Lys Asn His Pro His Gly Phe Val Ala Lys Pro Glu Thr Gly
            20                  25                  30

Pro Asp Gly Ser Val Asn Leu Met Val Trp His Cys Thr Ile Pro Gly
        35                  40                  45

Lys Ala Gly Thr Asp Trp Glu Gly Gly Tyr Phe Pro Leu Thr Leu His
    50                  55                  60
```

Phe Ser Glu Asp Tyr Pro Ser Lys Pro Pro Lys Cys Lys Phe Pro Gln
65 70 75 80

Gly Phe Phe His Pro Asn Val Tyr Pro Ser Gly Thr Val Cys Leu Ser
85 90 95

Ile Leu Asn Glu Asp Ser Gly Trp Arg Pro Ala Ile Thr Val Lys Gln
100 105 110

Ile Leu Val Gly Ile Gln Asp Leu Leu Asp Gln Pro Asn Pro Ala Asp
115 120 125

Pro Ala Gln Thr Asp Gly Tyr Gln Leu Phe Ile Gln Glu Pro Ala Glu
130 135 140

Tyr Lys Arg Arg Val Arg Gln Gln Ala Lys Gln Tyr Pro Pro Leu Val
145 150 155 160

<210> 221
<211> 483
<212> DNA
<213> Nicotiana benthamiana

<400> 221
atgtcaggag gtatagcccg tggccgtctt gcagaggagc gcaaagcttg gcgcaaaaat 60

cacccccatg ggtttgtagc aaagccagag acgctttcgg atgggtcagt taacttgatg 120

gtttggcact gcagtattcc tggtaaagca ggaacggact gggaaggcgg tttttatccg 180

gttacgatac acttcagtga agattatcct agcaaaccac ctaagtgcaa attcccacaa 240

ggcttcttcc atccgaatgt ctatccatca ggaacagttt gcttgtcgat cctcaacgaa 300

gatagcggtt ggagacctgc cattacagtg aaacagatac tggttggtat ccaagacttg 360

ttagatcagc caaaccctgc tgatcctgcc caaaccgaag ggtatcatct ctttattcag 420

gatgctattg agtacaagaa gcgggttagg ctgcaggcca agcagtatcc tcctctggtg 480

tag 483

<210> 222
<211> 160
<212> PRT
<213> Nicotiana benthamiana

<400> 222

Met Ser Gly Gly Ile Ala Arg Gly Arg Leu Ala Glu Glu Arg Lys Ala
1 5 10 15

Trp Arg Lys Asn His Pro His Gly Phe Val Ala Lys Pro Glu Thr Leu
20 25 30

```
Ser Asp Gly Ser Val Asn Leu Met Val Trp His Cys Ser Ile Pro Gly
        35                  40                  45

Lys Ala Gly Thr Asp Trp Glu Gly Gly Phe Tyr Pro Val Thr Ile His
    50                  55                  60

Phe Ser Glu Asp Tyr Pro Ser Lys Pro Pro Lys Cys Lys Phe Pro Gln
65                  70                  75                  80

Gly Phe Phe His Pro Asn Val Tyr Pro Ser Gly Thr Val Cys Leu Ser
                85                  90                  95

Ile Leu Asn Glu Asp Ser Gly Trp Arg Pro Ala Ile Thr Val Lys Gln
            100                 105                 110

Ile Leu Val Gly Ile Gln Asp Leu Leu Asp Gln Pro Asn Pro Ala Asp
        115                 120                 125

Pro Ala Gln Thr Glu Gly Tyr His Leu Phe Ile Gln Asp Ala Ile Glu
    130                 135                 140

Tyr Lys Lys Arg Val Arg Leu Gln Ala Lys Gln Tyr Pro Pro Leu Val
145                 150                 155                 160


<210>  223
<211>  483
<212>  DNA
<213>  Populus x canadensis

<400>  223
atgtcaggtg gcatcgcacg tggtcgtctt gctgaggaaa ggaagtcctg gcgcaaaaac      60

cacctcatg gttttgtggc gaaaccagag acacagccag atggaacagt aaatttgatg     120

gtctggcatt gcacaatccc tggaaaactt ggtactgatt gggaaggtgg ttattttcct     180

cttacactca acttcagtga agattatcct agcaagccac caaagtgtaa atttcctcag     240

ggtttcttcc accctaatgt atatccatct ggaactgttt gcttgtcaat ccttaacgag     300

gacagtggat ggagaccagc catcacagtg aagcagattc ttgtgggtat ccaggacttg     360

ctggaccagc caaatcctgc tgatcctgcc caaactgaag gttatcatct gtttatccag     420

gatgctgcag agtacaagaa aagagttcgc cagcaagcta agcaataccc ttctcttgtc     480

taa                                                                   483


<210>  224
<211>  160
<212>  PRT
<213>  Populus x canadensis
```

<400> 224

Met Ser Gly Gly Ile Ala Arg Gly Arg Leu Ala Glu Glu Arg Lys Ser
1 5 10 15

Trp Arg Lys Asn His Pro His Gly Phe Val Ala Lys Pro Glu Thr Gln
20 25 30

Pro Asp Gly Thr Val Asn Leu Met Val Trp His Cys Thr Ile Pro Gly
35 40 45

Lys Leu Gly Thr Asp Trp Glu Gly Gly Tyr Phe Pro Leu Thr Leu Asn
50 55 60

Phe Ser Glu Asp Tyr Pro Ser Lys Pro Pro Lys Cys Lys Phe Pro Gln
65 70 75 80

Gly Phe Phe His Pro Asn Val Tyr Pro Ser Gly Thr Val Cys Leu Ser
85 90 95

Ile Leu Asn Glu Asp Ser Gly Trp Arg Pro Ala Ile Thr Val Lys Gln
100 105 110

Ile Leu Val Gly Ile Gln Asp Leu Leu Asp Gln Pro Asn Pro Ala Asp
115 120 125

Pro Ala Gln Thr Glu Gly Tyr His Leu Phe Ile Gln Asp Ala Ala Glu
130 135 140

Tyr Lys Lys Arg Val Arg Gln Gln Ala Lys Gln Tyr Pro Ser Leu Val
145 150 155 160

<210> 225
<211> 486
<212> DNA
<213> Triticum turgidum

<400> 225
atgtcttccg gtgggatcgc gcgcggccgc ctcgcggagg agcgcaaggc ctggcggaag 60

aaccaccccc acggcttcgt cgccaagccg gagacgctgg gcgacggcac ggtcaacctc 120

atggtctggc actgcaccat ccccggcaag caagggactg attgggaagg tggatacttc 180

cctctcaccc ttcatttcag cgaggattac cccagcaagc ctcccaagtg caagttccct 240

acaaatttct tccacccgaa tgtctatcct tcggggacag tctgcctttc aatcctcaat 300

gaggacagcg gctggagacc tgctattact gtgaagcaaa tccttgttgg aattcaggac 360

ttgcttgatc agcccaaccc ggctgaccct gctcagactg atggttatca ccttttcatc 420

caggatccag ctgagtacaa gaggcgtgtt cgggcgcagg caaagcagta tcccgcattg 480

gtctga 486

<210> 226
<211> 161
<212> PRT
<213> Triticum turgidum

<400> 226

Met Ser Ser Gly Gly Ile Ala Arg Gly Arg Leu Ala Glu Glu Arg Lys
1 5 10 15

Ala Trp Arg Lys Asn His Pro His Gly Phe Val Ala Lys Pro Glu Thr
20 25 30

Leu Gly Asp Gly Thr Val Asn Leu Met Val Trp His Cys Thr Ile Pro
35 40 45

Gly Lys Gln Gly Thr Asp Trp Glu Gly Gly Tyr Phe Pro Leu Thr Leu
50 55 60

His Phe Ser Glu Asp Tyr Pro Ser Lys Pro Pro Lys Cys Lys Phe Pro
65 70 75 80

Thr Asn Phe Phe His Pro Asn Val Tyr Pro Ser Gly Thr Val Cys Leu
85 90 95

Ser Ile Leu Asn Glu Asp Ser Gly Trp Arg Pro Ala Ile Thr Val Lys
100 105 110

Gln Ile Leu Val Gly Ile Gln Asp Leu Leu Asp Gln Pro Asn Pro Ala
115 120 125

Asp Pro Ala Gln Thr Asp Gly Tyr His Leu Phe Ile Gln Asp Pro Ala
130 135 140

Glu Tyr Lys Arg Arg Val Arg Ala Gln Ala Lys Gln Tyr Pro Ala Leu
145 150 155 160

Val

<210> 227
<211> 486
<212> DNA
<213> Populus trichocarpa

<400> 227
atgtcaggag gaggcatagc tcgtggtcgt cttgctgaag agagaaagtc atggcgtaag 60

aatcatcccc acggttttgt ggctaagcct gataatgcac aagatggttc tcttgatttg 120

atggtgtgga agtgcatcat acctggcaaa cccgggacag attgggaggg tggcttcttc 180

cccctctcgc ttcatttcag tgaggactac ccaagcaaac ctccaaagtg caagttcccc 240

caaggtttct tccaccctaa tgtctaccct tcaggaactg tgtgcttatc tattctcaat 300

gaggactatg gctggagacc agccattact gtgaagcaaa ttttagttgg cattcaggat 360

ttgcttgatc aaccaaatcc ttctgatcct gcgcaaactg atggctatca gctttttgtc 420

caggacccga ctgagtacag gagaagggtg cgccaacaag ccaagcaata tccacctgcg 480

ctctga 486

<210> 228
<211> 161
<212> PRT
<213> Populus trichocarpa

<400> 228

Met Ser Gly Gly Gly Ile Ala Arg Gly Arg Leu Ala Glu Glu Arg Lys
1 5 10 15

Ser Trp Arg Lys Asn His Pro His Gly Phe Val Ala Lys Pro Asp Asn
20 25 30

Ala Gln Asp Gly Ser Leu Asp Leu Met Val Trp Lys Cys Ile Ile Pro
35 40 45

Gly Lys Pro Gly Thr Asp Trp Glu Gly Gly Phe Phe Pro Leu Ser Leu
50 55 60

His Phe Ser Glu Asp Tyr Pro Ser Lys Pro Pro Lys Cys Lys Phe Pro
65 70 75 80

Gln Gly Phe Phe His Pro Asn Val Tyr Pro Ser Gly Thr Val Cys Leu
85 90 95

Ser Ile Leu Asn Glu Asp Tyr Gly Trp Arg Pro Ala Ile Thr Val Lys
100 105 110

Gln Ile Leu Val Gly Ile Gln Asp Leu Leu Asp Gln Pro Asn Pro Ser
115 120 125

Asp Pro Ala Gln Thr Asp Gly Tyr Gln Leu Phe Val Gln Asp Pro Thr
130 135 140

Glu Tyr Arg Arg Arg Val Arg Gln Gln Ala Lys Gln Tyr Pro Pro Ala
145 150 155 160

Leu

<210> 229
<211> 486
<212> DNA
<213> Populus trichocarpa

<400> 229
atgtcaggag gaggcatagc tcgtggtcgt cttgctgaag agagaaaggc atggcgtaag 60

aatcaccctc acggttttgt ggctaagcct gataatgctc cagatggttc tctagatttg 120

atgatgtgga agtgcattat acctggcaaa cccgggactg attgggaggg tggctacttc 180

cccctcactc ttcatttcag tgaggactac ccaagcaaac ctccaaagtg caagttcccc 240

caaggtttct tccaccctaa tgtctaccct tcaggaactg tgtgcttatc tatcctcaat 300

gaggactatg gctggagacc agccattaca gtgaagcaaa tattaattgg cattcaggat 360

ttgcttgatc aaccaaatcc ttctgatcct gcacaaactg atggctatca gctttttgtc 420

caggaccctg ctgagtacag gagaagggtg cgccaacaag ccaagctata cccacctacg 480

ctctga 486

<210> 230
<211> 161
<212> PRT
<213> Populus trichocarpa

<400> 230

Met Ser Gly Gly Gly Ile Ala Arg Gly Arg Leu Ala Glu Glu Arg Lys
1               5                   10                  15

Ala Trp Arg Lys Asn His Pro His Gly Phe Val Ala Lys Pro Asp Asn
            20                  25                  30

Ala Pro Asp Gly Ser Leu Asp Leu Met Met Trp Lys Cys Ile Ile Pro
        35                  40                  45

Gly Lys Pro Gly Thr Asp Trp Glu Gly Gly Tyr Phe Pro Leu Thr Leu
    50                  55                  60

His Phe Ser Glu Asp Tyr Pro Ser Lys Pro Pro Lys Cys Lys Phe Pro
65                  70                  75                  80

Gln Gly Phe Phe His Pro Asn Val Tyr Pro Ser Gly Thr Val Cys Leu
                85                  90                  95

Ser Ile Leu Asn Glu Asp Tyr Gly Trp Arg Pro Ala Ile Thr Val Lys

100 105 110

Gln Ile Leu Ile Gly Ile Gln Asp Leu Leu Asp Gln Pro Asn Pro Ser
115 120 125

Asp Pro Ala Gln Thr Asp Gly Tyr Gln Leu Phe Val Gln Asp Pro Ala
130 135 140

Glu Tyr Arg Arg Arg Val Arg Gln Gln Ala Lys Leu Tyr Pro Pro Thr
145 150 155 160

Leu

<210> 231
<211> 483
<212> DNA
<213> Physcomitrella patens

<400> 231
atgtcaggag gaatcgcacg cggtcgtctt gcggaggagc gcaaggcctg gcgcaagaat 60

catcctcatg gatttgtagc gaggccggag acaggtgcag atggagctct aaatttgatg 120

gtttggcagt gcactttgcc tggaaaagtt gggacggact gggaaggtgg attttaccct 180

gtagcaattc acttcagtga ggattatccc agcaagcccc ccaagtgcaa gtttccacag 240

ggtttttttcc accccaacgt ttatccttca ggcacagttt gcctatccat ccttaatgaa 300

gattctggtt ggagaccagc tatcactgta aaacagatcc ttgtgggtat tcaggagctt 360

ctcgacgctc cgaacccagc agatcccgct caaaccgaag cctatcagct ttttattcaa 420

gatccagttg aatacaagcg tcgtgttagg cagcaagcca agcagtaccc accgccaatt 480

taa 483

<210> 232
<211> 160
<212> PRT
<213> Physcomitrella patens

<400> 232

Met Ser Gly Gly Ile Ala Arg Gly Arg Leu Ala Glu Glu Arg Lys Ala
1 5 10 15

Trp Arg Lys Asn His Pro His Gly Phe Val Ala Arg Pro Glu Thr Gly
20 25 30

Ala Asp Gly Ala Leu Asn Leu Met Val Trp Gln Cys Thr Leu Pro Gly
35 40 45

```
Lys Val Gly Thr Asp Trp Glu Gly Gly Phe Tyr Pro Val Ala Ile His
    50                  55                  60

Phe Ser Glu Asp Tyr Pro Ser Lys Pro Pro Lys Cys Lys Phe Pro Gln
65                  70                  75                  80

Gly Phe Phe His Pro Asn Val Tyr Pro Ser Gly Thr Val Cys Leu Ser
                85                  90                  95

Ile Leu Asn Glu Asp Ser Gly Trp Arg Pro Ala Ile Thr Val Lys Gln
            100                 105                 110

Ile Leu Val Gly Ile Gln Glu Leu Leu Asp Ala Pro Asn Pro Ala Asp
        115                 120                 125

Pro Ala Gln Thr Glu Ala Tyr Gln Leu Phe Ile Gln Asp Pro Val Glu
    130                 135                 140

Tyr Lys Arg Arg Val Arg Gln Gln Ala Lys Gln Tyr Pro Pro Pro Ile
145                 150                 155                 160
```

<210> 233
<211> 483
<212> DNA
<213> Physcomitrella patens

<400> 233

```
atgtcgggag gaattgcgcg gggtcgactt gcggaggagc gcaaggcctg gcggaagaat      60

catcctcatg ggtttgtggc taggcctgag acatgtgcag atggagctct taatttgatg     120

gtttggcagt gtactttacc tggaaaagtt gggaccgact gggaaggtgg attctatcct     180

gtagcaattc actttactga agattatccc agcaagcctc ctaagtgcaa attcccacag     240

ggtttcttcc accccaacgt gtatccttca ggcacagttt gcctctccat cctgaatgaa     300

gattcgggtt ggagaccagc tatcaccgtg aagcagatcc tcgtcggtat ccaggagctg     360

ctcgacgctc caaacccagc agatcccgct cagactgaag cttatcagct tttattcag      420

gatccagttg aatacaagcg acgagtaagg cagcaagcca agcaataccc accaccaatc     480

taa                                                                   483
```

<210> 234
<211> 160
<212> PRT
<213> Physcomitrella patens

<400> 234

```
Met Ser Gly Gly Ile Ala Arg Gly Arg Leu Ala Glu Glu Arg Lys Ala
1               5                   10                  15
```

```
Trp Arg Lys Asn His Pro His Gly Phe Val Ala Arg Pro Glu Thr Cys
            20                  25                  30

Ala Asp Gly Ala Leu Asn Leu Met Val Trp Gln Cys Thr Leu Pro Gly
        35                  40                  45

Lys Val Gly Thr Asp Trp Glu Gly Gly Phe Tyr Pro Val Ala Ile His
    50                  55                  60

Phe Thr Glu Asp Tyr Pro Ser Lys Pro Pro Lys Cys Lys Phe Pro Gln
65                  70                  75                  80

Gly Phe Phe His Pro Asn Val Tyr Pro Ser Gly Thr Val Cys Leu Ser
                85                  90                  95

Ile Leu Asn Glu Asp Ser Gly Trp Arg Pro Ala Ile Thr Val Lys Gln
            100                 105                 110

Ile Leu Val Gly Ile Gln Glu Leu Leu Asp Ala Pro Asn Pro Ala Asp
        115                 120                 125

Pro Ala Gln Thr Glu Ala Tyr Gln Leu Phe Ile Gln Asp Pro Val Glu
    130                 135                 140

Tyr Lys Arg Arg Val Arg Gln Gln Ala Lys Gln Tyr Pro Pro Pro Ile
145                 150                 155                 160
```

<210> 235
<211> 480
<212> DNA
<213> Chlamydomonas reinhardtii

<400> 235

```
atgtctggcg tcgcacgctc acgcttgcaa gaggagcgga aagcctggcg gagggataag     60

ccgttcggct tccatgctcg accagaaacc gcagacgacg ggagcgtgaa cctgatgaag    120

tggaagtgcc acatccccgg gaaacaaggc acggactggg agggcggctt ctacccgctc    180

accatggagt tcagcgagga ctaccccacc aagccgccca agtgcaagtt ccccgcgggc    240

ttcttccacc ccaacatcta cccctccggt accgtgtgcc tcagcatcct caacgaggac    300

gagggctggc ggccctccat caccatcaag cagctgctgt tgggcatcca ggagctgctg    360

gacacgccca accccggcag ccccgcccag tccgacgcct tcgtgctgtt cacgcagcag    420

aaggccgagt acgtcaagaa ggtgaagcgc caggcgctca actacccgcc accctcgtga    480
```

<210> 236
<211> 159
<212> PRT

<213> Chlamydomonas reinhardtii

<400> 236

```
Met Ser Gly Val Ala Arg Ser Arg Leu Gln Glu Glu Arg Lys Ala Trp
1               5                   10                  15

Arg Arg Asp Lys Pro Phe Gly Phe His Ala Arg Pro Glu Thr Ala Asp
            20                  25                  30

Asp Gly Ser Val Asn Leu Met Lys Trp Lys Cys His Ile Pro Gly Lys
        35                  40                  45

Gln Gly Thr Asp Trp Glu Gly Gly Phe Tyr Pro Leu Thr Met Glu Phe
    50                  55                  60

Ser Glu Asp Tyr Pro Thr Lys Pro Pro Lys Cys Lys Phe Pro Ala Gly
65                  70                  75                  80

Phe Phe His Pro Asn Ile Tyr Pro Ser Gly Thr Val Cys Leu Ser Ile
                85                  90                  95

Leu Asn Glu Asp Glu Gly Trp Arg Pro Ser Ile Thr Ile Lys Gln Leu
            100                 105                 110

Leu Leu Gly Ile Gln Glu Leu Leu Asp Thr Pro Asn Pro Gly Ser Pro
        115                 120                 125

Ala Gln Ser Asp Ala Phe Val Leu Phe Thr Gln Gln Lys Ala Glu Tyr
    130                 135                 140

Val Lys Lys Val Lys Arg Gln Ala Leu Asn Tyr Pro Pro Pro Ser
145                 150                 155
```

<210> 237
<211> 381
<212> DNA
<213> Prunus armeniaca

<400> 237

```
gggacggtga atttgatggt gtggcattgc acgattcctg gcaagaccgg tactgactgg      60

gaggggggtt ttttcccact tacccttcac ttcagtgaag actaccctag caagcctcca     120

aagtgtaaat tcccaccagg tttcttccac ccaaatgtat atccatctgg aactgtttgt     180

ctatcaattc ttaatgagga cagtggttgg agaccagcaa taaccgtgaa gcaaattctt     240

gtgggcattc aggatttact ggatcagcca aatcctgctg atcctgcaca gacagaaggg     300

tatcacctct tcattcagga tgccacggag tacaagaaaa gggttcggca gcaggccaag     360

caatacccac ctctagttta a                                               381
```

<210> 238
<211> 126
<212> PRT
<213> Prunus armeniaca

<400> 238

```
Gly Thr Val Asn Leu Met Val Trp His Cys Thr Ile Pro Gly Lys Thr
1               5                   10                  15

Gly Thr Asp Trp Glu Gly Gly Phe Phe Pro Leu Thr Leu His Phe Ser
            20                  25                  30

Glu Asp Tyr Pro Ser Lys Pro Pro Lys Cys Lys Phe Pro Pro Gly Phe
        35                  40                  45

Phe His Pro Asn Val Tyr Pro Ser Gly Thr Val Cys Leu Ser Ile Leu
    50                  55                  60

Asn Glu Asp Ser Gly Trp Arg Pro Ala Ile Thr Val Lys Gln Ile Leu
65                  70                  75                  80

Val Gly Ile Gln Asp Leu Leu Asp Gln Pro Asn Pro Ala Asp Pro Ala
                85                  90                  95

Gln Thr Glu Gly Tyr His Leu Phe Ile Gln Asp Ala Thr Glu Tyr Lys
            100                 105                 110

Lys Arg Val Arg Gln Gln Ala Lys Gln Tyr Pro Pro Leu Val
        115                 120                 125
```

<210> 239
<211> 480
<212> DNA
<213> Ostreococus tauri

<400> 239

```
atggcgtcgg tcgccctcgc gcgcctgggc gaggagcgcc gaaactggcg tcgcgaccac      60

ccgcccggat tcttcgcgcg tcccgagaaa acggcgagcg gggagacgaa tctgtttcga     120

tggcggtgct cgataccggg cgcgcgcggg acgcgctggg agggcgcgtt cgtgccgctg     180

acgatggagt tcccgaggga gtacccggcc aagccgatga agtgtaaatt tcctgcggga     240

ttttatcacc cgaacgtgta cccgagcggg acggtgtgcc tgagcattct gaacgaggac     300

gaggggtggc ggccgagcgt gacggtgaag caggtggcgc tggggataca ggaactgctg     360

gataatccga acgagaagtc gccggcgcag agcgatgcgt acgtgacgta cacgacggat     420

agggcgaagt acgagcggag ggtgagggag gaggtggcga agtatccgcc gccggagtag     480
```

<210> 240
<211> 159
<212> PRT
<213> Ostreococus tauri

<400> 240

Met Ala Ser Val Ala Leu Ala Arg Leu Gly Glu Glu Arg Arg Asn Trp
1 5 10 15

Arg Arg Asp His Pro Pro Gly Phe Phe Ala Arg Pro Glu Lys Thr Ala
20 25 30

Ser Gly Glu Thr Asn Leu Phe Arg Trp Arg Cys Ser Ile Pro Gly Ala
35 40 45

Arg Gly Thr Arg Trp Glu Gly Ala Phe Val Pro Leu Thr Met Glu Phe
50 55 60

Pro Arg Glu Tyr Pro Ala Lys Pro Met Lys Cys Lys Phe Pro Ala Gly
65 70 75 80

Phe Tyr His Pro Asn Val Tyr Pro Ser Gly Thr Val Cys Leu Ser Ile
85 90 95

Leu Asn Glu Asp Glu Gly Trp Arg Pro Ser Val Thr Val Lys Gln Val
100 105 110

Ala Leu Gly Ile Gln Glu Leu Leu Asp Asn Pro Asn Glu Lys Ser Pro
115 120 125

Ala Gln Ser Asp Ala Tyr Val Thr Tyr Thr Thr Asp Arg Ala Lys Tyr
130 135 140

Glu Arg Arg Val Arg Glu Glu Val Ala Lys Tyr Pro Pro Pro Glu
145 150 155

<210> 241
<211> 483
<212> DNA
<213> Picea sitchensis

<400> 241
atgtctggag gcatagctcg aggtcgactt gctgaggagc ggaaggcctg gcgtaagaac 60

catccccatg gtttcgtagc tagacctgac actcagccag atggttccct taacttaatg 120

gtgtggcaat gcatcattcc tggcaaatct gggacggatt gggagggtgg ttactttcct 180

ctaacaatta atttcagtga ggattaccca agtaaacctc caaaatgcaa gttccctcaa 240

gggtttttcc atcctaatgt ttatccatca ggaactgttt gtctgtctat ccttaatgag 300

gattctgggt ggcggccagc cattactgtg aagcaaatac ttataggtat tcaagacctt 360

ttagatcagc caaatccagg tgatcctgca caaacggatg gctaccatct ttttatccaa 420

gacctcacag aatacaagcg gagagttcga caacaggcaa aacaataccc acctctggtg 480

tga 483

<210> 242
<211> 160
<212> PRT
<213> Picea sitchensis

<400> 242

Met Ser Gly Gly Ile Ala Arg Gly Arg Leu Ala Glu Glu Arg Lys Ala
1 5 10 15

Trp Arg Lys Asn His Pro His Gly Phe Val Ala Arg Pro Asp Thr Gln
20 25 30

Pro Asp Gly Ser Leu Asn Leu Met Val Trp Gln Cys Ile Ile Pro Gly
35 40 45

Lys Ser Gly Thr Asp Trp Glu Gly Gly Tyr Phe Pro Leu Thr Ile Asn
50 55 60

Phe Ser Glu Asp Tyr Pro Ser Lys Pro Pro Lys Cys Lys Phe Pro Gln
65 70 75 80

Gly Phe Phe His Pro Asn Val Tyr Pro Ser Gly Thr Val Cys Leu Ser
85 90 95

Ile Leu Asn Glu Asp Ser Gly Trp Arg Pro Ala Ile Thr Val Lys Gln
100 105 110

Ile Leu Ile Gly Ile Gln Asp Leu Leu Asp Gln Pro Asn Pro Gly Asp
115 120 125

Pro Ala Gln Thr Asp Gly Tyr His Leu Phe Ile Gln Asp Leu Thr Glu
130 135 140

Tyr Lys Arg Arg Val Arg Gln Gln Ala Lys Gln Tyr Pro Pro Leu Val
145 150 155 160

<210> 243
<211> 54
<212> DNA
<213> Artificial sequence

<220>

<223> pimer 1

<400> 243
ggggacaagt ttgtacaaaa aagcaggctt aaacaatggc tagtggaatc gctc 54

<210> 244
<211> 49
<212> DNA
<213> Artificial sequence

<220>
<223> pimer 2

<400> 244
ggggaccact ttgtacaaga aagctgggta tcagttttgg tgcgttctc 49

<210> 245
<211> 2194
<212> DNA
<213> Oryza sativa

<400> 245
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag attttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctccttctc ccatctataa attcctcccc ccttttcccc tctctatata 1020

ggaggcatcc aagccaagaa gagggagagc accaaggaca cgcgactagc agaagccgag 1080

cgaccgcctt ctcgatccat atcttccggt cgagttcttg gtcgatctct tccctcctcc 1140

acctcctcct cacagggtat gtgcctccct tcggttgttc ttggatttat tgttctaggt 1200

tgtgtagtac gggcgttgat gttaggaaag gggatctgta tctgtgatga ttcctgttct 1260

tggatttggg atagaggggt tcttgatgtt gcatgttatc ggttcggttt gattagtagt 1320

atggttttca atcgtctgga gagctctatg gaaatgaaat ggtttaggga tcggaatctt 1380

gcgattttgt gagtaccttt tgtttgaggt aaaatcagag caccggtgat tttgcttggt 1440

gtaataaagt acggttgttt ggtcctcgat tctggtagtg atgcttctcg atttgacgaa 1500

gctatccttt gtttattccc tattgaacaa aaataatcca actttgaaga cggtcccgtt 1560

gatgagattg aatgattgat tcttaagcct gtccaaaatt tcgcagctgg cttgtttaga 1620

tacagtagtc cccatcacga aattcatgga aacagttata atcctcagga acaggggatt 1680

ccctgttctt ccgatttgct ttagtcccag aatttttttt cccaaatatc ttaaaaagtc 1740

actttctggt tcagttcaat gaattgattg ctacaaataa tgcttttata gcgttatcct 1800

agctgtagtt cagttaatag gtaatacccc tatagtttag tcaggagaag aacttatccg 1860

atttctgatc tccattttta attatatgaa atgaactgta gcataagcag tattcatttg 1920

gattattttt tttattagct ctcacccctt cattattctg agctgaaagt ctggcatgaa 1980

ctgtcctcaa ttttgttttc aaattcacat cgattatcta tgcattatcc tcttgtatct 2040

acctgtagaa gtttcttttt ggttattcct tgactgcttg attacagaaa gaaatttatg 2100

aagctgtaat cgggatagtt atactgcttg ttcttatgat tcatttcctt tgtgcagttc 2160

ttggtgtagc ttgccacttt caccagcaaa gttc 2194

<210> 246
<211> 2196
<212> DNA
<213> Lycopersicon esculentum

<400> 246
atggatgctt atgaagctac aaaaattgtt tttcaaagga ttcaaagttt ggatcctgaa 60

aatgcatcaa aaattatggg gattcttctg atgcaagacc atggtgagaa agaaatgatt 120

cgattagctt ttggtccaga agctttagtt cactcggtga ttcttaaagc aagaaaggag 180

cttggtgttt cttcaaactc accttctaca ccttcaactc cttcttcacc ttcacctttt 240

ggtggttcaa tgtgtttttc aaggcagaat tcttcttctt cagctacttc tggtaggatt 300

cttgggggtc ttagccttcc ttcacctctt agcataacta gtaacaacaa ccactcttca 360

aatgtttctg cttcttggag taccagtcct agtttctctg agtttcaaga agctgatctt 420

gttagtccta gtgcttccaa catctcatat actgctgcta ctactactaa tggaatgacc 480

aattccacca tgaattcctc agctcctccc ttttattgca atggtgaagt agacttgata 540

gatgagtttc aactacagga ccagctttct ttcttgaatg atgggtcacc aaccttgggg 600

cctaagaatc ctgatgttta ttaccagcaa cagcagcaac aacaagattt agcctcaagt 660

```
ccaagtgggg attccatgct tttctcttca tataactggg gtggtggttg caactcagtc     720
aacggcctct ctcatagaag gagctgctct gtgagtgatg tatgcttggg ggctgatgac     780
ccaagtggag gacttggctg gaaaccttgt ctctattttg ccagagggta ttgcaagaat     840
ggaagtagct gtaggttcct tcatggtgct gggcctggtg aaggtgaagt tgggtcacca     900
aacaagtttg agatgatgga acattgccaa gaacttctca gatctaagtc tgctcaccag     960
caaagactag ccacagcttc tcagctcgtg gcttcttcta actttcctct ctctcccatg    1020
gctgctaaca aatgcatgaa ctttcttcag cagcaacagt tgcagtctgc tgaaagccca    1080
agggcagctg ctgcattgat gatgggtgat gacatgcata aattgagcag aagtcgtttt    1140
gaaagagggg attttggact gaatggtgga gttggaatag caaatccagg ttcaaggcaa    1200
atttacttga catttccagc tgatagtact ttcaaagaag aggatgtttc caattatttc    1260
agcacttatg ggcctgttca agatgtgagg attccatatc agcaaaagag gatgtttggt    1320
tttgttacat ttgtttatcc agagactgtg aagaccattc ttgccaaagg aaatcctcat    1380
tttgtatgtg atgctagggt gcttgtcaag ccttacaaag agaagggcaa agtcccagag    1440
aagtttagga agcaacacca acagcagatg gagaggggag aattcactgg atgcggtagt    1500
cctactggtc tggactccag tgatccttat gatcttcagc ttggtgcaag aatgttttac    1560
aacactcaag atgcgctgtg gaggagaaaa ttggaggaac aagctgatct gcaacaggca    1620
attgagctcc aaagcaggag attgctgaat ttacagcttc ttgatgtcaa aaggagcaac    1680
catcatcgtg ccctttccat gagtgctgtt atcccatccc caccgcattc tccaggcttc    1740
ttcaatcaga atatggttcg ctccacagac tttggcagcc gagaagagaa tggttttgca    1800
ccaaaaaatgg ccaattttgc tgctgttact gctgagcaaa agaatgcaaa tcttactgcc    1860
aaggagagag aatgcttcac aggtaaagat gaaaatagca gtggcaaaga aagttccaag    1920
aaggaagcaa gtgattttca agaaagcttg gagcataatc tcccagatag tccatttgca    1980
tcacctaaag cagttgggga cttcatcaca actttctcaa atgaagctgc tggagatgtt    2040
gacaaaggtg ctggattaaa tgcatcatcc tctgctaaca ataatatgat cccttcttcc    2100
tccttgtcaa ctagtactct agacatgact cctttcaaat catgttactt ccaagtgcct    2160
aggttccctt ccggacatgg cgccattgga atgtag                              2196
```

```
<210>  247
<211>  731
<212>  PRT
<213>  Lycopersicon esculentum

<400>  247

Met Asp Ala Tyr Glu Ala Thr Lys Ile Val Phe Gln Arg Ile Gln Ser
1               5                   10                  15
```

```
Leu Asp Pro Glu Asn Ala Ser Lys Ile Met Gly Ile Leu Leu Met Gln
            20                  25                  30

Asp His Gly Glu Lys Glu Met Ile Arg Leu Ala Phe Gly Pro Glu Ala
        35                  40                  45

Leu Val His Ser Val Ile Leu Lys Ala Arg Lys Glu Leu Gly Val Ser
    50                  55                  60

Ser Asn Ser Pro Ser Thr Pro Ser Thr Pro Ser Ser Pro Ser Pro Phe
65                  70                  75                  80

Gly Gly Ser Met Cys Phe Ser Arg Gln Asn Ser Ser Ser Ser Ala Thr
                85                  90                  95

Ser Gly Arg Ile Leu Gly Gly Leu Ser Leu Pro Ser Pro Leu Ser Ile
            100                 105                 110

Thr Ser Asn Asn Asn His Ser Ser Asn Val Ser Ala Ser Trp Ser Thr
        115                 120                 125

Ser Pro Ser Phe Ser Glu Phe Gln Glu Ala Asp Leu Val Ser Pro Ser
    130                 135                 140

Ala Ser Asn Ile Ser Tyr Thr Ala Ala Thr Thr Thr Asn Gly Met Thr
145                 150                 155                 160

Asn Ser Thr Met Asn Ser Ser Ala Pro Pro Phe Tyr Cys Asn Gly Glu
                165                 170                 175

Val Asp Leu Ile Asp Glu Phe Gln Leu Gln Asp Gln Leu Ser Phe Leu
            180                 185                 190

Asn Asp Gly Ser Pro Thr Leu Gly Pro Lys Asn Pro Asp Val Tyr Tyr
        195                 200                 205

Gln Gln Gln Gln Gln Gln Gln Asp Leu Ala Ser Ser Pro Ser Gly Asp
    210                 215                 220

Ser Met Leu Phe Ser Ser Tyr Asn Trp Gly Gly Gly Cys Asn Ser Val
225                 230                 235                 240

Asn Gly Leu Ser His Arg Arg Ser Cys Ser Val Ser Asp Val Cys Leu
                245                 250                 255

Gly Ala Asp Asp Pro Ser Gly Gly Leu Gly Trp Lys Pro Cys Leu Tyr
```

260 265 270

Phe Ala Arg Gly Tyr Cys Lys Asn Gly Ser Ser Cys Arg Phe Leu His
275 280 285

Gly Ala Gly Pro Gly Glu Gly Glu Val Gly Ser Pro Asn Lys Phe Glu
290 295 300

Met Met Glu His Cys Gln Glu Leu Leu Arg Ser Lys Ser Ala His Gln
305 310 315 320

Gln Arg Leu Ala Thr Ala Ser Gln Leu Val Ala Ser Ser Asn Phe Pro
325 330 335

Leu Ser Pro Met Ala Ala Asn Lys Cys Met Asn Phe Leu Gln Gln Gln
340 345 350

Gln Leu Gln Ser Ala Glu Ser Pro Arg Ala Ala Ala Ala Leu Met Met
355 360 365

Gly Asp Asp Met His Lys Leu Ser Arg Ser Arg Phe Glu Arg Gly Asp
370 375 380

Phe Gly Leu Asn Gly Gly Val Gly Ile Ala Asn Pro Gly Ser Arg Gln
385 390 395 400

Ile Tyr Leu Thr Phe Pro Ala Asp Ser Thr Phe Lys Glu Glu Asp Val
405 410 415

Ser Asn Tyr Phe Ser Thr Tyr Gly Pro Val Gln Asp Val Arg Ile Pro
420 425 430

Tyr Gln Gln Lys Arg Met Phe Gly Phe Val Thr Phe Val Tyr Pro Glu
435 440 445

Thr Val Lys Thr Ile Leu Ala Lys Gly Asn Pro His Phe Val Cys Asp
450 455 460

Ala Arg Val Leu Val Lys Pro Tyr Lys Glu Lys Gly Lys Val Pro Glu
465 470 475 480

Lys Phe Arg Lys Gln His Gln Gln Gln Met Glu Arg Gly Glu Phe Thr
485 490 495

Gly Cys Gly Ser Pro Thr Gly Leu Asp Ser Ser Asp Pro Tyr Asp Leu
500 505 510

Gln Leu Gly Ala Arg Met Phe Tyr Asn Thr Gln Asp Ala Leu Trp Arg
515 520 525

Arg Lys Leu Glu Glu Gln Ala Asp Leu Gln Gln Ala Ile Glu Leu Gln
530 535 540

Ser Arg Arg Leu Leu Asn Leu Gln Leu Leu Asp Val Lys Arg Ser Asn
545 550 555 560

His His Arg Ala Leu Ser Met Ser Ala Val Ile Pro Ser Pro Pro His
565 570 575

Ser Pro Gly Phe Phe Asn Gln Asn Met Val Arg Ser Thr Asp Phe Gly
580 585 590

Ser Arg Glu Glu Asn Gly Phe Ala Pro Lys Met Ala Asn Phe Ala Ala
595 600 605

Val Thr Ala Glu Gln Lys Asn Ala Asn Leu Thr Ala Lys Glu Arg Glu
610 615 620

Cys Phe Thr Gly Lys Asp Glu Asn Ser Ser Gly Lys Glu Ser Ser Lys
625 630 635 640

Lys Glu Ala Ser Asp Phe Gln Glu Ser Leu Glu His Asn Leu Pro Asp
645 650 655

Ser Pro Phe Ala Ser Pro Lys Ala Val Gly Asp Phe Ile Thr Thr Phe
660 665 670

Ser Asn Glu Ala Ala Gly Asp Val Asp Lys Gly Ala Gly Leu Asn Ala
675 680 685

Ser Ser Ser Ala Asn Asn Asn Met Ile Pro Ser Ser Ser Leu Ser Thr
690 695 700

Ser Thr Leu Asp Met Thr Pro Phe Lys Ser Cys Tyr Phe Gln Val Pro
705 710 715 720

Arg Phe Pro Ser Gly His Gly Ala Ile Gly Met
725 730

<210> 248
<211> 1866
<212> DNA
<213> Pinus radiata

<400> 248
atggatgcct atgaagctac aaggattgtg ttctccagga tccagagctt agagccagaa 60

aatgtgtcta aaattattgg gtacttgtta ttacaagacc atggtgaaca ggaaatgatt 120

aggttggctt tcagtcctga ttccttgatt cagtccatga tcatcaaggt taagaaagat 180

ctaggtttga tgcaacagca aggtactcca gctccaactg tttcatctta cctatccaga 240

atcaatcgtc tccccaactt gccactgcag tctgctcaga tttctcaatc cagagctttc 300

tcttctccaa ccgccctttc acctcatgca gctccatggg gatctcatat ttctcaacag 360

actaggcctt tatccaacaa ttttaactcg atcttgaatg agatacagac taacactagt 420

acttctagta ctataaattc tactagcaat ggctatctta atttcagtct gccatccatg 480

ccagatcaag ctaatagcct tccttacagc gagcatcctg gccttgtaga tgaatttcaa 540

ttgcaagatc agcttccctt tctcaatgat tctccagagt ctgcccaatc tcatgctaat 600

tatctcaact atcctgagat gttgcaggca tattgcaatg gcaatcagcc attgactata 660

gaccatgtaa gcccaacagc agctaataat agctatcctg gtactactca tatacccaag 720

cagcctagtt caatttcaga tatttacaat ctaacttcag aatctgcacc tgggtcagcc 780

ttggcctgga agccatgcat gtactttgct aggggttact gcaagaatgg gagcaattgc 840

aggtttcttc atggtaacta tggtggtcat gtaaggtcag agagcaataa tgaccacagt 900

gaaaagttca tgggatccag tagtggccca ttggaaaaac tggagttaga attgaaggaa 960

ttgctcagag gaagagggtc tccagtttca gttgcttctc tacctcagtt ctatagtgag 1020

aggcttggga aggctcttca ggccgagagg tttacaaggt atagaagtga acgagattca 1080

tctgatcact tggccagttc tgcttccaat tctggatctc gccaaatata cttgactttt 1140

cctgcagaga gtactttcag ggaggaggac gtatcaaatt atttcagcat ttttggaccc 1200

gtgcaggatg taaggattcc ttatcagcag aagaggatgt ttgggtttgt gacatttgta 1260

tatcaagaga ctgttaagat tattttggca aaaggcaatc ctcattatgt ctgtgatgcc 1320

cgtgttcttg tcaaaccata caaagaaaaa ggatccaaac ccgcagagag aatgaagtat 1380

accgactgta ggggcgatta ttcaggatat gtgacaactc acaatcttga tatcaaggac 1440

agcaatttgc aacttggccc tcccagattt gttgaaaaca gcttagacct ggtgacaaga 1500

aggcagttgg aggaggagca ggatcatgta gagcaagccg ttgagcttca aacaaaacga 1560

cttgcagagc tgcagcttgg tgacagaaag agaccacagc ttgtaccttc agatcctcaa 1620

gtttctatgg cttcaacaaa ctccggcccg gctcagcatt atcaaaatca gttctcaaat 1680

ggacccaata atcattcaga agaggacgca acaacctcag aagattttag cagttctaca 1740

ttagcagaac attttggtta tgtgctacag gttttagata gtgaatctgt ctatgaggaa 1800

cacccaaaac ctgtcaacca tcaccatgac aggcttccta ttactaatgg tgcaatgaga 1860

ctgtaa 1866

<210> 249
<211> 621
<212> PRT
<213> Pinus radiata

<400> 249

Met Asp Ala Tyr Glu Ala Thr Arg Ile Val Phe Ser Arg Ile Gln Ser
1               5                   10                  15

Leu Glu Pro Glu Asn Val Ser Lys Ile Ile Gly Tyr Leu Leu Leu Gln
            20                  25                  30

Asp His Gly Glu Gln Glu Met Ile Arg Leu Ala Phe Ser Pro Asp Ser
        35                  40                  45

Leu Ile Gln Ser Met Ile Ile Lys Val Lys Lys Asp Leu Gly Leu Met
    50                  55                  60

Gln Gln Gln Gly Thr Pro Ala Pro Thr Val Ser Ser Tyr Leu Ser Arg
65                  70                  75                  80

Ile Asn Arg Leu Pro Asn Leu Pro Leu Gln Ser Ala Gln Ile Ser Gln
                85                  90                  95

Ser Arg Ala Phe Ser Ser Pro Thr Ala Leu Ser Pro His Ala Ala Pro
            100                 105                 110

Trp Gly Ser His Ile Ser Gln Gln Thr Arg Pro Leu Ser Asn Asn Phe
        115                 120                 125

Asn Ser Ile Leu Asn Glu Ile Gln Thr Asn Thr Ser Thr Ser Ser Thr
    130                 135                 140

Ile Asn Ser Thr Ser Asn Gly Tyr Leu Asn Phe Ser Leu Pro Ser Met
145                 150                 155                 160

Pro Asp Gln Ala Asn Ser Leu Pro Tyr Ser Glu His Pro Gly Leu Val
                165                 170                 175

Asp Glu Phe Gln Leu Gln Asp Gln Leu Pro Phe Leu Asn Asp Ser Pro
            180                 185                 190

Glu Ser Ala Gln Ser His Ala Asn Tyr Leu Asn Tyr Pro Glu Met Leu
        195                 200                 205

Gln Ala Tyr Cys Asn Gly Asn Gln Pro Leu Thr Ile Asp His Val Ser
    210                 215                 220

Pro Thr Ala Ala Asn Asn Ser Tyr Pro Gly Thr Thr His Ile Pro Lys
225 230 235 240

Gln Pro Ser Ser Ile Ser Asp Ile Tyr Asn Leu Thr Ser Glu Ser Ala
245 250 255

Pro Gly Ser Ala Leu Ala Trp Lys Pro Cys Met Tyr Phe Ala Arg Gly
260 265 270

Tyr Cys Lys Asn Gly Ser Asn Cys Arg Phe Leu His Gly Asn Tyr Gly
275 280 285

Gly His Val Arg Ser Glu Ser Asn Asn Asp His Ser Glu Lys Phe Met
290 295 300

Gly Ser Ser Ser Gly Pro Leu Glu Lys Leu Glu Leu Glu Leu Lys Glu
305 310 315 320

Leu Leu Arg Gly Arg Gly Ser Pro Val Ser Val Ala Ser Leu Pro Gln
325 330 335

Phe Tyr Ser Glu Arg Leu Gly Lys Ala Leu Gln Ala Glu Arg Phe Thr
340 345 350

Arg Tyr Arg Ser Glu Arg Asp Ser Ser Asp His Leu Ala Ser Ser Ala
355 360 365

Ser Asn Ser Gly Ser Arg Gln Ile Tyr Leu Thr Phe Pro Ala Glu Ser
370 375 380

Thr Phe Arg Glu Glu Asp Val Ser Asn Tyr Phe Ser Ile Phe Gly Pro
385 390 395 400

Val Gln Asp Val Arg Ile Pro Tyr Gln Gln Lys Arg Met Phe Gly Phe
405 410 415

Val Thr Phe Val Tyr Gln Glu Thr Val Lys Ile Ile Leu Ala Lys Gly
420 425 430

Asn Pro His Tyr Val Cys Asp Ala Arg Val Leu Val Lys Pro Tyr Lys
435 440 445

Glu Lys Gly Ser Lys Pro Ala Glu Arg Met Lys Tyr Thr Asp Cys Arg
450 455 460

Gly Asp Tyr Ser Gly Tyr Val Thr Thr His Asn Leu Asp Ile Lys Asp
465 470 475 480

Ser Asn Leu Gln Leu Gly Pro Pro Arg Phe Val Glu Asn Ser Leu Asp
485 490 495

Leu Val Thr Arg Arg Gln Leu Glu Glu Glu Gln Asp His Val Glu Gln
500 505 510

Ala Val Glu Leu Gln Thr Lys Arg Leu Ala Glu Leu Gln Leu Gly Asp
515 520 525

Arg Lys Arg Pro Gln Leu Val Pro Ser Asp Pro Gln Val Ser Met Ala
530 535 540

Ser Thr Asn Ser Gly Pro Ala Gln His Tyr Gln Asn Gln Phe Ser Asn
545 550 555 560

Gly Pro Asn Asn His Ser Glu Glu Asp Ala Thr Thr Ser Glu Asp Phe
565 570 575

Ser Ser Ser Thr Leu Ala Glu His Phe Gly Tyr Val Leu Gln Val Leu
580 585 590

Asp Ser Glu Ser Val Tyr Glu Glu His Pro Lys Pro Val Asn His His
595 600 605

His Asp Arg Leu Pro Ile Thr Asn Gly Ala Met Arg Leu
610 615 620

<210> 250
<211> 2274
<212> DNA
<213> Eucalyptus grandis

<400> 250
atggacgcat atgaagccac aaggattgtc ttctcaagaa tccaaagttt agaccctgag 60

aatgcctcca agatcatggg tctcctcctc atccaagatc atggtgagaa ggagatgatc 120

aggctggctc ttggaccgga gactctgctt cactcagtgg tcctcaaggc aaggaaggac 180

ataatccttc cgtcaaactc tccctcaacg ccctccacac cttcttctcc ctctcctttc 240

atgtctacca accccatctc catctcctcc aggcctaaag gaagcaactt ttcgccatct 300

tctctctcaa atatccccag cccatcttct tggggggtg gtggtggtgg tggtggttct 360

ttctctgatc tctcaagtgg agatgatttg atcaattctt cctcttgttt gtatggaaat 420

ggaggcagtg acaccatgat tgatgagctt cagctccaag accagctttc cttcctcaac 480

gataactccc cacccttgg acccaacagc aaccctgata tgttctgccc ccagcaggac 540

ttgctgtcca gtcccaccgc cgtatacggc ggagcggccg cgggctgggg cgccccggtg 600

caccggagga gctgctcggt cagcgatgtg tgctcgggtt cctcagaaga cccatcttgt 660
ggagtcgggt ggaggccatg cttgtattat gctagagggt actgcaagaa tgggatcagc 720
tgcaggttct tgcacagtgg tggacttggc gatgccgctt ctgtggtcgg cagcccggac 780
ggcagtgcgt ccgcggtggt cggctccccg agtaaagtgg acatgatggg ccagtgccat 840
gaagctgttc tgaggtccaa atctgctcag cagcagagac tagctgctgc ttctcagctc 900
ataggttctg caaccttccc ttacactccc aaatccatga atttacttct ccatcaccag 960
caaaatgatg ctcatagggc tgctgctgct gctgcgctga tgatgggtga tgacttttac 1020
aagtatggca gatcaaggct agaaaggagt gatttttcag tgaatggttg tgtgaatcct 1080
gcttctaggc agatttactt gactttccca gccgacagta ctttcaagga ggaagatgtt 1140
tccaactatt tcagcaactt tgggccggtg caagatgtga gaattcctta ccagcagaag 1200
aggatgtttg gctttgttac atttgtttac ccagaaacgg tgaagctcat tttggccaaa 1260
gggaaccctc attttgtttg tgatgctaga gttctcgtca agccttacaa agagaaggga 1320
aaagtgccag acaagttcag gaagcagtcc cagctggtgg aaaggggtga tttttcgcct 1380
tgtggaactc caactgggtt ggattcgagg gggggaccat ttgacctcaa cctcggagcg 1440
aggccgtttt acaattctca ggacatgctg tggaggagga gattcgagga gcaagctgat 1500
cttcaacaag cccttgaata ccagagtcaa cggctgatga gtctgcagct tctagatgtc 1560
aagaagcatc atcatcagag ggctctctca actggctccc ccattccatc tcctgctcaa 1620
tcgcctactt tgttcaacaa tccaaccttc ctcaacattc cttcggttcg cagcctgggc 1680
gtcacagaag agaatggttc tagccctggc ttatccgaca gtcagccctt gaactaccag 1740
tctgtgattg tctctgctgg gaaagatttg actggaagtg acaagagtaa tgggaatgac 1800
aaggaaagct cccatactga agataaaggc ttggctgaaa gtttggagca taaccttcct 1860
gatagtccct ttgcatctcc tactaaagcc tccgcagagc acttctcttc cttaaccaat 1920
gtagtcagcg aggctgaaaa ggatggtgta ggttcggcct catcttctcc caacagcaat 1980
aacccagtct cttcaccctt gatcccgggc acctccgcca tggacatggc ttcattcacg 2040
tctttcaact gccagattcc tgccatagga atgtatgccg gtgctggagg gccaacatgc 2100
ccagtgggta tatagctagc tcttccttga ctgagggaga ttaacaacaa taaccaaaca 2160
aacccgttat caaagaccag atctgtagag aatccgtacc actaccatca cctccaccac 2220
tacctcgatt atccatacta tactactgga taccatacaa aaatgcatac gtaa 2274

<210> 251
<211> 755
<212> PRT
<213> Eucalyptus grandis

<400> 251

```
Met Asp Ala Tyr Glu Ala Thr Arg Ile Val Phe Ser Arg Ile Gln Ser
1               5                   10                  15

Leu Asp Pro Glu Asn Ala Ser Lys Ile Met Gly Leu Leu Leu Ile Gln
            20                  25                  30

Asp His Gly Glu Lys Glu Met Ile Arg Leu Ala Leu Gly Pro Glu Thr
        35                  40                  45

Leu Leu His Ser Val Val Leu Lys Ala Arg Lys Asp Ile Ile Leu Pro
    50                  55                  60

Ser Asn Ser Pro Ser Thr Pro Ser Thr Pro Ser Ser Pro Ser Pro Phe
65                  70                  75                  80

Met Ser Thr Asn Pro Ile Ser Ile Ser Ser Arg Pro Lys Gly Ser Asn
                85                  90                  95

Phe Ser Pro Ser Ser Leu Ser Asn Ile Pro Ser Pro Ser Ser Trp Gly
            100                 105                 110

Gly Gly Gly Gly Gly Gly Gly Ser Phe Ser Asp Leu Ser Ser Gly Asp
        115                 120                 125

Asp Leu Ile Asn Ser Ser Ser Cys Leu Tyr Gly Asn Gly Gly Ser Asp
    130                 135                 140

Thr Met Ile Asp Glu Leu Gln Leu Gln Asp Gln Leu Ser Phe Leu Asn
145                 150                 155                 160

Asp Asn Ser Pro Pro Leu Gly Pro Asn Ser Asn Pro Asp Met Phe Cys
                165                 170                 175

Pro Gln Gln Asp Leu Leu Ser Ser Pro Thr Ala Val Tyr Gly Gly Ala
            180                 185                 190

Ala Ala Gly Trp Gly Ala Pro Val His Arg Arg Ser Cys Ser Val Ser
        195                 200                 205

Asp Val Cys Ser Gly Ser Ser Glu Asp Pro Ser Cys Gly Val Gly Trp
    210                 215                 220

Arg Pro Cys Leu Tyr Tyr Ala Arg Gly Tyr Cys Lys Asn Gly Ile Ser
225                 230                 235                 240

Cys Arg Phe Leu His Ser Gly Gly Leu Gly Asp Ala Ala Ser Val Val
```

245 250 255

Gly Ser Pro Asp Gly Ser Ala Ser Ala Val Val Gly Ser Pro Ser Lys
260 265 270

Val Asp Met Met Gly Gln Cys His Glu Ala Val Leu Arg Ser Lys Ser
275 280 285

Ala Gln Gln Gln Arg Leu Ala Ala Ala Ser Gln Leu Ile Gly Ser Ala
290 295 300

Thr Phe Pro Tyr Thr Pro Lys Ser Met Asn Leu Leu Leu His His Gln
305 310 315 320

Gln Asn Asp Ala His Arg Ala Ala Ala Ala Ala Ala Leu Met Met Gly
325 330 335

Asp Asp Phe Tyr Lys Tyr Gly Arg Ser Arg Leu Glu Arg Ser Asp Phe
340 345 350

Ser Val Asn Gly Cys Val Asn Pro Ala Ser Arg Gln Ile Tyr Leu Thr
355 360 365

Phe Pro Ala Asp Ser Thr Phe Lys Glu Glu Asp Val Ser Asn Tyr Phe
370 375 380

Ser Asn Phe Gly Pro Val Gln Asp Val Arg Ile Pro Tyr Gln Gln Lys
385 390 395 400

Arg Met Phe Gly Phe Val Thr Phe Val Tyr Pro Glu Thr Val Lys Leu
405 410 415

Ile Leu Ala Lys Gly Asn Pro His Phe Val Cys Asp Ala Arg Val Leu
420 425 430

Val Lys Pro Tyr Lys Glu Lys Gly Lys Val Pro Asp Lys Phe Arg Lys
435 440 445

Gln Ser Gln Leu Val Glu Arg Gly Asp Phe Ser Pro Cys Gly Thr Pro
450 455 460

Thr Gly Leu Asp Ser Arg Gly Gly Pro Phe Asp Leu Asn Leu Gly Ala
465 470 475 480

Arg Pro Phe Tyr Asn Ser Gln Asp Met Leu Trp Arg Arg Arg Phe Glu
485 490 495

```
Glu Gln Ala Asp Leu Gln Gln Ala Leu Glu Tyr Gln Ser Gln Arg Leu
            500                 505                 510

Met Ser Leu Gln Leu Leu Asp Val Lys Lys His His His Gln Arg Ala
        515                 520                 525

Leu Ser Thr Gly Ser Pro Ile Pro Ser Pro Ala Gln Ser Pro Thr Leu
    530                 535                 540

Phe Asn Asn Pro Thr Phe Leu Asn Ile Pro Ser Val Arg Ser Leu Gly
545                 550                 555                 560

Val Thr Glu Glu Asn Gly Ser Ser Pro Gly Leu Ser Asp Ser Gln Pro
                565                 570                 575

Leu Asn Tyr Gln Ser Val Ile Val Ser Ala Gly Lys Asp Leu Thr Gly
            580                 585                 590

Ser Asp Lys Ser Asn Gly Asn Asp Lys Glu Ser Ser His Thr Glu Asp
        595                 600                 605

Lys Gly Leu Ala Glu Ser Leu Glu His Asn Leu Pro Asp Ser Pro Phe
    610                 615                 620

Ala Ser Pro Thr Lys Ala Ser Ala Glu His Phe Ser Ser Leu Thr Asn
625                 630                 635                 640

Val Val Ser Glu Ala Glu Lys Asp Gly Val Gly Ser Ala Ser Ser Ser
                645                 650                 655

Pro Asn Ser Asn Asn Pro Val Ser Ser Pro Leu Ile Pro Gly Thr Ser
            660                 665                 670

Ala Met Asp Met Ala Ser Phe Thr Ser Phe Asn Cys Gln Ile Pro Ala
        675                 680                 685

Ile Gly Met Tyr Ala Gly Ala Gly Gly Pro Thr Cys Pro Val Gly Ile
    690                 695                 700

Leu Ala Leu Pro Leu Arg Glu Ile Asn Asn Asn Asn Gln Thr Asn Pro
705                 710                 715                 720

Leu Ser Lys Thr Arg Ser Val Glu Asn Pro Tyr His Tyr His His Leu
                725                 730                 735

His His Tyr Leu Asp Tyr Pro Tyr Tyr Thr Thr Gly Tyr His Thr Lys
            740                 745                 750
```

Met His Thr
        755

<210> 252
<211> 1806
<212> DNA
<213> Pinus radiata

<400> 252
atggatacat atgaagcaac aaggattgtg ttcacaagga ttcagagcat agaaccagag 60

aatgtgtcca agatcattgg gtatttgctt cttcaagacc tgggagatca agaaatgatt 120

cgcctggcat ttgggcctaa tacactctta cagtccatga tttccaaggc caagacagag 180

cttggtttat catctccgtc ccctcttcag tcacctctgc gctacactca gttttctaat 240

ttgttgtctc gctcattctc ttctccagca cccaatggct ttgatgattg tcagctccaa 300

gatcatctct tctatcccac tgagaatctt gattcctaca gcctaccaaa tgatagtcat 360

gtctatacag agatgctctc tttcttgaat ggaagcaaga caggattgaa tcacaggcga 420

tcttactcca tgacagacgt ttctgtaagc tgtgagccag tttcttggaa accatgcctg 480

tattttgcca ggggatattg caagcatgga tccagctgcc gttttactca cagttattca 540

cgctctgaca acgtttcgag ttcaattccc ttggatcccc gcttcgaaga agctttctct 600

gtggaatcat tggaaagatt agagttagaa cttcaagaat tattgagagg aagacgggcc 660

cctgtttcca ttgcttccct acctcaactc tattacgaga gatttgggaa aaccctgcaa 720

gccgagggat acttgactga gagtcagagg catgggaaag caggatacag cttgacaaat 780

ctactagcac gcttgaagaa tacagtaagc ctcattgata ggcctcatgg tcagcacgcc 840

attgttttgg cagaggatgc taacaggttc acaacttata ggccaagtga acgagatccc 900

tactatttga gtggtgtcag ctctggatcc cgtcaaattt acatgacatt tcctgctgaa 960

agcaccttca cggaggagga tgtttccaac tatttcagga tatacggacc tgtagaggat 1020

gtgagaattc cataccaaca gaagcgtatg tttggatttg taacatatgt tttcccagag 1080

actgtcaaat tgatactggc taaaggaaat cctcactatg tctgcggtgc tcgtgttctg 1140

gttaagccat acaaggagag aaacaagcat ggagacagga aaaatggcga tagaggagaa 1200

caatatgcaa gatacttgct gccatcttac aatgtagatt caaaggacta tgatctatgt 1260

ccagctccta ggatgtttca gaattccgaa ctgatcagaa ggcatataga agagcaagag 1320

caagccatcg aattggaaag actacgcctg acagagttgc atctggctga ccgtgcgcag 1380

agaactcaaa ataatgccat cactctgcaa caacaaaatt ctcattctaa tgggctactc 1440

aatgtagagg aagaagaaac tcaggtttca gaagagctaa acagctttga tccgcccacg 1500

gatcactttg gttatctgct ggatgtgctg gatagtgagc agaaccctga agaagagcct 1560

aaacaacaga aagccgacaa tgacgaagaa tgcaacgggc acaaccttcc tgatagccct 1620

tttgggtttt ctcattccat taaaacaaca ttgccccatc ccgagaaaac gaacaacttt 1680

tcctttttcg acaccagccc agcacaagaa tcatccactt ccatcatgac aaaggaaggg 1740

acttgttcca tgtgcctcga ttccattgtg gagcaggtgc ggttagagtg caagcatgtg 1800

aggtga 1806

<210> 253
<211> 601
<212> PRT
<213> Pinus radiata

<400> 253

```
Met Asp Thr Tyr Glu Ala Thr Arg Ile Val Phe Thr Arg Ile Gln Ser
1               5                   10                  15

Ile Glu Pro Glu Asn Val Ser Lys Ile Ile Gly Tyr Leu Leu Leu Gln
            20                  25                  30

Asp Leu Gly Asp Gln Glu Met Ile Arg Leu Ala Phe Gly Pro Asn Thr
        35                  40                  45

Leu Leu Gln Ser Met Ile Ser Lys Ala Lys Thr Glu Leu Gly Leu Ser
    50                  55                  60

Ser Pro Ser Pro Leu Gln Ser Pro Leu Arg Tyr Thr Gln Phe Ser Asn
65                  70                  75                  80

Leu Leu Ser Arg Ser Phe Ser Ser Pro Ala Pro Asn Gly Phe Asp Asp
                85                  90                  95

Cys Gln Leu Gln Asp His Leu Phe Tyr Pro Thr Glu Asn Leu Asp Ser
            100                 105                 110

Tyr Ser Leu Pro Asn Asp Ser His Val Tyr Thr Glu Met Leu Ser Phe
        115                 120                 125

Leu Asn Gly Ser Lys Thr Gly Leu Asn His Arg Arg Ser Tyr Ser Met
    130                 135                 140

Thr Asp Val Ser Val Ser Cys Glu Pro Val Ser Trp Lys Pro Cys Leu
145                 150                 155                 160

Tyr Phe Ala Arg Gly Tyr Cys Lys His Gly Ser Ser Cys Arg Phe Thr
                165                 170                 175

His Ser Tyr Ser Arg Ser Asp Asn Val Ser Ser Ser Ile Pro Leu Asp
```

180 185 190

Pro Arg Phe Glu Glu Ala Phe Ser Val Glu Ser Leu Glu Arg Leu Glu
195 200 205

Leu Glu Leu Gln Glu Leu Leu Arg Gly Arg Arg Ala Pro Val Ser Ile
210 215 220

Ala Ser Leu Pro Gln Leu Tyr Tyr Glu Arg Phe Gly Lys Thr Leu Gln
225 230 235 240

Ala Glu Gly Tyr Leu Thr Glu Ser Gln Arg His Gly Lys Ala Gly Tyr
245 250 255

Ser Leu Thr Asn Leu Leu Ala Arg Leu Lys Asn Thr Val Ser Leu Ile
260 265 270

Asp Arg Pro His Gly Gln His Ala Ile Val Leu Ala Glu Asp Ala Asn
275 280 285

Arg Phe Thr Thr Tyr Arg Pro Ser Glu Arg Asp Pro Tyr Tyr Leu Ser
290 295 300

Gly Val Ser Ser Gly Ser Arg Gln Ile Tyr Met Thr Phe Pro Ala Glu
305 310 315 320

Ser Thr Phe Thr Glu Glu Asp Val Ser Asn Tyr Phe Arg Ile Tyr Gly
325 330 335

Pro Val Glu Asp Val Arg Ile Pro Tyr Gln Gln Lys Arg Met Phe Gly
340 345 350

Phe Val Thr Tyr Val Phe Pro Glu Thr Val Lys Leu Ile Leu Ala Lys
355 360 365

Gly Asn Pro His Tyr Val Cys Gly Ala Arg Val Leu Val Lys Pro Tyr
370 375 380

Lys Glu Arg Asn Lys His Gly Asp Arg Lys Asn Gly Asp Arg Gly Glu
385 390 395 400

Gln Tyr Ala Arg Tyr Leu Leu Pro Ser Tyr Asn Val Asp Ser Lys Asp
405 410 415

Tyr Asp Leu Cys Pro Ala Pro Arg Met Phe Gln Asn Ser Glu Leu Ile
420 425 430

Arg Arg His Ile Glu Glu Gln Glu Gln Ala Ile Glu Leu Glu Arg Leu
435 440 445

Arg Leu Thr Glu Leu His Leu Ala Asp Arg Ala Gln Arg Thr Gln Asn
450 455 460

Asn Ala Ile Thr Leu Gln Gln Gln Asn Ser His Ser Asn Gly Leu Leu
465 470 475 480

Asn Val Glu Glu Glu Glu Thr Gln Val Ser Glu Glu Leu Asn Ser Phe
485 490 495

Asp Pro Pro Thr Asp His Phe Gly Tyr Leu Leu Asp Val Leu Asp Ser
500 505 510

Glu Gln Asn Pro Glu Glu Glu Pro Lys Gln Gln Lys Ala Asp Asn Asp
515 520 525

Glu Glu Cys Asn Gly His Asn Leu Pro Asp Ser Pro Phe Gly Phe Ser
530 535 540

His Ser Ile Lys Thr Thr Leu Pro His Pro Glu Lys Thr Asn Asn Phe
545 550 555 560

Ser Phe Phe Asp Thr Ser Pro Ala Gln Glu Ser Ser Thr Ser Ile Met
565 570 575

Thr Lys Glu Gly Thr Cys Ser Met Cys Leu Asp Ser Ile Val Glu Gln
580 585 590

Val Arg Leu Glu Cys Lys His Val Arg
595 600

<210> 254
<211> 2124
<212> DNA
<213> Populus trichocarpa

<400> 254
atggatggtt atgaagcaac aagaatagtt ttctcgagaa tccaaaacct agacccagaa 60

aatgcttcaa aaatcatggg tcttcttttg attcaggacc atggtgaaaa ggaaatgatt 120

aggttagctt ttggaccaga agcacttgtt cactcagtaa tccttaaagc gaggaaagaa 180

ctaggacttt gctctccaac aaacccttct aaaagtcctt cgccccttc gcctctatat 240

tcaagcaacc caataaccat ctctagacag aattcatctt cttcaacttc aagacttggg 300

tttaacatcc caccttcact tactatccca aacccttcat caaatttttc ttcttcttgg 360

agtgaccttc caaaccctga tgacttgatt agtcctaatg gtagttcact caatcctgct 420

```
tctgctcctt tctatgctaa tggagtaaga ggtggaggag agtctgattt gatggatgag     480
tttcagctcc aagaccagct ttcattcttg aatgacaatt cagcaaatct tggtccaaaa     540
agctcagatc ttttttactc tcaactggat gctttatcaa gtccaactgg tgctagtgat     600
tctgtgatgt ttccttctta ctggggtggg tctgtgcaca gaaggagctg ttctgtcagt     660
gatgttttgg ggtctgagga tccaaattca ggctttgggt ggagaccttg cctttacttt     720
gctagagggt actgtaagaa tggaagtaac tgtaggtttg ttcacggtgg gctcggagaa     780
tctgatggtg caggtgttgt tgtgggttca cctaatggta acaacaagat tgatatgatg     840
gaccagtgcc atgagttgct tagatccaag tctgctcaac agcaaaggtt agctgctgct     900
tctcagctca tgggtggctc tgctgcttct tttccttact ctcctaaaag catgaacttt     960
cttcttcaac aacagcaaaa tgatagccag agggctgctg ctgctttgat gatgggggag    1020
gacatgcaca aatttgcaag atctaggctt gataggaatg atttgattaa tcctgcttcc    1080
aggcagatct acttgacttt ccctgctgat agcactttta gagaggaaga tgtgtcaaat    1140
tacttcagta tttatgggcc agtgcaagat gtgaggattc cttatcagca gaagaggatg    1200
tttggatttg ttaccttttt gtatccagag accgtgaaga taatattggc caaagggaac    1260
cctcattttg tttgtgatgc aagggtgctt gttaagcctt acaaagagaa aggcaaagtc    1320
ccagacaaga agcaacagca gcaacaagtt gagaggggtg agttctcacc atgtggtact    1380
cctactggcc ttgattcaag agatccattt gatctccaac ttggtgcaag aatgttttac    1440
aacacacaag acatgttgtg gaggaggaag ctagaggagc aagctgattt gcagcaagcc    1500
cttgagcttc aaagtagaag attgatgagt ttgcagcttc ttgatgtcaa gaaacatcat    1560
catagggctc tttccactgg cagccctgtc ccctccccaa ctcactctcc aaatattttc    1620
aatcaatctc ttgcctttcc tccactccac agcaacacag aagttccaca agagaattgt    1680
tctagcccaa tgccagccat ttcagtggct gccccaactg aaaaacagat atcaaatgct    1740
aattctggga aagaatgtac tagcagtgaa gagaatggca gtggtaaaga gagctcccat    1800
ggtgaagaca gcgatttaca agaaagtttg gagcacaacc tccctgatag tccctttgca    1860
tctcctacca aaggctccgg ggactactac tctgccttca tccatggagt tcctgacctc    1920
tcccatgaga aggatgctaa catcccggct tcatcttctg ctaacaatag tttggtcact    1980
acaagtctaa tctctcctaa ttcttcacta gaaatggcat ccttcaagtc cttcaattgc    2040
caaatgccca ggttttcatc cgggcatgga gcaataggga tgtatgccaa cacagatgga    2100
cctacctgcc ctgttggaat ttag                                           2124
```

<210> 255
<211> 2196
<212> DNA

<213> Lycopersicon esculentum

<400> 255

```
atggatgctt atgaagctac aaaaattgtt tttcaaagga ttcaaagttt ggatcctgaa      60
aatgcatcaa aaattatggg gattcttctg atgcaagacc atggtgagaa agaaatgatt     120
cgattagctt ttggtccaga agctttagtt cactcggtga ttcttaaagc aagaaaggag     180
cttggtgttt cttcaaactc accttctaca ccttcaactc cttcttcacc ttcacctttt     240
ggtggttcaa tgtgtttttc aaggcagaat tcttcttctt cagctacttc tggtaggatt     300
cttgggggtc ttagccttcc ttcacctctt agcataacta gtaacaacaa ccactcttca     360
aatgtttctg cttcttggag taccagtcct agtttctctg agtttcaaga agctgatctt     420
gttagtccta gtgcttccaa catctcatat actgctgcta ctactactaa tggaatgacc     480
aattccacca tgaattcctc agctcctccc ttttattgca atggtgaagt agacttgata     540
gatgagtttc aactacagga ccagctttct ttcttgaatg atgggtcacc aaccttgggg     600
cctaagaatc ctgatgttta ttaccagcaa cagcagcaac aacaagattt agcctcaagt     660
ccaagtgggg attccatgct tttctcttca tataactggg gtggtggttg caactcagtc     720
aacggcctct ctcatagaag gagctgctct gtgagtgatg tatgcttggg ggctgatgac     780
ccaagtggag gacttggctg gaaaccttgt ctctattttg ccagagggta ttgcaagaat     840
ggaagtagct gtaggttcct tcatggtgct gggcctggtg aaggtgaagt tgggtcacca     900
aacaagtttg agatgatgga acattgccaa gaacttctca gatctaagtc tgctcaccag     960
caaagactag ccacagcttc tcagctcgtg gcttcttcta actttcctct ctctcccatg    1020
gctgctaaca aatgcatgaa ctttcttcag cagcaacagt tgcagtctgc tgaaagccca    1080
agggcagctg ctgcattgat gatgggtgat gacatgcata aattgagcag aagtcgtttt    1140
gaaagagggg attttggact gaatggtgga gttggaatag caaatccagg ttcaaggcaa    1200
atttacttga catttccagc tgatagtact ttcaaagaag aggatgtttc caattatttc    1260
agcacttatg ggcctgttca agatgtgagg attccatatc agcaaaagag gatgtttggt    1320
tttgttacat ttgtttatcc agagactgtg aagaccattc ttgccaaagg aaatcctcat    1380
tttgtatgtg atgctagggt gcttgtcaag ccttacaaag agaagggcaa agtcccagag    1440
aagtttagga agcaacacca acagcagatg gagaggggag aattcactgg atgcggtagt    1500
cctactggtc tggactccag tgatccttat gatcttcagc ttggtgcaag aatgttttac    1560
aacactcaag atgcgctgtg gaggagaaaa ttggaggaac aagctgatct gcaacaggca    1620
attgagctcc aaagcaggag attgctgaat ttacagcttc ttgatgtcaa aaggagcaac    1680
catcatcgtg ccctttccat gagtgctgtt atcccatccc caccgcattc tccaggcttc    1740
ttcaatcaga atatggttcg ctccacagac tttggcagcc gagaagagaa tggttttgca    1800
```

ccaaaaatgg ccaattttgc tgctgttact gctgagcaaa agaatgcaaa tcttactgcc 1860

aaggagagag aatgcttcac aggtaaagat gaaaatagca gtggcaaaga aagttccaag 1920

aaggaagcaa gtgattttca agaaagcttg gagcataatc tcccagatag tccatttgca 1980

tcacctaaag cagttgggga cttcatcaca actttctcaa atgaagctgc tggagatgtt 2040

gacaaaggtg ctggattaaa tgcatcatcc tctgctaaca ataatatgat cccttcttcc 2100

tccttgtcaa ctagtactct agacatgact cctttcaaat catgttactt ccaagtgcct 2160

aggttccctt ccggacatgg cgccattgga atgtag 2196

<210> 256
<211> 731
<212> PRT
<213> Lycopersicon esculentum

<400> 256

Met Asp Ala Tyr Glu Ala Thr Lys Ile Val Phe Gln Arg Ile Gln Ser
1 5 10 15

Leu Asp Pro Glu Asn Ala Ser Lys Ile Met Gly Ile Leu Leu Met Gln
20 25 30

Asp His Gly Glu Lys Glu Met Ile Arg Leu Ala Phe Gly Pro Glu Ala
35 40 45

Leu Val His Ser Val Ile Leu Lys Ala Arg Lys Glu Leu Gly Val Ser
50 55 60

Ser Asn Ser Pro Ser Thr Pro Ser Thr Pro Ser Ser Pro Ser Pro Phe
65 70 75 80

Gly Gly Ser Met Cys Phe Ser Arg Gln Asn Ser Ser Ser Ser Ala Thr
85 90 95

Ser Gly Arg Ile Leu Gly Gly Leu Ser Leu Pro Ser Pro Leu Ser Ile
100 105 110

Thr Ser Asn Asn Asn His Ser Ser Asn Val Ser Ala Ser Trp Ser Thr
115 120 125

Ser Pro Ser Phe Ser Glu Phe Gln Glu Ala Asp Leu Val Ser Pro Ser
130 135 140

Ala Ser Asn Ile Ser Tyr Thr Ala Ala Thr Thr Thr Asn Gly Met Thr
145 150 155 160

Asn Ser Thr Met Asn Ser Ser Ala Pro Pro Phe Tyr Cys Asn Gly Glu

165 170 175

Val Asp Leu Ile Asp Glu Phe Gln Leu Gln Asp Gln Leu Ser Phe Leu
180 185 190

Asn Asp Gly Ser Pro Thr Leu Gly Pro Lys Asn Pro Asp Val Tyr Tyr
195 200 205

Gln Gln Gln Gln Gln Gln Gln Asp Leu Ala Ser Ser Pro Ser Gly Asp
210 215 220

Ser Met Leu Phe Ser Ser Tyr Asn Trp Gly Gly Gly Cys Asn Ser Val
225 230 235 240

Asn Gly Leu Ser His Arg Arg Ser Cys Ser Val Ser Asp Val Cys Leu
245 250 255

Gly Ala Asp Asp Pro Ser Gly Gly Leu Gly Trp Lys Pro Cys Leu Tyr
260 265 270

Phe Ala Arg Gly Tyr Cys Lys Asn Gly Ser Ser Cys Arg Phe Leu His
275 280 285

Gly Ala Gly Pro Gly Glu Gly Glu Val Gly Ser Pro Asn Lys Phe Glu
290 295 300

Met Met Glu His Cys Gln Glu Leu Leu Arg Ser Lys Ser Ala His Gln
305 310 315 320

Gln Arg Leu Ala Thr Ala Ser Gln Leu Val Ala Ser Ser Asn Phe Pro
325 330 335

Leu Ser Pro Met Ala Ala Asn Lys Cys Met Asn Phe Leu Gln Gln Gln
340 345 350

Gln Leu Gln Ser Ala Glu Ser Pro Arg Ala Ala Ala Ala Leu Met Met
355 360 365

Gly Asp Asp Met His Lys Leu Ser Arg Ser Arg Phe Glu Arg Gly Asp
370 375 380

Phe Gly Leu Asn Gly Gly Val Gly Ile Ala Asn Pro Gly Ser Arg Gln
385 390 395 400

Ile Tyr Leu Thr Phe Pro Ala Asp Ser Thr Phe Lys Glu Glu Asp Val
405 410 415

```
Ser Asn Tyr Phe Ser Thr Tyr Gly Pro Val Gln Asp Val Arg Ile Pro
            420                 425                 430

Tyr Gln Gln Lys Arg Met Phe Gly Phe Val Thr Phe Val Tyr Pro Glu
        435                 440                 445

Thr Val Lys Thr Ile Leu Ala Lys Gly Asn Pro His Phe Val Cys Asp
    450                 455                 460

Ala Arg Val Leu Val Lys Pro Tyr Lys Glu Lys Gly Lys Val Pro Glu
465                 470                 475                 480

Lys Phe Arg Lys Gln His Gln Gln Gln Met Glu Arg Gly Glu Phe Thr
                485                 490                 495

Gly Cys Gly Ser Pro Thr Gly Leu Asp Ser Ser Asp Pro Tyr Asp Leu
            500                 505                 510

Gln Leu Gly Ala Arg Met Phe Tyr Asn Thr Gln Asp Ala Leu Trp Arg
        515                 520                 525

Arg Lys Leu Glu Glu Gln Ala Asp Leu Gln Gln Ala Ile Glu Leu Gln
    530                 535                 540

Ser Arg Arg Leu Leu Asn Leu Gln Leu Leu Asp Val Lys Arg Ser Asn
545                 550                 555                 560

His His Arg Ala Leu Ser Met Ser Ala Val Ile Pro Ser Pro Pro His
                565                 570                 575

Ser Pro Gly Phe Phe Asn Gln Asn Met Val Arg Ser Thr Asp Phe Gly
            580                 585                 590

Ser Arg Glu Glu Asn Gly Phe Ala Pro Lys Met Ala Asn Phe Ala Ala
        595                 600                 605

Val Thr Ala Glu Gln Lys Asn Ala Asn Leu Thr Ala Lys Glu Arg Glu
    610                 615                 620

Cys Phe Thr Gly Lys Asp Glu Asn Ser Ser Gly Lys Glu Ser Ser Lys
625                 630                 635                 640

Lys Glu Ala Ser Asp Phe Gln Glu Ser Leu Glu His Asn Leu Pro Asp
                645                 650                 655

Ser Pro Phe Ala Ser Pro Lys Ala Val Gly Asp Phe Ile Thr Thr Phe
            660                 665                 670
```

```
Ser Asn Glu Ala Ala Gly Asp Val Asp Lys Gly Ala Gly Leu Asn Ala
        675                 680                 685

Ser Ser Ser Ala Asn Asn Asn Met Ile Pro Ser Ser Ser Leu Ser Thr
    690                 695                 700

Ser Thr Leu Asp Met Thr Pro Phe Lys Ser Cys Tyr Phe Gln Val Pro
705                 710                 715                 720

Arg Phe Pro Ser Gly His Gly Ala Ile Gly Met
                725                 730


<210>  257
<211>  1866
<212>  DNA
<213>  Pinus radiata

<400>  257
atggatgcct atgaagctac aaggattgtg ttctccagga tccagagctt agagccagaa      60

aatgtgtcta aaattattgg gtacttgtta ttacaagacc atggtgaaca ggaaatgatt     120

aggttggctt tcagtcctga ttccttgatt cagtccatga tcatcaaggt taagaaagat     180

ctaggtttga tgcaacagca aggtactcca gctccaactg tttcatctta cctatccaga     240

atcaatcgtc tccccaactt gccactgcag tctgctcaga tttctcaatc cagagctttc     300

tcttctccaa ccgccctttc acctcatgca gctccatggg gatctcatat ttctcaacag     360

actaggcctt tatccaacaa ttttaactcg atcttgaatg agatacagac taacactagt     420

acttctagta ctataaattc tactagcaat ggctatctta atttcagtct gccatccatg     480

ccagatcaag ctaatagcct tccttacagc gagcatcctg gccttgtaga tgaatttcaa     540

ttgcaagatc agcttccctt tctcaatgat tctccagagt ctgcccaatc tcatgctaat     600

tatctcaact atcctgagat gttgcaggca tattgcaatg gcaatcagcc attgactata     660

gaccatgtaa gcccaacagc agctaataat agctatcctg gtactactca tatacccaag     720

cagcctagtt caatttcaga tatttacaat ctaacttcag aatctgcacc tgggtcagcc     780

ttggcctgga agccatgcat gtactttgct aggggttact gcaagaatgg gagcaattgc     840

aggtttcttc atggtaacta tggtggtcat gtaaggtcag agagcaataa tgaccacagt     900

gaaaagttca tgggatccag tagtggccca ttggaaaaac tggagttaga attgaaggaa     960

ttgctcagag gaagagggtc tccagtttca gttgcttctc tacctcagtt ctatagtgag    1020

aggcttggga aggctcttca ggccgagagg tttacaaggt atagaagtga acgagattca    1080

tctgatcact tggccagttc tgcttccaat tctggatctc gccaaatata cttgactttt    1140

cctgcagaga gtactttcag ggaggaggac gtatcaaatt atttcagcat tttggaccc     1200
```

gtgcaggatg taaggattcc ttatcagcag aagaggatgt ttgggtttgt gacatttgta 1260

tatcaagaga ctgttaagat tattttggca aaaggcaatc ctcattatgt ctgtgatgcc 1320

cgtgttcttg tcaaaccata caaagaaaaa ggatccaaac ccgcagagag aatgaagtat 1380

accgactgta ggggcgatta ttcaggatat gtgacaactc acaatcttga tatcaaggac 1440

agcaatttgc aacttggccc tcccagattt gttgaaaaca gcttagacct ggtgacaaga 1500

aggcagttgg aggaggagca ggatcatgta gagcaagccg ttgagcttca aacaaaacga 1560

cttgcagagc tgcagcttgg tgacagaaag agaccacagc ttgtaccttc agatcctcaa 1620

gtttctatgg cttcaacaaa ctccggcccg gctcagcatt atcaaaatca gttctcaaat 1680

ggacccaata atcattcaga agaggacgca acaacctcag aagattttag cagttctaca 1740

ttagcagaac attttggtta tgtgctacag gttttagata gtgaatctgt ctatgaggaa 1800

cacccaaaac ctgtcaacca tcaccatgac aggcttccta ttactaatgg tgcaatgaga 1860

ctgtaa 1866

<210> 258
<211> 621
<212> PRT
<213> Pinus radiata

<400> 258

Met Asp Ala Tyr Glu Ala Thr Arg Ile Val Phe Ser Arg Ile Gln Ser
1 5 10 15

Leu Glu Pro Glu Asn Val Ser Lys Ile Ile Gly Tyr Leu Leu Leu Gln
20 25 30

Asp His Gly Glu Gln Glu Met Ile Arg Leu Ala Phe Ser Pro Asp Ser
35 40 45

Leu Ile Gln Ser Met Ile Ile Lys Val Lys Lys Asp Leu Gly Leu Met
50 55 60

Gln Gln Gln Gly Thr Pro Ala Pro Thr Val Ser Ser Tyr Leu Ser Arg
65 70 75 80

Ile Asn Arg Leu Pro Asn Leu Pro Leu Gln Ser Ala Gln Ile Ser Gln
85 90 95

Ser Arg Ala Phe Ser Ser Pro Thr Ala Leu Ser Pro His Ala Ala Pro
100 105 110

Trp Gly Ser His Ile Ser Gln Gln Thr Arg Pro Leu Ser Asn Asn Phe
115 120 125

Asn Ser Ile Leu Asn Glu Ile Gln Thr Asn Thr Ser Thr Ser Ser Thr
130 135 140

Ile Asn Ser Thr Ser Asn Gly Tyr Leu Asn Phe Ser Leu Pro Ser Met
145 150 155 160

Pro Asp Gln Ala Asn Ser Leu Pro Tyr Ser Glu His Pro Gly Leu Val
165 170 175

Asp Glu Phe Gln Leu Gln Asp Gln Leu Pro Phe Leu Asn Asp Ser Pro
180 185 190

Glu Ser Ala Gln Ser His Ala Asn Tyr Leu Asn Tyr Pro Glu Met Leu
195 200 205

Gln Ala Tyr Cys Asn Gly Asn Gln Pro Leu Thr Ile Asp His Val Ser
210 215 220

Pro Thr Ala Ala Asn Asn Ser Tyr Pro Gly Thr Thr His Ile Pro Lys
225 230 235 240

Gln Pro Ser Ser Ile Ser Asp Ile Tyr Asn Leu Thr Ser Glu Ser Ala
245 250 255

Pro Gly Ser Ala Leu Ala Trp Lys Pro Cys Met Tyr Phe Ala Arg Gly
260 265 270

Tyr Cys Lys Asn Gly Ser Asn Cys Arg Phe Leu His Gly Asn Tyr Gly
275 280 285

Gly His Val Arg Ser Glu Ser Asn Asn Asp His Ser Glu Lys Phe Met
290 295 300

Gly Ser Ser Ser Gly Pro Leu Glu Lys Leu Glu Leu Glu Leu Lys Glu
305 310 315 320

Leu Leu Arg Gly Arg Gly Ser Pro Val Ser Val Ala Ser Leu Pro Gln
325 330 335

Phe Tyr Ser Glu Arg Leu Gly Lys Ala Leu Gln Ala Glu Arg Phe Thr
340 345 350

Arg Tyr Arg Ser Glu Arg Asp Ser Ser Asp His Leu Ala Ser Ser Ala
355 360 365

Ser Asn Ser Gly Ser Arg Gln Ile Tyr Leu Thr Phe Pro Ala Glu Ser
370 375 380

Thr Phe Arg Glu Glu Asp Val Ser Asn Tyr Phe Ser Ile Phe Gly Pro
385 390 395 400

Val Gln Asp Val Arg Ile Pro Tyr Gln Gln Lys Arg Met Phe Gly Phe
405 410 415

Val Thr Phe Val Tyr Gln Glu Thr Val Lys Ile Ile Leu Ala Lys Gly
420 425 430

Asn Pro His Tyr Val Cys Asp Ala Arg Val Leu Val Lys Pro Tyr Lys
435 440 445

Glu Lys Gly Ser Lys Pro Ala Glu Arg Met Lys Tyr Thr Asp Cys Arg
450 455 460

Gly Asp Tyr Ser Gly Tyr Val Thr Thr His Asn Leu Asp Ile Lys Asp
465 470 475 480

Ser Asn Leu Gln Leu Gly Pro Pro Arg Phe Val Glu Asn Ser Leu Asp
485 490 495

Leu Val Thr Arg Arg Gln Leu Glu Glu Glu Gln Asp His Val Glu Gln
500 505 510

Ala Val Glu Leu Gln Thr Lys Arg Leu Ala Glu Leu Gln Leu Gly Asp
515 520 525

Arg Lys Arg Pro Gln Leu Val Pro Ser Asp Pro Gln Val Ser Met Ala
530 535 540

Ser Thr Asn Ser Gly Pro Ala Gln His Tyr Gln Asn Gln Phe Ser Asn
545 550 555 560

Gly Pro Asn Asn His Ser Glu Glu Asp Ala Thr Thr Ser Glu Asp Phe
565 570 575

Ser Ser Ser Thr Leu Ala Glu His Phe Gly Tyr Val Leu Gln Val Leu
580 585 590

Asp Ser Glu Ser Val Tyr Glu Glu His Pro Lys Pro Val Asn His His
595 600 605

His Asp Arg Leu Pro Ile Thr Asn Gly Ala Met Arg Leu
610 615 620

<210> 259

<211> 2274
<212> DNA
<213> Eucalyptus grandis

<400> 259
atggacgcat atgaagccac aaggattgtc ttctcaagaa tccaaagttt agaccctgag 60

aatgcctcca agatcatggg tctcctcctc atccaagatc atggtgagaa ggagatgatc 120

aggctggctc ttggaccgga gactctgctt cactcagtgg tcctcaaggc aaggaaggac 180

ataatccttc cgtcaaactc tccctcaacg ccctccacac cttcttctcc ctctcctttc 240

atgtctacca accccatctc catctcctcc aggcctaaag gaagcaactt ttcgccatct 300

tctctctcaa atatccccag cccatcttct tggggggtg gtggtggtgg tggtggttct 360

ttctctgatc tctcaagtgg agatgatttg atcaattctt cctcttgttt gtatggaaat 420

ggaggcagtg acaccatgat tgatgagctt cagctccaag accagctttc cttcctcaac 480

gataactccc caccccttgg acccaacagc aaccctgata tgttctgccc ccagcaggac 540

ttgctgtcca gtcccaccgc cgtatacggc ggagcggccg cgggctgggg cgcccggtg 600

caccggagga gctgctcggt cagcgatgtg tgctcgggtt cctcagaaga cccatcttgt 660

ggagtcgggt ggaggccatg cttgtattat gctagagggt actgcaagaa tgggatcagc 720

tgcaggttct tgcacagtgg tggacttggc gatgccgctt ctgtggtcgg cagcccggac 780

ggcagtgcgt ccgcggtggt cggctccccg agtaaagtgg acatgatggg ccagtgccat 840

gaagctgttc tgaggtccaa atctgctcag cagcagagac tagctgctgc ttctcagctc 900

ataggttctg caaccttccc ttacactccc aaatccatga atttacttct ccatcaccag 960

caaaatgatg ctcatagggc tgctgctgct gctgcgctga tgatgggtga tgacttttac 1020

aagtatggca gatcaaggct agaaaggagt gatttttcag tgaatggttg tgtgaatcct 1080

gcttctaggc agatttactt gactttccca gccgacagta ctttcaagga ggaagatgtt 1140

tccaactatt tcagcaactt tgggccggtg caagatgtga gaattcctta ccagcagaag 1200

aggatgtttg gctttgttac atttgtttac ccagaaacgg tgaagctcat tttggccaaa 1260

gggaaccctc attttgtttg tgatgctaga gttctcgtca agccttacaa agagaaggga 1320

aaagtgccag acaagttcag gaagcagtcc cagctggtgg aaaggggtga tttttcgcct 1380

tgtggaactc caactgggtt ggattcgagg gggggaccat ttgacctcaa cctcggagcg 1440

aggccgtttt acaattctca ggacatgctg tggaggagga gattcgagga gcaagctgat 1500

cttcaacaag cccttgaata ccagagtcaa cggctgatga gtctgcagct tctagatgtc 1560

aagaagcatc atcatcagag ggctctctca actggctccc ccattccatc tcctgctcaa 1620

tcgcctactt tgttcaacaa tccaaccttc ctcaacattc cttcggttcg cagcctgggc 1680

gtcacagaag agaatggttc tagccctggc ttatccgaca gtcagccctt gaactaccag 1740

tctgtgattg tctctgctgg gaaagatttg actggaagtg acaagagtaa tgggaatgac 1800

aaggaaagct cccatactga agataaaggc ttggctgaaa gtttggagca taaccttcct 1860

gatagtccct ttgcatctcc tactaaagcc tccgcagagc acttctcttc cttaaccaat 1920

gtagtcagcg aggctgaaaa ggatggtgta ggttcggcct catcttctcc caacagcaat 1980

aacccagtct cttcaccctt gatcccgggc acctccgcca tggacatggc ttcattcacg 2040

tctttcaact gccagattcc tgccatagga atgtatgccg gtgctggagg gccaacatgc 2100

ccagtgggta tatagctagc tcttccttga ctgagggaga ttaacaacaa taaccaaaca 2160

aacccgttat caaagaccag atctgtagag aatccgtacc actaccatca cctccaccac 2220

tacctcgatt atccatacta tactactgga taccatacaa aaatgcatac gtaa 2274

<210> 260
<211> 755
<212> PRT
<213> Eucalyptus grandis

<400> 260

Met Asp Ala Tyr Glu Ala Thr Arg Ile Val Phe Ser Arg Ile Gln Ser
1 5 10 15

Leu Asp Pro Glu Asn Ala Ser Lys Ile Met Gly Leu Leu Leu Ile Gln
20 25 30

Asp His Gly Glu Lys Glu Met Ile Arg Leu Ala Leu Gly Pro Glu Thr
35 40 45

Leu Leu His Ser Val Val Leu Lys Ala Arg Lys Asp Ile Ile Leu Pro
50 55 60

Ser Asn Ser Pro Ser Thr Pro Ser Thr Pro Ser Ser Pro Ser Pro Phe
65 70 75 80

Met Ser Thr Asn Pro Ile Ser Ile Ser Ser Arg Pro Lys Gly Ser Asn
85 90 95

Phe Ser Pro Ser Ser Leu Ser Asn Ile Pro Ser Pro Ser Ser Trp Gly
100 105 110

Gly Gly Gly Gly Gly Gly Gly Ser Phe Ser Asp Leu Ser Ser Gly Asp
115 120 125

Asp Leu Ile Asn Ser Ser Ser Cys Leu Tyr Gly Asn Gly Gly Ser Asp
130 135 140

Thr Met Ile Asp Glu Leu Gln Leu Gln Asp Gln Leu Ser Phe Leu Asn

145 150 155 160

Asp Asn Ser Pro Pro Leu Gly Pro Asn Ser Asn Pro Asp Met Phe Cys
165 170 175

Pro Gln Gln Asp Leu Leu Ser Ser Pro Thr Ala Val Tyr Gly Gly Ala
180 185 190

Ala Ala Gly Trp Gly Ala Pro Val His Arg Arg Ser Cys Ser Val Ser
195 200 205

Asp Val Cys Ser Gly Ser Ser Glu Asp Pro Ser Cys Gly Val Gly Trp
210 215 220

Arg Pro Cys Leu Tyr Tyr Ala Arg Gly Tyr Cys Lys Asn Gly Ile Ser
225 230 235 240

Cys Arg Phe Leu His Ser Gly Gly Leu Gly Asp Ala Ala Ser Val Val
245 250 255

Gly Ser Pro Asp Gly Ser Ala Ser Ala Val Val Gly Ser Pro Ser Lys
260 265 270

Val Asp Met Met Gly Gln Cys His Glu Ala Val Leu Arg Ser Lys Ser
275 280 285

Ala Gln Gln Gln Arg Leu Ala Ala Ala Ser Gln Leu Ile Gly Ser Ala
290 295 300

Thr Phe Pro Tyr Thr Pro Lys Ser Met Asn Leu Leu Leu His His Gln
305 310 315 320

Gln Asn Asp Ala His Arg Ala Ala Ala Ala Ala Ala Leu Met Met Gly
325 330 335

Asp Asp Phe Tyr Lys Tyr Gly Arg Ser Arg Leu Glu Arg Ser Asp Phe
340 345 350

Ser Val Asn Gly Cys Val Asn Pro Ala Ser Arg Gln Ile Tyr Leu Thr
355 360 365

Phe Pro Ala Asp Ser Thr Phe Lys Glu Glu Asp Val Ser Asn Tyr Phe
370 375 380

Ser Asn Phe Gly Pro Val Gln Asp Val Arg Ile Pro Tyr Gln Gln Lys
385 390 395 400

Arg Met Phe Gly Phe Val Thr Phe Val Tyr Pro Glu Thr Val Lys Leu
405 410 415

Ile Leu Ala Lys Gly Asn Pro His Phe Val Cys Asp Ala Arg Val Leu
420 425 430

Val Lys Pro Tyr Lys Glu Lys Gly Lys Val Pro Asp Lys Phe Arg Lys
435 440 445

Gln Ser Gln Leu Val Glu Arg Gly Asp Phe Ser Pro Cys Gly Thr Pro
450 455 460

Thr Gly Leu Asp Ser Arg Gly Gly Pro Phe Asp Leu Asn Leu Gly Ala
465 470 475 480

Arg Pro Phe Tyr Asn Ser Gln Asp Met Leu Trp Arg Arg Arg Phe Glu
485 490 495

Glu Gln Ala Asp Leu Gln Gln Ala Leu Glu Tyr Gln Ser Gln Arg Leu
500 505 510

Met Ser Leu Gln Leu Leu Asp Val Lys Lys His His His Gln Arg Ala
515 520 525

Leu Ser Thr Gly Ser Pro Ile Pro Ser Pro Ala Gln Ser Pro Thr Leu
530 535 540

Phe Asn Asn Pro Thr Phe Leu Asn Ile Pro Ser Val Arg Ser Leu Gly
545 550 555 560

Val Thr Glu Glu Asn Gly Ser Ser Pro Gly Leu Ser Asp Ser Gln Pro
565 570 575

Leu Asn Tyr Gln Ser Val Ile Val Ser Ala Gly Lys Asp Leu Thr Gly
580 585 590

Ser Asp Lys Ser Asn Gly Asn Asp Lys Glu Ser Ser His Thr Glu Asp
595 600 605

Lys Gly Leu Ala Glu Ser Leu Glu His Asn Leu Pro Asp Ser Pro Phe
610 615 620

Ala Ser Pro Thr Lys Ala Ser Ala Glu His Phe Ser Ser Leu Thr Asn
625 630 635 640

Val Val Ser Glu Ala Glu Lys Asp Gly Val Gly Ser Ala Ser Ser Ser
645 650 655

Pro Asn Ser Asn Asn Pro Val Ser Ser Pro Leu Ile Pro Gly Thr Ser
660 665 670

Ala Met Asp Met Ala Ser Phe Thr Ser Phe Asn Cys Gln Ile Pro Ala
675 680 685

Ile Gly Met Tyr Ala Gly Ala Gly Gly Pro Thr Cys Pro Val Gly Ile
690 695 700

Leu Ala Leu Pro Leu Arg Glu Ile Asn Asn Asn Asn Gln Thr Asn Pro
705 710 715 720

Leu Ser Lys Thr Arg Ser Val Glu Asn Pro Tyr His Tyr His His Leu
725 730 735

His His Tyr Leu Asp Tyr Pro Tyr Tyr Thr Thr Gly Tyr His Thr Lys
740 745 750

Met His Thr
755

<210> 261
<211> 1806
<212> DNA
<213> Pinus radiata

<400> 261
atggatacat atgaagcaac aaggattgtg ttcacaagga ttcagagcat agaaccagag 60

aatgtgtcca agatcattgg gtatttgctt cttcaagacc tgggagatca agaaatgatt 120

cgcctggcat ttgggcctaa tacactctta cagtccatga tttccaaggc caagacagag 180

cttggtttat catctccgtc ccctcttcag tcacctctgc gctacactca gttttctaat 240

ttgttgtctc gctcattctc ttctccagca cccaatggct ttgatgattg tcagctccaa 300

gatcatctct tctatcccac tgagaatctt gattcctaca gcctaccaaa tgatagtcat 360

gtctatacag agatgctctc tttcttgaat ggaagcaaga caggattgaa tcacaggcga 420

tcttactcca tgacagacgt ttctgtaagc tgtgagccag tttcttggaa accatgcctg 480

tattttgcca ggggatattg caagcatgga tccagctgcc gttttactca cagttattca 540

cgctctgaca acgtttcgag ttcaattccc ttggatcccc gcttcgaaga agctttctct 600

gtggaatcat tggaaagatt agagttagaa cttcaagaat tattgagagg aagacgggcc 660

cctgtttcca ttgcttccct acctcaactc tattacgaga gatttgggaa aaccctgcaa 720

gccgagggat acttgactga gagtcagagg catgggaaag caggatacag cttgacaaat 780

ctactagcac gcttgaagaa tacagtaagc ctcattgata ggcctcatgg tcagcacgcc 840

attgttttgg cagaggatgc taacaggttc acaacttata ggccaagtga acgagatccc 900

tactatttga gtggtgtcag ctctggatcc cgtcaaattt acatgacatt tcctgctgaa 960

agcaccttca cggaggagga tgtttccaac tatttcagga tatacggacc tgtagaggat 1020

gtgagaattc cataccaaca gaagcgtatg tttggatttg taacatatgt tttcccagag 1080

actgtcaaat tgatactggc taaaggaaat cctcactatg tctgcggtgc tcgtgttctg 1140

gttaagccat acaaggagag aaacaagcat ggagacagga aaaatggcga tagaggagaa 1200

caatatgcaa gatacttgct gccatcttac aatgtagatt caaaggacta tgatctatgt 1260

ccagctccta ggatgtttca gaattccgaa ctgatcagaa ggcatataga agagcaagag 1320

caagccatcg aattggaaag actacgcctg acagagttgc atctggctga ccgtgcgcag 1380

agaactcaaa ataatgccat cactctgcaa caacaaaatt ctcattctaa tgggctactc 1440

aatgtagagg aagaagaaac tcaggtttca gaagagctaa acagctttga tccgcccacg 1500

gatcactttg gttatctgct ggatgtgctg gatagtgagc agaaccctga agaagagcct 1560

aaacaacaga aagccgacaa tgacgaagaa tgcaacgggc acaaccttcc tgatagccct 1620

tttgggtttt ctcattccat taaaacaaca ttgccccatc ccgagaaaac gaacaacttt 1680

tcctttttcg acaccagccc agcacaagaa tcatccactt ccatcatgac aaaggaaggg 1740

acttgttcca tgtgcctcga ttccattgtg gagcaggtgc ggttagagtg caagcatgtg 1800

aggtga 1806

<210> 262
<211> 601
<212> PRT
<213> Pinus radiata

<400> 262

Met Asp Thr Tyr Glu Ala Thr Arg Ile Val Phe Thr Arg Ile Gln Ser
1 5 10 15

Ile Glu Pro Glu Asn Val Ser Lys Ile Ile Gly Tyr Leu Leu Leu Gln
20 25 30

Asp Leu Gly Asp Gln Glu Met Ile Arg Leu Ala Phe Gly Pro Asn Thr
35 40 45

Leu Leu Gln Ser Met Ile Ser Lys Ala Lys Thr Glu Leu Gly Leu Ser
50 55 60

Ser Pro Ser Pro Leu Gln Ser Pro Leu Arg Tyr Thr Gln Phe Ser Asn
65 70 75 80

Leu Leu Ser Arg Ser Phe Ser Ser Pro Ala Pro Asn Gly Phe Asp Asp

85 90 95

Cys Gln Leu Gln Asp His Leu Phe Tyr Pro Thr Glu Asn Leu Asp Ser
100 105 110

Tyr Ser Leu Pro Asn Asp Ser His Val Tyr Thr Glu Met Leu Ser Phe
115 120 125

Leu Asn Gly Ser Lys Thr Gly Leu Asn His Arg Arg Ser Tyr Ser Met
130 135 140

Thr Asp Val Ser Val Ser Cys Glu Pro Val Ser Trp Lys Pro Cys Leu
145 150 155 160

Tyr Phe Ala Arg Gly Tyr Cys Lys His Gly Ser Ser Cys Arg Phe Thr
165 170 175

His Ser Tyr Ser Arg Ser Asp Asn Val Ser Ser Ser Ile Pro Leu Asp
180 185 190

Pro Arg Phe Glu Glu Ala Phe Ser Val Glu Ser Leu Glu Arg Leu Glu
195 200 205

Leu Glu Leu Gln Glu Leu Leu Arg Gly Arg Arg Ala Pro Val Ser Ile
210 215 220

Ala Ser Leu Pro Gln Leu Tyr Tyr Glu Arg Phe Gly Lys Thr Leu Gln
225 230 235 240

Ala Glu Gly Tyr Leu Thr Glu Ser Gln Arg His Gly Lys Ala Gly Tyr
245 250 255

Ser Leu Thr Asn Leu Leu Ala Arg Leu Lys Asn Thr Val Ser Leu Ile
260 265 270

Asp Arg Pro His Gly Gln His Ala Ile Val Leu Ala Glu Asp Ala Asn
275 280 285

Arg Phe Thr Thr Tyr Arg Pro Ser Glu Arg Asp Pro Tyr Tyr Leu Ser
290 295 300

Gly Val Ser Ser Gly Ser Arg Gln Ile Tyr Met Thr Phe Pro Ala Glu
305 310 315 320

Ser Thr Phe Thr Glu Glu Asp Val Ser Asn Tyr Phe Arg Ile Tyr Gly
325 330 335

```
Pro Val Glu Asp Val Arg Ile Pro Tyr Gln Gln Lys Arg Met Phe Gly
            340                 345                 350

Phe Val Thr Tyr Val Phe Pro Glu Thr Val Lys Leu Ile Leu Ala Lys
        355                 360                 365

Gly Asn Pro His Tyr Val Cys Gly Ala Arg Val Leu Val Lys Pro Tyr
    370                 375                 380

Lys Glu Arg Asn Lys His Gly Asp Arg Lys Asn Gly Asp Arg Gly Glu
385                 390                 395                 400

Gln Tyr Ala Arg Tyr Leu Leu Pro Ser Tyr Asn Val Asp Ser Lys Asp
                405                 410                 415

Tyr Asp Leu Cys Pro Ala Pro Arg Met Phe Gln Asn Ser Glu Leu Ile
            420                 425                 430

Arg Arg His Ile Glu Glu Gln Glu Gln Ala Ile Glu Leu Glu Arg Leu
        435                 440                 445

Arg Leu Thr Glu Leu His Leu Ala Asp Arg Ala Gln Arg Thr Gln Asn
    450                 455                 460

Asn Ala Ile Thr Leu Gln Gln Gln Asn Ser His Ser Asn Gly Leu Leu
465                 470                 475                 480

Asn Val Glu Glu Glu Glu Thr Gln Val Ser Glu Glu Leu Asn Ser Phe
                485                 490                 495

Asp Pro Pro Thr Asp His Phe Gly Tyr Leu Leu Asp Val Leu Asp Ser
            500                 505                 510

Glu Gln Asn Pro Glu Glu Glu Pro Lys Gln Gln Lys Ala Asp Asn Asp
        515                 520                 525

Glu Glu Cys Asn Gly His Asn Leu Pro Asp Ser Pro Phe Gly Phe Ser
    530                 535                 540

His Ser Ile Lys Thr Thr Leu Pro His Pro Glu Lys Thr Asn Asn Phe
545                 550                 555                 560

Ser Phe Phe Asp Thr Ser Pro Ala Gln Glu Ser Ser Thr Ser Ile Met
                565                 570                 575

Thr Lys Glu Gly Thr Cys Ser Met Cys Leu Asp Ser Ile Val Glu Gln
            580                 585                 590
```

```
Val Arg Leu Glu Cys Lys His Val Arg
        595                 600


<210>  263
<211>  2124
<212>  DNA
<213>  Populus trichocarpa

<400>  263
atggatggtt atgaagcaac aagaatagtt ttctcgagaa tccaaaacct agacccagaa      60

aatgcttcaa aaatcatggg tcttcttttg attcaggacc atggtgaaaa ggaaatgatt     120

aggttagctt ttggaccaga agcacttgtt cactcagtaa tccttaaagc gaggaaagaa     180

ctaggacttt gctctccaac aaacccttct aaaagtcctt cgccccccttc gcctctatat     240

tcaagcaacc caataaccat ctctagacag aattcatctt cttcaacttc aagacttggg     300

tttaacatcc caccttcact tactatccca aacccttcat caaatttttc ttcttcttgg     360

agtgaccttc caaaccctga tgacttgatt agtcctaatg gtagttcact caatcctgct     420

tctgctcctt tctatgctaa tggagtaaga ggtggaggag agtctgattt gatggatgag     480

tttcagctcc aagaccagct ttcattcttg aatgacaatt cagcaaatct tggtccaaaa     540

agctcagatc ttttttactc tcaactggat gctttatcaa gtccaactgg tgctagtgat     600

tctgtgatgt ttccttctta ctggggtggg tctgtgcaca gaaggagctg ttctgtcagt     660

gatgttttgg ggtctgagga tccaaattca ggctttgggt ggagaccttg cctttacttt     720

gctagagggt actgtaagaa tggaagtaac tgtaggtttg ttcacggtgg gctcggagaa     780

tctgatggtg caggtgttgt tgtgggttca cctaatggta acaacaagat tgatatgatg     840

gaccagtgcc atgagttgct tagatccaag tctgctcaac agcaaaggtt agctgctgct     900

tctcagctca tgggtggctc tgctgcttct tttccttact ctcctaaaag catgaacttt     960

cttcttcaac aacagcaaaa tgatagccag agggctgctg ctgctttgat gatgggggag    1020

gacatgcaca aatttgcaag atctaggctt gataggaatg atttgattaa tcctgcttcc    1080

aggcagatct acttgacttt ccctgctgat agcactttta gagaggaaga tgtgtcaaat    1140

tacttcagta tttatgggcc agtgcaagat gtgaggattc cttatcagca gaagaggatg    1200

tttggatttg ttacctttt gtatccagag accgtgaaga taatattggc caaagggaac    1260

cctcattttg tttgtgatgc aagggtgctt gttaagcctt acaaagagaa aggcaaagtc    1320

ccagacaaga agcaacagca gcaacaagtt gagaggggtg agttctcacc atgtggtact    1380

cctactggcc ttgattcaag agatccattt gatctccaac ttggtgcaag aatgttttac    1440

aacacacaag acatgttgtg gaggaggaag ctagaggagc aagctgattt gcagcaagcc    1500

cttgagcttc aaagtagaag attgatgagt ttgcagcttc ttgatgtcaa gaaacatcat    1560
```

```
catagggctc tttccactgg cagccctgtc ccctccccaa ctcactctcc aaatattttc   1620

aatcaatctc ttgcctttcc tccactccac agcaacacag aagttccaca agagaattgt   1680

tctagcccaa tgccagccat ttcagtggct gccccaactg aaaaacagat atcaaatgct   1740

aattctggga aagaatgtac tagcagtgaa gagaatggca gtggtaaaga gagctcccat   1800

ggtgaagaca gcgatttaca agaaagtttg gagcacaacc tccctgatag tccctttgca   1860

tctcctacca aaggctccgg ggactactac tctgccttca tccatggagt tcctgacctc   1920

tcccatgaga aggatgctaa catcccggct tcatcttctg ctaacaatag tttggtcact   1980

acaagtctaa tctctcctaa ttcttcacta gaaatggcat ccttcaagtc cttcaattgc   2040

caaatgccca ggttttcatc cgggcatgga gcaataggga tgtatgccaa cacagatgga   2100

cctacctgcc ctgttggaat ttag                                          2124
```

```
<210>  264
<211>  707
<212>  PRT
<213>  Populus trichocarpa

<400>  264

Met Asp Gly Tyr Glu Ala Thr Arg Ile Val Phe Ser Arg Ile Gln Asn
1               5                   10                  15

Leu Asp Pro Glu Asn Ala Ser Lys Ile Met Gly Leu Leu Leu Ile Gln
            20                  25                  30

Asp His Gly Glu Lys Glu Met Ile Arg Leu Ala Phe Gly Pro Glu Ala
        35                  40                  45

Leu Val His Ser Val Ile Leu Lys Ala Arg Lys Glu Leu Gly Leu Cys
    50                  55                  60

Ser Pro Thr Asn Pro Ser Lys Ser Pro Ser Pro Pro Ser Pro Leu Tyr
65                  70                  75                  80

Ser Ser Asn Pro Ile Thr Ile Ser Arg Gln Asn Ser Ser Ser Ser Thr
                85                  90                  95

Ser Arg Leu Gly Phe Asn Ile Pro Pro Ser Leu Thr Ile Pro Asn Pro
            100                 105                 110

Ser Ser Asn Phe Ser Ser Ser Trp Ser Asp Leu Pro Asn Pro Asp Asp
        115                 120                 125

Leu Ile Ser Pro Asn Gly Ser Ser Leu Asn Pro Ala Ser Ala Pro Phe
    130                 135                 140
```

Tyr Ala Asn Gly Val Arg Gly Gly Gly Glu Ser Asp Leu Met Asp Glu
145 150 155 160

Phe Gln Leu Gln Asp Gln Leu Ser Phe Leu Asn Asp Asn Ser Ala Asn
165 170 175

Leu Gly Pro Lys Ser Ser Asp Leu Phe Tyr Ser Gln Leu Asp Ala Leu
180 185 190

Ser Ser Pro Thr Gly Ala Ser Asp Ser Val Met Phe Pro Ser Tyr Trp
195 200 205

Gly Gly Ser Val His Arg Arg Ser Cys Ser Val Ser Asp Val Leu Gly
210 215 220

Ser Glu Asp Pro Asn Ser Gly Phe Gly Trp Arg Pro Cys Leu Tyr Phe
225 230 235 240

Ala Arg Gly Tyr Cys Lys Asn Gly Ser Asn Cys Arg Phe Val His Gly
245 250 255

Gly Leu Gly Glu Ser Asp Gly Ala Gly Val Val Val Gly Ser Pro Asn
260 265 270

Gly Asn Asn Lys Ile Asp Met Met Asp Gln Cys His Glu Leu Leu Arg
275 280 285

Ser Lys Ser Ala Gln Gln Gln Arg Leu Ala Ala Ala Ser Gln Leu Met
290 295 300

Gly Gly Ser Ala Ala Ser Phe Pro Tyr Ser Pro Lys Ser Met Asn Phe
305 310 315 320

Leu Leu Gln Gln Gln Gln Asn Asp Ser Gln Arg Ala Ala Ala Ala Leu
325 330 335

Met Met Gly Glu Asp Met His Lys Phe Ala Arg Ser Arg Leu Asp Arg
340 345 350

Asn Asp Leu Ile Asn Pro Ala Ser Arg Gln Ile Tyr Leu Thr Phe Pro
355 360 365

Ala Asp Ser Thr Phe Arg Glu Glu Asp Val Ser Asn Tyr Phe Ser Ile
370 375 380

Tyr Gly Pro Val Gln Asp Val Arg Ile Pro Tyr Gln Gln Lys Arg Met
385 390 395 400

Phe Gly Phe Val Thr Phe Leu Tyr Pro Glu Thr Val Lys Ile Ile Leu
405 410 415

Ala Lys Gly Asn Pro His Phe Val Cys Asp Ala Arg Val Leu Val Lys
420 425 430

Pro Tyr Lys Glu Lys Gly Lys Val Pro Asp Lys Lys Gln Gln Gln Gln
435 440 445

Gln Val Glu Arg Gly Glu Phe Ser Pro Cys Gly Thr Pro Thr Gly Leu
450 455 460

Asp Ser Arg Asp Pro Phe Asp Leu Gln Leu Gly Ala Arg Met Phe Tyr
465 470 475 480

Asn Thr Gln Asp Met Leu Trp Arg Arg Lys Leu Glu Glu Gln Ala Asp
485 490 495

Leu Gln Gln Ala Leu Glu Leu Gln Ser Arg Arg Leu Met Ser Leu Gln
500 505 510

Leu Leu Asp Val Lys Lys His His His Arg Ala Leu Ser Thr Gly Ser
515 520 525

Pro Val Pro Ser Pro Thr His Ser Pro Asn Ile Phe Asn Gln Ser Leu
530 535 540

Ala Phe Pro Pro Leu His Ser Asn Thr Glu Val Pro Gln Glu Asn Cys
545 550 555 560

Ser Ser Pro Met Pro Ala Ile Ser Val Ala Ala Pro Thr Glu Lys Gln
565 570 575

Ile Ser Asn Ala Asn Ser Gly Lys Glu Cys Thr Ser Ser Glu Glu Asn
580 585 590

Gly Ser Gly Lys Glu Ser Ser His Gly Glu Asp Ser Asp Leu Gln Glu
595 600 605

Ser Leu Glu His Asn Leu Pro Asp Ser Pro Phe Ala Ser Pro Thr Lys
610 615 620

Gly Ser Gly Asp Tyr Tyr Ser Ala Phe Ile His Gly Val Pro Asp Leu
625 630 635 640

Ser His Glu Lys Asp Ala Asn Ile Pro Ala Ser Ser Ser Ala Asn Asn

645 650 655

Ser Leu Val Thr Thr Ser Leu Ile Ser Pro Asn Ser Ser Leu Glu Met
660 665 670

Ala Ser Phe Lys Ser Phe Asn Cys Gln Met Pro Arg Phe Ser Ser Gly
675 680 685

His Gly Ala Ile Gly Met Tyr Ala Asn Thr Asp Gly Pro Thr Cys Pro
690 695 700

Val Gly Ile
705

<210> 265
<211> 2199
<212> DNA
<213> Populus trichocarpa

<400> 265
atggatgctt atgaagcaac aagaatagtt ttctcaagaa tccaaaatct agacccagaa 60

aatgcttcaa agatcatggg tcttcttttg attcaagacc atggtgaaaa ggaaatgatt 120

aggttagctt ttggaccaga agcacttgtt cactcagtga tccttaaagc caggaaagaa 180

ctaggactaa gctctccgac aaacctttct acaagtcctt cctctccttc tcctctttac 240

tcaagcaacc caatagccat ctctagacaa aatagttctt caacttcaag acttgggttt 300

aatatcccac cttcacttgc tatcccaaac ccttcatcaa ataattcttc ttcttggagt 360

gaccttccaa acccagatga cttgatgatt agtcctaatg atagctcact caatcctgct 420

tcagtgcctt tctatgctaa tggagtaaga ggtggagagt ctgatttgat ggatgagttt 480

cagctccaag accagctttc attcttaaat gataattcac aaaatctcgg tccaaaaagt 540

tcagatcttt tttaccctca gcttgatgct ctatcaagtc caactggtgc tagtgattct 600

atgatgtttc cttcttactg ggtgggtct gtgcacagaa ggagctgctc tgtcagtgat 660

gttttggggt ctgaggatcc aaattcaggg tttggatgga gaccatgtct ttactttgct 720

agagggtact gtaagaatgg aagtaattgt aggtttgttc atggtgggct tggagaacta 780

gatggtgcag gtgttgtcgg ttcacccaat agcaacaaca agattgatat gatggaccag 840

tgccatgagt tgcttagatc taagtctgct caccagcaaa ggttagctgc tgcttctcag 900

ctcatgagta gctctgctgc ttcttttcct tactctccta aaagcatgaa ctttcttctt 960

caacagcagc aaaatgatag ccagagggct gctgctactg ctttgatgat gggggaggac 1020

atgcacaaat ttggaagatc taggcttgac aggaatgatt tggttaatcc tgcttcaagg 1080

cagatctact tgactttccc agcggatagc acttttagag aggaagacgt gtcaaattat 1140

ttcagtattt atgggccagt gcaagatgtg aggattcctt atcagcagaa gaggatgttt 1200

ggatttgtta cctttgttta tccagagacg gtgaagataa tcttggccaa agggaatcct 1260

cattttgttt gtgatgcaag ggtgcttgtt aagccataca aagagaaagg caaagtccca 1320

gacaagaagc aacagcagca acaagttgag aggggtgagt tctcaccttg cggtactcct 1380

actggtcttg attcaagaga tccctttgat ctccagcttg gtgcaagaat gttttacaat 1440

actcaagaca tgctgtggag gaggaagcta gaggagcaag ctgatttgca gcaagccctt 1500

gagcttcaaa gtagaagatt aatgagcttg cagcttcttg atgtcaagaa acatcatcac 1560

agggctcttt ccaatggcag ccctgtcccc tctcctactc actctcccaa tattttcaat 1620

cactctcttg ccttccctcc actccacagc agcaccgaag ttccacaggg tatggctgtc 1680

tctttgttac tctactctat acaggtgaaa attgagcttt acaacctaac tttggattgt 1740

tttgtttcag agaattgttc tagctcaatg ccagccacgt cagtgactgc cccgcctgaa 1800

aaacagatat caaatgctac ttctggtaaa gaatatacta gcagtgaaga gaacggcagt 1860

ggaaaagaga gctcccatgg tgaagacagt gatttacaag aaagtttgga gcacaacctc 1920

cctgacagtc cctttgcatc tccaacaaaa ggcaccgggg actattactc tgccttcatc 1980

aatggactta ctgaggcccg tgagaaggat gctagcatcc caacttcaac ttctgctaac 2040

aataatttgg tcccctcaag tctaatctct cctaattctt cactggaaat ggcatccttc 2100

aaatccttca attgccaaat ccctaggttt tcatctgggc atggagcaat tgggatgtat 2160

gccagcacag atggacctac ctgtcccgtt ggaatttag 2199

<210> 266
<211> 732
<212> PRT
<213> Populus trichocarpa

<400> 266

Met Asp Ala Tyr Glu Ala Thr Arg Ile Val Phe Ser Arg Ile Gln Asn
1 5 10 15

Leu Asp Pro Glu Asn Ala Ser Lys Ile Met Gly Leu Leu Leu Ile Gln
20 25 30

Asp His Gly Glu Lys Glu Met Ile Arg Leu Ala Phe Gly Pro Glu Ala
35 40 45

Leu Val His Ser Val Ile Leu Lys Ala Arg Lys Glu Leu Gly Leu Ser
50 55 60

Ser Pro Thr Asn Leu Ser Thr Ser Pro Ser Ser Pro Ser Pro Leu Tyr
65 70 75 80

Ser Ser Asn Pro Ile Ala Ile Ser Arg Gln Asn Ser Ser Ser Thr Ser
                85                  90                  95

Arg Leu Gly Phe Asn Ile Pro Pro Ser Leu Ala Ile Pro Asn Pro Ser
            100                 105                 110

Ser Asn Asn Ser Ser Ser Trp Ser Asp Leu Pro Asn Pro Asp Asp Leu
        115                 120                 125

Met Ile Ser Pro Asn Asp Ser Ser Leu Asn Pro Ala Ser Val Pro Phe
    130                 135                 140

Tyr Ala Asn Gly Val Arg Gly Gly Glu Ser Asp Leu Met Asp Glu Phe
145                 150                 155                 160

Gln Leu Gln Asp Gln Leu Ser Phe Leu Asn Asp Asn Ser Gln Asn Leu
                165                 170                 175

Gly Pro Lys Ser Ser Asp Leu Phe Tyr Pro Gln Leu Asp Ala Leu Ser
            180                 185                 190

Ser Pro Thr Gly Ala Ser Asp Ser Met Met Phe Pro Ser Tyr Trp Gly
        195                 200                 205

Gly Ser Val His Arg Arg Ser Cys Ser Val Ser Asp Val Leu Gly Ser
    210                 215                 220

Glu Asp Pro Asn Ser Gly Phe Gly Trp Arg Pro Cys Leu Tyr Phe Ala
225                 230                 235                 240

Arg Gly Tyr Cys Lys Asn Gly Ser Asn Cys Arg Phe Val His Gly Gly
                245                 250                 255

Leu Gly Glu Leu Asp Gly Ala Gly Val Val Gly Ser Pro Asn Ser Asn
            260                 265                 270

Asn Lys Ile Asp Met Met Asp Gln Cys His Glu Leu Leu Arg Ser Lys
        275                 280                 285

Ser Ala His Gln Gln Arg Leu Ala Ala Ala Ser Gln Leu Met Ser Ser
    290                 295                 300

Ser Ala Ala Ser Phe Pro Tyr Ser Pro Lys Ser Met Asn Phe Leu Leu
305                 310                 315                 320

Gln Gln Gln Gln Asn Asp Ser Gln Arg Ala Ala Ala Thr Ala Leu Met
                325                 330                 335

Met Gly Glu Asp Met His Lys Phe Gly Arg Ser Arg Leu Asp Arg Asn
340 345 350

Asp Leu Val Asn Pro Ala Ser Arg Gln Ile Tyr Leu Thr Phe Pro Ala
355 360 365

Asp Ser Thr Phe Arg Glu Glu Asp Val Ser Asn Tyr Phe Ser Ile Tyr
370 375 380

Gly Pro Val Gln Asp Val Arg Ile Pro Tyr Gln Gln Lys Arg Met Phe
385 390 395 400

Gly Phe Val Thr Phe Val Tyr Pro Glu Thr Val Lys Ile Ile Leu Ala
405 410 415

Lys Gly Asn Pro His Phe Val Cys Asp Ala Arg Val Leu Val Lys Pro
420 425 430

Tyr Lys Glu Lys Gly Lys Val Pro Asp Lys Lys Gln Gln Gln Gln Gln
435 440 445

Val Glu Arg Gly Glu Phe Ser Pro Cys Gly Thr Pro Thr Gly Leu Asp
450 455 460

Ser Arg Asp Pro Phe Asp Leu Gln Leu Gly Ala Arg Met Phe Tyr Asn
465 470 475 480

Thr Gln Asp Met Leu Trp Arg Arg Lys Leu Glu Glu Gln Ala Asp Leu
485 490 495

Gln Gln Ala Leu Glu Leu Gln Ser Arg Arg Leu Met Ser Leu Gln Leu
500 505 510

Leu Asp Val Lys Lys His His His Arg Ala Leu Ser Asn Gly Ser Pro
515 520 525

Val Pro Ser Pro Thr His Ser Pro Asn Ile Phe Asn His Ser Leu Ala
530 535 540

Phe Pro Pro Leu His Ser Ser Thr Glu Val Pro Gln Gly Met Ala Val
545 550 555 560

Ser Leu Leu Leu Tyr Ser Ile Gln Val Lys Ile Glu Leu Tyr Asn Leu
565 570 575

Thr Leu Asp Cys Phe Val Ser Glu Asn Cys Ser Ser Ser Met Pro Ala

580 585 590

Thr Ser Val Thr Ala Pro Pro Glu Lys Gln Ile Ser Asn Ala Thr Ser
595 600 605

Gly Lys Glu Tyr Thr Ser Ser Glu Glu Asn Gly Ser Gly Lys Glu Ser
610 615 620

Ser His Gly Glu Asp Ser Asp Leu Gln Glu Ser Leu Glu His Asn Leu
625 630 635 640

Pro Asp Ser Pro Phe Ala Ser Pro Thr Lys Gly Thr Gly Asp Tyr Tyr
645 650 655

Ser Ala Phe Ile Asn Gly Leu Thr Glu Ala Arg Glu Lys Asp Ala Ser
660 665 670

Ile Pro Thr Ser Thr Ser Ala Asn Asn Asn Leu Val Pro Ser Ser Leu
675 680 685

Ile Ser Pro Asn Ser Ser Leu Glu Met Ala Ser Phe Lys Ser Phe Asn
690 695 700

Cys Gln Ile Pro Arg Phe Ser Ser Gly His Gly Ala Ile Gly Met Tyr
705 710 715 720

Ala Ser Thr Asp Gly Pro Thr Cys Pro Val Gly Ile
725 730

<210> 267
<211> 1623
<212> DNA
<213> Arabidopsis thaliana

<400> 267
atggatggat atgaagctac taggattgtg ctctctagaa tccaaagctt agaccctgaa 60

aacgcatcaa agatcatggg tcttctcctt cttcaagatc acggtgaaaa agagatgata 120

aggctagctt ttggtccaga gactcttgtt cactctgtta tagtgaaagc caagaaagag 180

ttaggtctca tgaactgttc aaggtctccg tggagtcatc aagatgagtt gattagccct 240

aagaacaacc gtggctcttc actcaatcca gcttctttgc ccttttacgc taatggagga 300

agatcttcta gggatttaac caacgatttc gagctcatgg atgatatgaa ctccagaagt 360

actgattttt tgggctctgt gcatgcgaga agcggtagct gcgttttgga cggtttaggg 420

tatggtggtg attctgattt agggtttgga ggtgtgccct gttcttactt cgctagaggc 480

ttctgcaaaa acggagctag ctgcagattc gtccacagtg atggaggagc tgatttggtt 540

ggctccccaa gcagaatcga gcttcttagg tctaactcgg taccccccaag acttgctcac 600

cacttcatga ctcgctcttc tctcccttct ttttcaacta aaggtgttaa cttgcagcaa 660

aacgatgttc aaagagctgc tgctgctttg atgataggag atgaattgca gaagcttgga 720

agatggagac ctgaaaggat tgatctttct gctatggctt gtccagcttc aagacagatc 780

tatctgacat tccctgccga cagtaggttc agggaggaag atgtgtccaa ttacttcagt 840

acttttggac cagttcaaga tgtgaggata ccatatcagc aaaagagaat gtttggtttt 900

gtgacatttg tgtaccctga gactgttaag agcattctcg ccaaagggaa tcctcacttt 960

gtgtgtgatt ccagagttct tgtcaagcct tacaaggaga aaggcaaagt ccctgacaaa 1020

tacagaacta accaaacaac agagcgagaa ctgtccccaa caggccttga ttctagccct 1080

agggacgttc taggagggag agggttttat aacaacactc aagatgtgtt gtggaggagt 1140

aagtttgaag aagagattct tgaacttcag agcagaaggc tgatgaatct gcagcttctt 1200

gacgtcaaga agcatttcca actcaattcc cctaccaaca ttcactctcc gaatcctttc 1260

agccaatcac ttatatctcc acgcccattg tccgtgatca agagagagta tgatggagga 1320

gagaaaggga aaggaagttc taaagaagga tctgatgatg atacaatgaa tctaccagag 1380

aggttggagg atagcttgcc agatagtccg tttgcatcgc ccgctcatca tttgcttctg 1440

tttgctgatt ctgctgacaa taacggatcg gatctgtggt cgccttcttc tgataatgat 1500

gataattcta ctccttctac actctccgac tccttcaact ctttcaacta ccaaatgcca 1560

aggttaccgg cgattggaat gttacccggt aggggtggac caacctgtcg tgttgggata 1620

taa 1623

<210> 268
<211> 540
<212> PRT
<213> Arabidopsis thaliana

<400> 268

Met Asp Gly Tyr Glu Ala Thr Arg Ile Val Leu Ser Arg Ile Gln Ser
1 5 10 15

Leu Asp Pro Glu Asn Ala Ser Lys Ile Met Gly Leu Leu Leu Leu Gln
20 25 30

Asp His Gly Glu Lys Glu Met Ile Arg Leu Ala Phe Gly Pro Glu Thr
35 40 45

Leu Val His Ser Val Ile Val Lys Ala Lys Lys Glu Leu Gly Leu Met
50 55 60

Asn Cys Ser Arg Ser Pro Trp Ser His Gln Asp Glu Leu Ile Ser Pro

```
65                  70                  75                  80

Lys Asn Asn Arg Gly Ser Ser Leu Asn Pro Ala Ser Leu Pro Phe Tyr
                85                  90                  95

Ala Asn Gly Gly Arg Ser Ser Arg Asp Leu Thr Asn Asp Phe Glu Leu
            100                 105                 110

Met Asp Asp Met Asn Ser Arg Ser Thr Asp Phe Leu Gly Ser Val His
        115                 120                 125

Ala Arg Ser Gly Ser Cys Val Leu Asp Gly Leu Gly Tyr Gly Gly Asp
    130                 135                 140

Ser Asp Leu Gly Phe Gly Gly Val Pro Cys Ser Tyr Phe Ala Arg Gly
145                 150                 155                 160

Phe Cys Lys Asn Gly Ala Ser Cys Arg Phe Val His Ser Asp Gly Gly
                165                 170                 175

Ala Asp Leu Val Gly Ser Pro Ser Arg Ile Glu Leu Leu Arg Ser Asn
            180                 185                 190

Ser Val Pro Pro Arg Leu Ala His His Phe Met Thr Arg Ser Ser Leu
        195                 200                 205

Pro Ser Phe Ser Thr Lys Gly Val Asn Leu Gln Gln Asn Asp Val Gln
    210                 215                 220

Arg Ala Ala Ala Ala Leu Met Ile Gly Asp Glu Leu Gln Lys Leu Gly
225                 230                 235                 240

Arg Trp Arg Pro Glu Arg Ile Asp Leu Ser Ala Met Ala Cys Pro Ala
                245                 250                 255

Ser Arg Gln Ile Tyr Leu Thr Phe Pro Ala Asp Ser Arg Phe Arg Glu
            260                 265                 270

Glu Asp Val Ser Asn Tyr Phe Ser Thr Phe Gly Pro Val Gln Asp Val
        275                 280                 285

Arg Ile Pro Tyr Gln Gln Lys Arg Met Phe Gly Phe Val Thr Phe Val
    290                 295                 300

Tyr Pro Glu Thr Val Lys Ser Ile Leu Ala Lys Gly Asn Pro His Phe
305                 310                 315                 320
```

```
Val Cys Asp Ser Arg Val Leu Val Lys Pro Tyr Lys Glu Lys Gly Lys
                325                 330                 335

Val Pro Asp Lys Tyr Arg Thr Asn Gln Thr Thr Glu Arg Glu Leu Ser
            340                 345                 350

Pro Thr Gly Leu Asp Ser Ser Pro Arg Asp Val Leu Gly Gly Arg Gly
        355                 360                 365

Phe Tyr Asn Asn Thr Gln Asp Val Leu Trp Arg Ser Lys Phe Glu Glu
    370                 375                 380

Glu Ile Leu Glu Leu Gln Ser Arg Arg Leu Met Asn Leu Gln Leu Leu
385                 390                 395                 400

Asp Val Lys Lys His Phe Gln Leu Asn Ser Pro Thr Asn Ile His Ser
                405                 410                 415

Pro Asn Pro Phe Ser Gln Ser Leu Ile Ser Pro Arg Pro Leu Ser Val
            420                 425                 430

Ile Lys Arg Glu Tyr Asp Gly Gly Glu Lys Gly Lys Gly Ser Ser Lys
        435                 440                 445

Glu Gly Ser Asp Asp Asp Thr Met Asn Leu Pro Glu Arg Leu Glu Asp
    450                 455                 460

Ser Leu Pro Asp Ser Pro Phe Ala Ser Pro Ala His His Leu Leu Leu
465                 470                 475                 480

Phe Ala Asp Ser Ala Asp Asn Asn Gly Ser Asp Leu Trp Ser Pro Ser
                485                 490                 495

Ser Asp Asn Asp Asp Asn Ser Thr Pro Ser Thr Leu Ser Asp Ser Phe
            500                 505                 510

Asn Ser Phe Asn Tyr Gln Met Pro Arg Leu Pro Ala Ile Gly Met Leu
        515                 520                 525

Pro Gly Arg Gly Gly Pro Thr Cys Arg Val Gly Ile
    530                 535                 540
```

<210> 269
<211> 1611
<212> DNA
<213> Arabidopsis thaliana

<400> 269
atgaatttca cagaatcaat gaacgttgtg cacgccagaa tccaacaact tgaaccagag 60

aatgcagcaa agatctttgg ttatctcttg ttgatgcaag aaaatggcaa ccgcgacatg 120
atccgtctcg cgttctgtcc tgattctgtt atgtgttctg tcatcaattg cgttaaatac 180
gagttagcta ggaattctca tcattaccac agccctcctt ctgatcacat tcctactccc 240
aaatttggat cattcaccgg ttcatcgcct ctttcggttt cggtttctcc tcccatgaaa 300
accggttttt gggagaattc aaccgagatg gataccttgc agaacaatct tcagttcttg 360
aattttgagg atcctttgac cagccctgaa ttctctaacg ggttcttctc tcaagaacgt 420
caatgtttgc ctttgcgaac tagccgaaga tcccccgagtt tacccgagtt cccggtaaaa 480
atctgccatt acttcaacaa agggttctgc aaacacggca acaactgtag gtacttccac 540
gggcagatta taccggagag ggagagtttt gctcagatgt ttaatccaaa caacaaccta 600
agtgacgaag agcatgttgt ttcccctgta tctcttgaga agctagaagg tgagatcatt 660
gagttactca agttaagaag aggagctcca atctccatag cttcattgcc aatgatgtac 720
tacgaaaaat acggtaggac tcttcaagct gaaggatatc tcactgagtc acaaagacat 780
ggcaaagctg gctatagtct caccaagctt cttgctcgct tgaagaacac gattcgcctc 840
gtcgacaggc ctcatgggca acattcagtt atattagcag aggatgcatc aaagtttgtg 900
gaatacactg gagagagaaa tgaacatgga gcaatccttg ctggttctag acagatttac 960
ttaacgtttc cggcagagag tagtttcact gaacatgatg tctcaatcta cttcacctca 1020
tatggacatg tggaagatgt gaggattcct tgccagcaga aaagaatgta tggatttgta 1080
acatttgctt cctcagaaac agttaaacac attcttgcta aaggcaatcc tcatttcatt 1140
tgcaatgcac gtgttctagt caagccttac cgggaaaaat cacgctctag tcgatacctc 1200
gacaattaca agcctctaca cggcatgcga tatggctcta aatttattga gagagacata 1260
gagatgaaca cattgccacc gcgggttagt gagagctcaa gaatgaggaa gccatttctt 1320
agtgagcctg aacaatcagt ttctaagtcc ttacctacta attactccta cctcggcttc 1380
tcctcggatg acttcaagtt aacatcaaat gcggagcaag aggaacaagc agaacggttg 1440
agctacctac tggactattt gaacacggaa gataatgtca tgaacataac cactaactac 1500
agagacaatg atcggagaac tcattgtgaa tcgttggaca gtcaagtcct gaatctaccc 1560
gagagtccgt tttcttccct ttcggggaag gagatttcaa cggttacata g 1611

<210> 270
<211> 536
<212> PRT
<213> Arabidopsis thaliana

<400> 270

Met Asn Phe Thr Glu Ser Met Asn Val Val His Ala Arg Ile Gln Gln
1 5 10 15

```
Leu Glu Pro Glu Asn Ala Ala Lys Ile Phe Gly Tyr Leu Leu Leu Met
            20                  25                  30

Gln Glu Asn Gly Asn Arg Asp Met Ile Arg Leu Ala Phe Cys Pro Asp
        35                  40                  45

Ser Val Met Cys Ser Val Ile Asn Cys Val Lys Tyr Glu Leu Ala Arg
    50                  55                  60

Asn Ser His His Tyr His Ser Pro Pro Ser Asp His Ile Pro Thr Pro
65                  70                  75                  80

Lys Phe Gly Ser Phe Thr Gly Ser Ser Pro Leu Ser Val Ser Val Ser
                85                  90                  95

Pro Pro Met Lys Thr Gly Phe Trp Glu Asn Ser Thr Glu Met Asp Thr
            100                 105                 110

Leu Gln Asn Asn Leu Gln Phe Leu Asn Phe Glu Asp Pro Leu Thr Ser
        115                 120                 125

Pro Glu Phe Ser Asn Gly Phe Phe Ser Gln Glu Arg Gln Cys Leu Pro
    130                 135                 140

Leu Arg Thr Ser Arg Arg Ser Pro Ser Leu Pro Glu Phe Pro Val Lys
145                 150                 155                 160

Ile Cys His Tyr Phe Asn Lys Gly Phe Cys Lys His Gly Asn Asn Cys
                165                 170                 175

Arg Tyr Phe His Gly Gln Ile Ile Pro Glu Arg Glu Ser Phe Ala Gln
            180                 185                 190

Met Phe Asn Pro Asn Asn Asn Leu Ser Asp Glu Glu His Val Val Ser
        195                 200                 205

Pro Val Ser Leu Glu Lys Leu Glu Gly Glu Ile Ile Glu Leu Leu Lys
    210                 215                 220

Leu Arg Arg Gly Ala Pro Ile Ser Ile Ala Ser Leu Pro Met Met Tyr
225                 230                 235                 240

Tyr Glu Lys Tyr Gly Arg Thr Leu Gln Ala Glu Gly Tyr Leu Thr Glu
                245                 250                 255

Ser Gln Arg His Gly Lys Ala Gly Tyr Ser Leu Thr Lys Leu Leu Ala
```

260 265 270

Arg Leu Lys Asn Thr Ile Arg Leu Val Asp Arg Pro His Gly Gln His
275 280 285

Ser Val Ile Leu Ala Glu Asp Ala Ser Lys Phe Val Glu Tyr Thr Gly
290 295 300

Glu Arg Asn Glu His Gly Ala Ile Leu Ala Gly Ser Arg Gln Ile Tyr
305 310 315 320

Leu Thr Phe Pro Ala Glu Ser Ser Phe Thr Glu His Asp Val Ser Ile
325 330 335

Tyr Phe Thr Ser Tyr Gly His Val Glu Asp Val Arg Ile Pro Cys Gln
340 345 350

Gln Lys Arg Met Tyr Gly Phe Val Thr Phe Ala Ser Ser Glu Thr Val
355 360 365

Lys His Ile Leu Ala Lys Gly Asn Pro His Phe Ile Cys Asn Ala Arg
370 375 380

Val Leu Val Lys Pro Tyr Arg Glu Lys Ser Arg Ser Ser Arg Tyr Leu
385 390 395 400

Asp Asn Tyr Lys Pro Leu His Gly Met Arg Tyr Gly Ser Lys Phe Ile
405 410 415

Glu Arg Asp Ile Glu Met Asn Thr Leu Pro Pro Arg Val Ser Glu Ser
420 425 430

Ser Arg Met Arg Lys Pro Phe Leu Ser Glu Pro Glu Gln Ser Val Ser
435 440 445

Lys Ser Leu Pro Thr Asn Tyr Ser Tyr Leu Gly Phe Ser Ser Asp Asp
450 455 460

Phe Lys Leu Thr Ser Asn Ala Glu Gln Glu Glu Gln Ala Glu Arg Leu
465 470 475 480

Ser Tyr Leu Leu Asp Tyr Leu Asn Thr Glu Asp Asn Val Met Asn Ile
485 490 495

Thr Thr Asn Tyr Arg Asp Asn Asp Arg Arg Thr His Cys Glu Ser Leu
500 505 510

Asp Ser Gln Val Leu Asn Leu Pro Glu Ser Pro Phe Ser Ser Leu Ser
515 520 525

Gly Lys Glu Ile Ser Thr Val Thr
530 535

<210> 271
<211> 2034
<212> DNA
<213> Oryza sativa

<400> 271
atggatgcat acgaggcgac caaggtggtg ttctcccgga tccaggcgct ggaccctgac 60

cacgccgcca agatcatggg cctcctgctc atccaggacc acggcgacaa ggagatgata 120

cgcctcgcct tcggccccga ggcgctgctc cacagcgtca tggcgcaggc gcgcaaggag 180

ctcgccctcc tgccgccgcc tcaggcggcg tcgtcgtcgc ccaccgtgcc ggcagcccac 240

tcgccgttcc tgctgtcgag gcagaactcc ggccgctgcc ccgcgccgtc gccgtcgtcg 300

tgggcgcagg cgcagccgtt ctcgaggagt aacagcatgg gcaatggcgg cgccgcggat 360

gagatggtcg gcgctgggga ggagctaatg agccccttga acggcggggg aggcgccgcg 420

gcgaacgccc cgcccttctt tcctcggggt ggggacgcgc tcctggacga cttcgagctg 480

caggagcagc tcgcgttcct gcacgacgga gccgggggcg tgaaccccgg gcatgctctc 540

caggcgttcg acggagcgga gtgccggagc cccggccccg gcgagagcgg cgggatgctc 600

ccctacggcc tcgcctgggc caacggcggc cctgggcacc gccgcagcgc gtcggtgaac 660

gagctctgcc tcggcggcga cggcttcggg tggaaacctt gcctgtacta cgcgcgcggg 720

ttctgcaaga acggcagcac ctgcaggttc gtccatggcg gcctctccga cgacgccgcc 780

atggacgcaa ccaccgccga acagcagcag tgccaggatt tcctcctccg ctccaagagc 840

cagcgcctcg gccccgccgc cttcccgttc acccccacag gctctctccc tgcctcgcca 900

tccgccacca gcaagtgcct cagcctcctc ctgcagcagc agcagcagca caacgacaac 960

caaagagccg ccgcggccgc gctgatgctc gccggcggcg acgaggcgca caagttcatg 1020

gggcggccgc gcctggaccg cgtcgatttc gccagcatga tgaaccccgg gtcgcgccag 1080

atttacctca ccttcccggc cgacagcacg ttccgcgagg aggacgtctc caactacttc 1140

agcatctacg ggccggtgca cgacgtgcgc atcccgtacc agcagaagcg catgttcggg 1200

ttcgtcacct tcgtttaccc ggagacggtg aagctgatct tggccaaggg caacccgcac 1260

ttcatctgcg acgcccgcgt gctcgtcaag ccctacaagg agaagggcaa ggtccccgac 1320

aagtacagga agcagcagca aggcgatttc tgctgcatgt cgcccacggg gttagacgcc 1380

agggacccct ttgattttca ccagctcggt gcaaggatgc tgcagcactc caacagcgcg 1440

aacgagctaa tgctgcggcg gaagctcgag gagcagcaac aggcggcgga gctgcagcag 1500

gctattgatc tccatagccg ccgcctcatt ggcctccagc tgctcgacct caagtcttct 1560

gccgctgtcc atgcggcgga gacgacaaca atgtctctgc ccactccgat caccaatgcc 1620

ttcacctccg gccaacccgg tgccaccaca atcgtcgagt caccgcctag ctctactggg 1680

caactgatgg cgagctgcgg ctctccatcg gaggggaaag ttgtcaatgg tggtaataag 1740

gcggattctg ccggtgaggt tacccgcaat gccgatagtg accaaagtgg tgagcacaac 1800

ttgccagaca gcccgtttgc ttcctctacc aagtcgaccg cattctttac cgcaaccgct 1860

gccactgcca ttggtagcga gggagatttc accaccggta gtagctgcaa tattggtggc 1920

agtgcggtcg gcagcgccaa ccctctccgg cctcccacat tggacatacc ttcgccaagg 1980

acctgcttct tccccatgcc caggctgtcc gagcacgggg cgatcgggat gtaa 2034

<210> 272
<211> 677
<212> PRT
<213> Oryza sativa

<400> 272

Met Asp Ala Tyr Glu Ala Thr Lys Val Val Phe Ser Arg Ile Gln Ala
1 5 10 15

Leu Asp Pro Asp His Ala Ala Lys Ile Met Gly Leu Leu Leu Ile Gln
20 25 30

Asp His Gly Asp Lys Glu Met Ile Arg Leu Ala Phe Gly Pro Glu Ala
35 40 45

Leu Leu His Ser Val Met Ala Gln Ala Arg Lys Glu Leu Ala Leu Leu
50 55 60

Pro Pro Pro Gln Ala Ala Ser Ser Ser Pro Thr Val Pro Ala Ala His
65 70 75 80

Ser Pro Phe Leu Leu Ser Arg Gln Asn Ser Gly Arg Cys Pro Ala Pro
85 90 95

Ser Pro Ser Ser Trp Ala Gln Ala Gln Pro Phe Ser Arg Ser Asn Ser
100 105 110

Met Gly Asn Gly Gly Ala Ala Asp Glu Met Val Gly Ala Gly Glu Glu
115 120 125

Leu Met Ser Pro Leu Asn Gly Gly Gly Gly Ala Ala Ala Asn Ala Pro
130 135 140

Pro Phe Phe Pro Arg Gly Gly Asp Ala Leu Leu Asp Asp Phe Glu Leu
145 150 155 160

Gln Glu Gln Leu Ala Phe Leu His Asp Gly Ala Gly Gly Val Asn Pro
165 170 175

Gly His Ala Leu Gln Ala Phe Asp Gly Ala Glu Cys Arg Ser Pro Gly
180 185 190

Pro Gly Glu Ser Gly Gly Met Leu Pro Tyr Gly Leu Ala Trp Ala Asn
195 200 205

Gly Gly Pro Gly His Arg Arg Ser Ala Ser Val Asn Glu Leu Cys Leu
210 215 220

Gly Gly Asp Gly Phe Gly Trp Lys Pro Cys Leu Tyr Tyr Ala Arg Gly
225 230 235 240

Phe Cys Lys Asn Gly Ser Thr Cys Arg Phe Val His Gly Gly Leu Ser
245 250 255

Asp Asp Ala Ala Met Asp Ala Thr Thr Ala Glu Gln Gln Gln Cys Gln
260 265 270

Asp Phe Leu Leu Arg Ser Lys Ser Gln Arg Leu Gly Pro Ala Ala Phe
275 280 285

Pro Phe Thr Pro Thr Gly Ser Leu Pro Ala Ser Pro Ser Ala Thr Ser
290 295 300

Lys Cys Leu Ser Leu Leu Leu Gln Gln Gln Gln Gln His Asn Asp Asn
305 310 315 320

Gln Arg Ala Ala Ala Ala Ala Leu Met Leu Ala Gly Gly Asp Glu Ala
325 330 335

His Lys Phe Met Gly Arg Pro Arg Leu Asp Arg Val Asp Phe Ala Ser
340 345 350

Met Met Asn Pro Gly Ser Arg Gln Ile Tyr Leu Thr Phe Pro Ala Asp
355 360 365

Ser Thr Phe Arg Glu Glu Asp Val Ser Asn Tyr Phe Ser Ile Tyr Gly
370 375 380

Pro Val His Asp Val Arg Ile Pro Tyr Gln Gln Lys Arg Met Phe Gly
385 390 395 400

```
Phe Val Thr Phe Val Tyr Pro Glu Thr Val Lys Leu Ile Leu Ala Lys
                405                 410                 415

Gly Asn Pro His Phe Ile Cys Asp Ala Arg Val Leu Val Lys Pro Tyr
            420                 425                 430

Lys Glu Lys Gly Lys Val Pro Asp Lys Tyr Arg Lys Gln Gln Gln Gly
        435                 440                 445

Asp Phe Cys Cys Met Ser Pro Thr Gly Leu Asp Ala Arg Asp Pro Phe
    450                 455                 460

Asp Phe His Gln Leu Gly Ala Arg Met Leu Gln His Ser Asn Ser Ala
465                 470                 475                 480

Asn Glu Leu Met Leu Arg Arg Lys Leu Glu Glu Gln Gln Gln Ala Ala
                485                 490                 495

Glu Leu Gln Gln Ala Ile Asp Leu His Ser Arg Arg Leu Ile Gly Leu
            500                 505                 510

Gln Leu Leu Asp Leu Lys Ser Ser Ala Ala Val His Ala Ala Glu Thr
        515                 520                 525

Thr Thr Met Ser Leu Pro Thr Pro Ile Thr Asn Ala Phe Thr Ser Gly
    530                 535                 540

Gln Pro Gly Ala Thr Thr Ile Val Glu Ser Pro Pro Ser Ser Thr Gly
545                 550                 555                 560

Gln Leu Met Ala Ser Cys Gly Ser Pro Ser Glu Gly Lys Val Val Asn
                565                 570                 575

Gly Gly Asn Lys Ala Asp Ser Ala Gly Glu Val Thr Arg Asn Ala Asp
            580                 585                 590

Ser Asp Gln Ser Gly Glu His Asn Leu Pro Asp Ser Pro Phe Ala Ser
        595                 600                 605

Ser Thr Lys Ser Thr Ala Phe Phe Thr Ala Thr Ala Ala Thr Ala Ile
    610                 615                 620

Gly Ser Glu Gly Asp Phe Thr Thr Gly Ser Ser Cys Asn Ile Gly Gly
625                 630                 635                 640

Ser Ala Val Gly Ser Ala Asn Pro Leu Arg Pro Pro Thr Leu Asp Ile
                645                 650                 655
```

```
Pro Ser Pro Arg Thr Cys Phe Phe Pro Met Pro Arg Leu Ser Glu His
            660                 665                 670

Gly Ala Ile Gly Met
        675


<210>  273
<211>  2049
<212>  DNA
<213>  Oryza sativa

<400>  273
atggacgcct acgaggcgac caaggtggtg ttctcgcgga tccaggcgct cgacccggac      60

cacgcagcca agatcatggg cttcctcctc atccaggacc atggcgagaa ggagatgata     120

cgcctcgctt tcggccccga ggcgctgctc cacaccgtca tggccaaggc caggaaggag     180

ctcggcctcc tcccggcgtc cgggcccggg acgcccacct ccgtggcggc ggccgcggcc     240

gccgcccact cgcccttcat gctctcgcgg cagaactccg ggcgctgcgg caccgcgccc     300

tcgccgctct cggtgtcctc cccctcctcg tgggcgcctc cccggtgtt ttcgaggaac      360

aacagcatca gcaatggcgc cggggaggag atggtcggcc tcggcgacga gctcatcagc     420

ccggccaacg gcgggggccc gccatcgccc ttcttcggcg gcgacccgct catggacgag     480

ctccagctgc aggaccagct cgcgttcctc aacgagggcg gcgtccccgc ggggcaccag     540

atgcccatgt tcgacggcgg cgagtgccgg agccccggcg gaggcgacgg cggccttttc     600

tcctacaacc tagggtgggc gaacggtggc cccggacacc gccggagcgc gtcggtcagc     660

gagctctgcc tcggcggcgc cgacggcctc ggctggaagc cgtgcctcta ctacgcgcgc     720

ggctactgca agaacggcag cgcttgccgg ttcgtccacg gcggcctccc cgacgacgcc     780

gccggcaaga tggacccctc cgccgtggag cagcagtgtc aggacttcct catccgctcc     840

aagtcccagc gcctcgccgc cgccgccttc ccctactcgc ccaccggctc actccccggc     900

tcgccatccg cagccaccaa gtgtttgagc ttgctcctcc agcagcagca gcagcagaac     960

gagagccaga gggcggcggc agcggcggcg ctgatgctag gcggcgacga ggcgcacaag    1020

ttcatggggc ggccgcggct ggagcgcgcc gacttcgcga gcatgatgaa ccccggctcg    1080

cgccagattt acctcacctt cccggcggat agcaccttcc gcgaggagga cgtctccaac    1140

tacttcagca tctacggccc cgtccacgac gttcgcatcc cgtaccagca gaagcgcatg    1200

ttcggcttcg tcaccttcgt ctacccggag acggtgaagc tgattctcgc caagggcaac    1260

ccgcacttca tctgcgacgc ccgcgtgctc gtcaagccat acaaggagaa gggcaaggtc    1320

cccgacaagt acaggaagca gcaccagccg ggcgagaggg tggacttctc cagctgcact    1380

actccaactg gactcgatgc cagagacccc ttcgacatgc accagctcgg tgcgagaatg    1440
```

ctgcagcact ccaacagcgc gaatgagatg ctgctgagga ggaagctgga ggagcagcag 1500

caggccgccg agctgcagca ggcgatcgag ctccacagcc gccgcctcat ggggctgcag 1560

ctgctagact tcaagtcgcg cgccgccgcg gcgccgaccc ccattggcaa tcctttcagc 1620

gcttctcaga ccgccgccaa cgcgaccggc gagtcgcctc ccgattccgg ggagctcggt 1680

aagggaagcg gcttccttct tgctcacaag aaggcggtca acggagccga taaggaggaa 1740

tccaccggag agtcctccag ccctaacaca gacagtgacc aaagtgtgga gcataatctg 1800

ccggacagcc cgtttgcgtc gccgaccaag tccgcgggat ttgctcgcga tcctttcgct 1860

cccactgagg cggagatctc cgccaccgcg tcgactggtt gtagcgccac ctatgttggc 1920

atcaacaatg gcgccagcaa tggcggcact aaccatctcc taccttctgc cttggacatg 1980

ccctcaccaa aaccttattt cttccccatg tccaggctgg cctccgatca cggcgcgatc 2040

ggaatgtaa 2049

<210> 274
<211> 682
<212> PRT
<213> Oryza sativa

<400> 274

Met Asp Ala Tyr Glu Ala Thr Lys Val Val Phe Ser Arg Ile Gln Ala
1 5 10 15

Leu Asp Pro Asp His Ala Ala Lys Ile Met Gly Phe Leu Leu Ile Gln
20 25 30

Asp His Gly Glu Lys Glu Met Ile Arg Leu Ala Phe Gly Pro Glu Ala
35 40 45

Leu Leu His Thr Val Met Ala Lys Ala Arg Lys Glu Leu Gly Leu Leu
50 55 60

Pro Ala Ser Gly Pro Gly Thr Pro Thr Ser Val Ala Ala Ala Ala Ala
65 70 75 80

Ala Ala His Ser Pro Phe Met Leu Ser Arg Gln Asn Ser Gly Arg Cys
85 90 95

Gly Thr Ala Pro Ser Pro Leu Ser Val Ser Ser Pro Ser Ser Trp Ala
100 105 110

Pro Pro Pro Val Phe Ser Arg Asn Asn Ser Ile Ser Asn Gly Ala Gly
115 120 125

Glu Glu Met Val Gly Leu Gly Asp Glu Leu Ile Ser Pro Ala Asn Gly
    130                 135                 140

Gly Gly Pro Pro Ser Pro Phe Phe Gly Gly Asp Pro Leu Met Asp Glu
145                 150                 155                 160

Leu Gln Leu Gln Asp Gln Leu Ala Phe Leu Asn Glu Gly Gly Val Pro
                165                 170                 175

Ala Gly His Gln Met Pro Met Phe Asp Gly Gly Glu Cys Arg Ser Pro
            180                 185                 190

Gly Gly Gly Asp Gly Gly Leu Phe Ser Tyr Asn Leu Gly Trp Ala Asn
        195                 200                 205

Gly Gly Pro Gly His Arg Arg Ser Ala Ser Val Ser Glu Leu Cys Leu
    210                 215                 220

Gly Gly Ala Asp Gly Leu Gly Trp Lys Pro Cys Leu Tyr Tyr Ala Arg
225                 230                 235                 240

Gly Tyr Cys Lys Asn Gly Ser Ala Cys Arg Phe Val His Gly Gly Leu
                245                 250                 255

Pro Asp Asp Ala Ala Gly Lys Met Asp Pro Ser Ala Val Glu Gln Gln
            260                 265                 270

Cys Gln Asp Phe Leu Ile Arg Ser Lys Ser Gln Arg Leu Ala Ala Ala
        275                 280                 285

Ala Phe Pro Tyr Ser Pro Thr Gly Ser Leu Pro Gly Ser Pro Ser Ala
    290                 295                 300

Ala Thr Lys Cys Leu Ser Leu Leu Leu Gln Gln Gln Gln Gln Gln Asn
305                 310                 315                 320

Glu Ser Gln Arg Ala Ala Ala Ala Ala Ala Leu Met Leu Gly Gly Asp
                325                 330                 335

Glu Ala His Lys Phe Met Gly Arg Pro Arg Leu Glu Arg Ala Asp Phe
            340                 345                 350

Ala Ser Met Met Asn Pro Gly Ser Arg Gln Ile Tyr Leu Thr Phe Pro
        355                 360                 365

Ala Asp Ser Thr Phe Arg Glu Glu Asp Val Ser Asn Tyr Phe Ser Ile
    370                 375                 380

```
Tyr Gly Pro Val His Asp Val Arg Ile Pro Tyr Gln Gln Lys Arg Met
385                 390                 395                 400

Phe Gly Phe Val Thr Phe Val Tyr Pro Glu Thr Val Lys Leu Ile Leu
                405                 410                 415

Ala Lys Gly Asn Pro His Phe Ile Cys Asp Ala Arg Val Leu Val Lys
            420                 425                 430

Pro Tyr Lys Glu Lys Gly Lys Val Pro Asp Lys Tyr Arg Lys Gln His
        435                 440                 445

Gln Pro Gly Glu Arg Val Asp Phe Ser Ser Cys Thr Thr Pro Thr Gly
    450                 455                 460

Leu Asp Ala Arg Asp Pro Phe Asp Met His Gln Leu Gly Ala Arg Met
465                 470                 475                 480

Leu Gln His Ser Asn Ser Ala Asn Glu Met Leu Leu Arg Arg Lys Leu
                485                 490                 495

Glu Glu Gln Gln Gln Ala Ala Glu Leu Gln Gln Ala Ile Glu Leu His
            500                 505                 510

Ser Arg Arg Leu Met Gly Leu Gln Leu Leu Asp Phe Lys Ser Arg Ala
        515                 520                 525

Ala Ala Ala Pro Thr Pro Ile Gly Asn Pro Phe Ser Ala Ser Gln Thr
    530                 535                 540

Ala Ala Asn Ala Thr Gly Glu Ser Pro Pro Asp Ser Gly Glu Leu Gly
545                 550                 555                 560

Lys Gly Ser Gly Phe Leu Leu Ala His Lys Lys Ala Val Asn Gly Ala
                565                 570                 575

Asp Lys Glu Glu Ser Thr Gly Glu Ser Ser Ser Pro Asn Thr Asp Ser
            580                 585                 590

Asp Gln Ser Val Glu His Asn Leu Pro Asp Ser Pro Phe Ala Ser Pro
        595                 600                 605

Thr Lys Ser Ala Gly Phe Ala Arg Asp Pro Phe Ala Pro Thr Glu Ala
    610                 615                 620

Glu Ile Ser Ala Thr Ala Ser Thr Gly Cys Ser Ala Thr Tyr Val Gly
625                 630                 635                 640
```

```
Ile Asn Asn Gly Ala Ser Asn Gly Gly Thr Asn His Leu Leu Pro Ser
                645                 650                 655

Ala Leu Asp Met Pro Ser Pro Lys Pro Tyr Phe Phe Pro Met Ser Arg
            660                 665                 670

Leu Ala Ser Asp His Gly Ala Ile Gly Met
        675                 680
```

<210> 275
<211> 2067
<212> DNA
<213> Oryza sativa

<400> 275

```
atggacgcct acgaggcgac caaggtggtg ttctcccgga tccaggcgct ggaccctgac      60
cacgccgcca agatcatggg cctcctgctc atccaggacc acggcgacaa ggagatgata     120
cgcctcgcct tcggccccga ggcgctgctc cacagcgtca tggcgcaggc tcgcaaggag     180
ctcgccctcc tcccgccgcc gccgccgccg tcgtcgtcgt cgcccaccgt gccggcggcc     240
cactcgccgt tcctgctgtc gcggcagaac tccggccgcg gccccgcgcc gtcgccgtcg     300
ccgctgtccg cgtcctcgcc gtcctcgtgg gcgcaggcgc agccgttctc gaggagcaat     360
gggtccgtgg atgaggtggt gggcgctggg gaggagctaa tcagcccggc gaacagcggg     420
ggaggcgccg cggcgaacgc gccgcccttc tttcctcggg gtggggacgt gctcctggac     480
gacttccagc tgcaggagca gctcgcgttc ctcaacgagg ggggcgtcaa ccccagccac     540
cctctccagg ggttcgatgg agcggagtgc cggagccccg gtcccggcga gggcggcggg     600
atgttcccgt acggtctcgg ctgggcaaac ggtggccctg gcaccgccg gagcgcgtcg      660
gtcaacgagc tctgcctcgg cggcggcagc agcgacggct tcgggtggaa gccttgcctg     720
tactacgcgc gcgggttctg caagaacggc agcagctgca ggttcgtcca cggcgacgac     780
gccgccgctc tgacgggcgc cgccatggac gcggccaccg ccgagcagca gcagtgccag     840
gatttcctcc tccgttccaa gagccagcgc ctcggccctg ctgccttccc ctattcaccc     900
acagggtcgc tccccggctc gccatccgcc gccaccaagt gcctcagcct cttgctgcag     960
cagcagcaca acgacaacca aagagccgcg gcggcggcgg cgctgatgct cggcggcagc    1020
gacgaggcgc acaagttcat ggggcggccg cgcctggacc gcgtcgactt tgccagcatg    1080
atgaaccccg gatcgcgcca gatttacctc accttcccgg ccgacagcac gttccgcgag    1140
gaggacgtct ccaactactt cagcatctac ggtccggtgc acgacgtgcg catcccgtac    1200
cagcagaagc gcatgttcgg gttcgtcacc ttcgtgtacc ctgagacggt gaagctgatc    1260
ttggccaagg gcaatccgca tttcatctgc gacgcccgcg tgctcgtcaa gccttacaag    1320
```

```
gagaagggca aggtccccga caagtacagg aaacatcaag gtgacttctc cggctgcacg   1380

acccccactg gcctggacgg cagagaccca ttcgatctgc atcagctcgg tgcgaggatg   1440

ctgcagcact ccaatagcac aaacgagatg atgcttcgta ggaaactgga ggagcagcag   1500

caggctgccg agctgcaaca ggccatcgaa ctccacagtc gccgtctcat ggacctccag   1560

ctgctcgacc tcaagaatag agccgcagct gctgtcacaa cagcaatggc gatgacaatt   1620

cccaccgcca atgccttcgg ttccagtcag cctcttgcca ccaccatggt cgagtcaccg   1680

cctgattctg gcgagcagct caagggaaca ggttacttca cagaagagag gaaaatggtc   1740

aacggaggag gtgataagga agaatctgct ggtgaggcga gcctgaatgc tgatagcgac   1800

caaagcttgg agcacaattt gccggacagc ccgtttgctt cgccgactaa gtcctctgtc   1860

tcagctcacc aaagtttcac caccactgat accggtgtcg tcgcgacaag tagctgcagt   1920

gcatctcatg taggcatcag cgcgggcact aatgctggag gtggaatcaa ccatctgcgg   1980

ccctctactt tggatatacc ttcgccaaga gacttcttct cggtttccag caggctggcc   2040

tctgatcacg gagcgattgg gatgtaa                                         2067


<210>  276
<211>  688
<212>  PRT
<213>  Oryza sativa

<400>  276

Met Asp Ala Tyr Glu Ala Thr Lys Val Val Phe Ser Arg Ile Gln Ala
1               5                   10                  15


Leu Asp Pro Asp His Ala Ala Lys Ile Met Gly Leu Leu Leu Ile Gln
            20                  25                  30


Asp His Gly Asp Lys Glu Met Ile Arg Leu Ala Phe Gly Pro Glu Ala
        35                  40                  45


Leu Leu His Ser Val Met Ala Gln Ala Arg Lys Glu Leu Ala Leu Leu
    50                  55                  60


Pro Pro Pro Pro Pro Pro Ser Ser Ser Ser Pro Thr Val Pro Ala Ala
65                  70                  75                  80


His Ser Pro Phe Leu Leu Ser Arg Gln Asn Ser Gly Arg Gly Pro Ala
                85                  90                  95


Pro Ser Pro Ser Pro Leu Ser Ala Ser Ser Pro Ser Ser Trp Ala Gln
            100                 105                 110
```

```
Ala Gln Pro Phe Ser Arg Ser Asn Gly Ser Val Asp Glu Val Val Gly
        115                 120                 125

Ala Gly Glu Glu Leu Ile Ser Pro Ala Asn Ser Gly Gly Gly Ala Ala
    130                 135                 140

Ala Asn Ala Pro Pro Phe Phe Pro Arg Gly Gly Asp Val Leu Leu Asp
145                 150                 155                 160

Asp Phe Gln Leu Gln Glu Gln Leu Ala Phe Leu Asn Glu Gly Gly Val
                165                 170                 175

Asn Pro Ser His Pro Leu Gln Gly Phe Asp Gly Ala Glu Cys Arg Ser
            180                 185                 190

Pro Gly Pro Gly Glu Gly Gly Gly Met Phe Pro Tyr Gly Leu Gly Trp
        195                 200                 205

Ala Asn Gly Gly Pro Gly His Arg Arg Ser Ala Ser Val Asn Glu Leu
    210                 215                 220

Cys Leu Gly Gly Gly Ser Ser Asp Gly Phe Gly Trp Lys Pro Cys Leu
225                 230                 235                 240

Tyr Tyr Ala Arg Gly Phe Cys Lys Asn Gly Ser Ser Cys Arg Phe Val
                245                 250                 255

His Gly Asp Asp Ala Ala Ala Leu Thr Gly Ala Ala Met Asp Ala Ala
            260                 265                 270

Thr Ala Glu Gln Gln Gln Cys Gln Asp Phe Leu Leu Arg Ser Lys Ser
        275                 280                 285

Gln Arg Leu Gly Pro Ala Ala Phe Pro Tyr Ser Pro Thr Gly Ser Leu
    290                 295                 300

Pro Gly Ser Pro Ser Ala Ala Thr Lys Cys Leu Ser Leu Leu Leu Gln
305                 310                 315                 320

Gln Gln His Asn Asp Asn Gln Arg Ala Ala Ala Ala Ala Ala Leu Met
                325                 330                 335

Leu Gly Gly Ser Asp Glu Ala His Lys Phe Met Gly Arg Pro Arg Leu
            340                 345                 350

Asp Arg Val Asp Phe Ala Ser Met Met Asn Pro Gly Ser Arg Gln Ile
        355                 360                 365
```

```
Tyr Leu Thr Phe Pro Ala Asp Ser Thr Phe Arg Glu Glu Asp Val Ser
    370                 375                 380

Asn Tyr Phe Ser Ile Tyr Gly Pro Val His Asp Val Arg Ile Pro Tyr
385                 390                 395                 400

Gln Gln Lys Arg Met Phe Gly Phe Val Thr Phe Val Tyr Pro Glu Thr
                405                 410                 415

Val Lys Leu Ile Leu Ala Lys Gly Asn Pro His Phe Ile Cys Asp Ala
            420                 425                 430

Arg Val Leu Val Lys Pro Tyr Lys Glu Lys Gly Lys Val Pro Asp Lys
        435                 440                 445

Tyr Arg Lys His Gln Gly Asp Phe Ser Gly Cys Thr Thr Pro Thr Gly
    450                 455                 460

Leu Asp Gly Arg Asp Pro Phe Asp Leu His Gln Leu Gly Ala Arg Met
465                 470                 475                 480

Leu Gln His Ser Asn Ser Thr Asn Glu Met Met Leu Arg Arg Lys Leu
                485                 490                 495

Glu Glu Gln Gln Gln Ala Ala Glu Leu Gln Gln Ala Ile Glu Leu His
            500                 505                 510

Ser Arg Arg Leu Met Asp Leu Gln Leu Leu Asp Leu Lys Asn Arg Ala
        515                 520                 525

Ala Ala Ala Val Thr Thr Ala Met Ala Met Thr Ile Pro Thr Ala Asn
    530                 535                 540

Ala Phe Gly Ser Ser Gln Pro Leu Ala Thr Thr Met Val Glu Ser Pro
545                 550                 555                 560

Pro Asp Ser Gly Glu Gln Leu Lys Gly Thr Gly Tyr Phe Thr Glu Glu
                565                 570                 575

Arg Lys Met Val Asn Gly Gly Gly Asp Lys Glu Glu Ser Ala Gly Glu
            580                 585                 590

Ala Ser Leu Asn Ala Asp Ser Asp Gln Ser Leu Glu His Asn Leu Pro
        595                 600                 605

Asp Ser Pro Phe Ala Ser Pro Thr Lys Ser Ser Val Ser Ala His Gln
    610                 615                 620
```

Ser Phe Thr Thr Thr Asp Thr Gly Val Val Ala Thr Ser Ser Cys Ser
625 630 635 640

Ala Ser His Val Gly Ile Ser Ala Gly Thr Asn Ala Gly Gly Gly Ile
645 650 655

Asn His Leu Arg Pro Ser Thr Leu Asp Ile Pro Ser Pro Arg Asp Phe
660 665 670

Phe Ser Val Ser Ser Arg Leu Ala Ser Asp His Gly Ala Ile Gly Met
675 680 685

<210> 277
<211> 2037
<212> DNA
<213> Zea mays

<400> 277
atggacgcct acgaagccac caaggtggtg ttctcgcgga tccaggcgct ggacccggac 60

cacgccgcca agatcatggg cttcctgctc atccaggacc acggcgagaa ggagatgata 120

cgcctcgcct tcggccccga ggcgctgctg cacaccgtca tggccaaggc gcgcaaggac 180

ctgggcctgc tcccgtcgcc aggcccgggg acgcccacat ccgtcaccgc cgccgcaacg 240

cactcgccgt tcctcctctc gcgccagaac tccgggcgct gcggcgccgg caccgcgccg 300

tcgccgctct cggtgtcgtc gccctcgtcg tgggcgccgc cgccccactt ctccaggacc 360

aacagcgtcg tcagcaatgg cgcgccggcg gaggccttgg ccgccgacct catgagcccg 420

gccgccgccg ggaacgcgcc gccgtcgccc ttctttgccg ccggggaacc gctcctcgac 480

gagctccagc tgcaggagca gctcgcgttc ctcagcgacg ccgccgccgg cggccaccag 540

ctgcccctgt tcgacgccag cgagtgccgg agccccggct caggagacgc cgccgggttc 600

ttcccgtacg gcgccctcgg gtgggccaac ggcggcccgg ggcaccgccg gagctcgtcg 660

gtcagcgagc tctgcctcgg cggggccgac gggctcggct ggaagccctg cctgtactac 720

gcccgcgggt actgcaagaa cggcagcgct tgccggttcg tgcacggcgg cctcaccgac 780

gacgccaccg ctaagatgga caccgccacc ttggagcagc agtgccagga catcctgctc 840

cgctccaagt cccagcgcct tgccgccttc ccctactccc cgaccggctc ggtcccgggc 900

tccccgtccg cggccaccaa gtgcctgagc ttgctcttgc accagcagca gcagcagaac 960

gagaaccaga gggtggcagc tgcagcggcc gccgcggcgc tgatgctggg cggcgacgac 1020

gcgcacaagt tcattgggcg gccgcggctg gaccgcgccg acttggcgag cttggtgaac 1080

ccgggctcgc gccagattta cctcaccttc ccggcagaca gcaccttccg cgaggaggac 1140

gtgtccaact acttcagcat ctacggcccc gtccacgacg tgcgcatccc gtaccagcag 1200

aagcgcatgt tcgggttcgt caccttcgtg tacccggaga cggtgaagct gatcctggcc 1260

aagggcaacc cgcacttcat ctgcgacgcg cgcgtgctcg tgaagcccta caaggagaag 1320

ggcaaggtcc ccgacaagta caggaagcag cagctgcaag gcgagagggc ggtggatttc 1380

ttctccaacg ggttagacgg cagagaaaac cacttggatc tgcaccagct gggtgcgagg 1440

atgctccagc actcgcacag cgcgaacgag atgctgctga ggaggaagct ggaggagcag 1500

cagcaggccg ctgctgccga gctgcagcag gccatggagc tccagagccg ccgcctgatg 1560

aggctgcagc tgctggacct gaagccgcgc gcgtcgccga gccccatcgg cagcatgccc 1620

ctgggcccca cccaaagggc cgtcgactcg ccgcccgatt ccggcaggga ggagtcgtcc 1680

gccggcgacg cgagcccgaa cgcggacagc gaccaaagcg ccgagcacaa cctgccggac 1740

agcccgttcg cgtcgccgac gaggtccgcg gccttggctc gcgacccttt cgcggccatc 1800

gaccgggaga tggctgcctc gcccggtcgt cgaaacggcg ccggttcctt cgctggcatc 1860

agcagcagca gcggcgtcct cgccggccat ctgaggccgt cggctctgga catcccctcc 1920

ccgttcttcc ccatgtcgat gaccaggctg tcctccgatc acggcgccgg gcgcgatcgg 1980

gatgtaaagt tatatagtcc tcgctgctac ttgcctaatc atagaatact taactag 2037

<210> 278
<211> 678
<212> PRT
<213> Zea mays

<400> 278

Met Asp Ala Tyr Glu Ala Thr Lys Val Val Phe Ser Arg Ile Gln Ala
1 5 10 15

Leu Asp Pro Asp His Ala Ala Lys Ile Met Gly Phe Leu Leu Ile Gln
20 25 30

Asp His Gly Glu Lys Glu Met Ile Arg Leu Ala Phe Gly Pro Glu Ala
35 40 45

Leu Leu His Thr Val Met Ala Lys Ala Arg Lys Asp Leu Gly Leu Leu
50 55 60

Pro Ser Pro Gly Pro Gly Thr Pro Thr Ser Val Thr Ala Ala Ala Thr
65 70 75 80

His Ser Pro Phe Leu Leu Ser Arg Gln Asn Ser Gly Arg Cys Gly Ala
85 90 95

Gly Thr Ala Pro Ser Pro Leu Ser Val Ser Ser Pro Ser Ser Trp Ala
100 105 110

Pro Pro Pro His Phe Ser Arg Thr Asn Ser Val Val Ser Asn Gly Ala
115 120 125

Pro Ala Glu Ala Leu Ala Ala Asp Leu Met Ser Pro Ala Ala Ala Gly
130 135 140

Asn Ala Pro Pro Ser Pro Phe Phe Ala Ala Gly Glu Pro Leu Leu Asp
145 150 155 160

Glu Leu Gln Leu Gln Glu Gln Leu Ala Phe Leu Ser Asp Ala Ala Ala
165 170 175

Gly Gly His Gln Leu Pro Leu Phe Asp Ala Ser Glu Cys Arg Ser Pro
180 185 190

Gly Ser Gly Asp Ala Ala Gly Phe Phe Pro Tyr Gly Ala Leu Gly Trp
195 200 205

Ala Asn Gly Gly Pro Gly His Arg Arg Ser Ser Ser Val Ser Glu Leu
210 215 220

Cys Leu Gly Gly Ala Asp Gly Leu Gly Trp Lys Pro Cys Leu Tyr Tyr
225 230 235 240

Ala Arg Gly Tyr Cys Lys Asn Gly Ser Ala Cys Arg Phe Val His Gly
245 250 255

Gly Leu Thr Asp Asp Ala Thr Ala Lys Met Asp Thr Ala Thr Leu Glu
260 265 270

Gln Gln Cys Gln Asp Ile Leu Leu Arg Ser Lys Ser Gln Arg Leu Ala
275 280 285

Ala Phe Pro Tyr Ser Pro Thr Gly Ser Val Pro Gly Ser Pro Ser Ala
290 295 300

Ala Thr Lys Cys Leu Ser Leu Leu Leu His Gln Gln Gln Gln Gln Asn
305 310 315 320

Glu Asn Gln Arg Val Ala Ala Ala Ala Ala Ala Ala Ala Leu Met Leu
325 330 335

Gly Gly Asp Asp Ala His Lys Phe Ile Gly Arg Pro Arg Leu Asp Arg
340 345 350

Ala Asp Leu Ala Ser Leu Val Asn Pro Gly Ser Arg Gln Ile Tyr Leu

355 360 365

Thr Phe Pro Ala Asp Ser Thr Phe Arg Glu Glu Asp Val Ser Asn Tyr
370 375 380

Phe Ser Ile Tyr Gly Pro Val His Asp Val Arg Ile Pro Tyr Gln Gln
385 390 395 400

Lys Arg Met Phe Gly Phe Val Thr Phe Val Tyr Pro Glu Thr Val Lys
405 410 415

Leu Ile Leu Ala Lys Gly Asn Pro His Phe Ile Cys Asp Ala Arg Val
420 425 430

Leu Val Lys Pro Tyr Lys Glu Lys Gly Lys Val Pro Asp Lys Tyr Arg
435 440 445

Lys Gln Gln Leu Gln Gly Glu Arg Ala Val Asp Phe Phe Ser Asn Gly
450 455 460

Leu Asp Gly Arg Glu Asn His Leu Asp Leu His Gln Leu Gly Ala Arg
465 470 475 480

Met Leu Gln His Ser His Ser Ala Asn Glu Met Leu Leu Arg Arg Lys
485 490 495

Leu Glu Glu Gln Gln Gln Ala Ala Ala Ala Glu Leu Gln Gln Ala Met
500 505 510

Glu Leu Gln Ser Arg Arg Leu Met Arg Leu Gln Leu Leu Asp Leu Lys
515 520 525

Pro Arg Ala Ser Pro Ser Pro Ile Gly Ser Met Pro Leu Gly Pro Thr
530 535 540

Gln Arg Ala Val Asp Ser Pro Pro Asp Ser Gly Arg Glu Glu Ser Ser
545 550 555 560

Ala Gly Asp Ala Ser Pro Asn Ala Asp Ser Asp Gln Ser Ala Glu His
565 570 575

Asn Leu Pro Asp Ser Pro Phe Ala Ser Pro Thr Arg Ser Ala Ala Leu
580 585 590

Ala Arg Asp Pro Phe Ala Ala Ile Asp Arg Glu Met Ala Ala Ser Pro
595 600 605

Gly Arg Arg Asn Gly Ala Gly Ser Phe Ala Gly Ile Ser Ser Ser Ser
610 615 620

Gly Val Leu Ala Gly His Leu Arg Pro Ser Ala Leu Asp Ile Pro Ser
625 630 635 640

Pro Phe Phe Pro Met Ser Met Thr Arg Leu Ser Ser Asp His Gly Ala
645 650 655

Gly Arg Asp Arg Asp Val Lys Leu Tyr Ser Pro Arg Cys Tyr Leu Pro
660 665 670

Asn His Arg Ile Leu Asn
675

<210> 279
<211> 1671
<212> DNA
<213> Vitis vinifera

<400> 279
atggattttt ctgagtccac agcagttgtt ttcaatagaa ttcaaaaact agaaccagag 60

aatgtttcaa agattatagg gtatcttctt gttaagggtt ttagtaaggg agaaatgatt 120

cggttggctt ttggtcccga taaggcgatt cgtatgataa ttgaaggggt caaaatagag 180

cttggtttga ttccaaatcc accatctcca atctctcctc acttccctcc tctctctcct 240

tcaactggtt cttggttccc ttcatcttct ccgtctcccg tgaaccgata tctccaacat 300

gctacagagc aactaccaaa agattatagt ctccaaagtc agcctttggg tttagaggaa 360

cagttagaac aggtcaaccc agcaatttta ggagtttccg gtgattacta ttacccggag 420

acggcagtgg aaaatttgag tgtgaggacg ggtccaagat ctctgattgg ttcggaattt 480

ccagttaagg tttgtcacta tttcaacaag gggttttgta agcatggaaa taattgtcga 540

tacttgcatg cacaagtctt tcctgaggtt ttaagcccta ttgcaaatga tcttgctaat 600

gatgatcata ttttctcacc tggttcaatc gagaagttgg aattggagtt aacagagctc 660

ctgaaatcaa gaagaggcaa tcctgtctcc attgcctctc tgcctatgat gtattatgag 720

agatatggta ggggtcttca ggctgaaggg tacctcactg agagccaacg acatgggaaa 780

gctggttata gtttgacaaa gcttcttgct cggttgagaa ccattcgtct aattgacagg 840

cctcatgggc agcattcagt aattttggca gaagacgtgc caaaatacat ggaaagccgg 900

agtgagagga gtgaccctgg tccaattgta agtggttccc ggcagatata tctgacattt 960

ccagctgaga gtacttttac tgaagaagat gtctctgact acttcagcac ctttggactg 1020

gttgaggatg tgaggattcc ctgccagcag aaacggatgt ttgggtttgt aacctttgac 1080

agttctgata ccgtgaagag cattttggcc aagggaagtc cacattatgt ttgtggggct 1140

```
cgtgttcttg tgaaacctta cagagaaaag ccaaggactg gtgataggaa atattcagag   1200

aaatttgagt cttcaatgta ttatcctttg caatatgcag acatggattc cgagcttcac   1260

atgatgccta gaggaatgga gacctcaaga ttgctcagaa agcagattat ggaagagcaa   1320

gagtttgcac aggatcttga atttgagaca aggcgtctct ccaagctgca gctagcacga   1380

aatcctctgg ccaatcagct ccaccatggc tattccttgg atgaactaaa agtcttggaa   1440

gccattattc catcattctg gaattgttct ctctcttctg acttgacgct tttcttggt    1500

cttgttcaaa ccatagcaca tgcaaatcat tccaagttcc cgactgttgt ccattccaat   1560

tatccattgg atgtttcaaa caatggctct acaagtgatg ataagccatg gcgtgcagtc   1620

aacaacccca tcgatcataa gaggtataaa tgcatttcta ttagtgatta g            1671


<210>  280
<211>  556
<212>  PRT
<213>  Vitis vinifera

<400>  280

Met Asp Phe Ser Glu Ser Thr Ala Val Val Phe Asn Arg Ile Gln Lys
1               5                   10                  15


Leu Glu Pro Glu Asn Val Ser Lys Ile Ile Gly Tyr Leu Leu Val Lys
            20                  25                  30


Gly Phe Ser Lys Gly Glu Met Ile Arg Leu Ala Phe Gly Pro Asp Lys
        35                  40                  45


Ala Ile Arg Met Ile Ile Glu Gly Val Lys Ile Glu Leu Gly Leu Ile
    50                  55                  60


Pro Asn Pro Pro Ser Pro Ile Ser Pro His Phe Pro Pro Leu Ser Pro
65                  70                  75                  80


Ser Thr Gly Ser Trp Phe Pro Ser Ser Ser Pro Ser Pro Val Asn Arg
                85                  90                  95


Tyr Leu Gln His Ala Thr Glu Gln Leu Pro Lys Asp Tyr Ser Leu Gln
            100                 105                 110


Ser Gln Pro Leu Gly Leu Glu Glu Gln Leu Glu Gln Val Asn Pro Ala
        115                 120                 125


Ile Leu Gly Val Ser Gly Asp Tyr Tyr Tyr Pro Glu Thr Ala Val Glu
    130                 135                 140
```

```
Asn Leu Ser Val Arg Thr Gly Pro Arg Ser Leu Ile Gly Ser Glu Phe
145                 150                 155                 160

Pro Val Lys Val Cys His Tyr Phe Asn Lys Gly Phe Cys Lys His Gly
                165                 170                 175

Asn Asn Cys Arg Tyr Leu His Ala Gln Val Phe Pro Glu Val Leu Ser
            180                 185                 190

Pro Ile Ala Asn Asp Leu Ala Asn Asp Asp His Ile Phe Ser Pro Gly
        195                 200                 205

Ser Ile Glu Lys Leu Glu Leu Glu Leu Thr Glu Leu Leu Lys Ser Arg
    210                 215                 220

Arg Gly Asn Pro Val Ser Ile Ala Ser Leu Pro Met Met Tyr Tyr Glu
225                 230                 235                 240

Arg Tyr Gly Arg Gly Leu Gln Ala Glu Gly Tyr Leu Thr Glu Ser Gln
                245                 250                 255

Arg His Gly Lys Ala Gly Tyr Ser Leu Thr Lys Leu Leu Ala Arg Leu
            260                 265                 270

Arg Thr Ile Arg Leu Ile Asp Arg Pro His Gly Gln His Ser Val Ile
        275                 280                 285

Leu Ala Glu Asp Val Pro Lys Tyr Met Glu Ser Arg Ser Glu Arg Ser
    290                 295                 300

Asp Pro Gly Pro Ile Val Ser Gly Ser Arg Gln Ile Tyr Leu Thr Phe
305                 310                 315                 320

Pro Ala Glu Ser Thr Phe Thr Glu Glu Asp Val Ser Asp Tyr Phe Ser
                325                 330                 335

Thr Phe Gly Leu Val Glu Asp Val Arg Ile Pro Cys Gln Gln Lys Arg
            340                 345                 350

Met Phe Gly Phe Val Thr Phe Asp Ser Ser Asp Thr Val Lys Ser Ile
        355                 360                 365

Leu Ala Lys Gly Ser Pro His Tyr Val Cys Gly Ala Arg Val Leu Val
    370                 375                 380

Lys Pro Tyr Arg Glu Lys Pro Arg Thr Gly Asp Arg Lys Tyr Ser Glu
385                 390                 395                 400
```

Lys Phe Glu Ser Ser Met Tyr Tyr Pro Leu Gln Tyr Ala Asp Met Asp
405 410 415

Ser Glu Leu His Met Met Pro Arg Gly Met Glu Thr Ser Arg Leu Leu
420 425 430

Arg Lys Gln Ile Met Glu Glu Gln Glu Phe Ala Gln Asp Leu Glu Phe
435 440 445

Glu Thr Arg Arg Leu Ser Lys Leu Gln Leu Ala Arg Asn Pro Leu Ala
450 455 460

Asn Gln Leu His His Gly Tyr Ser Leu Asp Glu Leu Lys Val Leu Glu
465 470 475 480

Ala Ile Ile Pro Ser Phe Trp Asn Cys Ser Leu Ser Ser Asp Leu Thr
485 490 495

Leu Phe Leu Gly Leu Val Gln Thr Ile Ala His Ala Asn His Ser Lys
500 505 510

Phe Pro Thr Val Val His Ser Asn Tyr Pro Leu Asp Val Ser Asn Asn
515 520 525

Gly Ser Thr Ser Asp Asp Lys Pro Trp Arg Ala Val Asn Asn Pro Ile
530 535 540

Asp His Lys Arg Tyr Lys Cys Ile Ser Ile Ser Asp
545 550 555

<210> 281
<211> 4830
<212> DNA
<213> Vitis vinifera

<400> 281
atggcttctg cttctttgat ctatgctgtt aattttatca accctccaaa atgtattttg 60

cattcgaaaa gggattattt ttctttcaat atgagttata cttgtacaaa atccaaaacc 120

tcacaaagac agatgagaaa taagcaatca agaatggcta caattgctgc tggaaatcaa 180

gaaatggact tcactgatcc ctgttggaag accaaatttc aagaggattt cgagttgaga 240

tttaatttgc ctcaccttaa ggatgtattg cccataaagc caaggcctac gacgttttcc 300

ctgaaaaata gaaatgctca attagtgaat ggcgccaatg tgcttgagga tcggaaaaat 360

ggttatgtta atgaggatga tagagcactt ctaaaggtta tcagatattc ttcaccaact 420

tctgctggag ctgagtgcat tgatcctgat tgcagctggg tggagcaatg ggtacatcgt 480

gctgggccac gtgaggagat attctttgag cctggggaag tgaaagctgg aattgttacc 540
tgtggagggc tctgtcctgg tctcaatgat gtcattagac agattgtttt cactctggaa 600
ctctatgggg ttaagaagat tgttggaatc cagtatggtt atcgtggact tttgatcgg 660
ggcttagctg aaatagagct tttccgtgaa gtggttcaaa acattaatct tgccggtgga 720
agtctgcttg gagtttcccg tggaggtgct gatatcagtg agattgtaga tagcatacag 780
gccaggggaa ttgatatgat tttcatactt gggggcaatg gtacacatgc aggagcaaat 840
gcaatacaca acgagtgccg caggaggaag atgaaagtat cggttatatg tgttccaaaa 900
acaattgata atgatattct gttaatggat aaaacctttg gatttgatac tgctgtagaa 960
gaagctcaaa gggctattaa ttctgcatat attgagtgca tatcatggca tcgggcttgt 1020
aaaactgatg ggaagaagca gcggctttat agcaatgcac gcttcgcttt caagcggtac 1080
catttcaaat tgagggacct tataggtgtc ttacgacatt tagagcatct catagagact 1140
aaagggtcag ctgtgctctg tgtggctgaa ggagcaggac aagattttgt agaaaagacg 1200
aattcgacgg atgcatctgg gaatgcgaga cttggagaca ttggtgttta tctccaacag 1260
cagatcaaga aacattttag gaggattggc gttccagctg atgttaaata cattgatccc 1320
acttatatga ttcgagcgtg tcgagcaaat gcatctgatg ctgttctttg cactgttctt 1380
ggccagaatg ctgtccatgg agcatttgca gggttcagtg gaatcactgt tggaatatgt 1440
aacagccact acgtctactt accaatcccg gaagtgatcg cctctccaag agtcgtcgat 1500
ccagacagcc ggatgtggca ccgtaactgc cgctttcatg gtgttgccat aatactattg 1560
caggaagtgg caaggttcac gttcccagag ctatttagct ctcttccgaa atgcgtctat 1620
tcttcatcta ccagtgaaga ttcacaatct ccaaccgacg atgagtttcc attgtttgga 1680
ttcgaatttt ttgactctgc gcaaatagag aagagcctca tcaaatcaat ctggagggat 1740
gacagagctc agtttctaac tcaatcaaag cgactctttc agaaccctga gaatcgagag 1800
agcttggaag acgacgaaaa cagagactca ccagaactac tggaccgaag cattgttgca 1860
aagcttctgc agtggtgttg caggtttgat tcagtggact gcgccacagc tttgctaaat 1920
ggagcggttg gaatgttgcc tctcatcaac gaaatggatg aaaayggacg aacggcactg 1980
catacggcgg cggaggctca cgcggccaga tgcgtcgagc ttctcctacg caaacgcgct 2040
cgtacggacc tcaagtccaa ggacggttgc tcacagcttg ctttggagct gtctctgtct 2100
agcagaagga tggatgtact gtggaatcca gatracgcca ttgaagactt gatcgttctg 2160
cttcgtgaaa aggacctaac ggcggtaaaa tttctgacgg agaaaacaaa agatatcgcg 2220
gaagtcgctt acgtgaccgc catggagggg cgtgtgattg cgttagctgc tctgctagtg 2280
gttgccgcag acaaggttaa cgcttcgata ctggtgttgc aaagcgacgg ggacttgagt 2340
tccaaggaga agaccagcat ttacgaatgc gtggttaaag aggccttatc tctgggccgg 2400

acggagacct caaagtcgac cacatgcaaa tggaaaagcg aggaagtgga gaagagggca 2460

ctcctgctat gcgagatgga gctgcttcag ctcttcggag ctgtagctca caacagttgt 2520

acggaaagga aggtgacgtc acctctcatt cgtgcagccc aggctggaga tgaagctgta 2580

atcaatttac ttctgaagac gagtatagaa gttgacgaca cagatgcaga aggaaactct 2640

gctcttcaat gttcccttaa gacgtccatg gcctcagttg ctaagcagat aagaattgta 2700

tggctccttc taaagcatgg tgctcgagtg agccacagaa ataaattggg gctaaatgca 2760

atccatattg ctgccgcaaa tggtaattca gaggccctcc atttgcttct actggaagag 2820

ccagacggtg tgaatgcaac aactgaaatg aaagaaaccc cactattttt tgcagtaaag 2880

aacaattata tggactgtgc tgagcttctt ttgcgctggg gagcaaatag ccaagtcctc 2940

aacttacgta gacaaagacc tattgacttg gcaaagtcgc aagagatgcg gttcatgcta 3000

agtccaacca atattggcct taggcaccga gctttcccca tgcaacggaa atttactact 3060

tacttccata accatgagat gatttcagaa acctgtgagt cactcccaaa cgtgatagaa 3120

gaaggcaccc ttcctgagag ctctagaact tgctcgatcg cgaagacagg aatctgcaga 3180

tactttgaat ctccaggagg ttgtgtgcga ggggctaagt gcttctatgc acatggggaa 3240

gatgagcttc ggcggctgaa gcagggaaca agaacacagc actcaagtac aattgaggag 3300

cttaaaagga aaatttttgt aggaggcctg ccctcttcac tggacactgt cacctttgac 3360

gaatgtcttt ttctctatca atcttgtgaa gcagactcat tggctaagat ttttgaagaa 3420

caatttggtt cagtagaaga agccatagtc atgggtgatc aaatgggcga ccaaatacac 3480

tctcgaggat ttggtttcgt catctttaaa catgagaaat ccgcctcaga tgctgttcaa 3540

gcgcactaca ttaccattat gggcaagcaa gttgagataa aaagtgctgt tccaaaatgc 3600

atattatttg cagagctcca aacactacca cctcaacaag aagatcagga acaaggacaa 3660

attattcagt ttcagccaca agcagcaaca cctgatgaga agaatactga tgatggtgaa 3720

cctaggcaga tgtcttgggt tgataaatta ctccagaatc cgccaaatac atgtttcagt 3780

gaacctcaga ttcttcttaa ttctacaact ccaaaccaaa gcatgcctaa atgggtcaga 3840

attttcaaga ggtggcttcc cagcttctta aatgatgttt caaagcgtct taaggaggga 3900

gagtggtacc ccctctcttc tctaaaagct gattttaggg ctacatgtgg ccaagaactg 3960

gatcacacct ctctgggcta tcccaagctt agtgacttca tgagatcttt ccctggccta 4020

tgtcgcatga agattgtccc tgtaggtgga cgaggacctg ccactcacat ggtcctccag 4080

cctaaccatc agcaacagac ccaaccactt ccaatgcggt gtttttctcc caccccttca 4140

ccccttgatg actatgatga tgatggttcc attgatttga agtctcttgg tgaatttcta 4200

ccagtttctt atgataatgc tggctccctt ggtggcagct ttgaggatgg ggactcactc 4260

catgggactc ttgaagagag tcctgctcac aaggatgcaa aacatggtgt ccacccatgg 4320

ttcttggaat ttttaaagcc agatacactc ctgggtcaac catggtttag gaatgaaaat 4380

gcagctgcag gagatgatta taaaagcaag gagctcaggc aacaaaaaag gcacctggtt 4440

ctagaggccc ttgcaagaga aaaaaacaac acctctgtct tcttcttgcg tgagtttgat 4500

ttttatgaga attacaagtc aagtgtggct cagggaaagt gctttgcatg caatagaagt 4560

gaaatgttct gggctaattt tccgtgcaaa cacttgctgt ggtgcggcaa ctgtaagata 4620

catgctattc aggcagccag cattttggag cacaaatgcg tggtgtgtga tgctcaagtg 4680

cagaatattg gtccactccc atggactgag aaatatcagc aaatctgtga tgtccccaac 4740

aacgacttcc ctcctttcga ccctaaccct ataagaatgt acgcccattc catgcctaca 4800

ttttcccaca agtgtatgat cgtttcatag 4830

<210> 282
<211> 697
<212> PRT
<213> Vitis vinifera

<220>
<221> UNSURE
<222> (542)..(542)
<223> Xaa can be any naturally occurring amino acid

<220>
<221> UNSURE
<222> (564)..(564)
<223> Xaa can be any naturally occurring amino acid

<400> 282

Met Asp Ser Tyr Glu Ala Thr Arg Ile Val Phe Ser Arg Ile Gln Ala
1 5 10 15

Leu Asp Pro Glu Asn Ala Ser Lys Ile Met Gly Tyr Ile Leu Leu Ile
20 25 30

Gln Asp His Gly Glu Lys Glu Met Ile Arg Leu Ala Phe Gly Pro Glu
35 40 45

Thr Leu Leu His Asn Leu Ile Leu Lys Ala Lys Thr Gln Leu Gly Ile
50 55 60

Leu Ser Asn Thr Pro Ser Thr Pro Thr Ser Pro Ser Pro Phe Asn Pro
65 70 75 80

Ile Ser Lys Pro Thr Arg Leu Pro Thr Asn Asn Gly Phe Asn Pro Ser
85 90 95

```
Ser Ser Trp Pro Val Ser Gly Phe Ser Asp Leu Arg Ser Pro Asn Ser
            100                 105                 110

Thr Thr Ala Gln Leu Ser Tyr Ala Ala Val Val Asn Gly Ala Thr Asn
        115                 120                 125

Val Ser Asp Leu Gly Thr Val Ser Ser Ser Pro Ala Ser Ile Pro Tyr
    130                 135                 140

Tyr Asn Asn Cys Ser Gly Ser Asn Asn Ser Ser Val Val Cys Asn Asp
145                 150                 155                 160

Asn Val Met Asp Asp Tyr Gln Leu Gln Asp His Leu Ser Phe Leu Asn
                165                 170                 175

Asp Ala Ser Lys Pro Glu Asp Leu Phe Asp Pro Arg Leu Glu Leu Ala
            180                 185                 190

Met Ser Pro Ser Phe Gly Glu Thr Gln Leu His Arg Arg Ser Tyr Ser
        195                 200                 205

Phe Asn Asp Ala Cys Tyr Gly Ser Asp Asp Gly Ala Ser Gly Phe Gly
    210                 215                 220

Trp Lys Pro Cys Leu Tyr Phe Ala Arg Gly Phe Cys Lys Asn Gly Asn
225                 230                 235                 240

Thr Cys Lys Phe Leu His Gly Gly Phe Ala Asp Ser Val Glu Ala Ser
                245                 250                 255

Ser Ala Ala Ser Ala Ala Ile Val Gly Ser Pro Gly Lys Leu Asp Gly
            260                 265                 270

Phe Glu Gln Glu Met Leu Arg Ser Gln Gln Gln Arg Leu Ala Val Ala
        275                 280                 285

Ser Gln Leu Met Ala Gly Leu Asn Phe Pro Tyr Asn Lys Cys Met Asn
    290                 295                 300

Phe Phe Met Gln Gln Asn Glu Thr Gln Arg Ser Ala Ala Ala Ala Leu
305                 310                 315                 320

Met Met Gly Glu Glu Leu His Lys Phe Gly Arg Cys Arg Pro Glu Arg
                325                 330                 335

Asn Asp Phe Ser Gly Met Gly Leu Gly Gly Ala Val Asn Pro Gly Ser
            340                 345                 350
```

```
Arg Gln Ile Tyr Leu Thr Phe Pro Ala Asp Ser Thr Phe Arg Glu Glu
        355                 360                 365

Asp Val Ser Asn Tyr Phe Ser Ile Phe Gly Pro Val Gln Asp Val Arg
    370                 375                 380

Ile Pro Tyr Gln Gln Lys Arg Met Phe Gly Phe Val Thr Phe Val Tyr
385                 390                 395                 400

Pro Glu Thr Val Lys Leu Ile Leu Ala Lys Gly Asn Pro His Phe Val
                405                 410                 415

Cys Asp Ser Arg Val Leu Val Lys Pro Tyr Lys Glu Lys Gly Lys Val
            420                 425                 430

Pro Glu Lys Lys Gln Gln His Gln Gln Gln Gln Gln Gln Gln Gln Gln
        435                 440                 445

Gln Gln Gln Leu Glu Arg Gly Glu Tyr Ser Thr Cys Ser Ser Pro Ser
    450                 455                 460

Gly Ile Asp Pro Arg Glu Pro Tyr Asp Leu His Leu Gly Ala Arg Met
465                 470                 475                 480

Phe Tyr Asn Thr Gln Glu Met Leu Leu Arg Arg Lys Leu Glu Glu Gln
                485                 490                 495

Ala Asp Leu Gln Gln Ala Ile Glu Leu Gln Gly Arg Arg Leu Met Asn
            500                 505                 510

Leu Gln Leu Leu Asp Leu Lys Asn His Gln His Gln His Gln His His
        515                 520                 525

Leu His Asn Leu Ser Gly Gly Ala Pro Val Ala Ser Pro Xaa Gln Ser
    530                 535                 540

Ser Ile His Asn Asn Gln Ser Leu Gly Leu Pro Ser Asp Gly Asn Asn
545                 550                 555                 560

Gln Glu Val Xaa Glu Glu Asn Ser Ser Ser Pro Ala Ala Thr Thr Ser
                565                 570                 575

Pro Thr Ala Ala Ala Asp Lys Pro Leu Arg Gln Glu Val Asn Ile Ser
            580                 585                 590

Cys Asn Ser Asn Ser Gly Asn Asp Ser Gly Asn Asn Ser Thr Glu Glu
        595                 600                 605
```

Ser Ser Asn Pro Ala Asp Phe Asp Leu His Glu Ser Leu Glu His Ile
610 615 620

Leu Pro Asp Ser Leu Phe Ala Ser Pro Thr Lys Ser Ala Gly Asp Arg
625 630 635 640

Ser Val Phe Ser Thr Ala Ser Ala Ser Val Asp Glu Ser Thr Thr Ile
645 650 655

Ser Ile Thr Pro Ala Ser Asn Asn Asn Pro Val Leu Pro Gly Thr Thr
660 665 670

Leu Asn Met Ala Ser Leu Lys Ser Cys Phe Phe Glu Met Pro Arg Phe
675 680 685

Pro Ser Gly His Gly Ala Ile Glu Met
690 695

<210> 283
<211> 20
<212> PRT
<213> Artificial sequence

<220>
<223> C3H consensus

<220>
<221> UNSURE
<222> (2)..(8)
<223> Xaa can be any naturally occurring amino acid

<220>
<221> UNSURE
<222> (9)..(9)
<223> Xaa can be a gap or any naturally occurring amino acid

<220>
<221> UNSURE
<222> (11)..(15)
<223> Xaa can be any naturally occurring amino acid

<220>
<221> UNSURE
<222> (17)..(19)
<223> Xaa can be any naturally occurring amino acid

<400> 283

Cys Xaa Xaa Xaa Xaa Xaa Xaa Xaa Xaa Cys Xaa Xaa Xaa Xaa Xaa Cys
1 5 10 15

Xaa Xaa Xaa His
20

<210> 284
<211> 5
<212> PRT
<213> Artificial sequence

<220>
<223> Motif I

<400> 284

Met Ile Arg Leu Ala
1 5

<210> 285
<211> 17
<212> PRT
<213> Artificial sequence

<220>
<223> Motif II

<220>
<221> VARIANT
<222> (16)..(16)
<223> / repalce = "Lys"

<220>
<221> VARIANT
<222> (17)..(17)
<223> / repalce = "Ala"

<400> 285

Glu Ser Leu Glu His Asn Leu Pro Asp Ser Pro Phe Ala Ser Pro Thr
1 5 10 15

Lys

<210> 286
<211> 56
<212> DNA
<213> Artificial sequence

<220>
<223> primer 1

<400> 286
ggggacaagt ttgtacaaaa aagcaggctt aaacaatgga tgcttatgaa gctaca 56

<210> 287
<211> 50
<212> DNA
<213> Artificial sequence

<220>

<223> primer 2

<400> 287
ggggaccact ttgtacaaga aagctgggta cgtaacataa catgctgtcc 50

<210> 288
<211> 2194
<212> DNA
<213> Oryza sativa

<400> 288
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag atttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctccttctc ccatctataa attcctcccc ccttttcccc tctctatata 1020

ggaggcatcc aagccaagaa gagggagagc accaaggaca cgcgactagc agaagccgag 1080

cgaccgcctt ctcgatccat atcttccggt cgagttcttg gtcgatctct tccctcctcc 1140

acctcctcct cacagggtat gtgcctccct tcggttgttc ttggatttat tgttctaggt 1200

tgtgtagtac gggcgttgat gttaggaaag gggatctgta tctgtgatga ttcctgttct 1260

tggatttggg atagaggggt tcttgatgtt gcatgttatc ggttcggttt gattagtagt 1320

atggttttca atcgtctgga gagctctatg gaaatgaaat ggtttaggga tcggaatctt 1380

gcgattttgt gagtaccttt tgtttgaggt aaaatcagag caccggtgat tttgcttggt 1440

gtaataaagt acggttgttt ggtcctcgat tctggtagtg atgcttctcg atttgacgaa 1500

gctatccttt gtttattccc tattgaacaa aaataatcca actttgaaga cggtcccgtt 1560

gatgagattg aatgattgat tcttaagcct gtccaaaatt tcgcagctgg cttgtttaga 1620

tacagtagtc cccatcacga aattcatgga aacagttata atcctcagga acaggggatt 1680

ccctgttctt ccgatttgct ttagtcccag aattttttt cccaaatatc ttaaaaagtc 1740

actttctggt tcagttcaat gaattgattg ctacaaataa tgcttttata gcgttatcct 1800

agctgtagtt cagttaatag gtaatacccc tatagtttag tcaggagaag aacttatccg 1860

atttctgatc tccattttta attatatgaa atgaactgta gcataagcag tattcatttg 1920

gattattttt tttattagct ctcacccctt cattattctg agctgaaagt ctggcatgaa 1980

ctgtcctcaa ttttgttttc aaattcacat cgattatcta tgcattatcc tcttgtatct 2040

acctgtagaa gtttcttttt ggttattcct tgactgcttg attacagaaa gaaatttatg 2100

aagctgtaat cgggatagtt atactgcttg ttcttatgat tcatttcctt tgtgcagttc 2160

ttggtgtagc ttgccacttt caccagcaaa gttc 2194

<210> 289
<211> 309
<212> DNA
<213> Arabidopsis thaliana

<400> 289
atggatatga taacgaagat ggtgatggag agaccggtgg tgatttacag caagagctct 60

tgctgtatgt ctcacacgat caagactttg ctctgcgatt tcggagcaaa tccagcggtt 120

tacgagctgg atgagatatc tagagggagg gagatcgagc aggcgttgtt gcggctcggg 180

tgtagccccg cagttccggg cgttttcatt ggtggagagt tggtcggtgg agccaacgag 240

gtcatgagtc tacatcttaa cggatccttg attcccatgc ttaagcgggc tggtgcattg 300

tgggtttga 309

<210> 290
<211> 102
<212> PRT
<213> Arabidopsis thaliana

<400> 290

Met Asp Met Ile Thr Lys Met Val Met Glu Arg Pro Val Val Ile Tyr
1 5 10 15

Ser Lys Ser Ser Cys Cys Met Ser His Thr Ile Lys Thr Leu Leu Cys
20 25 30

Asp Phe Gly Ala Asn Pro Ala Val Tyr Glu Leu Asp Glu Ile Ser Arg
35 40 45

Gly Arg Glu Ile Glu Gln Ala Leu Leu Arg Leu Gly Cys Ser Pro Ala

50 55 60

Val Pro Gly Val Phe Ile Gly Gly Glu Leu Val Gly Gly Ala Asn Glu
65 70 75 80

Val Met Ser Leu His Leu Asn Gly Ser Leu Ile Pro Met Leu Lys Arg
85 90 95

Ala Gly Ala Leu Trp Val
100

<210> 291
<211> 309
<212> DNA
<213> Arabidopsis thaliana

<400> 291
atggagaaga tatcaaattt gttagaagac aagcccgtgg tgatattcag caagacgtcc 60

tgctgtatga gtcactcgat caagtcgctt atatctggtt acggtgcgaa ttcaacagtg 120

tatgagctag acgaaatgtc taatggacca gagatcgaac gagcacttgt agagcttggg 180

tgcaaaccga ctgtgccagc tgtctttata gggcaagagc tcgtaggtgg tgcaaatcaa 240

cttatgtctc ttcaagtcag gaaccaacta gcttcgttgc tccgaagagc tggagccata 300

tggatttaa 309

<210> 292
<211> 102
<212> PRT
<213> Arabidopsis thaliana

<400> 292

Met Glu Lys Ile Ser Asn Leu Leu Glu Asp Lys Pro Val Val Ile Phe
1 5 10 15

Ser Lys Thr Ser Cys Cys Met Ser His Ser Ile Lys Ser Leu Ile Ser
20 25 30

Gly Tyr Gly Ala Asn Ser Thr Val Tyr Glu Leu Asp Glu Met Ser Asn
35 40 45

Gly Pro Glu Ile Glu Arg Ala Leu Val Glu Leu Gly Cys Lys Pro Thr
50 55 60

Val Pro Ala Val Phe Ile Gly Gln Glu Leu Val Gly Gly Ala Asn Gln
65 70 75 80

Leu Met Ser Leu Gln Val Arg Asn Gln Leu Ala Ser Leu Leu Arg Arg
85 90 95

```
Ala Gly Ala Ile Trp Ile
            100


<210>  293
<211>  887
<212>  DNA
<213>  Arabidopsis thaliana

<400>  293
aaacgaaccc aactacacaa gtctctctct cttttcccat ttctccctct ctctctttgt     60

ctctctatca tcaattatgc aaaaagcaat tcgaccatac gagtcaccgt ggacgaagac    120

cgtgccgggc aatagcattt tccttttaaa gaatgaagat aaaccatcat catcatcatc    180

atcattatca tggttaacat caggatcacc aaagccaaca tctataagca ataagagatc    240

aagcaaccta gttgtgatgg agaatgctgt ggtggtgttt gcaaggagag gctgttgttt    300

gggacacgtg gcaaaacggc tgctactgac acatggcgtg aatccagtgg tggttgagat    360

tggtgaagaa gacaacaaca actacgacaa tatcgtaagt gataaagaga aattacctat    420

gatgtacata ggaggaaagt tgtttggagg attggaaaat ctgatggctg ctcatattaa    480

tggccactcc atcaagattc gaaccgatac atggtcgtcg ttctcagtcg ccaccgtcga    540

ccgaatccgc tggtgagaat agcgtaagaa gcatccacgg taacgaatca acaagaaggg    600

ttaaattaga agagaccata actaaatcac gataagagaa tgctttcctc cgctcaaatc    660

ttacggatag gttctgagat gaagaacgaa gttgtttcag agataattta gtaatggatc    720

atcatcggag atcttaaaat tcgtagataa aatatggatt tatttttgt cgtttagaag     780

aagaagaata gcaaattttc gagtatttcc ttttttccga ctaggttacg aaaaaggaaa    840

tttcattaat tatgctaaaa acaaaaaaat atggaaattt tctcaca                  887


<210>  294
<211>  150
<212>  PRT
<213>  Arabidopsis thaliana

<400>  294

Met Gln Lys Ala Ile Arg Pro Tyr Glu Ser Pro Trp Thr Lys Thr Val
1               5                   10                  15


Pro Gly Asn Ser Ile Phe Leu Leu Lys Asn Glu Asp Lys Pro Ser Ser
            20                  25                  30


Ser Ser Ser Ser Leu Ser Trp Leu Thr Ser Gly Ser Pro Lys Pro Thr
        35                  40                  45


Ser Ile Ser Asn Lys Arg Ser Ser Asn Leu Val Val Met Glu Asn Ala
```

50 55 60

Val Val Val Phe Ala Arg Arg Gly Cys Cys Leu Gly His Val Ala Lys
65 70 75 80

Arg Leu Leu Leu Thr His Gly Val Asn Pro Val Val Val Glu Ile Gly
85 90 95

Glu Glu Asp Asn Asn Asn Tyr Asp Asn Ile Val Ser Asp Lys Glu Lys
100 105 110

Leu Pro Met Met Tyr Ile Gly Gly Lys Leu Phe Gly Gly Leu Glu Asn
115 120 125

Leu Met Ala Ala His Ile Asn Gly Asp Leu Val Pro Thr Leu Arg Gln
130 135 140

Ala Gly Ala Leu Trp Leu
145 150

<210> 295
<211> 505
<212> DNA
<213> Arabidopsis thaliana

<400> 295
aatccatata cttcttcttc ttcaccttat gcaagataat ggacaaagtt atgagaatgt 60

cgtccgaaaa aggggtggtt atatttacca agagctcctg ttgtttgtcc tatgcggttc 120

aagttctctt ccaagatctt ggtgttaacc ctaagatcca cgagattgat aaggaccctg 180

aatgccgaga gatagagaag gctcttatga ggctagggtg ttcaaagccg gtcccagccg 240

tcttcattgg tggcaagctc gttggttcga ccaacgaagt aatgtccatg cacctaagca 300

gctcgctcgt tcccctagtg aagccatatt tatgttaaac aacaacgaag gagtatttat 360

gatattaatt agctatgtat atgttattca ataaggaaca aaattgagcc aaatctttgt 420

aatgtgtttt ttggtattat tattggttgt ataacattgg gaaagtgtac gtataattat 480

aagactgtta tattgattcg aaggt 505

<210> 296
<211> 99
<212> PRT
<213> Arabidopsis thaliana

<400> 296

Met Asp Lys Val Met Arg Met Ser Ser Glu Lys Gly Val Val Ile Phe
1 5 10 15

Thr Lys Ser Ser Cys Cys Leu Ser Tyr Ala Val Gln Val Leu Phe Gln
20 25 30

Asp Leu Gly Val Asn Pro Lys Ile His Glu Ile Asp Lys Asp Pro Glu
35 40 45

Cys Arg Glu Ile Glu Lys Ala Leu Met Arg Leu Gly Cys Ser Lys Pro
50 55 60

Val Pro Ala Val Phe Ile Gly Gly Lys Leu Val Gly Ser Thr Asn Glu
65 70 75 80

Val Met Ser Met His Leu Ser Ser Ser Leu Val Pro Leu Val Lys Pro
85 90 95

Tyr Leu Cys

<210> 297
<211> 600
<212> DNA
<213> Arabidopsis thaliana

<400> 297
atctatcttt aaaaacatac ttgaaaatgc aaggaacgat ttcttgtgca agaaattata 60

acatgacgac aaccgtcggg gaatctctgc ggccgctatc gcttaaaacg cagggaaacg 120

gcgagagagt tcggatggtg gtggaggaga acgcggtgat tgtgattgga cggagaggat 180

gttgcatgtg tcatgtggtg aggaggctgc ttcttggact tggagtgaat ccggcggtcc 240

ttgagattga tgaggagagg gaagatgaag ttttgagtga gttggagaat attggagttc 300

aaggcggcgg aggtacggtg aagttaccgg cggtttatgt aggagggagg ttgtttggag 360

ggttagatag ggttatggct actcatatct ccggtgagtt agttccaatt cttaaggaag 420

ttggggctct gtggttgtga ttgtaaatta ataatttaaa attatttttt tttcttttaa 480

ttaagaatct tgattggtaa ttgttgttta cggtttataa ttgaatcgtt tcatatatat 540

gtatataaag aaataaataa aagaaaagtc tcaagttgaa atttgctaga gattgtaccc 600

<210> 298
<211> 137
<212> PRT
<213> Arabidopsis thaliana

<400> 298

Met Gln Gly Thr Ile Ser Cys Ala Arg Asn Tyr Asn Met Thr Thr Thr
1 5 10 15

Val Gly Glu Ser Leu Arg Pro Leu Ser Leu Lys Thr Gln Gly Asn Gly

```
            20                  25                  30

Glu Arg Val Arg Met Val Val Glu Glu Asn Ala Val Ile Val Ile Gly
        35                  40                  45

Arg Arg Gly Cys Cys Met Cys His Val Val Arg Arg Leu Leu Leu Gly
    50                  55                  60

Leu Gly Val Asn Pro Ala Val Leu Glu Ile Asp Glu Glu Arg Glu Asp
65                  70                  75                  80

Glu Val Leu Ser Glu Leu Glu Asn Ile Gly Val Gln Gly Gly Gly Gly
                85                  90                  95

Thr Val Lys Leu Pro Ala Val Tyr Val Gly Gly Arg Leu Phe Gly Gly
            100                 105                 110

Leu Asp Arg Val Met Ala Thr His Ile Ser Gly Glu Leu Val Pro Ile
        115                 120                 125

Leu Lys Glu Val Gly Ala Leu Trp Leu
    130                 135


<210>  299
<211>  680
<212>  DNA
<213>  Arabidopsis thaliana

<400>  299
ctcaggcaac actgagctta atactgtagt acacacacac acacacacac acacacacac      60

aaaaccctct tttcttcaaa caggaacccc aaaagcgagg tttaatctca ggcgttttca     120

gttctaaatc tctttcaatc cacaagagaa ggaagatttt gtttcttctt ctccaagaaa     180

caatccctag attgatcgag acctccttca agaaaccatg gacaaagttg tgagaatgtc     240

gtcagagaaa ggagtggtta ttttcagcaa gagctcgtgt tgcatgtcct atgcggtcca     300

agtacttttc caagaccttg ggggttcaccc aacagtccat gagatcgata aagaccctga     360

atgtcgtgag atcgagaaag ccctaatgag gttagggtgt tccacgccgg tcccagccat     420

ctttgtgggt gggaagctca ttggttcgac caatgaagtc atgtcgcttc acttaagcgg     480

ctcgctggtt ccgctagtta agccgtttca agccaatcta tgttaaaaag gtgttctaat     540

tttctatact acaaaaatgt atttcaataa gaaacacaaa tcttatagcc tatgcaacct     600

ttgtaatgta gctttatata tatattgttt gttctgtaat ttcaggtaat atccaataat     660

aattgactaa tttctactca                                                 680


<210>  300
```

<211> 102
<212> PRT
<213> Arabidopsis thaliana

<400> 300

Met Asp Lys Val Val Arg Met Ser Ser Glu Lys Gly Val Val Ile Phe
1 5 10 15

Ser Lys Ser Ser Cys Cys Met Ser Tyr Ala Val Gln Val Leu Phe Gln
20 25 30

Asp Leu Gly Val His Pro Thr Val His Glu Ile Asp Lys Asp Pro Glu
35 40 45

Cys Arg Glu Ile Glu Lys Ala Leu Met Arg Leu Gly Cys Ser Thr Pro
50 55 60

Val Pro Ala Ile Phe Val Gly Gly Lys Leu Ile Gly Ser Thr Asn Glu
65 70 75 80

Val Met Ser Leu His Leu Ser Gly Ser Leu Val Pro Leu Val Lys Pro
85 90 95

Phe Gln Ala Asn Leu Cys
100

<210> 301
<211> 312
<212> DNA
<213> Arabidopsis thaliana

<400> 301
atggaacgag taagagattt ggcatcggag aaggcggctg tgatattcac gaagagctcg 60

tgttgcatgt gtcatagcat caagactctc ttctacgaac tcggggcgag tcctgccatc 120

catgagcttg acaaggaccc gcaaggccct gacatggaac gggccctctt ccgggtattc 180

gggtctaacc ctgctgtccc tgcggttttc gtaggaggaa ggtacgtcgg ctcagctaaa 240

gacgtcatct ccttccacgt ggatggctcc ctcaagcaga tgttaaaggc ctctaacgcc 300

atatggttgt ga 312

<210> 302
<211> 103
<212> PRT
<213> Arabidopsis thaliana

<400> 302

Met Glu Arg Val Arg Asp Leu Ala Ser Glu Lys Ala Ala Val Ile Phe
1 5 10 15

```
Thr Lys Ser Ser Cys Cys Met Cys His Ser Ile Lys Thr Leu Phe Tyr
            20                  25                  30

Glu Leu Gly Ala Ser Pro Ala Ile His Glu Leu Asp Lys Asp Pro Gln
        35                  40                  45

Gly Pro Asp Met Glu Arg Ala Leu Phe Arg Val Phe Gly Ser Asn Pro
    50                  55                  60

Ala Val Pro Ala Val Phe Val Gly Gly Arg Tyr Val Gly Ser Ala Lys
65                  70                  75                  80

Asp Val Ile Ser Phe His Val Asp Gly Ser Leu Lys Gln Met Leu Lys
                85                  90                  95

Ala Ser Asn Ala Ile Trp Leu
            100


<210>  303
<211>  632
<212>  DNA
<213>  Arabidopsis thaliana

<400>  303
atactcaaaa caaaacaaaa catacatcaa aacgctaaag tttaaacccc tagccatcat      60

cagatcttca gacttctgag gatcatggac aaagtgatga gaatgtcttc agagaaagga     120

gtggtgatct tcacgaagag ctcatgttgt ctctgctacg ccgttcaaat cctgttccgt     180

gaccttaggg ttcaaccaac catccacgag atcgacaacg acccggactg ccgtgagatc     240

gagaaggctc ttctccggct cggctgttcc acggcggttc cagctgtctt tgtcggaggc     300

aagcttgttg gctccaccaa tgaagtcatg tcccttcacc ttagtggctc tcttgtccca     360

ttgatcaaac cctatcagtc catcctttac tagcaaaatt aaaccaactc aatatataat     420

atctaattat tagctagtga gaataaacac agttacagct agagtgtgag ctagctagat     480

attcagtgag gacttcgtct gaattaatgt ttatcgtttg tatgttctat tgtttagctt     540

ctctcgtgtt tcagtttagt taatcaactg gtgtatgttg atgtatgact ctctgtttat     600

gctaatgaaa atagtattga aacttttaca tt                                   632


<210>  304
<211>  102
<212>  PRT
<213>  Arabidopsis thaliana

<400>  304

Met Asp Lys Val Met Arg Met Ser Ser Glu Lys Gly Val Val Ile Phe
1               5                   10                  15
```

```
Thr Lys Ser Ser Cys Cys Leu Cys Tyr Ala Val Gln Ile Leu Phe Arg
            20                  25                  30

Asp Leu Arg Val Gln Pro Thr Ile His Glu Ile Asp Asn Asp Pro Asp
        35                  40                  45

Cys Arg Glu Ile Glu Lys Ala Leu Leu Arg Leu Gly Cys Ser Thr Ala
    50                  55                  60

Val Pro Ala Val Phe Val Gly Gly Lys Leu Val Gly Ser Thr Asn Glu
65                  70                  75                  80

Val Met Ser Leu His Leu Ser Gly Ser Leu Val Pro Leu Ile Lys Pro
                85                  90                  95

Tyr Gln Ser Ile Leu Tyr
            100
```

<210> 305
<211> 748
<212> DNA
<213> Arabidopsis thaliana

<400> 305

```
ccaacaaact ttagccaatc cctctttctc tcttattcgt gtttaatcta gtttctttcc      60

aaacaaaagt gtaacaagag aaagagaaga gatatcaaag atgcaatacc agacagaatc     120

gtggggatca tacaagatga gcagcttagg attcggcggt ttggggatgg tggctgacac     180

tggtctgctt cgtatagagt ccctggcttc tgagagcgcg gtggtgatct tcagcgtgag     240

cacgtgctgc atgtgccacg ccgtgaaggg tctcttcaga ggaatgggcg tcagccccgc     300

cgtccacgag ctcgacctcc acccctacgg cggcgacatc cagcgagccc tcattcgtct     360

cctcggctgc tccggctcct cttctccagg gtctctcccg gtcgtcttca tcggcggtaa     420

actggttgga gctatggaca gagtcatggc ttctcacata aacggctctc tcgttcctct     480

tctcaaagac gccggcgctc tctggctctg atcccttcct ctgctttctt ttttcttttc     540

tatttgaagt tttcttgtaa gagaatgtgg tggaggaaga ttaggaaact agtcaatggc     600

tgtaatgaca ggttttagat tatagtttgt aattagagag agagttgttt taagctcacc     660

tttctctgtc ttcctcttct tcatctctta ttgatctttc gaatgctctc attaaatcat     720

aatagtaaac actttgcatc tttattaa                                        748
```

<210> 306
<211> 136
<212> PRT
<213> Arabidopsis thaliana

<400> 306

Met Gln Tyr Gln Thr Glu Ser Trp Gly Ser Tyr Lys Met Ser Ser Leu
1 5 10 15

Gly Phe Gly Gly Leu Gly Met Val Ala Asp Thr Gly Leu Leu Arg Ile
20 25 30

Glu Ser Leu Ala Ser Glu Ser Ala Val Val Ile Phe Ser Val Ser Thr
35 40 45

Cys Cys Met Cys His Ala Val Lys Gly Leu Phe Arg Gly Met Gly Val
50 55 60

Ser Pro Ala Val His Glu Leu Asp Leu His Pro Tyr Gly Gly Asp Ile
65 70 75 80

Gln Arg Ala Leu Ile Arg Leu Leu Gly Cys Ser Gly Ser Ser Ser Pro
85 90 95

Gly Ser Leu Pro Val Val Phe Ile Gly Gly Lys Leu Val Gly Ala Met
100 105 110

Asp Arg Val Met Ala Ser His Ile Asn Gly Ser Leu Val Pro Leu Leu
115 120 125

Lys Asp Ala Gly Ala Leu Trp Leu
130 135

<210> 307
<211> 813
<212> DNA
<213> Arabidopsis thaliana

<400> 307
atggaggaat taaggcaagc aacgaacaat tttgacatgg ataatactat tgggcatgga 60

agtcacggaa cagtttacac tgggctgatc aaggacatgc ccgttgtgat caaaagggag 120

tggtgtagtc cccaacctag attcttggat gaggtacaca caaacaaaaa attggtttct 180

tacatccaac ataaaaattt ggttaatctg ctcggttatt gttgcgacga cgagaaccaa 240

tcgctcgtct ttgagtatat ggtcaacggt agcgtccgag attatctaga gtctagcgac 300

ttaacgttca agaaaagaat atctatagct cttgatgcag ccaaagggtt actacacttg 360

cacaacttag atcctccagt acaacacaag agatttacgg cgagtaaagt tttgctcgat 420

gccaacctca atgccaaggt atcagatggg gcaatgttgg gactgcttca agcaagtgat 480

attcatctcg ctaatccaag aggtggttca gaggagacaa ttgatgtgta tagcttcggg 540

ttgttccttc tagagcttat cacctgtcaa aaccctggac tgttacaatc agactatgaa 600

gttttacaat ggaatatacc tgcagaaaca ttcacgagac catcgttcca agcttatctg 660

ataattacgt cggaatgctt ggaatatccc ccgataaatc gcccaaagat ggatgtggtc 720

gtgaccgagc ttgagacgat ttaccttaat gtcatcagtg agaaccatga ggtttctcta 780

ggaagtgagc tttttaacat aaccattgag taa 813

<210> 308
<211> 100
<212> PRT
<213> Arabidopsis thaliana

<400> 308

Met Asp Val Val Ala Arg Leu Ala Ser Gln Arg Ala Val Val Ile Phe
1 5 10 15

Ser Lys Ser Thr Cys Cys Met Ser His Ala Ile Lys Arg Leu Phe Tyr
20 25 30

Glu Gln Gly Val Ser Pro Ala Ile Val Glu Ile Asp Gln Asp Met Tyr
35 40 45

Gly Lys Asp Ile Glu Trp Ala Leu Ala Arg Leu Gly Cys Ser Pro Thr
50 55 60

Val Pro Ala Val Phe Val Gly Gly Lys Phe Val Gly Thr Ala Asn Thr
65 70 75 80

Val Met Thr Leu His Leu Asn Gly Ser Leu Lys Ile Leu Leu Lys Glu
85 90 95

Ala Gly Ala Leu
100

<210> 309
<211> 309
<212> DNA
<213> Arabidopsis thaliana

<400> 309
atggatgtgg tagcaagatt agcgtcgcaa agagcggtgg tgatattcag caagagtacg 60

tgttgcatgt ctcatgcaat taaacggttg ttttacgagc aaggtgtgag cccggcaatt 120

gtagagatcg accaagacat gtatgggaaa gatatcgagt gggccttggc ccgattaggc 180

tgtagcccta cggttcctgc ggtttttgtt ggagggaaat tcgtaggaac ggccaatact 240

gtcatgactc ttcatctcaa tggatcattg aaaatattgc tcaaggaggc tggtgctttg 300

tggctttag 309

<210> 310
<211> 102
<212> PRT
<213> Arabidopsis thaliana

<400> 310

Met Asp Val Val Ala Arg Leu Ala Ser Gln Arg Ala Val Val Ile Phe
1               5                   10                  15

Ser Lys Ser Thr Cys Cys Met Ser His Ala Ile Lys Arg Leu Phe Tyr
            20                  25                  30

Glu Gln Gly Val Ser Pro Ala Ile Val Glu Ile Asp Gln Asp Met Tyr
        35                  40                  45

Gly Lys Asp Ile Glu Trp Ala Leu Ala Arg Leu Gly Cys Ser Pro Thr
    50                  55                  60

Val Pro Ala Val Phe Val Gly Gly Lys Phe Val Gly Thr Ala Asn Thr
65                  70                  75                  80

Val Met Thr Leu His Leu Asn Gly Ser Leu Lys Ile Leu Leu Lys Glu
                85                  90                  95

Ala Gly Ala Leu Trp Leu
            100

<210> 311
<211> 486
<212> DNA
<213> Arabidopsis thaliana

<400> 311
aaagcaaaaa caaacataaa gatccttgag ttcccactca catagtcaca ctacatttgt 60

atcacattta tatacataga aatggaaagc gttagaagtt tagttgaaga caaaccagtg 120

gtgatattca gcaaaagctc ttgctgcatg agccactcaa ttcaaacact gatctcaggg 180

tttggggcaa agatgacggt ctacgagcta gaccaattct caaacggtca ggagatcgag 240

aaggcattgg tacagatggg gtgtaaaccc agtgtaccag ctgtgttcat agggcaacaa 300

ttcatcggtg gtgctaacca agtaatgact cttcaggtca agaaccagct agccgcaatg 360

ctaagaagag ccggagccat atgggtgtaa cagaagacaa gagagtcaaa tgcaaactag 420

gatagaataa gatgagaatg atacatatcg ttgttttgct tcttctttaa ttttcggggt 480

ttcgag 486

<210> 312

<211> 102
<212> PRT
<213> Arabidopsis thaliana

<400> 312

Met Glu Ser Val Arg Ser Leu Val Glu Asp Lys Pro Val Val Ile Phe
1 5 10 15

Ser Lys Ser Ser Cys Cys Met Ser His Ser Ile Gln Thr Leu Ile Ser
20 25 30

Gly Phe Gly Ala Lys Met Thr Val Tyr Glu Leu Asp Gln Phe Ser Asn
35 40 45

Gly Gln Glu Ile Glu Lys Ala Leu Val Gln Met Gly Cys Lys Pro Ser
50 55 60

Val Pro Ala Val Phe Ile Gly Gln Gln Phe Ile Gly Gly Ala Asn Gln
65 70 75 80

Val Met Thr Leu Gln Val Lys Asn Gln Leu Ala Ala Met Leu Arg Arg
85 90 95

Ala Gly Ala Ile Trp Val
100

<210> 313
<211> 686
<212> DNA
<213> Arabidopsis thaliana

<400> 313
ctcattacaa aaaaaacaac acttattgat cactaacttt cttgaccatc gcaaatggag 60

agaataagag atttgtcgtc gaagaaagcg gcggtgatat tcacaaagag ctcatgttgt 120

atgtgccata gcatcaagac gctattctac gaactaggcg ctagtccggc gatccatgag 180

ctcgacaaag accctgaagg ccgtgaaatg gaacgggccc tacgtgccct cggctcatcg 240

aacccggcgg ttccagctgt tttcgttgga ggaaggtaca tcggatcagc caaagacatc 300

atctcattcc acgtggacgg gtcactcaag cagatgctta aagacgctaa ggccatttgg 360

ttatagcgtc cgatcatcgt acatgtatgt gtatatatct atatatatat atatatatgt 420

atgcacatgt aattgtgtca ataatcgatg tgctaagagc gcatagatga taaatataat 480

cgggagggag catagatgta ttaaaatgct ttgctttctt cggatggatc gtagttattg 540

gataataatt tatatacatg tatagatgta tatatgtatg ttgacttacg tacattgtac 600

ggatgaaaag acaagtgtac ctttaagcac tgttgtataa cattactgtt cgaattccac 660

gtaaagagta aactcaaatc ttaaaa 686

<210> 314
<211> 103
<212> PRT
<213> Arabidopsis thaliana

<400> 314

```
Met Glu Arg Ile Arg Asp Leu Ser Ser Lys Lys Ala Ala Val Ile Phe
1               5                   10                  15

Thr Lys Ser Ser Cys Cys Met Cys His Ser Ile Lys Thr Leu Phe Tyr
            20                  25                  30

Glu Leu Gly Ala Ser Pro Ala Ile His Glu Leu Asp Lys Asp Pro Glu
        35                  40                  45

Gly Arg Glu Met Glu Arg Ala Leu Arg Ala Leu Gly Ser Ser Asn Pro
    50                  55                  60

Ala Val Pro Ala Val Phe Val Gly Gly Arg Tyr Ile Gly Ser Ala Lys
65                  70                  75                  80

Asp Ile Ile Ser Phe His Val Asp Gly Ser Leu Lys Gln Met Leu Lys
                85                  90                  95

Asp Ala Lys Ala Ile Trp Leu
            100
```

<210> 315
<211> 615
<212> DNA
<213> Arabidopsis thaliana

<400> 315

```
atcaaagcta catatactaa aagatatata atttctcgat tgtcctgagc cctcagacca      60

aaatctgacc atggacaagg ttatgagaat gtcatcggag aaaggagtcg tgatcttcac     120

caaaagttca tgttgtctct gctacgccgt gcaaatcctt ttccgtgatc ttagggttca     180

accaacaatc cacgagatcg acaacgatcc tgactgccgt gagatcgaga aggccttagt     240

tcgtcttggc tgcgccaacg cggttcctgc tgtctttgta agtggcaagc tcgtgggttc     300

gaccaacgat gtcatgtcgc ttcacctaag tggctccctc gttcccttga tcaagccgta     360

tcagtcattt cataactaga aaaacaatat cgatgcttaa gaaagataat tagtatatac     420

tattaattgg acagtgagaa taatgatgga attagataac acgtaaatgt gtatcaggtt     480

cttatttata gctagtgctt atatcttgtt tagcttatgt ctaagaaatt gatgagctag     540

ctttgtattt ccagcttaac taatcggtgg atgtactgat gtgtactatc tatttaatgg     600
```

aaaaattatt gagca 615

<210> 316
<211> 102
<212> PRT
<213> Arabidopsis thaliana

<400> 316

Met Asp Lys Val Met Arg Met Ser Ser Glu Lys Gly Val Val Ile Phe
1 5 10 15

Thr Lys Ser Ser Cys Cys Leu Cys Tyr Ala Val Gln Ile Leu Phe Arg
20 25 30

Asp Leu Arg Val Gln Pro Thr Ile His Glu Ile Asp Asn Asp Pro Asp
35 40 45

Cys Arg Glu Ile Glu Lys Ala Leu Val Arg Leu Gly Cys Ala Asn Ala
50 55 60

Val Pro Ala Val Phe Val Ser Gly Lys Leu Val Gly Ser Thr Asn Asp
65 70 75 80

Val Met Ser Leu His Leu Ser Gly Ser Leu Val Pro Leu Ile Lys Pro
85 90 95

Tyr Gln Ser Phe His Asn
100

<210> 317
<211> 505
<212> DNA
<213> Arabidopsis thaliana

<400> 317
cagtctcctt gattgtctct aattttccaa tctttcattt ctgattttgc attgtaatcg 60

aacataccca ctaatatcct ttgcaagccg cttttcaaac aacctattac attataacac 120

caatggagaa gatacaaaag atgatctccg agaagtcggt agtaatattt agcaataact 180

cttgttgcat gtcacacaca atcaagactc tcttcttaga ccttggcgtg aacccgacaa 240

tctatgagct agacgagatc aacagaggaa aagagataga gtatgcattg gctcagcttg 300

gctgcagccc gactgtgcca gtggtgttca taggagggca gcttgttggt ggagccaatc 360

aagtcatgag tctccatctc aaccgttctc tcattccaat gcttaaacgc tttggggctt 420

tatggctttg ataaaatata aaagaatagt tacttattga tcgtagcttg gtaataatga 480

tgtcatgaaa ccaatcaagg atgac 505

<210> 318
<211> 102
<212> PRT
<213> Arabidopsis thaliana

<400> 318

```
Met Glu Lys Ile Gln Lys Met Thr Ser Glu Lys Ser Leu Val Ile Phe
1               5                   10                  15

Ser Lys Asn Ser Cys Cys Met Ser His Thr Ile Lys Thr Leu Phe Leu
            20                  25                  30

Asp Leu Gly Val Asn Pro Thr Ile Tyr Glu Leu Asp Glu Ile Asn Arg
        35                  40                  45

Gly Lys Glu Ile Glu Gln Ala Leu Ala Gln Leu Gly Cys Ser Pro Thr
    50                  55                  60

Val Pro Val Val Phe Ile Gly Gly Gln Leu Val Gly Gly Ala Asn Gln
65                  70                  75                  80

Val Met Ser Leu His Leu Asn Arg Ser Leu Ile Pro Met Leu Lys Arg
                85                  90                  95

Phe Gly Ala Leu Trp Leu
            100
```

<210> 319
<211> 587
<212> DNA
<213> Arabidopsis thaliana

<400> 319

```
cataacctat ttcatcctcc tcaagtcact tttcaaacac actttcaaat taacaaaaat      60

ggagaagcta cagaagatga cctcggagaa gtcgttagtg atatttagca aaaactcatg     120

ctgcatgtcg cacacaatca agactctctt cttagacctt ggcgtaaatc cgacgattta     180

tgagctagat gagatcaaca gaggaaaaga gatagagcaa gcattggctc agcttggctg     240

cagcccgacc gtgccagtgg tgttcatagg agggcagctt gtcggtggag ccaatcaagt     300

catgagtctc catctcaacc gttctctcat tccaatgctt aaacgcgttg gggcgttatg     360

gctttgacaa aatataaaga aatagttact tattgattgt agattggtaa taataatgta     420

ttgaaaccaa tcatatacat atgaatgttt tgtgtctatc taagttttga aaggatagat     480

tatgaggcag ggtactgatt tcgtaacgtt cttggtatac ttagtgttgt atttctattt     540

tcagttttta tgaatacaaa catgcattta agaaaagtgt atgccat                  587
```

<210> 320

<211> 102
<212> PRT
<213> Arabidopsis thaliana

<400> 320

```
Met Glu Lys Ile Gln Lys Met Ile Ser Glu Lys Ser Val Val Ile Phe
1               5                   10                  15

Ser Asn Asn Ser Cys Cys Met Ser His Thr Ile Lys Thr Leu Phe Leu
            20                  25                  30

Asp Leu Gly Val Asn Pro Thr Ile Tyr Glu Leu Asp Glu Ile Asn Arg
        35                  40                  45

Gly Lys Glu Ile Glu Tyr Ala Leu Ala Gln Leu Gly Cys Ser Pro Thr
    50                  55                  60

Val Pro Val Val Phe Ile Gly Gly Gln Leu Val Gly Gly Ala Asn Gln
65                  70                  75                  80

Val Met Ser Leu His Leu Asn Arg Ser Leu Ile Pro Met Leu Lys Arg
                85                  90                  95

Val Gly Ala Leu Trp Leu
            100
```

<210> 321
<211> 644
<212> DNA
<213> Arabidopsis thaliana

<400> 321

```
atcaaaaaca aactctaagt catctcttat atatcgtcag ccaaagactt caagttctct      60
agcttaccaa tttcacccct tttctctcat ttcaataaaa aaaactaccc atttcatcct     120
cggcgagtcg ctcttcgaac acatttcaca ttataatacc aatggataag ctacagaaga     180
tgatctccga gaagtcggta gtgatcttta gcaaaaactc atgttgcatg tctcacacta     240
tcaagactct cttcatagac tttggcgtga atccaacgat ctatgagcta gatgagatca     300
acagaggaaa ggagatagag caagcattgg ctcagcttgg ctgcagccca accgtgcctg     360
tggtgtttat tggagggcag cttgttggtg gagccaatca agtcatgagt ctccatctca     420
atcgctctct ggttcctatg ctaaagaggg ttggagcact atggctttga tttcaagata     480
aggggatatt tacgttatga acgtagcatg gtaataataa tgtaatgaaa gtaatcaaag     540
atgacaaaca aaaacacata tacatgtatg ttgtctatct aagttttgga aggatagact     600
atgactatag gatacctatt ttgtaacgtg tgggtaaact ttat                      644
```

<210> 322
<211> 102
<212> PRT
<213> Arabidopsis thaliana

<400> 322

Met Asp Lys Leu Gln Lys Met Ile Ser Glu Lys Ser Val Val Ile Phe
1 5 10 15

Ser Asn Asn Ser Cys Cys Met Ser His Thr Ile Lys Thr Leu Phe Ile
20 25 30

Asp Phe Gly Val Asn Pro Thr Ile Tyr Glu Leu Asp Glu Ile Asn Arg
35 40 45

Gly Lys Glu Ile Glu Gln Ala Leu Ala Gln Leu Gly Cys Ser Pro Thr
50 55 60

Val Pro Val Val Phe Ile Gly Gly Gln Leu Val Gly Gly Ala Asn Gln
65 70 75 80

Val Met Ser Leu His Leu Asn Arg Ser Leu Ile Pro Met Leu Lys Arg
85 90 95

Val Gly Ala Leu Trp Leu
100

<210> 323
<211> 598
<212> DNA
<213> Arabidopsis thaliana

<400> 323
atcttcaccc aaagattata agctctctac actttttgt agtatagtct cttatttcag 60

tcaaaagaca cacatttgca tcctccgtga atcactttct tcagaaaatt tacaaaaaca 120

atggagaacc tacagaagat gatctctgag aagtcggtag taatttttag caagaactca 180

tgctgcatgt ctcatacaat taagactctc ttcttagact ttggcgtgaa cccgactatc 240

tatgagctcg acgagatcaa cataggaagg gagatagagc aagcattggc tcagctcgga 300

tgcagcccga ccgttccggt ggtgttcatt ggagggcagc ttgttggtgg agccaatcaa 360

gtcatgagtc tccatctcaa ccgctccctt gttcctatgc ttaaacgtgc tggagcatta 420

tggctttaac ttcaaaataa atgtaatttc aaatatataa tgcaattaag ttactataat 480

taaagtgaac aaagaaaaca tacacaagaa tgtatgtatg agttaatgct atgtctatct 540

aagttttaaa aagatagatt atccggttgc ctattttgta aacactggtt ctattata 598

<210> 324

<211> 102
<212> PRT
<213> Arabidopsis thaliana

<400> 324

Met Glu Asn Leu Gln Lys Met Ile Ser Glu Lys Ser Val Val Ile Phe
1 5 10 15

Ser Lys Asn Ser Cys Cys Met Ser His Thr Ile Lys Thr Leu Phe Ile
20 25 30

Asp Phe Gly Val Asn Pro Thr Ile Tyr Glu Leu Asp Glu Ile Asn Arg
35 40 45

Gly Lys Glu Ile Glu Gln Ala Leu Ala Gln Leu Gly Cys Ser Pro Thr
50 55 60

Val Pro Val Val Phe Ile Gly Gly Gln Leu Val Gly Gly Ala Asn Gln
65 70 75 80

Val Met Ser Leu His Leu Asn Arg Ser Leu Ile Pro Met Leu Lys Arg
85 90 95

Phe Gly Ala Leu Trp Leu
100

<210> 325
<211> 500
<212> DNA
<213> Arabidopsis thaliana

<400> 325
gtcacttctc attttcaccc aaatatatca ataatctctc tataagttat ctagttttcc 60

catattcatc tctaatctag cattttgacc aaacacacct atttttctcc tttgcaagac 120

agttttcaaa tatctatcac gttatattac taatggagaa tctacaaaag atgatctccg 180

agaagtcggt agtgatcttt agcaagaact cttgctgcat gtctcacaca atcaagactc 240

tcttcttaga ccttggcgtg aacccgacga tctatgaact cgatgagatt agcagaggaa 300

aggagataga gcatgcattg gctcagctcg ggtgcagccc gacagtgcca gtggtgttca 360

taggagggca gcttgttggt ggagccaatc aagtcatgag tctccatctc aaccgctccc 420

ttgttccaat gcttaagcgc gctggagctt tatggctttg acttcaaaat aaatggaatt 480

gcaaatgact taggttctga 500

<210> 326
<211> 102
<212> PRT
<213> Arabidopsis thaliana

<400> 326

Met Glu Asn Leu Gln Lys Met Ile Ser Glu Lys Ser Val Val Ile Phe
1 5 10 15

Ser Lys Asn Ser Cys Cys Met Ser His Thr Ile Lys Thr Leu Phe Leu
20 25 30

Asp Leu Gly Val Asn Pro Thr Ile Tyr Glu Leu Asp Glu Ile Ser Arg
35 40 45

Gly Lys Glu Ile Glu His Ala Leu Ala Gln Leu Gly Cys Ser Pro Thr
50 55 60

Val Pro Val Val Phe Ile Gly Gly Gln Leu Val Gly Gly Ala Asn Gln
65 70 75 80

Val Met Ser Leu His Leu Asn Arg Ser Leu Val Pro Met Leu Lys Arg
85 90 95

Ala Gly Ala Leu Trp Leu
100

<210> 327
<211> 770
<212> DNA
<213> Arabidopsis thaliana

<400> 327
gattccttct tttgttttaa acttcttttt gattctttct atatatacaa atacaaaatc 60

tcctcttctt cttgaaaaag ctctttacgt ttctctctct ctctctaatt gcctgctatg 120

atgcaagaat taggcttaca acgtttctca aacgacgtcg ttcgcttaga cctcactcct 180

ccttctcaaa cctcatctac ttctctttcc atcgacgaag aggaatcaac ggaagccaag 240

atccgacggc tgatatcgga gcatcctgtg atcatcttca gtagatcttc atgttgcatg 300

tgccacgtca tgaagagact cttagcaacg atcggcgtaa tccccaccgt catcgagctc 360

gatgatcacg aggtttcctc tcttcccacg gctctacaag atgaatattc cggtggcgtc 420

tccgtcgttg gtcctccgcc ggcggttttc attggccgtg agtgcgtcgg aggtcttgag 480

tcccttgtcg ctcttcactt aagtggtcaa cttgttccta agcttgtcca agttggagct 540

ctttgggtat gattgtaatt ttcatcgtct tcttacttgt gttaaaatca ataggctttt 600

gcagtatcaa aagaaaacaa aaattagggt ttcacttcaa ttggtcatga aagccaaatt 660

ctgattatca tcagatgatc ataattaaac acccatgtag aaaatgaatt atgaataatt 720

aaagatgtgt aattagtaaa ttttatatga tttgcttctt ttgataaagt 770

<210> 328
<211> 144
<212> PRT
<213> Arabidopsis thaliana

<400> 328

Met Met Gln Glu Leu Gly Leu Gln Arg Phe Ser Asn Asp Val Val Arg
1 5 10 15

Leu Asp Leu Thr Pro Pro Ser Gln Thr Ser Ser Thr Ser Leu Ser Ile
20 25 30

Asp Glu Glu Glu Ser Thr Glu Ala Lys Ile Arg Arg Leu Ile Ser Glu
35 40 45

His Pro Val Ile Ile Phe Ser Arg Ser Ser Cys Cys Met Cys His Val
50 55 60

Met Lys Arg Leu Leu Ala Thr Ile Gly Val Ile Pro Thr Val Ile Glu
65 70 75 80

Leu Asp Asp His Glu Val Ser Ser Leu Pro Thr Ala Leu Gln Asp Glu
85 90 95

Tyr Ser Gly Gly Val Ser Val Val Gly Pro Pro Pro Ala Val Phe Ile
100 105 110

Gly Arg Glu Cys Val Gly Gly Leu Glu Ser Leu Val Ala Leu His Leu
115 120 125

Ser Gly Gln Leu Val Pro Lys Leu Val Gln Val Gly Ala Leu Trp Val
130 135 140

<210> 329
<211> 706
<212> DNA
<213> Arabidopsis thaliana

<400> 329
ctctctctcc ctctctaagt actctttctc tctaatgaag acgatgcgag gtttacgaaa 60

ctgctcaaac gacgccgtaa cgctagacct gacggttcat cctcctcctc ctcctcctct 120

tcctcctcca gcaccatcaa cagtctcctc ctccaccgcc tcgacgagcc tgtcgttcga 180

tgaagaggaa acatcagagt caaagatcgg acggctgata tcagagcatc cagtcatcat 240

attcactcga ttttcctcat gttgcatgtg ccacgtcatg aagaagcttc tatcgaccgt 300

tggagttcac ccaacagtga tcgagatcga cgacggagaa attgcttacc tcgccgttga 360

agccgctccg gtgcttttca tcggtggtac ttgcgtcggt ggcttcgagt cacttgtagc 420

acttcaccta agtggtcagc ttattcctag actcgtcgag gttggagcct tatgggcata 480

attgtaattt tctgtatttt tctttctttc tttcaagtct agcctattta taaccttaag 540

aaattctgat aataaaatcc attgtaaaat tttccattaa tccactcttt ctttctgaaa 600

cacaaaaatg tttttttttc tttatctttt gccagtcggt aagtattgtt tagatcatca 660

agtgtaagat ttgttccatc ataattatta caatttttgt gaatga 706

<210> 330
<211> 145
<212> PRT
<213> Arabidopsis thaliana

<400> 330

Met Arg Gly Leu Arg Asn Cys Ser Asn Asp Ala Val Thr Leu Asp Leu
1 5 10 15

Thr Val His Pro Pro Pro Pro Pro Pro Leu Pro Pro Pro Ala Pro Ser
20 25 30

Thr Val Ser Ser Ser Thr Ala Ser Thr Ser Leu Ser Phe Asp Glu Glu
35 40 45

Glu Thr Ser Glu Ser Lys Ile Gly Arg Leu Ile Ser Glu His Pro Val
50 55 60

Ile Ile Phe Thr Arg Phe Ser Ser Cys Cys Met Cys His Val Met Lys
65 70 75 80

Lys Leu Leu Ser Thr Val Gly Val His Pro Thr Val Ile Glu Ile Asp
85 90 95

Asp Gly Glu Ile Ala Tyr Leu Ala Val Glu Ala Ala Pro Val Leu Phe
100 105 110

Ile Gly Gly Thr Cys Val Gly Gly Phe Glu Ser Leu Val Ala Leu His
115 120 125

Leu Ser Gly Gln Leu Ile Pro Arg Leu Val Glu Val Gly Ala Leu Trp
130 135 140

Ala
145

<210> 331
<211> 679
<212> DNA
<213> Arabidopsis thaliana

<400> 331
caaccaactc tcacacaaat tctctcgctc tctcactctc ttattctctt tctctttttc 60

tctaagaata caaaaaaaga tgcaatacaa aacagaaact cgagggtcgt tgtcctacaa 120

caacaacagt aaggtgatga acaacatgaa tgtgtttccg tcggagacac tggcgaagat 180

agagtcgatg gcggcagaga atgcggtggt tatattcagc gtgagcactt gctgcatgtg 240

ccatgccatc aagcgtctct tccgtggaat gggcgtcagc cccgccgtcc acgagctcga 300

cctcctccct tacggagttg aaatccaccg agctctcctc cgtctccttg gctgttccag 360

cggtggcgcc acatctccgg gggcacttcc ggtggtgttc atcggaggga agatggtagg 420

agcaatggag agagtgatgg cttcacatat caatggctca ctcgtccctc ttctcaaaga 480

tgctggcgct ctttggctct gatgagtgct aatctcatcc tccaaatatc aacctttggt 540

ttatctttgg tttttaagga cagaagaaat aggttaatcc cagtgttgaa ttagagacag 600

tgagagagaa gagtgatgcg tttgttttaa gcttagctct ttgtctatct taaatcacac 660

taatatataa atgttaact 679

<210> 332
<211> 140
<212> PRT
<213> Arabidopsis thaliana

<400> 332

Met Gln Tyr Lys Thr Glu Thr Arg Gly Ser Leu Ser Tyr Asn Asn Asn
1 5 10 15

Ser Lys Val Met Asn Asn Met Asn Val Phe Pro Ser Glu Thr Leu Ala
20 25 30

Lys Ile Glu Ser Met Ala Ala Glu Asn Ala Val Val Ile Phe Ser Val
35 40 45

Ser Thr Cys Cys Met Cys His Ala Ile Lys Arg Leu Phe Arg Gly Met
50 55 60

Gly Val Ser Pro Ala Val His Glu Leu Asp Leu Leu Pro Tyr Gly Val
65 70 75 80

Glu Ile His Arg Ala Leu Leu Arg Leu Leu Gly Cys Ser Ser Gly Gly
85 90 95

Ala Thr Ser Pro Gly Ala Leu Pro Val Val Phe Ile Gly Gly Lys Met
100 105 110

Val Gly Ala Met Glu Arg Val Met Ala Ser His Ile Asn Gly Ser Leu

115 120 125

Val Pro Leu Leu Lys Asp Ala Gly Ala Leu Trp Leu
130 135 140

<210> 333
<211> 505
<212> DNA
<213> Brassica napus

<400> 333
ggcaagcaaa aaccaaactc caactcccgc tttggtttca ggaacctcct tcattttctt 60

gaagacagct aaaaccaaga agaaaatgga caaggttctg agaatgtcat cggaaaaagg 120

agtggttata ttcagcaaga gctcgtgttg cttgtcctac gcggttcaag tcctcttcca 180

agatcttggg gttagcccta agatccacga gatcgacaag gaccccgaat gccgagagat 240

ggagaaggca ctgatgaagc taggctgctc aaagccagtt ccagccgtct tcattggtgg 300

taagctcgtt ggttccacca acgaagtcat gtccatgcac ctaagcagct ccttggttcc 360

cttagtgaag ccatatctat gttaaaagaa aaggtcggaa tgtatctcaa taaggaaaca 420

aatgtgagcc aaatcttcgt aatgtgtttt agtaattata ttggctgtgt aaccttaaaa 480

gttatataaa atgtcttttc gtcca 505

<210> 334
<211> 99
<212> PRT
<213> Brassica napus

<400> 334

Met Asp Lys Val Leu Arg Met Ser Ser Glu Lys Gly Val Val Ile Phe
1 5 10 15

Ser Lys Ser Ser Cys Cys Leu Ser Tyr Ala Val Gln Val Leu Phe Gln
20 25 30

Asp Leu Gly Val Ser Pro Lys Ile His Glu Ile Asp Lys Asp Pro Glu
35 40 45

Cys Arg Glu Met Glu Lys Ala Leu Met Lys Leu Gly Cys Ser Lys Pro
50 55 60

Val Pro Ala Val Phe Ile Gly Gly Lys Leu Val Gly Ser Thr Asn Glu
65 70 75 80

Val Met Ser Met His Leu Ser Ser Ser Leu Val Pro Leu Val Lys Pro
85 90 95

Tyr Leu Cys

<210> 335
<211> 603
<212> DNA
<213> Brassica napus

<400> 335
cggcacgagg caacataata tctcgaccgt tgggagccgc aagatcagaa actgatcatg 60

gacaaggtta tgagaatgtc atcagggaaa ggagttgtga tcttcaccaa aaactcatgt 120

tgtctgtgct acgccgtgca gatacttttt cgtgacctta gggttcaacc aacaatccac 180

gagattgaca acgatcctga ctgcctcgag atcgagaagg ccttagtccg tcttggctgc 240

cccaacgcag ttcctgctgt ttttgtaagt ggtaagctgg tgggttctac caatgaagtc 300

atgtcgcttc acctaagtgg ctctctcgtt cccttgatca agccgtatca gttatttcat 360

aactagaaat aaatggatct ttaaggaaaa gaaagataat tgttgtatgt tgagattgga 420

tagtaaataa tgatggaaag attacacttg aatgtgtatc atgttatata tatagctgat 480

tttatatttt gtttcgctca tgtccaagaa attaatttgc tatctttgta ttttccagct 540

taactaatca gtagatgtac tgctgtatta tctaatatct atagtaatga agaaaattat 600

act 603

<210> 336
<211> 102
<212> PRT
<213> Brassica napus

<400> 336

Met Asp Lys Val Met Arg Met Ser Ser Gly Lys Gly Val Val Ile Phe
1 5 10 15

Thr Lys Asn Ser Cys Cys Leu Cys Tyr Ala Val Gln Ile Leu Phe Arg
20 25 30

Asp Leu Arg Val Gln Pro Thr Ile His Glu Ile Asp Asn Asp Pro Asp
35 40 45

Cys Leu Glu Ile Glu Lys Ala Leu Val Arg Leu Gly Cys Pro Asn Ala
50 55 60

Val Pro Ala Val Phe Val Ser Gly Lys Leu Val Gly Ser Thr Asn Glu
65 70 75 80

Val Met Ser Leu His Leu Ser Gly Ser Leu Val Pro Leu Ile Lys Pro
85 90 95

Tyr Gln Leu Phe His Asn
100

<210> 337
<211> 833
<212> DNA
<213> Brassica napus

<400> 337
cggcacgagg gtttcttttc tctctctgat tgcaactatg atgcaagaat tgggcttaga 60
acgtttctcc aacgatgtct ttcccttaga cctcactcct ccttctcaaa cctcatctac 120
ttctctttcc atcgacgaag aggaatcttc ggaggccaag atccggcggc tgatatcgga 180
gcatccagtg atcatcttca gcagatcttc ttgttgcatg tgccacgtca tgaaaagcct 240
cctttcaaca atcggagtcg tccccaccgt catcgagctt gatgaccacg aggtttcctc 300
tctccccatg gctcttgaag aagaatattc cggcggccgc tccgtcgttg ttcctccgcc 360
ggcggttttc attggccgtg agtatgtcgg tggtcttgag tcccttgttg ctcttcatct 420
aagtagtcac ttggtcccta agcttgtcca agttggagct ctttggttat gacttgcttt 480
ttaatagtat caaaaagcag aaacttaggg tttttttctg attattatcc gatgatcaga 540
accaaacacc catgtataaa atgaattatg agaaataata attaaagatg tgtaagtaaa 600
aaaaaaaaaa aaaaaactcg agcgtcgagg aggataaaga cctaaaagga gaaatggtgc 660
agcgccttgt ataccgttcg cgtcacagct acgccaccaa gtccaaccag cacaggatcg 720
tcaaaacccc aggtggtaaa ttgacatacc agaccactaa gaagcgtgca agtggaccaa 780
aatgccccgt taccggcaag cgtatccagg ggatccctca cttgaggcct gct 833

<210> 338
<211> 144
<212> PRT
<213> Brassica napus

<400> 338

Met Met Gln Glu Leu Gly Leu Glu Arg Phe Ser Asn Asp Val Phe Pro
1 5 10 15

Leu Asp Leu Thr Pro Pro Ser Gln Thr Ser Ser Thr Ser Leu Ser Ile
20 25 30

Asp Glu Glu Glu Ser Ser Glu Ala Lys Ile Arg Arg Leu Ile Ser Glu
35 40 45

His Pro Val Ile Ile Phe Ser Arg Ser Ser Cys Cys Met Cys His Val
50 55 60

Met Lys Ser Leu Leu Ser Thr Ile Gly Val Val Pro Thr Val Ile Glu
65 70 75 80

Leu Asp Asp His Glu Val Ser Ser Leu Pro Met Ala Leu Glu Glu Glu
85 90 95

Tyr Ser Gly Gly Arg Ser Val Val Val Pro Pro Pro Ala Val Phe Ile
100 105 110

Gly Arg Glu Tyr Val Gly Gly Leu Glu Ser Leu Val Ala Leu His Leu
115 120 125

Ser Ser His Leu Val Pro Lys Leu Val Gln Val Gly Ala Leu Trp Leu
130 135 140

<210> 339
<211> 665
<212> DNA
<213> Brassica napus

<400> 339
actttctctc ctctctctcc tcccttctct ctcatacaac aattatgcaa aaagcagtaa 60

gaccctacga gtcatcgtgg acgaagacca taccggggaa tagcattttc cgtccagaga 120

atgaagataa accatcatca tccttatcat ggttaacatc atcaccacaa aaaccatcat 180

ctttaagcat caagaaacca aacaacgtat tggtgatgga gaatgctgcg gtagtgtttg 240

ccaggaaagg ttgttgtatg ggacatgtag ccaaacggtt gttactgaca catggagtga 300

acccattggt agttgagatt gatgaaggag acaacaacgg tgacaatatc atcatgagtg 360

agctgggtaa taacgtgatt agtaaagaga aattaccagt catgttcatt ggagggaagt 420

tgtttggagg attagagaat ctgatggctg ctcatattaa tggtgattta ggacctactc 480

tcagacgagc tggggcttta tggctttgat tcatcattgc aattctcata taaaagttta 540

tttagttgcc ttttgatttt ttttttctc tttgttgcat ttgcttgttg atattgtatg 600

caatttttta aacctatgtg aatttgtgat tggttgttat gtgattggtt tgatcataaa 660

caaca 665

<210> 340
<211> 154
<212> PRT
<213> Brassica napus

<400> 340

Met Gln Lys Ala Val Arg Pro Tyr Glu Ser Ser Trp Thr Lys Thr Ile
1 5 10 15

Pro Gly Asn Ser Ile Phe Arg Pro Glu Asn Glu Asp Lys Pro Ser Ser

20 25 30

Ser Leu Ser Trp Leu Thr Ser Ser Pro Gln Lys Pro Ser Ser Leu Ser
35 40 45

Ile Lys Lys Pro Asn Asn Val Leu Val Met Glu Asn Ala Ala Val Val
50 55 60

Phe Ala Arg Lys Gly Cys Cys Met Gly His Val Ala Lys Arg Leu Leu
65 70 75 80

Leu Thr His Gly Val Asn Pro Leu Val Val Glu Ile Asp Glu Gly Asp
85 90 95

Asn Asn Gly Asp Asn Ile Ile Met Ser Glu Leu Gly Asn Asn Val Ile
100 105 110

Ser Lys Glu Lys Leu Pro Val Met Phe Ile Gly Gly Lys Leu Phe Gly
115 120 125

Gly Leu Glu Asn Leu Met Ala Ala His Ile Asn Gly Asp Leu Gly Pro
130 135 140

Thr Leu Arg Arg Ala Gly Ala Leu Trp Leu
145 150

<210> 341
<211> 666
<212> DNA
<213> Brassica napus

<400> 341
gaacaaacct ctctacgttt ctctctctct ctaattgttg caatgatgca agaattaggc 60

ttagaacgtt tctccaacga cgtcgtttcc ttagacctca ctcttccttc tcaaacctca 120

tccacctctc tctccatcga cgaagaggaa tcatctgagg ccaagatccg acggctcata 180

accgagcatc ccgtgatcat attcagcaga tcttcttgtt gcatgtgcca cgtcatgaaa 240

agactcttgg caacgatcgg tgtcatcccc accgtcatcg agctcgatga tcacgaggtt 300

tcctctctcc ccttggctct tggagaagaa tattccggcg gtggctccgg cgttgttcct 360

cctccggcgg ttttcattgg ccgtgagtgt gtaggaggtc tcgagtccct cgtggcgctc 420

catctaagtg gtcaccttgt ccctaagctt gtccaagttg gagctctttg ggtatgatat 480

tgtaatttta cttctttgag tttcaatagt attttgcagt atcaaaagca taaatttagg 540

gtttctcttt tctggttatt atctgatgat cataattaaa cacccatgta ctatatatga 600

ataataataa taattaaaga atgtgtaagt agtaaatttt atataatttg cttcctctcg 660

gaggag 666

<210> 342
<211> 144
<212> PRT
<213> Brassica napus

<400> 342

Met Met Gln Glu Leu Gly Leu Glu Arg Phe Ser Asn Asp Val Val Ser
1 5 10 15

Leu Asp Leu Thr Leu Pro Ser Gln Thr Ser Ser Thr Ser Leu Ser Ile
20 25 30

Asp Glu Glu Glu Ser Ser Glu Ala Lys Ile Arg Arg Leu Ile Thr Glu
35 40 45

His Pro Val Ile Ile Phe Ser Arg Ser Ser Cys Cys Met Cys His Val
50 55 60

Met Lys Arg Leu Leu Ala Thr Ile Gly Val Ile Pro Thr Val Ile Glu
65 70 75 80

Leu Asp Asp His Glu Val Ser Ser Leu Pro Leu Ala Leu Gly Glu Glu
85 90 95

Tyr Ser Gly Gly Gly Ser Gly Val Val Pro Pro Pro Ala Val Phe Ile
100 105 110

Gly Arg Glu Cys Val Gly Gly Leu Glu Ser Leu Val Ala Leu His Leu
115 120 125

Ser Gly His Leu Val Pro Lys Leu Val Gln Val Gly Ala Leu Trp Val
130 135 140

<210> 343
<211> 596
<212> DNA
<213> Brassica napus

<400> 343
tttctctctc tctaaatgaa gacgatgcaa ggtttacgga acttcacaaa cgacaatgtt 60

tcgctagacc tgacgtttcc tcccccagca ccaccaccca tctcctcctc caccgcctcc 120

acgagcctat ccttcgatga ggaggagacg tcagcgtcga agatcgaacg gctgatatct 180

gagcacccgg tcatcatatt cactagatca tccacctgct gcatgtgtca cgtcatgaag 240

aagcttctat caaccgttgg agtccaccca acagtgatcg agatcgacga ggaagagatc 300

gcttgcctcg ccgttcaagc cgctccggtg ctcttcatag gtggtgcttg tgtcggtggg 360

tttgagtctc ttgtggcact tcaccttagt ggtcatctta ttcctagact cgtcgaggtt 420

ggagccttgt gggaataatt gtgtatgttt aacttataac tatttaagaa aatctggtaa 480

taatatccat tgtaaatctc atgatctact actattttct gttcttgatt ctgaaacata 540

aaaatgtatt tttcattttc ccttgtgtac tttttttaa tatctttcac cacttg 596

<210> 344
<211> 140
<212> PRT
<213> Brassica napus

<400> 344

Met Lys Thr Met Gln Gly Leu Arg Asn Phe Thr Asn Asp Asn Val Ser
1               5                   10                  15

Leu Asp Leu Thr Phe Pro Pro Pro Ala Pro Pro Pro Ile Ser Ser Ser
            20                  25                  30

Thr Ala Ser Thr Ser Leu Ser Phe Asp Glu Glu Glu Thr Ser Ala Ser
        35                  40                  45

Lys Ile Glu Arg Leu Ile Ser Glu His Pro Val Ile Ile Phe Thr Arg
    50                  55                  60

Ser Ser Thr Cys Cys Met Cys His Val Met Lys Lys Leu Leu Ser Thr
65                  70                  75                  80

Val Gly Val His Pro Thr Val Ile Glu Ile Asp Glu Glu Glu Ile Ala
                85                  90                  95

Cys Leu Ala Val Gln Ala Ala Pro Val Leu Phe Ile Gly Gly Ala Cys
            100                 105                 110

Val Gly Gly Phe Glu Ser Leu Val Ala Leu His Leu Ser Gly His Leu
        115                 120                 125

Ile Pro Arg Leu Val Glu Val Gly Ala Leu Trp Glu
    130                 135                 140

<210> 345
<211> 808
<212> DNA
<213> Medicago truncatula

<400> 345
ggacaacaca atccaactat atcacaaaga aaaaaccaca cgttccttct tctctcatca 60

ctcaacaaac aaccatgcaa caagcaattc cttataggtc atggacacac aacacttcca 120

```
ccactcactt caatgttatc aaaccacaca tattaactac aactaaaatc cacaatacaa    180
ttgatgagtc ttctcatagg ccctcctcct ttaattttaa tgaagaggac aaaacaatgt    240
ttcataacat ggtatcagag aacgcagtta tagtctttgc tagacgtgga tgttgtatga    300
gccatgtcgt gaagcgcttg cttctcggtc ttggtgttaa tcctgctgta catgaggttg    360
aggagaaaga tgaagttggt ttggttaaag aattggaatc aattgcaaat gaagagaagg    420
ttcaatttcc agcagtgttt ataggtggaa atttgtttgg aggactggat cgaattatgg    480
ccactcatat ttctggtgaa ttggtcccca ttcttaaaca agcaggagct ttatggcttt    540
gactcattaa tatcatattt ttttccacta aatttttcat ttccggatat attgattcca    600
tcctttggaa tttgtagaga aaaatatcag gagtgttttt caaaaattat tcatgtcttt    660
attggatgca aatttttggc tcgactatgt caagttgtta agagtccaac actgaaaaaa    720
atatgatttc cgtaaattta taaatgagag atatcatcac catagtaact gattttgtaa    780
ggtcgtatta gactcgattt aaatctaa                                      808
```

```
<210>  346
<211>  155
<212>  PRT
<213>  Medicago truncatula

<400>  346

Met Gln Gln Ala Ile Pro Tyr Arg Ser Trp Thr His Asn Thr Ser Thr
1               5                   10                  15

Thr His Phe Asn Val Ile Lys Pro His Ile Leu Thr Thr Thr Lys Ile
            20                  25                  30

His Asn Thr Ile Asp Glu Ser Ser His Arg Pro Ser Ser Phe Asn Phe
        35                  40                  45

Asn Glu Glu Asp Lys Thr Met Phe His Asn Met Val Ser Glu Asn Ala
    50                  55                  60

Val Ile Val Phe Ala Arg Arg Gly Cys Cys Met Ser His Val Val Lys
65                  70                  75                  80

Arg Leu Leu Leu Gly Leu Gly Val Asn Pro Ala Val His Glu Val Glu
                85                  90                  95

Glu Lys Asp Glu Val Gly Leu Val Lys Glu Leu Glu Ser Ile Ala Asn
            100                 105                 110

Glu Glu Lys Val Gln Phe Pro Ala Val Phe Ile Gly Gly Asn Leu Phe
        115                 120                 125
```

Gly Gly Leu Asp Arg Ile Met Ala Thr His Ile Ser Gly Glu Leu Val
130 135 140

Pro Ile Leu Lys Gln Ala Gly Ala Leu Trp Leu
145 150 155

<210> 347
<211> 378
<212> DNA
<213> Oryza sativa

<400> 347
atgcaggcgg tggcggcggc ggcggggatg atgcggcggg ggagcctgac gatagacccg 60

gcgggggagg aggaggcgcc ggccgagagg gtggggcggc tggtgcggga gagccccgtg 120

gtggtgttcg cgcggcgggg gtgctacatg gcgcacgtca tgaggcgcct cctcgccgcc 180

gtcggcgcgc acgccaccgt catcgagctg gagggcggcg cggcggagga ggaggaggcg 240

gcgctgggcg gcggcgccgc gctccccgcg ctcttcgtcg gcggcgaccc cgtcggcggc 300

ctcgagggcc tcatgggcct ccacctcagc ggccgcctcg tcccgcgcct cagagaggtc 360

ggcgccctct gcacctag 378

<210> 348
<211> 125
<212> PRT
<213> Oryza sativa

<400> 348

Met Gln Ala Val Ala Ala Ala Ala Gly Met Met Arg Arg Gly Ser Leu
1 5 10 15

Thr Ile Asp Pro Ala Gly Glu Glu Glu Ala Pro Ala Glu Arg Val Gly
20 25 30

Arg Leu Val Arg Glu Ser Pro Val Val Val Phe Ala Arg Arg Gly Cys
35 40 45

Tyr Met Ala His Val Met Arg Arg Leu Leu Ala Ala Val Gly Ala His
50 55 60

Ala Thr Val Ile Glu Leu Glu Gly Gly Ala Ala Glu Glu Glu Glu Ala
65 70 75 80

Ala Leu Gly Gly Gly Ala Ala Leu Pro Ala Leu Phe Val Gly Gly Asp
85 90 95

Pro Val Gly Gly Leu Glu Gly Leu Met Gly Leu His Leu Ser Gly Arg
100 105 110

Leu Val Pro Arg Leu Arg Glu Val Gly Ala Leu Cys Thr
115 120 125

<210> 349
<211> 411
<212> DNA
<213> Oryza sativa

<400> 349
atgcaaggag caaggtcggc ggcggcgatg gcggcggcgg cggccgacga ggagagggag 60

gtgcggaggg cggtggagga gaagccggtt gtggtggtgg ggcggcgcgg gtgctgcatg 120

gcgcacgtcg cgcggcgcct gctgctgggg cagggcgcga acccggcggt gctcgaggtc 180

ggcgacgacg ccgacccggc ggcgctcgtc gacgccgcgc tgcaggcccg ccggcgcaag 240

gacggcggcg acaaggctgc ggcgggcgac ggaggcggcg gagcggcggt ggcattcccg 300

gcggtgttca tcggcgggag gctggtgggc gggctcgatc ggctcatggc catgcacatg 360

gccggcgagc tcgtgccggt cttgaagcag gcaggagccc tgtggctctg a 411

<210> 350
<211> 136
<212> PRT
<213> Oryza sativa

<400> 350

Met Gln Gly Ala Arg Ser Ala Ala Ala Met Ala Ala Ala Ala Ala Asp
1 5 10 15

Glu Glu Arg Glu Val Arg Arg Ala Val Glu Glu Lys Pro Val Val Val
20 25 30

Val Gly Arg Arg Gly Cys Cys Met Ala His Val Ala Arg Arg Leu Leu
35 40 45

Leu Gly Gln Gly Ala Asn Pro Ala Val Leu Glu Val Gly Asp Asp Ala
50 55 60

Asp Pro Ala Ala Leu Val Asp Ala Ala Leu Gln Ala Arg Arg Arg Lys
65 70 75 80

Asp Gly Gly Asp Lys Ala Ala Ala Gly Asp Gly Gly Gly Gly Ala Ala
85 90 95

Val Ala Phe Pro Ala Val Phe Ile Gly Gly Arg Leu Val Gly Gly Leu
100 105 110

Asp Arg Leu Met Ala Met His Met Ala Gly Glu Leu Val Pro Val Leu

115 120 125

Lys Gln Ala Gly Ala Leu Trp Leu
130 135

<210> 351
<211> 312
<212> DNA
<213> Oryza sativa

<400> 351
atggacaggg tgaacaggct ggcggcgcag cgggcggtgg tgatcttcag catgagctcg 60

tgctgcatgt gccacaccgt gacgcgcctc ttctgcgagc tcggggtgaa cccgacggtg 120

gtggagctgg acgaggaccc gagggggaag gagatggaga aggcgctggc gaggctcctc 180

ggccgcagcc ccgccgtgcc ggcggtgttc atcggcggga ggctcgtcgg ctccaccgac 240

aaggtcatgt cgctgcacct cagcggcaac cttgtcccgc tgcttcgcaa tgcgggtgcc 300

ctctgggtgt ag 312

<210> 352
<211> 103
<212> PRT
<213> Oryza sativa

<400> 352

Met Asp Arg Val Asn Arg Leu Ala Ala Gln Arg Ala Val Val Ile Phe
1 5 10 15

Ser Met Ser Ser Cys Cys Met Cys His Thr Val Thr Arg Leu Phe Cys
20 25 30

Glu Leu Gly Val Asn Pro Thr Val Val Glu Leu Asp Glu Asp Pro Arg
35 40 45

Gly Lys Glu Met Glu Lys Ala Leu Ala Arg Leu Leu Gly Arg Ser Pro
50 55 60

Ala Val Pro Ala Val Phe Ile Gly Gly Arg Leu Val Gly Ser Thr Asp
65 70 75 80

Lys Val Met Ser Leu His Leu Ser Gly Asn Leu Val Pro Leu Leu Arg
85 90 95

Asn Ala Gly Ala Leu Trp Val
100

<210> 353
<211> 444

<212> DNA
<213> Oryza sativa

<400> 353
atgtaccagg cgatcccgta cagcagcacc cggccgtggc tcaggccgga gccggcggcg 60

agcgtggtcg acgtcgtgaa ggtggagacg acgacggccg tcgcgggtcg gggcggtgag 120

gcggaggtcg tgggggagga ggaggcggcg gaggtgcgga gggcggtggc ggagagcccg 180

gtgctggtgg tggggaggcg cgggtgctgc ctcatccacg tggtgaagcg gctgctgcag 240

gggctcgggg tcaacccggc cgtgcacgag gtcgccggcg aggccgcgct caagggggtt 300

gtgccggccg gtggggaggc cgcggcgctc cccgccgtgt tcgtcggggg gaagctcctc 360

ggcgggctcg accgcctcat ggccgtccac atctccggcg agctcgtgcc catcctcaag 420

aaggccggtg ccctctggct ttaa 444

<210> 354
<211> 147
<212> PRT
<213> Oryza sativa

<400> 354

Met Tyr Gln Ala Ile Pro Tyr Ser Ser Thr Arg Pro Trp Leu Arg Pro
1 5 10 15

Glu Pro Ala Ala Ser Val Val Asp Val Val Lys Val Glu Thr Thr Thr
20 25 30

Ala Val Ala Gly Arg Gly Gly Glu Ala Glu Val Val Gly Glu Glu Glu
35 40 45

Ala Ala Glu Val Arg Arg Ala Val Ala Glu Ser Pro Val Leu Val Val
50 55 60

Gly Arg Arg Gly Cys Cys Leu Ile His Val Val Lys Arg Leu Leu Gln
65 70 75 80

Gly Leu Gly Val Asn Pro Ala Val His Glu Val Ala Gly Glu Ala Ala
85 90 95

Leu Lys Gly Val Val Pro Ala Gly Gly Glu Ala Ala Ala Leu Pro Ala
100 105 110

Val Phe Val Gly Gly Lys Leu Leu Gly Gly Leu Asp Arg Leu Met Ala
115 120 125

Val His Ile Ser Gly Glu Leu Val Pro Ile Leu Lys Lys Ala Gly Ala
130 135 140

Leu Trp Leu
145

<210> 355
<211> 378
<212> DNA
<213> Oryza sativa

<400> 355
atggcggaga gggtggcgcg gctgtcgtcg cagagggcgg tggtgatctt cggggcgagc 60

aactgcttca tgtgccacgt ggtgaagacg ctcttctcgg agctcggggt gagctgggcg 120

gtgcacgagg tggacaagga ccccaacggc aaggacgtcg agagggcgct cgccggaatg 180

gtcggccgga cgccgccggt gccggccgtc ttcatcggcg gcaagctcgt cgggcccacc 240

gaccaggtca tgtcgctcca cctcgccggc aagctcgtcc cgctcctccg cgaagccggc 300

gccctctggc tcagagatac gaagtactcc tatatactac cagctaatca attaattaac 360

tatcgatcaa ttaattaa 378

<210> 356
<211> 125
<212> PRT
<213> Oryza sativa

<400> 356

Met Ala Glu Arg Val Ala Arg Leu Ser Ser Gln Arg Ala Val Val Ile
1 5 10 15

Phe Gly Ala Ser Asn Cys Phe Met Cys His Val Val Lys Thr Leu Phe
20 25 30

Ser Glu Leu Gly Val Ser Trp Ala Val His Glu Val Asp Lys Asp Pro
35 40 45

Asn Gly Lys Asp Val Glu Arg Ala Leu Ala Gly Met Val Gly Arg Thr
50 55 60

Pro Pro Val Pro Ala Val Phe Ile Gly Gly Lys Leu Val Gly Pro Thr
65 70 75 80

Asp Gln Val Met Ser Leu His Leu Ala Gly Lys Leu Val Pro Leu Leu
85 90 95

Arg Glu Ala Gly Ala Leu Trp Leu Arg Asp Thr Lys Tyr Ser Tyr Ile
100 105 110

Leu Pro Ala Asn Gln Leu Ile Asn Tyr Arg Ser Ile Asn
115 120 125

<210> 357
<211> 408
<212> DNA
<213> Oryza sativa

<400> 357
atgcagtacg gagcggcggc cgagcaggcg tggtacatgc cggcggcggc gccggcaccg 60

atggtggaga gcgcggtggc gcgggtggag cggctggcgt cggagagcgc ggtggtggtg 120

ttcagcgtga gcagctgctg catgtgccac gccgtgaagc gcctcttctg cggcatgggg 180

gtgcacccga cggtgcacga gctggacctc gacccgcgcg gccgcgagct ggagcgcgcc 240

ctggcgcgcc tcgtcgggta cggcggcccc gccgccgcgt cgccgcccgt cgtccccgtc 300

gtcttcatcg gcggcaagct cgtcggcgcc atggaccgcg tcatggccgc gcacatcaac 360

ggctccctcg tccccctcct caaggaggcc ggcgcgctct ggctctag 408

<210> 358
<211> 135
<212> PRT
<213> Oryza sativa

<400> 358

Met Gln Tyr Gly Ala Ala Ala Glu Gln Ala Trp Tyr Met Pro Ala Ala
1 5 10 15

Ala Pro Ala Pro Met Val Glu Ser Ala Val Ala Arg Val Glu Arg Leu
20 25 30

Ala Ser Glu Ser Ala Val Val Val Phe Ser Val Ser Ser Cys Cys Met
35 40 45

Cys His Ala Val Lys Arg Leu Phe Cys Gly Met Gly Val His Pro Thr
50 55 60

Val His Glu Leu Asp Leu Asp Pro Arg Gly Arg Glu Leu Glu Arg Ala
65 70 75 80

Leu Ala Arg Leu Val Gly Tyr Gly Gly Pro Ala Ala Ala Ser Pro Pro
85 90 95

Val Val Pro Val Val Phe Ile Gly Gly Lys Leu Val Gly Ala Met Asp
100 105 110

Arg Val Met Ala Ala His Ile Asn Gly Ser Leu Val Pro Leu Leu Lys
115 120 125

Glu Ala Gly Ala Leu Trp Leu

130 135

<210> 359
<211> 408
<212> DNA
<213> Oryza sativa

<400> 359
atgcagtacg gcgcggcggc ggcggagcag gcgtggtaca tgccggcggc ggcgatggtg 60

gtggcagcgg cggcggagac ggcggcggag cgggtggaga ggctggcgtc ggagagcgcg 120

gtggtggtgt tcagcgtgag cagctgctgc atgtgccacg ccgtgaagcg cctcttctgc 180

ggcatgggcg tgcacccggc ggtgcacgag ctggacctcg acccgcgcgg ccgcgacctg 240

gagcgcgccc tggcgcgcct cgtcggcgcc ggcggcgccg ccgctgccgc cgtgcccgtc 300

gtgttcatcg gcggcaagct ggtcggcgcc atggaccgcg tcatggccgc gcacatcaac 360

ggctccctcg tgccgctgct caaggaggcc ggcgcgctct ggctttag 408

<210> 360
<211> 135
<212> PRT
<213> Oryza sativa

<400> 360

Met Gln Tyr Gly Ala Ala Ala Ala Glu Gln Ala Trp Tyr Met Pro Ala
1 5 10 15

Ala Ala Met Val Val Ala Ala Ala Ala Glu Thr Ala Ala Glu Arg Val
20 25 30

Glu Arg Leu Ala Ser Glu Ser Ala Val Val Val Phe Ser Val Ser Ser
35 40 45

Cys Cys Met Cys His Ala Val Lys Arg Leu Phe Cys Gly Met Gly Val
50 55 60

His Pro Ala Val His Glu Leu Asp Leu Asp Pro Arg Gly Arg Asp Leu
65 70 75 80

Glu Arg Ala Leu Ala Arg Leu Val Gly Ala Gly Gly Ala Ala Ala Ala
85 90 95

Ala Val Pro Val Val Phe Ile Gly Gly Lys Leu Val Gly Ala Met Asp
100 105 110

Arg Val Met Ala Ala His Ile Asn Gly Ser Leu Val Pro Leu Leu Lys
115 120 125

Glu Ala Gly Ala Leu Trp Leu
130 135

<210> 361
<211> 345
<212> DNA
<213> Oryza sativa

<400> 361
atggacaggg tgacaaggct ggcgtcgcag aaggcggtgg tggtgttcag caagagctcg 60

tgcggcatgt cccacgccgt gacgcgcctg ctccgggagc tcggcgtcga cgcccgcgtg 120

gtggagctcg acgaggagcc cgccggcgcc gacatggaga acgcgctcgc cgggatgttg 180

ctcgccggca ccgccgccaa cggcggtggc cgcggccgcg gcgtcgtggt gccgacagtg 240

ttcatcggcg gcaggctcgt cggctccacc gaccgggtca tgtcgctcca cgtcgccggc 300

ggccttgtcc cgctcctccg cgacgccggc gcgctctggg tgtag 345

<210> 362
<211> 114
<212> PRT
<213> Oryza sativa

<400> 362

Met Asp Arg Val Thr Arg Leu Ala Ser Gln Lys Ala Val Val Val Phe
1 5 10 15

Ser Lys Ser Ser Cys Gly Met Ser His Ala Val Thr Arg Leu Leu Arg
20 25 30

Glu Leu Gly Val Asp Ala Arg Val Val Glu Leu Asp Glu Glu Pro Ala
35 40 45

Gly Ala Asp Met Glu Asn Ala Leu Ala Gly Met Leu Leu Ala Gly Thr
50 55 60

Ala Ala Asn Gly Gly Gly Arg Gly Arg Gly Val Val Val Pro Thr Val
65 70 75 80

Phe Ile Gly Gly Arg Leu Val Gly Ser Thr Asp Arg Val Met Ser Leu
85 90 95

His Val Ala Gly Gly Leu Val Pro Leu Leu Arg Asp Ala Gly Ala Leu
100 105 110

Trp Val

<210> 363

<211> 417
<212> DNA
<213> Oryza sativa

<400> 363
atgcaaggag gaggcggcgt gagctgcgcg gtggccgggg acgcgccgtc gtcgacgagg 60

gggggaggcg gcggagggat gctggggctg acgctgttcg acccgccggg aggggagcag 120

ccggcggaga ggatcgggag gctggtgcgg gagagccccg tggtgatctt cgcgaggagg 180

gggtgctgca tgtgccacgt catgcgccgc ctcctggccg ccgtgggggc gcacgccacc 240

gtcatcgagc tcgacgaggc cgccgaggag gccgcggcgt ccgcggccgc cgccgccgcc 300

gtcccggcgc tcttcgtcgg cggcgcccca gtcggcggcc tcgacggcct catgggcctc 360

cacctcagcg gccgcctcgt cccccgcctc agggaggtcg gcgccctctg cggctag 417

<210> 364
<211> 138
<212> PRT
<213> Oryza sativa

<400> 364

Met Gln Gly Gly Gly Gly Val Ser Cys Ala Val Ala Gly Asp Ala Pro
1 5 10 15

Ser Ser Thr Arg Gly Gly Gly Gly Gly Gly Met Leu Gly Leu Thr Leu
20 25 30

Phe Asp Pro Pro Gly Gly Glu Gln Pro Ala Glu Arg Ile Gly Arg Leu
35 40 45

Val Arg Glu Ser Pro Val Val Ile Phe Ala Arg Arg Gly Cys Cys Met
50 55 60

Cys His Val Met Arg Arg Leu Leu Ala Ala Val Gly Ala His Ala Thr
65 70 75 80

Val Ile Glu Leu Asp Glu Ala Ala Glu Glu Ala Ala Ala Ser Ala Ala
85 90 95

Ala Ala Ala Ala Val Pro Ala Leu Phe Val Gly Gly Ala Pro Val Gly
100 105 110

Gly Leu Asp Gly Leu Met Gly Leu His Leu Ser Gly Arg Leu Val Pro
115 120 125

Arg Leu Arg Glu Val Gly Ala Leu Cys Gly
130 135

<210> 365
<211> 399
<212> DNA
<213> Oryza sativa

<400> 365
atgtaccagg cgatcccgta caacgcgaac cgggcttggc cggcggcgag ccggccggcg 60

acggcggcgg cggcgccgcc gccgccgccg ccgcgtggcg aggaggagga ggtgaggagg 120

gcggtggcgg agtgcccggt ggtggtggtg ggtcggagcg ggtgctgcct gagccacgtc 180

gtgaagcggc tgctgcaggg gctcggggtc aacccggcgg tgcacgaggt cgccggcgag 240

gccgagctcg ccggggtggt cgccggcggc ggcggcgtcg cgctgccggc ggtgttcgtc 300

ggcgggaggc tcctcggcgg gctcgaccgg ctcatggccg tgcacatctc cggcgagctc 360

gtgcccattc tgaaggaggc cggtgcactc tggctctga 399

<210> 366
<211> 132
<212> PRT
<213> Oryza sativa

<400> 366

Met Tyr Gln Ala Ile Pro Tyr Asn Ala Asn Arg Ala Trp Pro Ala Ala
1 5 10 15

Ser Arg Pro Ala Thr Ala Ala Ala Ala Pro Pro Pro Pro Pro Pro Arg
20 25 30

Gly Glu Glu Glu Glu Val Arg Arg Ala Val Ala Glu Cys Pro Val Val
35 40 45

Val Val Gly Arg Ser Gly Cys Cys Leu Ser His Val Val Lys Arg Leu
50 55 60

Leu Gln Gly Leu Gly Val Asn Pro Ala Val His Glu Val Ala Gly Glu
65 70 75 80

Ala Glu Leu Ala Gly Val Val Ala Gly Gly Gly Gly Val Ala Leu Pro
85 90 95

Ala Val Phe Val Gly Gly Arg Leu Leu Gly Gly Leu Asp Arg Leu Met
100 105 110

Ala Val His Ile Ser Gly Glu Leu Val Pro Ile Leu Lys Glu Ala Gly
115 120 125

Ala Leu Trp Leu
130

<210> 367
<211> 579
<212> DNA
<213> Oryza sativa

<400> 367
atgtcagagc gtgtgttcgc cgagctcgcg accatccact accaaaaaag ccttccatgt 60

cgccactcct ttgacccccc tcgcaccaca ccaattctcc atctatatat catccacctt 120

cttcttcctc ctctcattgc cattgtgtgt ttgtgttaca ttgcaatcgt gccatttgaa 180

gaagaggagg agaggatgag gatgcaggtg gtggagacgg cggcggtgga ggaggaggag 240

gcggcggcgg cgatgatgtc ggtgtacgag agggtggcga ggatggcgag cgggaacgcg 300

gtggtggtgt tcagcgcgag cgggtgctgc atgtgccacg tcgtcaagcg cctcctcctc 360

ggcctcggcg tcggccccgc cgtctacgag ctcgaccagc tcgccgccgc cgccgacatc 420

caggccgcgc tgtcgcagct cctcccgccg ggccagccgc cggtgcccgt cgtgttcgtc 480

ggcggcaggc tcctcggcgg cgtcgagaag gtgatggcgt gccacatcaa tggcaccctc 540

gtcccccctcc tcaagcaggc cggcgccctc tggctctga 579

<210> 368
<211> 192
<212> PRT
<213> Oryza sativa

<400> 368

Met Ser Glu Arg Val Phe Ala Glu Leu Ala Thr Ile His Tyr Gln Lys
1 5 10 15

Ser Leu Pro Cys Arg His Ser Phe Asp Pro Pro Arg Thr Thr Pro Ile
20 25 30

Leu His Leu Tyr Ile Ile His Leu Leu Leu Pro Pro Leu Ile Ala Ile
35 40 45

Val Cys Leu Cys Tyr Ile Ala Ile Val Pro Phe Glu Glu Glu Glu Glu
50 55 60

Arg Met Arg Met Gln Val Val Glu Thr Ala Ala Val Glu Glu Glu Glu
65 70 75 80

Ala Ala Ala Ala Met Met Ser Val Tyr Glu Arg Val Ala Arg Met Ala
85 90 95

Ser Gly Asn Ala Val Val Val Phe Ser Ala Ser Gly Cys Cys Met Cys
100 105 110

His Val Val Lys Arg Leu Leu Leu Gly Leu Gly Val Gly Pro Ala Val
115 120 125

Tyr Glu Leu Asp Gln Leu Ala Ala Ala Ala Asp Ile Gln Ala Ala Leu
130 135 140

Ser Gln Leu Leu Pro Pro Gly Gln Pro Pro Val Pro Val Val Phe Val
145 150 155 160

Gly Gly Arg Leu Leu Gly Gly Val Glu Lys Val Met Ala Cys His Ile
165 170 175

Asn Gly Thr Leu Val Pro Leu Leu Lys Gln Ala Gly Ala Leu Trp Leu
180 185 190

<210> 369
<211> 327
<212> DNA
<213> Oryza sativa

<400> 369
atggagaggg tggcgaagct ggcgtcggag agggcggtgg tggtgttcac ggcgagcaac 60

tgcggcatgt gccacgccgt gacgagcctc ctcgtcggcg agctgggcgt caacgccgcc 120

gtgcacgagc tcgacaagga cccccgcggc agggacatgg agagggagct cgccaggagg 180

ctcaacggcg gcggcggcgg cggccgcgcc ctgccggcgg tgttcgtcgg aggcaacctc 240

gtcggcggcg ccaaccgggt catgtcgctc cacctcgccg gcgagctcgt ccccatgctc 300

aagaacgccg gcgcgctctg gctctag 327

<210> 370
<211> 108
<212> PRT
<213> Oryza sativa

<400> 370

Met Glu Arg Val Ala Lys Leu Ala Ser Glu Arg Ala Val Val Val Phe
1 5 10 15

Thr Ala Ser Asn Cys Gly Met Cys His Ala Val Thr Ser Leu Leu Val
20 25 30

Gly Glu Leu Gly Val Asn Ala Ala Val His Glu Leu Asp Lys Asp Pro
35 40 45

Arg Gly Arg Asp Met Glu Arg Glu Leu Ala Arg Arg Leu Asn Gly Gly
50 55 60

Gly Gly Gly Gly Arg Ala Leu Pro Ala Val Phe Val Gly Gly Asn Leu

65 70 75 80

Val Gly Gly Ala Asn Arg Val Met Ser Leu His Leu Ala Gly Glu Leu
85 90 95

Val Pro Met Leu Lys Asn Ala Gly Ala Leu Trp Leu
100 105

<210> 371
<211> 330
<212> DNA
<213> Oryza sativa

<400> 371
atggcggaga tggtggcgag gctggcgtcg gagagggcgg tggtggtgtt caccaagagc 60

ggctgctgca tgtgcaccgc ggtgacgacg ctgctcggcg agctcgccgt cagcgccgcc 120

gtgcacgagc tcgacaggga cccgctcggg aaggagatgg agaaggagct cgccaggagg 180

ctctacggct ccagcggccg cggcgggccc gccgtgccgg cggtgttcat cggcgggagc 240

ctcgtcggcg gcaccagcaa ggtgatggcc atgcacctca agggcgagct cgtgcccttg 300

ctcaagagcg ccggtgcact gtggctttga 330

<210> 372
<211> 109
<212> PRT
<213> Oryza sativa

<400> 372

Met Ala Glu Met Val Ala Arg Leu Ala Ser Glu Arg Ala Val Val Val
1 5 10 15

Phe Thr Lys Ser Gly Cys Cys Met Cys Thr Ala Val Thr Thr Leu Leu
20 25 30

Gly Glu Leu Ala Val Ser Ala Ala Val His Glu Leu Asp Arg Asp Pro
35 40 45

Leu Gly Lys Glu Met Glu Lys Glu Leu Ala Arg Arg Leu Tyr Gly Ser
50 55 60

Ser Gly Arg Gly Gly Pro Ala Val Pro Ala Val Phe Ile Gly Gly Ser
65 70 75 80

Leu Val Gly Gly Thr Ser Lys Val Met Ala Met His Leu Lys Gly Glu
85 90 95

Leu Val Pro Leu Leu Lys Ser Ala Gly Ala Leu Trp Leu
100 105

<210> 373
<211> 330
<212> DNA
<213> Oryza sativa

<400> 373
atggcggaga tggtggcgag gctggcgtcg gagagggcgg tggtggtgtt caccaagagc 60

ggctgctgca tgtgcaccgc ggtgacgacg ctgctcggcg agctcgccgt cagcgccgcc 120

gtgcacgagc tcgacaggga gccgctcggg aaggagatgg agagggagct cgccaggagg 180

ctctacggct ccggcggccg cggcgggccc gccgtgccgg cggtgttcat cggcgggagc 240

ctcgtcggcg gcaccagcaa ggtgatgacc gtgcacctca agggggagct cgtgccaatg 300

ctcaagagtg ccggtgcact gtggctttga 330

<210> 374
<211> 109
<212> PRT
<213> Oryza sativa

<400> 374

Met Ala Glu Met Val Ala Arg Leu Ala Ser Glu Arg Ala Val Val Val
1 5 10 15

Phe Thr Lys Ser Gly Cys Cys Met Cys Thr Ala Val Thr Thr Leu Leu
20 25 30

Gly Glu Leu Ala Val Ser Ala Ala Val His Glu Leu Asp Arg Glu Pro
35 40 45

Leu Gly Lys Glu Met Glu Arg Glu Leu Ala Arg Arg Leu Tyr Gly Ser
50 55 60

Gly Gly Arg Gly Gly Pro Ala Val Pro Ala Val Phe Ile Gly Gly Ser
65 70 75 80

Leu Val Gly Gly Thr Ser Lys Val Met Thr Val His Leu Lys Gly Glu
85 90 95

Leu Val Pro Met Leu Lys Ser Ala Gly Ala Leu Trp Leu
100 105

<210> 375
<211> 330
<212> DNA
<213> Oryza sativa

<400> 375
atggcggaga tggtggcgag gctggcgtcg gagagggcgg tggtggtgtt caccaagagc 60

ggctgctgca tgtgcaccgc ggtgacgacg ctgctcggcg agctcgccgt cagcgccgcc 120

gtgcacgagc tcgacaggga gccgctcggg aaggagatgg agagggagct cgccaggagg 180

ctctacggct ccggcggccg cggcgggccc gccgtgccgg cggtgttcat cggcgggagc 240

ctcgtcggca gcaccagcaa ggtgatggcc atgcatctca agggggagct cgtgccaatg 300

ctcaagaacg ccggtgcact gtggctttaa 330

<210> 376
<211> 109
<212> PRT
<213> Oryza sativa

<400> 376

Met Ala Glu Met Val Ala Arg Leu Ala Ser Glu Arg Ala Val Val Val
1 5 10 15

Phe Thr Lys Ser Gly Cys Cys Met Cys Thr Ala Val Thr Thr Leu Leu
20 25 30

Gly Glu Leu Ala Val Ser Ala Ala Val His Glu Leu Asp Arg Glu Pro
35 40 45

Leu Gly Lys Glu Met Glu Arg Glu Leu Ala Arg Arg Leu Tyr Gly Ser
50 55 60

Gly Gly Arg Gly Gly Pro Ala Val Pro Ala Val Phe Ile Gly Gly Ser
65 70 75 80

Leu Val Gly Ser Thr Ser Lys Val Met Ala Met His Leu Lys Gly Glu
85 90 95

Leu Val Pro Met Leu Lys Asn Ala Gly Ala Leu Trp Leu
100 105

<210> 377
<211> 312
<212> DNA
<213> Oryza sativa

<400> 377
atggaccgtg tgatgaagct agcatccgag cgtgcggtgg tgatcttcac cttgagctca 60

tgctgcatgt gccacaccgt gacacgcctc ttctgtgatc tcggtgtcaa cgcgctagtg 120

catgagctgg accaagaccc taggggcaag gagatggaga gggcactcct caagctgctc 180

ggaagggggc cgcctgtgcc ggtggtgttc attggtggga agctcgttgg gggaaccaac 240

aagatcatgt ccctccacct tggaggtgag ctgatcccca tgctcaagaa tgcgggagcc 300

ctctggctgt ag 312

<210> 378
<211> 103
<212> PRT
<213> Oryza sativa

<400> 378

Met Asp Arg Val Met Lys Leu Ala Ser Glu Arg Ala Val Val Ile Phe
1 5 10 15

Thr Leu Ser Ser Cys Cys Met Cys His Thr Val Thr Arg Leu Phe Cys
20 25 30

Asp Leu Gly Val Asn Ala Leu Val His Glu Leu Asp Gln Asp Pro Arg
35 40 45

Gly Lys Glu Met Glu Arg Ala Leu Leu Lys Leu Leu Gly Arg Gly Pro
50 55 60

Pro Val Pro Val Val Phe Ile Gly Gly Lys Leu Val Gly Gly Thr Asn
65 70 75 80

Lys Ile Met Ser Leu His Leu Gly Gly Glu Leu Ile Pro Met Leu Lys
85 90 95

Asn Ala Gly Ala Leu Trp Leu
100

<210> 379
<211> 315
<212> DNA
<213> Oryza sativa

<400> 379
atggagaggg tggcgaagct gtcgacggag aaggcggtgg tgatcttcac ggcgagcaac 60

tgcccgatgt gccacacggt ggtgagcctc ttctccgacc tcggcgtcgg cgccgccgtc 120

cacgagctcg accgcgaccc gctccacggc cgggacatgg agcgcgacct cgcccgccgc 180

ctcggccgct ccccgcccgt ccccgccgtc ttcatcgccg gcaagctcgt cggctccacc 240

gaccgcgtca tgtcgctcca cctcgccggc aagctcgtcc ccatgctcaa ggccgccggc 300

gccatttggc tctag 315

<210> 380
<211> 104
<212> PRT
<213> Oryza sativa

<400> 380

```
Met Glu Arg Val Ala Lys Leu Ser Thr Glu Lys Ala Val Val Ile Phe
1               5                   10                  15

Thr Ala Ser Asn Cys Pro Met Cys His Thr Val Val Ser Leu Phe Ser
            20                  25                  30

Asp Leu Gly Val Gly Ala Ala Val His Glu Leu Asp Arg Asp Pro Leu
        35                  40                  45

His Gly Arg Asp Met Glu Arg Asp Leu Ala Arg Arg Leu Gly Arg Ser
    50                  55                  60

Pro Pro Val Pro Ala Val Phe Ile Ala Gly Lys Leu Val Gly Ser Thr
65                  70                  75                  80

Asp Arg Val Met Ser Leu His Leu Ala Gly Lys Leu Val Pro Met Leu
                85                  90                  95

Lys Ala Ala Gly Ala Ile Trp Leu
            100


<210>  381
<211>  892
<212>  DNA
<213>  Picea abies

<400>  381
cgatacagta gttctgccga ttccgacgtc acagtgggcg acgacgcagc gtgcaagctt      60

cggcctgcaa agcagctaga gctcatgggc tgcaatctgt taatgaaaac tgttgtcgag     120

tctcccgtgg acaagattcg caggcttact acggagaacg ccgtgctggt attcagcatg     180

acttcttgct gcatgtgcca tgtcgtgaag cggctcttgt gcagcctggg cgtacatcct     240

actgtttgtg aattggacga ggaagaagaa ggagtggaga tggagaagat actgcgggcg     300

ttggtgggag cacagaaatc gtcggtgccc gctgtgttca tcggagggaa tctcataggc     360

ggcctggacc gggtcatggc catgcacatc gaaggcgatt tggtaccgaa attgaaagaa     420

gccaaagctc tatggctttg atgagctgtt agggtaaggt aatcatgtta tgtccatagt     480

taaaaatcct tttctagtga ggcggtgttt ctggagcaga ttttctttgg ttgaagttcg     540

agtccggctg aatcttgttg gattaccagc tgttattggt caatcgtgtt gtcgtcttcc     600

atctggacat tctaaaaaag agggattttg caagattttc cttgttgatt gcctctatgt     660

cgcctcctcc tcctccagag acatctttat atgtaatttt ctaaccgatg gcgtcgggtt     720

tttcgacttt gcgatctttt attttgcagc tttcaatcca gggtttgtat attcagatga     780

tcatcatgaa gaaactacgc tgtaggtttc tgaattctgt aaagctagaa ttctactttg     840
```

attgtcaaat caaagcccgt gtcgcagctt ttaatatatt gcatttttaa cc 892

<210> 382
<211> 118
<212> PRT
<213> Picea abies

<400> 382

Met Gly Cys Asn Leu Leu Met Lys Thr Val Val Glu Ser Pro Val Asp
1 5 10 15

Lys Ile Arg Arg Leu Thr Thr Glu Asn Ala Val Leu Val Phe Ser Met
20 25 30

Thr Ser Cys Cys Met Cys His Val Val Lys Arg Leu Leu Cys Ser Leu
35 40 45

Gly Val His Pro Thr Val Cys Glu Leu Asp Glu Glu Glu Glu Gly Val
50 55 60

Glu Met Glu Lys Ile Leu Arg Ala Leu Val Gly Ala Gln Lys Ser Ser
65 70 75 80

Val Pro Ala Val Phe Ile Gly Gly Asn Leu Ile Gly Gly Leu Asp Arg
85 90 95

Val Met Ala Met His Ile Glu Gly Asp Leu Val Pro Lys Leu Lys Glu
100 105 110

Ala Lys Ala Leu Trp Leu
115

<210> 383
<211> 761
<212> DNA
<213> Picea abies

<400> 383
tcttttttt tccatgcgta acgacgctct gccttgtctt atagtcactc gttcttcctg 60

agcgagcttc cgcaagcttc cgcgagcttc agatttcctt tccttacatt ccaagctcga 120

atctccgcta ccctacttgt gtttttcttc cttcctgcat atatacaata tgcagtatca 180

cgctcgggcc tacaatcagt tcggcgaggg ttcgtatggt gagcgaacgt ctctacatct 240

ggcaagtcag tcgggttcgt cctccagcag cctcatgtct ccggtggaaa gaatccatca 300

gttggcctcc gagagcgccg tggtggtctt cagcataagc tcctgctgca tgtgccatgt 360

tgtcaagagg ctcttctgtg gcctgggagt caatcccacc gtctacgaac tggacgagga 420

gcacggcggc aaggagatcg agaaggccct gctcaggctg ctgggcggaa gcccagccgt 480

tcccgccgtc tttgtaggcg gaaagcttgt gggaggactg gaccgcgtaa tggcttctca 540

tataaacggc tctctcgtgc ctctcttgaa ggaagctgga gcactgtggc tctgagccct 600

ttttcataat ctgcgggcat tttcattaat tccttgtcgt gtctactttt ttgtatatcg 660

tcaatttcgt ctctggtcat gctcagggtc tcctttttttg taatatcgga gattcactat 720

atatatataa aatataagta acgctgtaga tttttcttgg c 761

<210> 384
<211> 141
<212> PRT
<213> Picea abies

<400> 384

Met Gln Tyr His Ala Arg Ala Tyr Asn Gln Phe Gly Glu Gly Ser Tyr
1 5 10 15

Gly Glu Arg Thr Ser Leu His Leu Ala Ser Gln Ser Gly Ser Ser Ser
20 25 30

Ser Ser Leu Met Ser Pro Val Glu Arg Ile His Gln Leu Ala Ser Glu
35 40 45

Ser Ala Val Val Val Phe Ser Ile Ser Ser Cys Cys Met Cys His Val
50 55 60

Val Lys Arg Leu Phe Cys Gly Leu Gly Val Asn Pro Thr Val Tyr Glu
65 70 75 80

Leu Asp Glu Glu His Gly Gly Lys Glu Ile Glu Lys Ala Leu Leu Arg
85 90 95

Leu Leu Gly Gly Ser Pro Ala Val Pro Ala Val Phe Val Gly Gly Lys
100 105 110

Leu Val Gly Gly Leu Asp Arg Val Met Ala Ser His Ile Asn Gly Ser
115 120 125

Leu Val Pro Leu Leu Lys Glu Ala Gly Ala Leu Trp Leu
130 135 140

<210> 385
<211> 788
<212> DNA
<213> Picea abies

<400> 385
tcgtccctca tcggtgctgg ggaaaggggg aacgttgtct tcatcgttgt catcatgatg 60

caggggtttac aatacagggc cggcggatcg tctgctccag gcaccgtggc ctccgcggcc 120

tgcaggaggc ctgcagccgc cacgctgcat ctggggcaga gctccgtgga ggacgacgaa 180

gagacgatga cgaggacggc catgatgagt atgagccctt tggaaagagt gcagcgcctg 240

gcctccgata acgcggtgct cgtattcagc gtcacttcct gctgcatgtg ccacgttgtt 300

aagcgcctct tctgcggcct tggggttaat ccggcggtgt tcgagctcga cgaggaaggg 360

gaaggtactg gaagctcaga tatggagaag gttcttgtca gattggtcgg caaaaagccc 420

gcggtgcctg cggttttcat cggcggagag ctcgtcggcg gcctcgatcg cctcatggcc 480

gcgcacatca gcggcgagct cgtgcccaaa ttgaagcaag ccggagctct gtggctttaa 540

agaatcagaa ttcgcccgcc tcatttttgc tttcattgtg cgacagatca atgaattaat 600

caccatctct cctgcataga aggcgagtat ataattaatt aatatttgtt gggggtcttg 660

acaattagaa ttctgttctt cttgttctgt aaatattctc tcggctttac agagataatt 720

ataacatata aatagtgaaa atctctcacg ccgatacaat tttgaataga ttataaatgc 780

tattctcc 788

<210> 386
<211> 161
<212> PRT
<213> Picea abies

<400> 386

Met Met Gln Gly Leu Gln Tyr Arg Ala Gly Gly Ser Ser Ala Pro Gly
1 5 10 15

Thr Val Ala Ser Ala Ala Cys Arg Arg Pro Ala Ala Ala Thr Leu His
20 25 30

Leu Gly Gln Ser Ser Val Glu Asp Asp Glu Glu Thr Met Thr Arg Thr
35 40 45

Ala Met Met Ser Met Ser Pro Leu Glu Arg Val Gln Arg Leu Ala Ser
50 55 60

Asp Asn Ala Val Leu Val Phe Ser Val Thr Ser Cys Cys Met Cys His
65 70 75 80

Val Val Lys Arg Leu Phe Cys Gly Leu Gly Val Asn Pro Ala Val Phe
85 90 95

Glu Leu Asp Glu Glu Gly Glu Gly Thr Gly Ser Ser Asp Met Glu Lys
100 105 110

Val Leu Val Arg Leu Val Gly Lys Lys Pro Ala Val Pro Ala Val Phe

115 120 125

Ile Gly Gly Glu Leu Val Gly Gly Leu Asp Arg Leu Met Ala Ala His
130 135 140

Ile Ser Gly Glu Leu Val Pro Lys Leu Lys Gln Ala Gly Ala Leu Trp
145 150 155 160

Leu

<210> 387
<211> 727
<212> DNA
<213> Picea abies

<400> 387
aatcataaat aacctacagg agagcatatt tccaatcata tacattgagg aattgcaggc 60

ccagagggga ttggattggg ttcattgatg cagggcctcc agtacaggcc gggcgcggcc 120

ggttccgcgc catgcaagaa gaaaagcgca gcgcttcaat tgaataagcc gttacagagg 180

ttggagtctc cgctggaggc ggtgcaaagg ctcgcctcag aaaacgccgt gctcgttttc 240

agcatgagtt cctgctgcat gtgccatgtg gttaagcggc tcctgtgcag cctcggggtc 300

aacccggccg tgtgcgagct ggacgaggaa gatcaagagg aggaggagga gacccacgga 360

aaattgcgcg cccacaggga tgatatagag aaggcgctct tcagattagt cgggcagagg 420

ccgcccgtgc cggcggtttt tataggcgga cagctcgtgg gtggcctgga ccagctcatg 480

gccgcacata tcagcggaga gcttgtgcct agattgaaag aggccggcgc tctctggctt 540

taataaagct caattgcttc gtttgtctgg ggaattttta ggtttatttg tttggggttc 600

gagggtttga tcagttcttc ttgtatattg ttctttgtaa tagctttgat ttcaaaagca 660

gtgggtagag tcgagttcat ttattgttct cgttcttttc aatatagaat gagattttta 720

gggatcg 727

<210> 388
<211> 151
<212> PRT
<213> Picea abies

<400> 388

Met Gln Gly Leu Gln Tyr Arg Pro Gly Ala Ala Gly Ser Ala Pro Cys
1 5 10 15

Lys Lys Lys Ser Ala Ala Leu Gln Leu Asn Lys Pro Leu Gln Arg Leu
20 25 30

```
Glu Ser Pro Leu Glu Ala Val Gln Arg Leu Ala Ser Glu Asn Ala Val
        35                  40                  45

Leu Val Phe Ser Met Ser Ser Cys Cys Met Cys His Val Val Lys Arg
    50                  55                  60

Leu Leu Cys Ser Leu Gly Val Asn Pro Ala Val Cys Glu Leu Asp Glu
65                  70                  75                  80

Glu Asp Gln Glu Glu Glu Glu Glu Thr His Gly Lys Leu Arg Ala His
                85                  90                  95

Arg Asp Asp Ile Glu Lys Ala Leu Phe Arg Leu Val Gly Gln Arg Pro
            100                 105                 110

Pro Val Pro Ala Val Phe Ile Gly Gly Gln Leu Val Gly Gly Leu Asp
        115                 120                 125

Gln Leu Met Ala Ala His Ile Ser Gly Glu Leu Val Pro Arg Leu Lys
    130                 135                 140

Glu Ala Gly Ala Leu Trp Leu
145                 150


<210>  389
<211>  309
<212>  DNA
<213>  Physcomitrella patens

<400>  389
atgcaggaga tagagaagct ggtgcaggaa aatgctgtgg ttgtgttcag ccagagcggg      60

tgctgcatgt gtcatgtggt aaagcgtctc ttctgcagtc tgggagtggg gccaactgtg     120

cacgaactcg atgaacggaa ggaaggtggc gacatggaga aggcattgct gcgcctcaac     180

aacaaagttg cgcttcctac cgtgtttgtg ggcggcaaac tggtgggggg cgtcgatgct     240

gtcatggctg cccacgtgag tgggaacctt gtcccccgct tgaaggaagc cggagctctt     300

tggctgtag                                                             309


<210>  390
<211>  102
<212>  PRT
<213>  Physcomitrella patens

<400>  390

Met Gln Glu Ile Glu Lys Leu Val Gln Glu Asn Ala Val Val Val Phe
1               5                   10                  15

Ser Gln Ser Gly Cys Cys Met Cys His Val Val Lys Arg Leu Phe Cys
```

20 25 30

Ser Leu Gly Val Gly Pro Thr Val His Glu Leu Asp Glu Arg Lys Glu
35 40 45

Gly Gly Asp Met Glu Lys Ala Leu Leu Arg Leu Asn Asn Lys Val Ala
50 55 60

Leu Pro Thr Val Phe Val Gly Gly Lys Leu Val Gly Gly Val Asp Ala
65 70 75 80

Val Met Ala Ala His Val Ser Gly Asn Leu Val Pro Arg Leu Lys Glu
85 90 95

Ala Gly Ala Leu Trp Leu
100

<210> 391
<211> 705
<212> DNA
<213> Pinus taeda

<400> 391
catttcttat cttcttcttc ttcgtctcgt tttcttcttc ttcttttttt cttgtttcgt 60

ggaagatgca gtaccatcat cccgcagcag aggcgtgggg aggctcctac ggcggcgacg 120

acatgtcttc ggggcggagg acgtccctac acatgccggc gggggccaa tctggctcga 180

tatcgccgct ggagcgggtg gagagacttg cttctgagaa cgcagtggtg gtcttcagca 240

tgagctcatg ttgtatgtgc catgtgatca agcgcctctt ctgcagcctc ggcgtcaatc 300

cgactgtgta cgaactggac gaggagcatc acggcaaaga catggagaag gctctgcaga 360

gactggtagg cggtggccag gccgtccctg ccgtgttcgt cggtggaaaa ttcttgggag 420

gaatggaccg cgtaatggcc tcccacatca atggcacgct cgtccctctt ttgaaggaag 480

cgggagcctt gtggctctga atcattccgg ccgcagatac ttcttgtttt ctcggttttc 540

cgctgatctt gtggctcaaa actgcgaccg agcctctgta accgtggttc cttcattttg 600

cattatgtcc agctttagaa ttttctttgc ggaattatta gttagcgtgt tgtcgaagct 660

ctgataaatg tgtatacatg ctttatcttt ctttctcatg gagct 705

<210> 392
<211> 144
<212> PRT
<213> Pinus taeda

<400> 392

Met Gln Tyr His His Pro Ala Ala Glu Ala Trp Gly Gly Ser Tyr Gly
1 5 10 15

Gly Asp Asp Met Ser Ser Gly Arg Arg Thr Ser Leu His Met Pro Ala
            20                  25                  30

Gly Gly Gln Ser Gly Ser Ile Ser Pro Leu Glu Arg Val Glu Arg Leu
        35                  40                  45

Ala Ser Glu Asn Ala Val Val Val Phe Ser Met Ser Ser Cys Cys Met
    50                  55                  60

Cys His Val Ile Lys Arg Leu Phe Cys Ser Leu Gly Val Asn Pro Thr
65                  70                  75                  80

Val Tyr Glu Leu Asp Glu Glu His His Gly Lys Asp Met Glu Lys Ala
                85                  90                  95

Leu Gln Arg Leu Val Gly Gly Gly Gln Ala Val Pro Ala Val Phe Val
            100                 105                 110

Gly Gly Lys Phe Leu Gly Gly Met Asp Arg Val Met Ala Ser His Ile
        115                 120                 125

Asn Gly Thr Leu Val Pro Leu Leu Lys Glu Ala Gly Ala Leu Trp Leu
    130                 135                 140

<210> 393
<211> 707
<212> DNA
<213> Pinus taeda

<400> 393
gcacgagcga ggtttcataa tccctcacca ttgctgggaa gcaggaatcg ttgccttcat 60

ctttgtcatc atgatgcagg gtttgcaata cagggccggc ggatcggcgg ctccaggcac 120

cgtggcctcc gcggcctgca ggaggcccgc cgctgctacg ctgcagttgg ggcggagctc 180

cgtggacgac gccgaggagg aaacgatgac cacgaagaca gccacgatga gcctgagccc 240

tttggaaaga gtgcagcgcc tcgcctccga taacgcggtg ctcgtattca gcgtcacttc 300

ctgctgcatg tgccacgtgg tcaagcgcct cttctgcggc ctcggggtta atccggccgt 360

cttcgagctc gacgaggaag gggaaggcgc cgggaactct gatatggaga aggttctggt 420

gagattggtc ggcaaaaagc ctgctgtgcc ggcggttttc atcggcggag agctcgtcgg 480

cggcctcgat cgcctcatgg ccgcgcacat cagcggcgag ctcgtgccca aattgaagca 540

agccggggct ctgtggcttt agagaatcag aattttcgcc ggcctcaatt tttgctgctt 600

tctttggcga tagatcaatt aattaatcac catttctcgt ggcatagaag gcgagtatat 660

aattaatgaa tatttgttgg gggtctcaac aattagaatg aaaaaaa 707

<210> 394
<211> 163
<212> PRT
<213> Pinus taeda

<400> 394

Met Met Gln Gly Leu Gln Tyr Arg Ala Gly Gly Ser Ala Ala Pro Gly
1               5                   10                  15

Thr Val Ala Ser Ala Ala Cys Arg Arg Pro Ala Ala Ala Thr Leu Gln
            20                  25                  30

Leu Gly Arg Ser Ser Val Asp Asp Ala Glu Glu Glu Thr Met Thr Thr
        35                  40                  45

Lys Thr Ala Thr Met Ser Leu Ser Pro Leu Glu Arg Val Gln Arg Leu
    50                  55                  60

Ala Ser Asp Asn Ala Val Leu Val Phe Ser Val Thr Ser Cys Cys Met
65                  70                  75                  80

Cys His Val Val Lys Arg Leu Phe Cys Gly Leu Gly Val Asn Pro Ala
                85                  90                  95

Val Phe Glu Leu Asp Glu Glu Gly Glu Gly Ala Gly Asn Ser Asp Met
            100                 105                 110

Glu Lys Val Leu Val Arg Leu Val Gly Lys Lys Pro Ala Val Pro Ala
        115                 120                 125

Val Phe Ile Gly Gly Glu Leu Val Gly Gly Leu Asp Arg Leu Met Ala
    130                 135                 140

Ala His Ile Ser Gly Glu Leu Val Pro Lys Leu Lys Gln Ala Gly Ala
145                 150                 155                 160

Leu Trp Leu

<210> 395
<211> 896
<212> DNA
<213> Pinus taeda

<400> 395
cggccgggga cacaccacat tctcattccc tctttatttt tcatgcgtaa cgacgctctc 60

ctctcccttg tcttctagtc tcagaccttc gttcttgttt cagcttgcgc gcgcccccct 120

```
tagcagtccg cccccccgtc cccccactat acaatatgca gtatcacgct cgggcctata     180
atcagttcgg cgaaggctcc tatggtgagc gaacgtctct gcatctggcg agccagtcgg     240
gttcgtccac cagcagcttg atgtcgcctg tggaaagaat ccatcagctg gccgccgaga     300
gcgccgtggt ggttttcagt ataagttctt gctgcatgtg ccatgttgtc aagaggctct     360
tctgtggtct gggagtcaat cctactgtct acgaactcga cgaggagcac ggcggtaagg     420
agattgagaa agccctgctc aggttgctgg gcgggagccc atcggttcct gctgtttttg     480
tcggcggaaa gcttgtggga ggactggacc gcgtaatggc ttctcatata aatggctcgc     540
tcgttcctct cttgaaggaa gccggagcac tgtggctctg agcccccttt ttccttaagg     600
ggctgtcatt ttcattaatt ccatgtcgcg tcgcgtctcc tttttgtata tcgttaattc     660
tgtctcgggt caagtcgggt ctccttttg taatatcgga gattcaatct ctctatatat     720
aaaaatatat atataaacta tgctgtagat ttttgttggc aactttagag cttatgggtt     780
ttcattttct gcacttgcaa ttttgtaaag aatcttgcgt ttggggtaag aagttgcctc     840
tgttcgggaa atttcttcat gcccaacgag taccgaatac aagtgttctg cgcact         896
```

```
<210>  396
<211>  141
<212>  PRT
<213>  Pinus taeda

<400>  396

Met Gln Tyr His Ala Arg Ala Tyr Asn Gln Phe Gly Glu Gly Ser Tyr
1               5                   10                  15

Gly Glu Arg Thr Ser Leu His Leu Ala Ser Gln Ser Gly Ser Ser Thr
            20                  25                  30

Ser Ser Leu Met Ser Pro Val Glu Arg Ile His Gln Leu Ala Ala Glu
        35                  40                  45

Ser Ala Val Val Val Phe Ser Ile Ser Ser Cys Cys Met Cys His Val
    50                  55                  60

Val Lys Arg Leu Phe Cys Gly Leu Gly Val Asn Pro Thr Val Tyr Glu
65                  70                  75                  80

Leu Asp Glu Glu His Gly Gly Lys Glu Ile Glu Lys Ala Leu Leu Arg
                85                  90                  95

Leu Leu Gly Gly Ser Pro Ser Val Pro Ala Val Phe Val Gly Gly Lys
            100                 105                 110

Leu Val Gly Gly Leu Asp Arg Val Met Ala Ser His Ile Asn Gly Ser
```

```
        115                 120                 125

Leu Val Pro Leu Leu Lys Glu Ala Gly Ala Leu Trp Leu
    130                 135                 140
```

<210> 397
<211> 427
<212> DNA
<213> Populus trichocarpa

<400> 397

```
tttgtacaaa aaagcaggct taaacaatgt atcaaaccga gtcatggggg tcttacatgc     60

cagcaagaac caaccttggt gacccattgg aacgcatagg aaggctagcc tcagagaatg    120

cagtggtgat ctttagcata agctcatgtt gcatgtgtca tgctattaag aggctctttt    180

gtggcatggg agtgaaccca acagtgtacg agctggatga agacccaaga ggtaaagaaa    240

tggagaaggc tctcatgagg cttctcggta gctcctctgc tgttcctgtt gttttcatcg    300

gtggcaagct tgtgggtgcc atggatagag tcatggcttc tcatattaac ggtactcttg    360

tccctcttct caaggaagcc ggtgctcttt ggctttaatt gatgctaccc agctttcttg    420

tacaaag                                                              427
```

<210> 398
<211> 123
<212> PRT
<213> Populus trichocarpa

<400> 398

```
Met Tyr Gln Thr Glu Ser Trp Gly Ser Tyr Met Pro Ala Arg Thr Asn
1               5                   10                  15

Leu Gly Asp Pro Leu Glu Arg Ile Gly Arg Leu Ala Ser Glu Asn Ala
            20                  25                  30

Val Val Ile Phe Ser Ile Ser Ser Cys Cys Met Cys His Ala Ile Lys
        35                  40                  45

Arg Leu Phe Cys Gly Met Gly Val Asn Pro Thr Val Tyr Glu Leu Asp
    50                  55                  60

Glu Asp Pro Arg Gly Lys Glu Met Glu Lys Ala Leu Met Arg Leu Leu
65                  70                  75                  80

Gly Ser Ser Ser Ala Val Pro Val Val Phe Ile Gly Gly Lys Leu Val
                85                  90                  95

Gly Ala Met Asp Arg Val Met Ala Ser His Ile Asn Gly Thr Leu Val
            100                 105                 110
```

```
Pro Leu Leu Lys Glu Ala Gly Ala Leu Trp Leu
        115                 120


<210>  399
<211>  416
<212>  DNA
<213>  Populus trichocarpa

<400>  399
ggcattagtt tcttgctcgt tgatttggaa atggagaggg tgacaaattt ggcatccgag      60

agaccagttg tgatcttcag caagagcact tgctgtatgt gccacaccat caagactctc     120

ttcaatgagt ttggggtgaa cgtggctgtc catgagctcg atgagatgcc tagaggaagg     180

gaaattgagc aagcactctc aaggtttgga tgcccaacat tgcctgccgt gttcattggt     240

ggtgaacttg tgggtggagc caatgaggtg atgagccttc accttaatcg ttccttaatc     300

ccaatgctta aacgtgctgg cgcgttatgg gtttgatcca tcgatgaact agcttagcta     360

cgcatcaggc actaaccaaa taaattatga aacatgttct ggttgctcat aacata         416


<210>  400
<211>  101
<212>  PRT
<213>  Populus trichocarpa

<400>  400

Met Glu Arg Val Thr Asn Leu Ala Ser Glu Arg Pro Val Val Ile Phe
1               5                   10                  15


Ser Lys Ser Thr Cys Cys Met Cys His Thr Ile Lys Thr Leu Phe Asn
            20                  25                  30


Glu Phe Gly Val Asn Val Ala Val His Glu Leu Asp Glu Met Pro Arg
        35                  40                  45


Gly Arg Glu Ile Glu Gln Ala Leu Ser Arg Phe Gly Cys Pro Thr Leu
    50                  55                  60


Pro Ala Val Phe Ile Gly Gly Glu Leu Val Gly Gly Ala Asn Glu Val
65                  70                  75                  80


Met Ser Leu His Leu Asn Arg Ser Leu Ile Pro Met Leu Lys Arg Ala
                85                  90                  95


Gly Ala Leu Trp Val
            100


<210>  401
```

<211> 491
<212> DNA
<213> Populus trichocarpa

<400> 401
ggttagtgtg taaattacaa ggaaattaag atgcaatatc atcaggctga gtcatggggt 60

taccatgtgc caacgaggac ctgcatggca tcagacccat tggagaaggt tgcgaggctg 120

gcatcggaga gtgctgttgt ggtatttagt atcagcagct gttgcatgtg tcatgccgtg 180

aagagactct tttgtgggat gggtgtgaac ccaactgttt atgagctcga ccatgaccca 240

agaggggaag agattgagaa ggcattaatg aggctgcttg ggaattcaac ttctgtgcct 300

gttgtattca ttggagggaa gttaattggt gccatggaac gagtcatggc ttcccatatc 360

agtggaactc tcgtgcccct cctcaaggaa gctggagcac tctggctttg attttgatca 420

tcgatcaaca aagttaacat cacaaactgg gtccaaaaca tggaaattat cattacaagg 480

aaaaaagagg a 491

<210> 402
<211> 126
<212> PRT
<213> Populus trichocarpa

<400> 402

Met Gln Tyr His Gln Ala Glu Ser Trp Gly Tyr His Val Pro Thr Arg
1               5                   10                  15

Thr Cys Met Ala Ser Asp Pro Leu Glu Lys Val Ala Arg Leu Ala Ser
            20                  25                  30

Glu Ser Ala Val Val Val Phe Ser Ile Ser Ser Cys Cys Met Cys His
        35                  40                  45

Ala Val Lys Arg Leu Phe Cys Gly Met Gly Val Asn Pro Thr Val Tyr
    50                  55                  60

Glu Leu Asp His Asp Pro Arg Gly Glu Glu Ile Glu Lys Ala Leu Met
65                  70                  75                  80

Arg Leu Leu Gly Asn Ser Thr Ser Val Pro Val Val Phe Ile Gly Gly
                85                  90                  95

Lys Leu Ile Gly Ala Met Glu Arg Val Met Ala Ser His Ile Ser Gly
            100                 105                 110

Thr Leu Val Pro Leu Leu Lys Glu Ala Gly Ala Leu Trp Leu
        115                 120                 125

<210> 403
<211> 419
<212> DNA
<213> Populus trichocarpa

<400> 403
accaactctt tgaacgctct gtgacattaa atggatgtgt taaatgtgat gatacaagaa 60

aagccagtgg tgattttcag caagagttct tgctgcatga gccactccat caagtcgctt 120

atacgtggat ttggagcaaa tccaacagtt tatgagctcg acaggattcc aaatggacaa 180

caaattgaaa gggcactagt gcagctgggg tttggacaga gcgtacctgc tgtgttcata 240

ggccaacggc tggttggtaa tgaaaggcag gtgatgagcc tccacgtcca gaaccagctg 300

gtcccattgc ttatacaagc cggtgctatt tggatttaga ataagtagtt tctcactgac 360

ataggacatg gatcattgtc atgcatctca aaatttaggt aagttgctgc tgctagcta 419

<210> 404
<211> 102
<212> PRT
<213> Populus trichocarpa

<400> 404

Met Asp Val Leu Asn Val Met Ile Gln Glu Lys Pro Val Val Ile Phe
1 5 10 15

Ser Lys Ser Ser Cys Cys Met Ser His Ser Ile Lys Ser Leu Ile Arg
20 25 30

Gly Phe Gly Ala Asn Pro Thr Val Tyr Glu Leu Asp Arg Ile Pro Asn
35 40 45

Gly Gln Gln Ile Glu Arg Ala Leu Val Gln Leu Gly Phe Gly Gln Ser
50 55 60

Val Pro Ala Val Phe Ile Gly Gln Arg Leu Val Gly Asn Glu Arg Gln
65 70 75 80

Val Met Ser Leu His Val Gln Asn Gln Leu Val Pro Leu Leu Ile Gln
85 90 95

Ala Gly Ala Ile Trp Ile
100

<210> 405
<211> 428
<212> DNA
<213> Populus trichocarpa

<400> 405
ttcaaactct ttcaatcgat cttcgtgaaa atggacgtgg tgaatgtaat gattcaagga 60

aagcctgtcg tgattttcag gaagagttct tgctgcatga gccactccgt cgagtcactt 120

atacgtggat ttggagcaaa tctaactatt tatgagctcg acagaataac aaatggacaa 180

caaattgaaa gggcactagt gcaactgggg tttcgacaga gcttacccgc tgtgttcata 240

ggccagcagc tggttggcaa tgaaaggcag gtgatgagcc tccacgtcca gaaccagctg 300

gtaccattgc ttatacaagc aggtgctata tggatgtgga ataagtagtt tctcactgac 360

atatataggg cacggatcaa tatcatgctg ctgaacattt tgataagcta ctacttttgt 420

tttttgtt 428

<210> 406
<211> 105
<212> PRT
<213> Populus trichocarpa

<400> 406

Met Asp Val Val Asn Val Met Ile Gln Gly Lys Pro Val Val Ile Phe
1 5 10 15

Arg Lys Ser Ser Cys Cys Met Ser His Ser Val Glu Ser Leu Ile Arg
20 25 30

Gly Phe Gly Ala Asn Leu Thr Ile Tyr Glu Leu Asp Arg Ile Thr Asn
35 40 45

Gly Gln Gln Ile Glu Arg Ala Leu Val Gln Leu Gly Phe Arg Gln Ser
50 55 60

Leu Pro Ala Val Phe Ile Gly Gln Gln Leu Val Gly Asn Glu Arg Gln
65 70 75 80

Val Met Ser Leu His Val Gln Asn Gln Leu Val Pro Leu Leu Ile Gln
85 90 95

Ala Gly Ala Ile Trp Met Trp Asn Lys
100 105

<210> 407
<211> 422
<212> DNA
<213> Populus trichocarpa

<400> 407
tcactttgcc aacttctatt cttcgtagac atggatatgg tgaacaggtt ggttgctgac 60

aggccagtgg tggtctttag caggagcact tgttgcatga gccactccat taagacactt 120

atatctagct ttggggcaaa tcctacagtt tatgagctgg atcaaatacc aaatgggaag 180

caaattgaaa aggcattagt gcagcagcta ggatgtcagc caagtgtacc agctgttttc 240

ataggccaag agtttgttgg tggtgataag caagtcatga gcttacaagt caggaatgag 300

ctagccccat tgctcaggaa ggcgggagct atatggattt gatcaaatgg cagtcattag 360

tcaaatttgc acttgcttta attcagtact gtttgctagt gctcgaattt tgatgggctt 420

ga 422

<210> 408
<211> 103
<212> PRT
<213> Populus trichocarpa

<400> 408

Met Asp Met Val Asn Arg Leu Val Ala Asp Arg Pro Val Val Val Phe
1 5 10 15

Ser Arg Ser Thr Cys Cys Met Ser His Ser Ile Lys Thr Leu Ile Ser
20 25 30

Ser Phe Gly Ala Asn Pro Thr Val Tyr Glu Leu Asp Gln Ile Pro Asn
35 40 45

Gly Lys Gln Ile Glu Lys Ala Leu Val Gln Gln Leu Gly Cys Gln Pro
50 55 60

Ser Val Pro Ala Val Phe Ile Gly Gln Glu Phe Val Gly Gly Asp Lys
65 70 75 80

Gln Val Met Ser Leu Gln Val Arg Asn Glu Leu Ala Pro Leu Leu Arg
85 90 95

Lys Ala Gly Ala Ile Trp Ile
100

<210> 409
<211> 419
<212> DNA
<213> Populus trichocarpa

<400> 409
tcttatcaac accaggaaaa ggttttagaa atggataagg ttagagattt ggcatctagg 60

aacgctgcag tgatcttcac caagagctca tgctgcatgt gccacagcat caagacactt 120

ttttatgaac taggtgcaag ccccgcaatt catgagctag accgtgaagc caatggtaag 180

gaaatggagt gggctttacg tgggctaggg tgcaacccca ccgtcccagc tgtcttcata 240

ggtggaaaat gggtaggatc agccaaagat gtgctatccc tgcacctgga tgggtctttg 300

aaacaaatgc tcatggaagc caaggcaatc tggttctagc tatagtacct ctcaaataag 360

ccacacagta ctttagttta acgctgtatg atatggaaat agctcttatg ctatgttga 419

<210> 410
<211> 102
<212> PRT
<213> Populus trichocarpa

<400> 410

Met Asp Lys Val Arg Asp Leu Ala Ser Arg Asn Ala Ala Val Ile Phe
1 5 10 15

Thr Lys Ser Ser Cys Cys Met Cys His Ser Ile Lys Thr Leu Phe Tyr
20 25 30

Glu Leu Gly Ala Ser Pro Ala Ile His Glu Leu Asp Arg Glu Ala Asn
35 40 45

Gly Lys Glu Met Glu Trp Ala Leu Arg Gly Leu Gly Cys Asn Pro Thr
50 55 60

Val Pro Ala Val Phe Ile Gly Gly Lys Trp Val Gly Ser Ala Lys Asp
65 70 75 80

Val Leu Ser Leu His Leu Asp Gly Ser Leu Lys Gln Met Leu Met Glu
85 90 95

Ala Lys Ala Ile Trp Phe
100

<210> 411
<211> 606
<212> DNA
<213> Triticum aestivum

<400> 411
gctagctcaa gtgaagtgag ctgatcgatt gagatacaga ggagagatag ctaggcaatt 60

cagcaatggc ggagagggtg accgcgattg cgtcgcaggg cacagtggtg atctttgggg 120

cgagctgctg ctgcatgtcc cacaccatga cgaggctctt tgcggagctg ggtgtttttt 180

ctacggtgca cgagctggac aaggaccccc agagggagga cctggagagg gcgctcgctg 240

gcatggtggg ccagagcccg gcggtgccgg cagtattcat ccgtggcgcg cttgtcggtg 300

gcaccaggca ggtcatgcag ctgcatctcg gcggccatct cgtgccgctg ctccgccaag 360

ctggtgccct gtggtcctga ggcattatta ataataatgg ctgatgcgta cttgcatagt 420

aatctacgtg tgatcataac ctcaaaagct gtagctagtg atcgacaata cagtgtgtgc 480

cgctacttaa tgttgaacaa tgcttctcgc cggacattga acccaactct caggcttctt 540

gccagataga tatctgtctc actatgatga tctggcaaga aatataggac atgattgtta 600

ggaatc 606

<210> 412
<211> 104
<212> PRT
<213> Triticum aestivum

<400> 412

Met Ala Glu Arg Val Thr Ala Ile Ala Ser Gln Gly Thr Val Val Ile
1               5                   10                  15

Phe Gly Ala Ser Cys Cys Cys Met Ser His Thr Met Thr Arg Leu Phe
            20                  25                  30

Ala Glu Leu Gly Val Phe Ser Thr Val His Glu Leu Asp Lys Asp Pro
        35                  40                  45

Gln Arg Glu Asp Leu Glu Arg Ala Leu Ala Gly Met Val Gly Gln Ser
    50                  55                  60

Pro Ala Val Pro Ala Val Phe Ile Arg Gly Ala Leu Val Gly Gly Thr
65                  70                  75                  80

Arg Gln Val Met Gln Leu His Leu Gly Gly His Leu Val Pro Leu Leu
                85                  90                  95

Arg Gln Ala Gly Ala Leu Trp Ser
            100

<210> 413
<211> 448
<212> DNA
<213> Triticum aestivum

<400> 413
ttcggcacga ggcacaagct cactatagca gctacaagct actcaggagc tgaccaacac 60

ccttccaaga gcctccttcc atcactttgc cgccgagctc gaccactgcg aggttacatc 120

acaagatgga ccgtgtgatg aagctagcat ctgagcgtgc cgtggtggtg ttcaccctga 180

gtccctgctg catgtgccac acagtggagc gtctgtttcg tgaccagctt ggggtcaatg 240

cgctggtgca cgagctcgac caggacccta ggggcaagga gatggagaga gccctcctca 300

agatgctcgg caggggggccg tcggtgccgg tcgtcttcat cggcgggaag cttgttggtg 360

gcaccaacag gatcatgtct ctacacctcg gtggcgagct agtccccatg ctcaagagtg 420

ccggtgctct ctggctatag agagctag 448

<210> 414
<211> 104
<212> PRT
<213> Triticum aestivum

<400> 414

Met Asp Arg Val Met Lys Leu Ala Ser Glu Arg Ala Val Val Val Phe
1               5                   10                  15

Thr Leu Ser Pro Cys Cys Met Cys His Thr Val Glu Arg Leu Phe Arg
            20                  25                  30

Asp Gln Leu Gly Val Asn Ala Leu Val His Glu Leu Asp Gln Asp Pro
        35                  40                  45

Arg Gly Lys Glu Met Glu Arg Ala Leu Leu Lys Met Leu Gly Arg Gly
    50                  55                  60

Pro Ser Val Pro Val Val Phe Ile Gly Gly Lys Leu Val Gly Gly Thr
65                  70                  75                  80

Asn Arg Ile Met Ser Leu His Leu Gly Gly Glu Leu Val Pro Met Leu
                85                  90                  95

Lys Ser Ala Gly Ala Leu Trp Leu
            100

<210> 415
<211> 714
<212> DNA
<213> Triticum aestivum

<400> 415
cgtacacgta gcactgaaag acttctacat caacagcatt gttcttttga gttggtggtg 60

aagatcaagg tcgggtgagc ttgatggagc aggtgacgaa gctggcgggg cagcgggcgg 120

tggtgatttt cagcatgagc tcctgctgca tgtgccacac ggtggcgcga ctcttccggg 180

atctcggggc gaacccggcg gaggtggatc tcgacgagga ccctaggggg aaggagatgg 240

agaaggcgct ggcgaggctt ctcggtcgga acccggccgt gccggcggtg ttcatcggcg 300

gcaggctcgt cggatccacc gacaaggtca tgtcccttca cctcagcggc aagcttgtac 360

cgctgcttcg taacgcaggt gctgtctggg tctagtgcgc gggagatggt tttttaggtg 420

cctgatgcta gagaataatg taatacacgt atgcctacgt gttctgtttt ctctatcagg 480

tgatgatcaa agataaaaaa aggaaaccat gcaggtcgtt ttattttagc actgcagagg 540

gggagtacga ttctactccc tctgttccga attactccat ttcaacgacg agtaatttgg 600

aacggaggga gtatgactaa agttgtgact ggcatagtgg catgtggacc aatttgatgg 660

gggccccatg tcctttatct taaagtcgag agatttaagt ctaaggatct cagt 714

<210> 416
<211> 103
<212> PRT
<213> Triticum aestivum

<400> 416

Met Glu Gln Val Thr Lys Leu Ala Gly Gln Arg Ala Val Val Ile Phe
1 5 10 15

Ser Met Ser Ser Cys Cys Met Cys His Thr Val Ala Arg Leu Phe Arg
20 25 30

Asp Leu Gly Ala Asn Pro Ala Glu Val Asp Leu Asp Glu Asp Pro Arg
35 40 45

Gly Lys Glu Met Glu Lys Ala Leu Ala Arg Leu Leu Gly Arg Asn Pro
50 55 60

Ala Val Pro Ala Val Phe Ile Gly Gly Arg Leu Val Gly Ser Thr Asp
65 70 75 80

Lys Val Met Ser Leu His Leu Ser Gly Lys Leu Val Pro Leu Leu Arg
85 90 95

Asn Ala Gly Ala Val Trp Val
100

<210> 417
<211> 661
<212> DNA
<213> Triticum aestivum

<400> 417
ccacgcgtcc gggcaaacca ctaagtaaca ctagcttttt ttctctgaga aaaacactag 60

atttctttcc agagaaaaac accagctttc caactaggtc ctttcacaat ccatcaagaa 120

gatcgacgag aaaatatgga gagggtgacg aggctatcga cggagaaggc agtggtgatc 180

ttcacgccga gcaacgactg cccaatgagc tacacagtga cgaccctctt ctctggcctc 240

ggcgtttgcg cggccgtgca cgagctggac aaggaccccc ggggccgtga catggagcgc 300

gacctcgccc gtcgcctggg ccgcacgccg gccgtcccgg ccgtcttcat cggcggcaag 360

ctcgtcggtt ccaccgacag ggtcatgtcg ctgcaccttg gtgggaagct ggtgcccatg 420

ctcaaggccg ccggggcgat ctggctctga gcctgagagg ctgagagaag catcaagtta 480

actcccacgt ataagtaccg tttataatca aaattttaca ctgcatgcat gcgcgcgtag 540

agaggtttgg tttgtatttt gcctactaaa catcctcaca tgagtacgta atgaagacgg 600

gagcaccaga agttttggcc tctatcgata agggaatgac caagctgcgt atctttatga 660

g 661

<210> 418
<211> 104
<212> PRT
<213> Triticum aestivum

<400> 418

Met Glu Arg Val Thr Arg Leu Ser Thr Glu Lys Ala Val Val Ile Phe
1 5 10 15

Thr Pro Ser Asn Asp Cys Pro Met Ser Tyr Thr Val Thr Thr Leu Phe
20 25 30

Ser Gly Leu Gly Val Cys Ala Ala Val His Glu Leu Asp Lys Asp Pro
35 40 45

Arg Gly Arg Asp Met Glu Arg Asp Leu Ala Arg Arg Leu Gly Arg Thr
50 55 60

Pro Ala Val Pro Ala Val Phe Ile Gly Gly Lys Leu Val Gly Ser Thr
65 70 75 80

Asp Arg Val Met Ser Leu His Leu Gly Gly Lys Leu Val Pro Met Leu
85 90 95

Lys Ala Ala Gly Ala Ile Trp Leu
100

<210> 419
<211> 453
<212> DNA
<213> Vitis vinifera

<400> 419
atgcagttcc tcctccttcg ggcgtatacc atcacatttt ggacacccag ggcactgata 60

ttcaccagac actttgatat tgcatacctt gttggtgtat tttgggacta cattgttagt 120

tgctcagtca tggcggatcc gctggagcga gtggcgaggc tggcgtcgga gaatgcggtg 180

gtgatcttca gcctcagctc ctgctgcatg tgccatgccg tgaagaggct cttctgcgga 240

atgggcgtga acccgactgt gtacgagctg gaccaggacc ccagagggaa ggagattgag 300

cgggcgttga tgaggctgct ggggaactct ccggcggtgc ctgtggtgtt catcgggggg 360

aagctcgtgg ggtcaatgga cagtgtcatg gcttcacata tcaatgggac tctggtccct 420

ctcctcaagg aagccggagc tctctggctc tga 453

<210> 420
<211> 150
<212> PRT
<213> Vitis vinifera

<400> 420

```
Met Gln Phe Leu Leu Leu Arg Ala Tyr Thr Ile Thr Phe Trp Thr Pro
1               5                   10                  15

Arg Ala Leu Ile Phe Thr Arg His Phe Asp Ile Ala Tyr Leu Val Gly
            20                  25                  30

Val Phe Trp Asp Tyr Ile Val Ser Cys Ser Val Met Ala Asp Pro Leu
        35                  40                  45

Glu Arg Val Ala Arg Leu Ala Ser Glu Asn Ala Val Val Ile Phe Ser
    50                  55                  60

Leu Ser Ser Cys Cys Met Cys His Ala Val Lys Arg Leu Phe Cys Gly
65                  70                  75                  80

Met Gly Val Asn Pro Thr Val Tyr Glu Leu Asp Gln Asp Pro Arg Gly
                85                  90                  95

Lys Glu Ile Glu Arg Ala Leu Met Arg Leu Leu Gly Asn Ser Pro Ala
            100                 105                 110

Val Pro Val Val Phe Ile Gly Gly Lys Leu Val Gly Ser Met Asp Ser
        115                 120                 125

Val Met Ala Ser His Ile Asn Gly Thr Leu Val Pro Leu Leu Lys Glu
    130                 135                 140

Ala Gly Ala Leu Trp Leu
145                 150
```

<210> 421
<211> 312
<212> DNA
<213> Vitis vinifera

<400> 421

```
atggagaggg cagtggcaag gttggcatca gagaggccag tggtgatatt cagcaagagc      60

tcatgctgca tgtgccacac catcaagacc cttttctcgg actttggagt taacccggcc     120

gtccacgagc tggatgaaat gccgagaggg cgtgaaattg agcaggcctt ggccaggctt     180

gggtgcaacc ccacggtgcc aacagtgttc attggtggtg aacgggtggg tggaaccaat     240

gagatcatga cccttcacct caatagatcc ttaatcccca tgctcaagag ggctggtgcc     300
```

ttatgggtct ga 312

<210> 422
<211> 103
<212> PRT
<213> Vitis vinifera

<400> 422

Met Glu Arg Ala Val Ala Arg Leu Ala Ser Glu Arg Pro Val Val Ile
1 5 10 15

Phe Ser Lys Ser Ser Cys Cys Met Cys His Thr Ile Lys Thr Leu Phe
20 25 30

Ser Asp Phe Gly Val Asn Pro Ala Val His Glu Leu Asp Glu Met Pro
35 40 45

Arg Gly Arg Glu Ile Glu Gln Ala Leu Ala Arg Leu Gly Cys Asn Pro
50 55 60

Thr Val Pro Thr Val Phe Ile Gly Gly Glu Arg Val Gly Gly Thr Asn
65 70 75 80

Glu Ile Met Thr Leu His Leu Asn Arg Ser Leu Ile Pro Met Leu Lys
85 90 95

Arg Ala Gly Ala Leu Trp Val
100

<210> 423
<211> 309
<212> DNA
<213> Vitis vinifera

<400> 423
atggatcgtg ttgggaagtt ggcgtcgcaa aaggcagtgg tgatcttcag taagagttcc 60

tgttgcatga gccatgccat caagagattg ttctatgaac aaggggttag tccggcaatc 120

cacgagctcg acgaggactc cagagggaaa gaaatggagt gggctctgat gaggctaggg 180

tgcaacccct cagttccggc tgtgttcatt ggggggaaat ttgtgggctc tgcaaatact 240

gtgatgaccc ttcatctcaa tggctcactt aagaaaatgc tgaaagaagc cggagctata 300

tggctttag 309

<210> 424
<211> 102
<212> PRT
<213> Vitis vinifera

<400> 424

Met Asp Arg Val Gly Lys Leu Ala Ser Gln Lys Ala Val Val Ile Phe
1 5 10 15

Ser Lys Ser Ser Cys Cys Met Ser His Ala Ile Lys Arg Leu Phe Tyr
20 25 30

Glu Gln Gly Val Ser Pro Ala Ile His Glu Leu Asp Glu Asp Ser Arg
35 40 45

Gly Lys Glu Met Glu Trp Ala Leu Met Arg Leu Gly Cys Asn Pro Ser
50 55 60

Val Pro Ala Val Phe Ile Gly Gly Lys Phe Val Gly Ser Ala Asn Thr
65 70 75 80

Val Met Thr Leu His Leu Asn Gly Ser Leu Lys Lys Met Leu Lys Glu
85 90 95

Ala Gly Ala Ile Trp Leu
100

<210> 425
<211> 321
<212> DNA
<213> Vitis vinifera

<400> 425
atggattcgg tgatggcatt ggggactgca aagccagtgg tgatctttac caagaactca 60

ttatgttgca tgagccacag cattaagaca ctcataagca gctatggggc cagtcctacg 120

gtctatgagc ttgatgaaat gccaaacgga gagcaaatgg aaaaggccct accaatacta 180

gggtgtccga atttaccagc ggtattcata ggtaaaaagc tggtgggtgg ggctcgtgag 240

ataatgagcc tgcaagtcag gggcgagctc tctgatctgc tcatagaggc aaaagctata 300

ttttttttgga acaaaaaata g 321

<210> 426
<211> 106
<212> PRT
<213> Vitis vinifera

<400> 426

Met Asp Ser Val Met Ala Leu Gly Thr Ala Lys Pro Val Val Ile Phe
1 5 10 15

Thr Lys Asn Ser Leu Cys Cys Met Ser His Ser Ile Lys Thr Leu Ile
20 25 30

```
Ser Ser Tyr Gly Ala Ser Pro Thr Val Tyr Glu Leu Asp Glu Met Pro
        35                  40                  45

Asn Gly Glu Gln Met Glu Lys Ala Leu Pro Ile Leu Gly Cys Pro Asn
    50                  55                  60

Leu Pro Ala Val Phe Ile Gly Lys Lys Leu Val Gly Gly Ala Arg Glu
65                  70                  75                  80

Ile Met Ser Leu Gln Val Arg Gly Glu Leu Ser Asp Leu Leu Ile Glu
                85                  90                  95

Ala Lys Ala Ile Phe Phe Trp Asn Lys Lys
            100                 105


<210>  427
<211>  309
<212>  DNA
<213>  Vitis vinifera

<400>  427
atggataggg tggaggagtt ggcgaggaag aatgctgcag tgattttcac caagagctca      60

tgctgcatgt gccacagcat caagacactg ttctacgatc tgggtgcgag ccctgcgatt     120

catgagctcg ataaggacgc tagaggaagg gaaatggagt gggctttgcg gcggataggg     180

tgcaacccct ccgtccctgc tgtgtttgta ggcggaaaat ttgttgggtc tgctaaagat     240

gtgattacca gccatgttga tgggtctcta aagcaaatgc tcattgcagc cagagccatc     300

tggttctag                                                              309


<210>  428
<211>  102
<212>  PRT
<213>  Vitis vinifera

<400>  428

Met Asp Arg Val Glu Glu Leu Ala Arg Lys Asn Ala Ala Val Ile Phe
1               5                   10                  15

Thr Lys Ser Ser Cys Cys Met Cys His Ser Ile Lys Thr Leu Phe Tyr
            20                  25                  30

Asp Leu Gly Ala Ser Pro Ala Ile His Glu Leu Asp Lys Asp Ala Arg
        35                  40                  45

Gly Arg Glu Met Glu Trp Ala Leu Arg Arg Ile Gly Cys Asn Pro Ser
    50                  55                  60
```

Val Pro Ala Val Phe Val Gly Gly Lys Phe Val Gly Ser Ala Lys Asp
65 70 75 80

Val Ile Thr Ser His Val Asp Gly Ser Leu Lys Gln Met Leu Ile Ala
85 90 95

Ala Arg Ala Ile Trp Phe
100

<210> 429
<211> 309
<212> DNA
<213> Vitis vinifera

<400> 429
atggataagg tgacgagatt ggcttcagag aaaggggtgg tgatcttcag caagagctca 60

tgctgcctgt gctatgctgt caacattctg tttcaagaac ttggggtcac tcccacggtt 120

catgaaatcg accaagaccc cgatggaagg gaaatggaga aagccctctt gaggctggga 180

tgcaatgctc ctgtcccagc tgtgttcata ggtggaaagc tcgtgggatc caccaacgaa 240

gtcatgtccc gtcacctaag cggctccctc attcccctgc tgaagccata tcagcagact 300

ttatcttaa 309

<210> 430
<211> 102
<212> PRT
<213> Vitis vinifera

<400> 430

Met Asp Lys Val Thr Arg Leu Ala Ser Glu Lys Gly Val Val Ile Phe
1 5 10 15

Ser Lys Ser Ser Cys Cys Leu Cys Tyr Ala Val Asn Ile Leu Phe Gln
20 25 30

Glu Leu Gly Val Thr Pro Thr Val His Glu Ile Asp Gln Asp Pro Asp
35 40 45

Gly Arg Glu Met Glu Lys Ala Leu Leu Arg Leu Gly Cys Asn Ala Pro
50 55 60

Val Pro Ala Val Phe Ile Gly Gly Lys Leu Val Gly Ser Thr Asn Glu
65 70 75 80

Val Met Ser Arg His Leu Ser Gly Ser Leu Ile Pro Leu Leu Lys Pro
85 90 95

Tyr Gln Gln Thr Leu Ser

100

<210> 431
<211> 372
<212> DNA
<213> Vitis vinifera

<400> 431
atgcattatc agacggaatc gtggggctcc tacatgccaa caaggagcgt tggagaccca 60

ttggagcgca tagagagact ggcctcagag aatgcggtgg tgatattcag catcagttct 120

tgctgtatgt gccacgccat taagaggctc ttctgcggga tgggcgtgaa ccccacggtc 180

tacgagctcg acgaggaccc cagagggaag gagatggaga aggccttgat gaggcttctg 240

ggtagttcct cggcggttcc ggtggtcttc atcggcggga agctcgtcgg agcaatggac 300

agagtcatgg cctcccatat taatgggacg ctggtgcctc tcctcaagga cgccggtgct 360

ctctggcttt aa 372

<210> 432
<211> 123
<212> PRT
<213> Vitis vinifera

<400> 432

Met His Tyr Gln Thr Glu Ser Trp Gly Ser Tyr Met Pro Thr Arg Ser
1 5 10 15

Val Gly Asp Pro Leu Glu Arg Ile Glu Arg Leu Ala Ser Glu Asn Ala
20 25 30

Val Val Ile Phe Ser Ile Ser Ser Cys Cys Met Cys His Ala Ile Lys
35 40 45

Arg Leu Phe Cys Gly Met Gly Val Asn Pro Thr Val Tyr Glu Leu Asp
50 55 60

Glu Asp Pro Arg Gly Lys Glu Met Glu Lys Ala Leu Met Arg Leu Leu
65 70 75 80

Gly Ser Ser Ser Ala Val Pro Val Val Phe Ile Gly Gly Lys Leu Val
85 90 95

Gly Ala Met Asp Arg Val Met Ala Ser His Ile Asn Gly Thr Leu Val
100 105 110

Pro Leu Leu Lys Asp Ala Gly Ala Leu Trp Leu
115 120

<210> 433
<211> 404
<212> DNA
<213> Zea mays

<220>
<221> misc_feature
<222> (47)..(47)
<223> n is a, c, g, or t

<400> 433
cgaagagccg gtaaagaagt taatatcgtc tagcaatggc ccgggtnacg aaactggctt 60

ctcagcgccc ggtggtcatc ttcagcccga gctcctgctg tatgtgcccc ccggtgacgc 120

gcctgttccg cgagctcggg gtgaacccgc cggtggtgga gctggccgag gaccccaggg 180

ggaaggagat ggagaaggcc ctggcgaggc tgctcgggcg caaccccgct gtgccggcag 240

tgttcatcgg cggcaggctc gtcggctcca ccgacaaggt catgtcgctt cacctgagcg 300

gcaacctcgt tccgcttctg cgcaatgccg gcgcgctctg ggtgtacccg tgttgcatat 360

atatgtagct acccgttttc cattccatat gcatgctatc tatc 404

<210> 434
<211> 110
<212> PRT
<213> Zea mays

<400> 434

Met Ala Arg Val Thr Lys Leu Ala Ser Gln Arg Pro Val Val Ile Phe
1 5 10 15

Ser Pro Ser Ser Cys Cys Met Cys Pro Pro Val Thr Arg Leu Phe Arg
20 25 30

Glu Leu Gly Val Asn Pro Pro Val Val Glu Leu Ala Glu Asp Pro Arg
35 40 45

Gly Lys Glu Met Glu Lys Ala Leu Ala Arg Leu Leu Gly Arg Asn Pro
50 55 60

Ala Val Pro Ala Val Phe Ile Gly Gly Arg Leu Val Gly Ser Thr Asp
65 70 75 80

Lys Val Met Ser Leu His Leu Ser Gly Asn Leu Val Pro Leu Leu Arg
85 90 95

Asn Ala Gly Ala Leu Trp Val Tyr Pro Cys Cys Ile Tyr Met
100 105 110

<210> 435

<211>  595
<212>  DNA
<213>  Zea mays

<400>  435
aagtagtcaa agcatctagc tcgttacaag tggctcttgt cgcctacaat tttgccttct      60

tccaagtctt gtttgaagtt gctctctgcg aagagccggt agagaagtta atatcgtcta     120

gcaatggacc gggtgacgaa actggcttct cagcgcgcgg tggtcatctt cagcacgagc     180

tcctgctgta tgtgccacac ggtgacgcgc ctgttccgcg agctcggggt gaacccgacg     240

gtggtggagc tggacgagga ccccaggggg aaggagatgg agaaggcact ggcgaggctg     300

ctcgggcgca accccgctgt gccggcagtg ttcatcggcg gcaggctcgt cggctccacc     360

gacaaggtca tgtcgcttca cctgagcggc aacctcgttc cgcttctgcg caatgccggc     420

gcgctctggg tgtagccgtg ttgcatatat atgtagctag ccgtgttcca ttccatatgc     480

atgctatcta tcaggagagg agacacacgg agggaagtac gtagtaataa ttaattaagc     540

aggccttttt atctgcaccg taataatttt ccatagagga cggagtgtaa aaaaa          595

<210>  436
<211>  103
<212>  PRT
<213>  Zea mays

<400>  436

Met Asp Arg Val Thr Lys Leu Ala Ser Gln Arg Ala Val Val Ile Phe
1               5                   10                  15

Ser Thr Ser Ser Cys Cys Met Cys His Thr Val Thr Arg Leu Phe Arg
            20                  25                  30

Glu Leu Gly Val Asn Pro Thr Val Val Glu Leu Asp Glu Asp Pro Arg
        35                  40                  45

Gly Lys Glu Met Glu Lys Ala Leu Ala Arg Leu Leu Gly Arg Asn Pro
    50                  55                  60

Ala Val Pro Ala Val Phe Ile Gly Gly Arg Leu Val Gly Ser Thr Asp
65                  70                  75                  80

Lys Val Met Ser Leu His Leu Ser Gly Asn Leu Val Pro Leu Leu Arg
                85                  90                  95

Asn Ala Gly Ala Leu Trp Val
            100

<210>  437
<211>  579

<212> DNA
<213> Zea mays

<400> 437
aaacaagcag cagcagagca gaggatggca atggaccacg tggcgaggct ggcgtcggag 60

cgcgcggtgg tggtgttcac ggcgagcaac tgcagcatgg gcgacgtggt gacgtcgctg 120

ctgagcagcc tgggcgtcaa cgcggcggtg cacgacctgg acagggaccc ccggggcatg 180

gagatggagc gggagctggc caggaggctc ggcggcggcg gcgggcgcgg cacgacgacg 240

acccccaccg tgccggcggt gttcgtgggc ggtgacctcg tcggcgggac caacagggtc 300

atggctctgc acctctccgg cgagctcgtg cccatgctca ggaaagccgg cgccctctgg 360

ttgtagtaaa ttgagaacgc ccgccgatcg accgcgcgca tgcaagcatg gctgatgagc 420

tttttacatc agctgtgtgt atatgtatgt gaataaaaaa gaagcggtca aagaaggcaa 480

agagatggag agagaaggat ccacccacgt actaagagta cgtgatgaat gcgaggtttt 540

tgttgtgtcc ttggaataaa tgtcacctcc gttttcact 579

<210> 438
<211> 113
<212> PRT
<213> Zea mays

<400> 438

Met Ala Met Asp His Val Ala Arg Leu Ala Ser Glu Arg Ala Val Val
1 5 10 15

Val Phe Thr Ala Ser Asn Cys Ser Met Gly Asp Val Val Thr Ser Leu
20 25 30

Leu Ser Ser Leu Gly Val Asn Ala Ala Val His Asp Leu Asp Arg Asp
35 40 45

Pro Arg Gly Met Glu Met Glu Arg Glu Leu Ala Arg Arg Leu Gly Gly
50 55 60

Gly Gly Gly Arg Gly Thr Thr Thr Thr Pro Thr Val Pro Ala Val Phe
65 70 75 80

Val Gly Gly Asp Leu Val Gly Gly Thr Asn Arg Val Met Ala Leu His
85 90 95

Leu Ser Gly Glu Leu Val Pro Met Leu Arg Lys Ala Gly Ala Leu Trp
100 105 110

Leu

<210> 439
<211> 622
<212> DNA
<213> Zea mays

<400> 439
ccgggatctc aagcaacacg acgcattgca ctagctagac cttgggctcg caaacctcac 60

acacccttcc gcttaacctg cagcccttcg atcatcatca gcactcagca gtagcatagg 120

catcaaggta gagaccccga caatgcagta cggcgcggcg gcggcggagc aggcgtggtc 180

gtacatgccg gtggtggcac cgtcgtcggc cgtggagacg gcggcggagc gcgtggagcg 240

gctggcgtcg gagagcgcgg tggtggtgtt cagcgtgagc acctgctgca tgtgccacgc 300

cgtgaagcgc ctcttctgcg gcatgggcgt gcacccgacg gtgcacgagc tggaccacga 360

cccgcggggc cgcgagctgg agcgcgccct ggcctgcctc ctcggcgcct ccggagcctc 420

ggcggcgggc gcgccggtcg tgcccgtcgt gttcatcggc ggcaggctgg tcggcgccat 480

ggaccgcgtc atggccgcgc acatcaacgg caccctcgtg ccgctgctca aggacgccgg 540

cgcgctctgg ctgtgatctc atcgccggcc gctagctact agccattgcc cgcgttgcag 600

ctgttcaatc ggggggctag ct 622

<210> 440
<211> 137
<212> PRT
<213> Zea mays

<400> 440

Met Gln Tyr Gly Ala Ala Ala Ala Glu Gln Ala Trp Ser Tyr Met Pro
1 5 10 15

Val Val Ala Pro Ser Ser Ala Val Glu Thr Ala Ala Glu Arg Val Glu
20 25 30

Arg Leu Ala Ser Glu Ser Ala Val Val Val Phe Ser Val Ser Thr Cys
35 40 45

Cys Met Cys His Ala Val Lys Arg Leu Phe Cys Gly Met Gly Val His
50 55 60

Pro Thr Val His Glu Leu Asp His Asp Pro Arg Gly Arg Glu Leu Glu
65 70 75 80

Arg Ala Leu Ala Cys Leu Leu Gly Ala Ser Gly Ala Ser Ala Ala Gly
85 90 95

Ala Pro Val Val Pro Val Val Phe Ile Gly Gly Arg Leu Val Gly Ala
100 105 110

Met Asp Arg Val Met Ala Ala His Ile Asn Gly Thr Leu Val Pro Leu
115 120 125

Leu Lys Asp Ala Gly Ala Leu Trp Leu
130 135

<210> 441
<211> 997
<212> DNA
<213> Zea mays

<400> 441
ctccaagcac tcacctcctc agctcattgc cagcctttgg ttccttccag ctagcaaccg 60

gacggccagc aagccagcag actagtagct cttctcagtt ctcacaccta ctgtgtctgt 120

gtgtgctcgc ctttagcggc aagcgaccca cccccttcg gtagcgagcg acaatgcagt 180

acgcggcggc ggagcaggcg tggtacatgc cggcgaccac gacgatgatg gcggagagcg 240

cggtggcgcg cgtggagcgg ctggcgtcgg agagcgcggt ggtggtgttc agcgtgagca 300

gctgctgcat gtgccacgcc gtgaagcggc tcttctgcgg catgggcgtg cacccgacgg 360

tgcacgagct ggacctggac ccgcgcggac gagagctgga gcacgccctc gcccgcctca 420

tcggctacgg ccccgccggc gcgcccgtcg tccccgtcgt cttcatcggc gggaagctgg 480

tcggcgccat ggaccgggtc atggccgcgc atatcaacgg ctccctcgtc ccacttctca 540

aggaggccgg cgcgctctgg ctctgaactg taggcaggcc gcgcgccatt gctggtgact 600

cgtgtgccaa caggctacgc agcttcttcc gttgtctaag ttagttcctt gttgctgtag 660

aacctgatgt cacttgctgc aagctaatca aactacgtac taagctacgg tgataggagg 720

atacatccat ggacagggcc ggatggtggt agatcagtga tgcatcggat tgggcttaat 780

gtgtgtgagt agtgtgtgca agagagaaga gaggctggta ccgtgtgtgt gctttttttct 840

cactacctac tgcctccgtc ggtgtttgtg tgtgcgtgca tgtggtgttt gacgcatgcg 900

ctggatttgc tttgcttggg gtgctacact gttaccacca ctgttgttta atttgatatt 960

cctttaattt taataccttg tttcctctca aaaaaaa 997

<210> 442
<211> 130
<212> PRT
<213> Zea mays

<400> 442

Met Gln Tyr Ala Ala Ala Glu Gln Ala Trp Tyr Met Pro Ala Thr Thr
1 5 10 15

Thr Met Met Ala Glu Ser Ala Val Ala Arg Val Glu Arg Leu Ala Ser

20 25 30

Glu Ser Ala Val Val Val Phe Ser Val Ser Ser Cys Cys Met Cys His
35 40 45

Ala Val Lys Arg Leu Phe Cys Gly Met Gly Val His Pro Thr Val His
50 55 60

Glu Leu Asp Leu Asp Pro Arg Gly Arg Glu Leu Glu His Ala Leu Ala
65 70 75 80

Arg Leu Ile Gly Tyr Gly Pro Ala Gly Ala Pro Val Val Pro Val Val
85 90 95

Phe Ile Gly Gly Lys Leu Val Gly Ala Met Asp Arg Val Met Ala Ala
100 105 110

His Ile Asn Gly Ser Leu Val Pro Leu Leu Lys Glu Ala Gly Ala Leu
115 120 125

Trp Leu
130

<210> 443
<211> 1568
<212> DNA
<213> Oryza sativa

<400> 443
cccacgcgtc cgcccacgcg tccgggacac cagaaacata gtacacttga gctcactcca 60

aactcaaaca ctcacaccaa tggctctcca agttcaggcc gcactcctgc cctctgctct 120

ctctgtcccc aagaagggta acttgagcgc ggtggtgaag gagccggggt tccttagcgt 180

gagcagaagg ccaagaagcc gtcgctggtg gtgagggcgg tggcgacgcg gcgggccggt 240

ggcgagcccc ggcgcgggca cgtcgaaggc ggacgggaag aagacgctgc ggcagggggt 300

ggtggtgatc accggcgcgt cgtcggggct cgggctcgcg gcggcgaagg cgcttggcgg 360

agacggggaa gtggcacgtg gtgatggcgt tccgcgactt tcctgaaggc ggcgacggcg 420

gcgaaggcgg cggggatggc ggcggggagc tacaccgtca tgcacctgga cctcgcctcc 480

ctcgacagcg tccgccagtt cgtggacaac ttccggcgct ccggcatgcc gctcgacgcg 540

ctggtgtgca acgccgcaca tctaccggcc gacggcgcgg caaccgacgt tcaacgccga 600

cgggtacgag atgagcgtcg ggtgaacca cctgggccac ttcctcctcg cccgcctcat 660

gctcgacgac ctcaagaaat ccgactaccc gtcgcggcgg ctcatcatcc tcggctccat 720

caccggcaac accaacacct tcgccggcaa cgtccctccc aaggccgggc taggcgacct 780

ccgggggctc gccggcgggc tccgcgggca gaacgggtcg gcgatgatcg acggcgcgga 840

gagcttcgac ggcgccaagg cgtacaagga cagcaagatc tgtaacatgc tgacgatgca 900

ggagttccac cggagattcc acgaggagac cgggatcacg ttcgcgtcgc tgtacccggg 960

gtgcatcgcg acgacgggct tgttccgcga gcacatcccg ctgttccggc tgctgttccc 1020

gccgttccag cggttcgtga cgaaggggtt cgtgtcggag gcggagtccg ggaagcggct 1080

ggcgcaggtg gtgggcgacc cgagcctgac caagtccggc gtgtactgga gctggaacaa 1140

ggactcggcg tcgttcgaga accagctctc gcaggaggcc agcgacccgg agaaggccag 1200

gaagctctgg gacctcagcg agaagctcgt cggcctcgtc tgagtttatt atttacccat 1260

tcgtttcaac tgttaatttc ttcggggttt agggggtttc agctttcagt gagagaggcc 1320

tgtcaagtga tgtacaatta gtaatttttt tttacccgac aaatcatgca ataaaaccac 1380

aggcttacat tatcgatttg tccacctaaa ttaagtttca actgttaatt tcttcggggt 1440

ttagggggtt tcagctttca gtgagagagg cctgtcaagt gatgtacaat tagtaatttt 1500

tttttacccg acaaatcatg caataaaacc acaggcttac attatcgatt tgtccaccta 1560

aattaagt 1568

<210> 444
<211> 56
<212> DNA
<213> Artificial sequence

<220>
<223> primer: prm09053

<400> 444
ggggacaagt ttgtacaaaa aagcaggctt aaacaatgga tatgataacg aagatg 56

<210> 445
<211> 50
<212> DNA
<213> Artificial sequence

<220>
<223> primer: prm09054

<400> 445
ggggaccact ttgtacaaga aagctgggta aaaacatgat aagtcaaacc 50

<210> 446
<211> 799
<212> DNA
<213> Arabidopsis thaliana

<400> 446
ttgattgaga tacttgagat ccaagataaa tatgtcttta agtcgtagag atcctcttgt 60

ggtcggcagt gttgttggag atgttcttga tcctttcacg aggttggtct ctcttaaggt 120

cacttatggc catagagagg ttactaatgg cttggatcta aggccttctc aagttctgaa 180

caaaccaata gtggagattg gaggagacga cttcagaaat ttctacacct tggttatggt 240

ggatccagat gtgccgagtc caagcaaccc tcaccaacga gaatatctcc actggttggt 300

gactgatata cctgccacca ctggaaatgc ctttggcaat gaggtggtgt gctacgagag 360

tccacgtccc ccctcgggaa ttcatcgtat tgtgttggta ttgttccggc aactcggaag 420

acaaacggtt tatgcaccgg ggtggcgcca acagttcaac actcgtgagt ttgctgagat 480

ctacaatctt ggtcttcctg tggctgcctc ttacttcaac tgccagaggg agaatggctg 540

tgggggaaga agaacgtaga tgcgtaccta cttacgttaa ctaataatct aatcgtataa 600

tattccctta atgaagtatt taagcatcta tgtcaatgta ataagaattt aaagatacga 660

gctaaaaaaa atgatgcata tgctgacatc gatgtaaagt agtttacact tttaatgtaa 720

taactaggtt ttaacccgcg gtacaccgcg agactatttt gtttttttaa gaataaaaat 780

ataatttgtt tagtcgatt 799

<210> 447
<211> 175
<212> PRT
<213> Arabidopsis thaliana

<400> 447

Met Ser Leu Ser Arg Arg Asp Pro Leu Val Val Gly Ser Val Val Gly
1 5 10 15

Asp Val Leu Asp Pro Phe Thr Arg Leu Val Ser Leu Lys Val Thr Tyr
20 25 30

Gly His Arg Glu Val Thr Asn Gly Leu Asp Leu Arg Pro Ser Gln Val
35 40 45

Leu Asn Lys Pro Ile Val Glu Ile Gly Gly Asp Asp Phe Arg Asn Phe
50 55 60

Tyr Thr Leu Val Met Val Asp Pro Asp Val Pro Ser Pro Ser Asn Pro
65 70 75 80

His Gln Arg Glu Tyr Leu His Trp Leu Val Thr Asp Ile Pro Ala Thr
85 90 95

Thr Gly Asn Ala Phe Gly Asn Glu Val Val Cys Tyr Glu Ser Pro Arg
100 105 110

Pro Pro Ser Gly Ile His Arg Ile Val Leu Val Leu Phe Arg Gln Leu
115 120 125

Gly Arg Gln Thr Val Tyr Ala Pro Gly Trp Arg Gln Gln Phe Asn Thr
130 135 140

Arg Glu Phe Ala Glu Ile Tyr Asn Leu Gly Leu Pro Val Ala Ala Ser
145 150 155 160

Tyr Phe Asn Cys Gln Arg Glu Asn Gly Cys Gly Gly Arg Arg Thr
165 170 175

<210> 448
<211> 2194
<212> DNA
<213> Oryza sativa

<400> 448
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag atttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctccttctc ccatctataa attcctcccc ccttttcccc tctctatata 1020

ggaggcatcc aagccaagaa gagggagagc accaaggaca cgcgactagc agaagccgag 1080

cgaccgcctt ctcgatccat atcttccggt cgagttcttg gtcgatctct tccctcctcc 1140

acctcctcct cacagggtat gtgcctccct tcggttgttc ttggatttat tgttctaggt 1200

tgtgtagtac gggcgttgat gttaggaaag gggatctgta tctgtgatga ttcctgttct 1260

tggatttggg atagaggggt tcttgatgtt gcatgttatc ggttcggttt gattagtagt 1320

```
atggttttca atcgtctgga gagctctatg gaaatgaaat ggtttaggga tcggaatctt   1380
gcgattttgt gagtaccttt tgtttgaggt aaaatcagag caccggtgat tttgcttggt   1440
gtaataaagt acggttgttt ggtcctcgat tctggtagtg atgcttctcg atttgacgaa   1500
gctatccttt gtttattccc tattgaacaa aaataatcca actttgaaga cggtcccgtt   1560
gatgagattg aatgattgat tcttaagcct gtccaaaatt tcgcagctgg cttgtttaga   1620
tacagtagtc cccatcacga aattcatgga aacagttata atcctcagga acaggggatt   1680
ccctgttctt ccgatttgct ttagtcccag aatttttttt cccaaatatc ttaaaaagtc   1740
actttctggt tcagttcaat gaattgattg ctacaaataa tgcttttata gcgttatcct   1800
agctgtagtt cagttaatag gtaatacccc tatagtttag tcaggagaag aacttatccg   1860
atttctgatc tccattttta attatatgaa atgaactgta gcataagcag tattcatttg   1920
gattattttt tttattagct ctcacccctt cattattctg agctgaaagt ctggcatgaa   1980
ctgtcctcaa ttttgttttc aaattcacat cgattatcta tgcattatcc tcttgtatct   2040
acctgtagaa gtttcttttt ggttattcct tgactgcttg attacagaaa gaaatttatg   2100
aagctgtaat cgggatagtt atactgcttg ttcttatgat tcatttcctt tgtgcagttc   2160
ttggtgtagc ttgccacttt caccagcaaa gttc                               2194

<210>  449
<211>  58
<212>  DNA
<213>  Artificial sequence

<220>
<223>  primer: prm4759

<400>  449
ggggacaagt ttgtacaaaa aagcaggctt aaacaatgtc tttaagtcgt agagatcc       58

<210>  450
<211>  50
<212>  DNA
<213>  Artificial sequence

<220>
<223>  primer: prm4760

<400>  450
ggggaccact ttgtacaaga aagctgggtg tacgcatcta cgttcttctt                50
```

## Description

The present invention relates generally to the field of molecular biology and concerns a method for enhancing various yield-related traits by modulating expression in a plant of a nucleic acid encoding an SCE1 (SUMO Conjugating Enzyme 1). The present invention also concerns plants having modulated expression of a nucleic acid encoding an SCE1, which plants have enhanced yield-related traits relative to corresponding wild type plants or other control plants. The invention also provides hitherto unknown SCE1-encoding nucleic acids, and constructs comprising the same, useful in performing the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defense mechanisms.

In another embodiment has now been found that various yield-related traits may be improved in plants by modulating expression in a plant of a nucleic acid encoding an SCE1 (SUMO Conjugating Enzyme 1).

## Background

Eukaryotic protein function is regulated in part by posttranslational processes such as the covalent attachment of small polypeptides. The most frequent and best characterized is the modification by ubiquitin and ubiquitin-like proteins. SUMO, the small ubiquitin-like modifier is similar to ubiquitin in tertiary structure but differs in primary sequence. SUMO conjugation to target proteins, a process referred to as sumoylation, involves the sequential action of a number of enzymes, namely, activating (E1), conjugating (E2 or SUMO E2) and ligase (E3). The process is reversible, and desumoylation, that is, removal of SUMO from the substrate, is mediated by SUMO proteases. Mechanistically sumoylation comprises distinct phases. Initially the E1 enzyme complex activates SUMO by binding to it via a highly reactive sulfhydryl bond. Activated SUMO is then transferred to the E2 conjugating enzyme *via* trans-sterification reaction, involving a conserved cysteine residue in the E2 enzyme. Residue cysteine 94 is the conjugated residue in the Arabidopsis thaliana E2 enzyme, also named AtSCE1 protein. In the last step, SUMO is transferred to the substrate via an isopeptide bond.

While protein modification by ubiquitin often results in protein degradation, sumoylation, ie. conjugation of SUMO to proteins, is often associated with protein stabilization. Sumoylation function is best understood in yeast and animals where it plays a role in signal transduction, cell cycle DNA repair, transcriptional regulation, nuclear import and subsequent localization and in viral pathogenesis. In plants, sumoylation has been implicated in regulation of gene expression in response to development, hormonal and environmental changes (Miura et al. 2007. Current Opinion in Plant Biol. 10, 495-502).

Protein components of the sumoylation pathway are encoded in the genome of eukaryotes. In yeast and mammals there is a single SUMO E2 conjugating enzyme described. Although initially in Arabidopsis thaliana only a single SUMO E2, AtSCE1a, was found (Lois et al. 2003. The Plant Cell 15, 1347-1359), some plants may have multiple isoforms, as is the case for rice, for which three genes encoding E2 enzymes have been described (Miura et al. 2007). The AtSCE1a protein is characterized by the presence of a UBC domain and of an active site cysteine amino acid residue. In Arabidopsis thaliana there are more than 40 UBC domain-containing proteins, of which the great majority are thought to act as ubiquitin conjugating enzymes, and only four of them are predicted or shown to function on conjugation of ubiquitin-like proteins. Of the latter only AtSCE1a (At3g57870) and a truncated SCE1b protein (At5g02240) thought to be encoded by a pseudogene are proposed to act as SUMO E2 conjugating enzymes (Kraft et al. Plant Phys 2005, 1597-1611). In comparison to other UBC proteins, SCE1 a protein has higher amino acid identity to human UBC12 and UBC9. Phylogenetic analysis revealed that Arabidopsis proteins with a UBC domain and an active site cysteine amino acid residue can be divided into 16 groups, with group I functioning in SUMO conjugation pathway (Kraft et al. 2005).

Functional characterization of a Nicotiana SCE1 protein showed that it can activate SUMO *in vitro* and it can complement a yeast SUMO E2 mutant (Castilo et al. 2004. J. virology 78: 2758-2769). Arabidopsis thaliana transgenic plants overexpressing a modified AtSCE1a by a histidine tag were used to demonstrate nuclear co-localization of AtSCE1a and SUMO1/2 (Lois et al 2003). The authors showed altered behaviour of the transgenic plant response to specific stresses such as salt and the hormone ABA, but not the hormone Auxin. However the authors failed to state any growth difference between the control and the transgenic plants grown on control medium lacking the factor causing the stress.

## Summary

Surprisingly, it has now been found that modulating expression of a nucleic acid encoding an SCE1 polypeptide gives plants having enhanced yield-related traits relative to control plants.

According one embodiment, there is provided a method for enhancing yield related traits of a plant relative to control plants, comprising modulating expression of a nucleic acid encoding an SCE1 polypeptide in a plant. The enhanced yield related traits comprise increased shoot and root biomass and increase number of panicles and of seeds of a plant.

## Definitions

Polypeptide(s) / Protein(s)

The terms "polypeptide" and "protein" are used interchangeably herein and refer to amino acids in a polymeric form of any length, linked together by peptide bonds.

Polynucleotide(s) / Nucleic acid(s) / Nucleic acid sequence(s) / Nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break α-helical structures or β-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

Table 1: Examples of conserved amino acid substitutions

| **Residue** | **Conservative Substitutions** | **Residue** | **Conservative Substitutions** |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

Motif/Consensus sequence/Signature

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6x\log_{10}[Na^+]^a + 0.41x\%[G/C^b] - 500x[L^c]^{-1} - 0.61x\% \text{ formamide}$$

2) DNA-RNA or RNA-RNA hybrids:

$$Tm = 79.8 + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m = 2 (I_n)$
For 20-35 nucleotides: $T_m = 22 + 1.46 (I_n)$

a or for other monovalent cation, but only accurate in the 0.01-0.4 M range.
[b] only accurate for %GC in the 30% to 75% range.
[c] L = length of duplex in base pairs.
[d] oligo, oligonucleotide; $I_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

Table 2a: Examples of constitutive promoters

| **Gene Source** | **Reference** |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

Table 2b: Examples of root-specific promoters

| Gene Source | Reference |
|---|---|
| RCc3 | Plant Mol Biol. 1995 Jan;27(2):237-48 |
| Arabidopsis PHT1 | Kovama et al., 2005; Mudge et al. (2002, Plant J. 31:341) |
| Medicago phosphate transporter | Xiao et al., 2006 |
| Arabidopsis Pyk10 | Nitz et al. (2001) Plant Sci 161(2): 337-346 |
| root-expressible genes | Tingey et al., EMBO J. 6: 1, 1987. |
| tobacco auxin-inducible gene | Van der Zaal et al., Plant Mol. Biol. 16, 983, 1991. |
| β-tubulin | Oppenheimer, et al., Gene 63: 87, 1988. |
| tobacco root-specific genes | Conkling, et al., Plant Physiol. 93: 1203, 1990. |
| B. napus G1-3b gene | United States Patent No. 5, 401, 836 |
| SbPRP1 | Suzuki et al., Plant Mol. Biol. 21: 109-119, 1993. |
| LRX1 | Baumberger et al. 2001, Genes & Dev. 15:1128 |
| BTG-26 Brassica napus | US 20050044585 |
| LeAMT1 (tomato) | Lauter et al. (1996, PNAS 3:8139) |
| The LeNRT1-1 (tomato) | Lauter et al. (1996, PNAS 3:8139) |
| class I patatin gene (potato) | Liu et al., Plant Mol. Biol. 153:386-395, 1991. |
| KDC1 (Daucus carota) | Downey et al. (2000, J. Biol. Chem. 275:39420) |
| TobRB7 gene | W Song (1997) PhD Thesis, North Carolina State University, Raleigh, NC USA |
| OsRAB5a (rice) | Wang et al. 2002, Plant Sci. 163:273 |
| ALF5 (Arabidopsis) | Diener et al. (2001, Plant Cell 13:1625) |
| NRT2;1Np (N. plumbaginifolia) | Quesada et al. (1997, Plant Mol. Biol. 34:265) |

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm and/or aleurone and/or embryo-specific. Examples of seed-specific promoters *(endosperm/aleurone/embryo specific)* are shown in Tables 2c-f below. Further examples of seed-specific promoters are given in Qing Qu and Takaiwa (Plant Biotechnol. J. 2, 113-125, 2004), which disclosure is incorporated by reference herein as if fully set forth.

Table 2c: Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat $\alpha$, $\beta$, $\gamma$-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice a-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice $\alpha$-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum $\alpha$-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | unpublished |
| PRO0147, trypsin inhibitor ITR1 (barley) | unpublished |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |
| $\alpha$-amylase (Amy32b) | Lanahan et al, Plant Cell 4:203-211, 1992; Skriver et al, Proc Natl Acad Sci USA 88:7266-7270, 1991 |
| cathepsin $\beta$-like gene | Cejudo et al, Plant Mol Biol 20:849-856, 1992 |

(continued)

| Gene source | Reference |
|---|---|
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 2d:** examples of endosperm-specific promoters

| Gene source | Reference |
|---|---|
| glutelin (rice) | Takaiwa et al. (1986) Mol Gen Genet 208:15-22; Takaiwa et al. (1987) FEBS Letts. 221:43-47 |
| | |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 2e:** Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 2f:** Examples of aleurone-specific promoters:

| Gene source | Reference |
|---|---|
| α-amylase (Amy32b) | Lanahan et al, Plant Cell 4:203-211, 1992; Skriver et al, Proc Natl Acad Sci USA 88: 7266-7270, 1991 |
| | |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g below.

**Table 2g:** Examples of green tissue-specific promoters

| Gene | Expression | Reference |
|---|---|---|
| Maize Orthophosphate dikinase | Leaf specific | Fukavama et al., 2001 |
| Maize Phosphoenolpyruvate carboxylase | Leaf specific | Kausch et al., 2001 |
| Rice Phosphoenolpyruvate carboxylase | Leaf specific | Liu et al., 2003 |
| Rice small subunit Rubisco | Leaf specific | Nomura et al., 2000 |
| rice beta expansin EXBP9 | Shoot specific | WO 2004/070039 |
| Pigeonpea small subunit Rubisco | Leaf specific | Panguluri et al., 2005 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of green meristem-specific promoters which may be used to perform the methods of the invention are shown in Table 2h below.

Table 2h: Examples of meristem-specific promoters

| Gene source | Expression pattern | Reference |
|---|---|---|
| rice OSH1 | Shoot apical meristem, from embryo globular stage to seedling stage | Sato et al. (1996) Proc. Natl. Acad. Sci. USA, 93: 8117-8122 |
| Rice metallothionein | Meristem specific | BAD87835.1 |
| WAK1 & WAK 2 | Shoot and root apical meristems, and in expanding leaves and sepals | Wagner & Kohorn (2001) Plant Cell 13(2): 303-318 |

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Decreased expression

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants. Examples of various methods for the reduction or substantial elimination of expression in a plant of an endogenous gene, or for lowering levels and/or activity of a protein, are known to the skilled in the art. The person skilled in the art is aware of the different approaches that allow a reduction or substantial elimination of expression, such as, but not limited to gene silencing, RNA-mediated silencing, co-suppression or insertion mutagenesis. Methods for decreasing expression are known in the art and the skilled person would readily be able to adapt the known methods for silencing so as to achieve reduction ofexpression of an endogenous gene in a whole plant or in parts thereof through the use of an appropriate promoter, for example.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest, preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation (s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. The person skilled in the art would readily be able to adapt the aforementioned methods for silencing so as to achieve reduction of expression of an endogenous gene in a whole plant or in parts thereof through the use of an appropriate promoter. The skilled is also aware of the different approaches that allow a reduction or substantial elimination of expression, such as, but not limited to gene silencing, RNA-mediated silencing, co-suppression or insertion mutagenesis.

<u>Selectable marker (gene)/Reporter gene</u>

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die). The marker genes may be removed or excised from the transgenic cell once they are no longer needed. Techniques for marker gene removal are known in the art, useful techniques are described above in the definitions section.

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the IoxP sequences. If the marker gene is integrated between the IoxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for Agrobacterium-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis (Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8), and approaches exist that are generally applicable regardless of the target organism (Miller et al, Nature Biotechnol. 25, 778-785, 2007).

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The term "yield" of a plant may relate to vegetative biomass (root and/or shoot biomass), to reproductive organs, and/or to propagules (such as seeds) of that plant.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increase / Improve / Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), and g) increased number of primary panicles, which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased seed yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Tripsacum dactyloides, Triticale sp., Triticosecale rimpaui, Triticum* spp. *(e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

Detailed description of the invention

Surprisingly, it has now been found that modulating expression in a plant of a nucleic acid encoding an SCE1 polypeptide, gives plants having enhanced yield-related traits relative to control plants. According to a first embodiment, the present invention provides a method for enhancing yield-related traits in plants relative to control plants, comprising modulating expression in a plant of a nucleic acid encoding an SCE1 polypeptide.

A preferred method for modulating (preferably, increasing) expression of a nucleic acid encoding an SCE1 polypeptide is by introducing and expressing in a plant a nucleic acid encoding an SCE1 polypeptide.

Any reference hereinafter to a "protein useful in the methods of the invention" is taken to mean an SCE1 polypeptide as defined herein. Any reference hereinafter to a "nucleic acid useful in the methods of the invention" is taken to mean a nucleic acid capable of encoding such an SCE1 polypeptide. The nucleic acid to be introduced into a plant (and therefore useful in performing the methods of the invention) is any nucleic acid encoding the type of protein which will now be described, hereinafter also named *"SCE1* nucleic acid" or *"SCE1* gene".

A "SCE1 polypeptide" as defined herein refers to any polypeptide comprising a Ubiquitin-conjugating domain (UBC domain) and preferably having SUMO E2 conjugating activity.

The conserved UBC domain is approximately 140 to 150 amino acids long and corresponds to the entry with accession number IPR000608 in the InterPro database (InterPro (Mulder et al., (2003) Nucl. Acids. Res. 31, 315-318).

Examples of SCE1 polypeptides useful in the methods of the invention SCE1 polypeptides are given in Table A of Example 1 herein. Table C in Example 4 describes the UBC domains as present in the SCE1 polypeptides of Table A.

A preferred SCE1 polypeptide useful in the methods of the invention comprises an amino acid sequence having, in increasing order of preference, at least 50%, 55%, 60%, 65%, 70%, 75%, 80%, 85%, 90%, 95%, 96%, 97%, 98%, 99% or more sequence identity to the amino acid sequence of any of the UBC domains as set forth in Table C of Example 4.

Further preferably, the SCE1 polypeptide mentioned above is a polypeptide having, in increasing order of preference, at least 50%, 55%, 60%, 65%, 70%, 75%, 80%, 85%, 90%, 95%, 96%, 97%, 98%, 99% or more sequence identity to the amino acid sequence of any of the polypeptides of Table A. Most preferably, the SCE1 polypeptide is one of the polypeptides of Table A.

Alternatively, the homologue of an SCE1 protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71 %, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 200. The overall sequence identity is determined using a global alignment algorithm, such as the Needleman Wunsch algorithm in the program GAP (GCG Wisconsin Package, Accelrys), preferably with default parameters. Compared to overall sequence identity, the sequence identity will generally be higher when only conserved domains or motifs are considered.

Alternatively, the sequence of the SCE1 polypeptide useful in the methods of the invention when used in the construction of a phylogenetic tree, such as the one depicted in Figure 6 of Kraft et al. 2005, clusters with the group I comprising the amino acid sequence of AtSCE1a rather than with any other group.

The term "domain" and "motif" is defined in the "definitions" section herein. Specialist databases exist for the identification of domains, for example, SMART (Schultz et al. (1998) Proc. Natl. Acad. Sci. USA 95, 5857 - 5864; Letunic et al. (2002) Nucleic Acids Res 30, 242 - 244), InterPro (Mulder et al., (2003) Nucl. Acids. Res. 31, 315 - 318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp 53 - 61, AAAI Press, Menlo Park; Hulo et al., Nucl. Acids. Res. 32: D134 - D137, (2004)), or Pfam (Bateman et al., Nucleic Acids Research 30 (1): 276 - 280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31: 3784 - 3788 (2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443 - 453) to find the global (i.e. spanning the complete sequences) alignment of two sequences that maximizes the number of matches and minimizes the number of gaps. The BLAST algorithm (Altschul et al. (1990) J Mol Biol 215: 403 - 410) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10; 4: 29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147 (1); 195 - 197).

Furthermore, SCE1 polypeptides typically have sumoylation activity. Tools and techniques for measuring sumoylation activity are well known in the art. Further details are provided in Example 6.2.

In addition, SCE1 polypeptides, when expressed in rice according to the methods of the present invention as outlined in Examples 6 and 7, give plants having increased yield related traits, in particular increased shoot and/or root biomass.

The present invention is illustrated by transforming plants with the nucleic acid sequence represented by SEQ ID NO: 199, encoding the polypeptide sequence of SEQ ID NO: 200. However, performance of the invention is not restricted to these sequences; the methods of the invention may advantageously be performed using any SCE1-encoding nucleic acid or SCE1 polypeptide as defined herein.

Examples of nucleic acids encoding SCE1 polypeptides are given in Table A of Example 1 herein. Such nucleic acids are useful in performing the methods of the invention. The amino acid sequences given in Table A of Example 1 are example sequences of orthologues and paralogues of the SCE1 polypeptide represented by SEQ ID NO: 200, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 199 or SEQ ID NO: 200, the second BLAST would therefore be against Arabidopsis sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Nucleic acid variants may also be useful in practising the methods of the invention. Examples of such variants include nucleic acids encoding homologues and derivatives of any one of the amino acid sequences given in Table A of Example 1, the terms "homologue" and "derivative" being as defined herein. Also useful in the methods of the invention are nucleic acids encoding homologues and derivatives of orthologues or paralogues of any one of the amino acid sequences given in Table A of Example 1. Homologues and derivatives useful in the methods of the present invention have substantially the same biological and functional activity as the unmodified protein from which they are derived.

Nucleic acid variants useful in practising the methods of the invention include portions of nucleic acids encoding SCE1 polypeptides, nucleic acids hybridising to nucleic acids encoding SCE1 polypeptides, splice variants of nucleic acids encoding SCE1 polypeptides, allelic variants of nucleic acids encoding SCE1 polypeptides and variants of nucleic acids encoding SCE1 polypeptides obtained by gene shuffling. The terms hybridising sequence, splice variant, allelic variant and gene shuffling are as described herein.

Nucleic acids encoding SCE1 polypeptides need not be full-length nucleic acids, since performance of the methods of the invention does not rely on the use of full-length nucleic acid sequences. According to the present invention, there is provided a method for enhancing yield-related traits in plants, comprising introducing and expressing in a plant a portion of any one of the nucleic acid sequences given in any of Table A of Example 1, or a portion of a nucleic acid encoding an orthologue, paralogue or homologue of any of the amino acid sequences given in Table A of Example 1.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode an SCE1 polypeptide as defined herein, and have substantially the same biological activity as the amino acid sequences given in Table A of Example 1. Preferably, the portion is a portion of any one of the nucleic acids given in Table A of Example 1, or is a portion of a nucleic acid encoding an orthologue or paralogue of any one of the amino acid sequences given in Table A of Example 1. Preferably the portion is at least 100, 200, 300, 400, 500, 600, 700, 800, 900, 1000 consecutive nucleotides in length, the consecutive nucleotides being of any one of the nucleic acid sequences given in Table A of Example 1, or of a nucleic acid encoding an orthologue or paralogue of any one of the amino acid sequences given in Table A of Example 1. Most preferably the portion is a portion of the nucleic acid of SEQ ID NO: 199. Preferably, the portion encodes a fragment of an amino acid sequence which, when used in the construction of a phylogenetic tree, such as the one depicted in Figure 6 of Kraft et al. 2005, clusters with the group I comprising the amino acid sequence of AtSCE1a rather than with any other group.

Another nucleic acid variant useful in the methods of the invention is a nucleic acid capable of hybridising, under reduced stringency conditions, preferably under stringent conditions, with a nucleic acid encoding a SCE1 polypeptide, or a homologue thereof as defined herein, or with a portion as defined herein.

According to the present invention, there is provided a method for enhancing yield-related traits in plants, comprising introducing and expressing in a plant a nucleic acid capable of hybridizing to any one of the nucleic acids given in Table A of Example 1, or comprising introducing and expressing in a plant a nucleic acid capable of hybridising to a nucleic acid encoding an orthologue, paralogue or homologue of any of the nucleic acid sequences given in Table A of Example 1.

Hybridising sequences useful in the methods of the invention encode an SCE1 polypeptide as defined herein, having substantially the same biological activity as the amino acid sequences given in Table A of Example 1. Preferably, the hybridising sequence is capable of hybridising to any one of the nucleic acids given in Table A of Example 1, or to a portion of any of these sequences, a portion being as defined above, or the hybridising sequence is capable of hybridising to a nucleic acid encoding an orthologue or paralogue of any one of the amino acid sequences given in Table A of Example 1. Most preferably, the hybridising sequence is capable of hybridising to a nucleic acid as represented by SEQ ID NO: 199 or to a portion thereof.

Preferably, the hybridising sequence encodes a polypeptide with an amino acid sequence which, when used in the construction of a phylogenetic tree, such as the one depicted in Figure 6 from Kraft et al. 2005, clusters with the group I comprising the amino acid sequence of AtSCE1a rather than with any other group.

Another nucleic acid variant useful in the methods of the invention is a splice variant encoding a SCE1 polypeptide, or a homologue thereof as defined hereinabove, a splice variant being as defined herein.

According to the present invention, there is provided a method for enhancing yield-related traits in plants, comprising introducing and expressing in a plant a splice variant of any one of the nucleic acid sequences given in Table A of Example 1, or a splice variant of a nucleic acid encoding an orthologue, paralogue or homologue of any of the amino acid sequences given in Table A of Example 1.

Preferred splice variants are splice variants of a nucleic acid represented by SEQ ID NO: 199, or a splice variant of a nucleic acid encoding an orthologue or paralogue of SEQ ID NO: 200. Preferably, the amino acid sequence encoded by the splice variant, when used in the construction of a phylogenetic tree, such as the one depicted in Figure 6 from Kraft et al. 2005, clusters with the group I comprising the amino acid sequence of AtSCE1a rather than with any other group.

Another nucleic acid variant useful in performing the methods of the invention is an allelic variant of a nucleic acid encoding an SCE1 polypeptide, or a homologue thereof as defined hereinabove, an allelic variant being as defined herein.

According to the present invention, there is provided a method for enhancing yield-related traits in plants, comprising introducing and expressing in a plant an allelic variant of any one of the nucleic acids given in Table A of Example 1, or comprising introducing and expressing in a plant an allelic variant of a nucleic acid encoding an orthologue, paralogue or homologue of any of the amino acid sequences given in Table A of Example 1.

The allelic variants useful in the methods of the present invention have substantially the same biological activity as the SCE1 polypeptide of SEQ ID NO: 200 and any of the amino acids depicted in Table A of Example 1. Allelic variants exist in nature, and encompassed within the methods of the present invention is the use of these natural alleles. Preferably, the allelic variant is an allelic variant of SEQ ID NO: 199 or an allelic variant of a nucleic acid encoding an orthologue or paralogue of SEQ ID NO: 200. Preferably, the amino acid sequence encoded by the allelic variant when used in the construction of a phylogenetic tree, such as the one depicted in Figure 6 from Kraft et al. 2005, clusters with the group I comprising the amino acid sequence of AtSCE1a rather than with any other group.

Gene shuffling or directed evolution may also be used to generate variants of nucleic acids encoding SCE1 polypeptides, or homologues thereof as defined above; the term "gene shuffling" being as defined herein.

A to the present invention, there is provided a method for enhancing yield-related traits in plants, comprising introducing and expressing in a plant a variant of any one of the nucleic acid sequences given in Table A of Example 1, or comprising introducing and expressing in a plant a variant of a nucleic acid encoding an orthologue, paralogue or homologue of any of the amino acid sequences given in Table A of Example 1, which variant nucleic acid is obtained by gene shuffling.

Concerning SCE1 sequences, preferably, the amino acid sequence encoded by the variant nucleic acid obtained by gene shuffling, when used in the construction of a phylogenetic tree, such as the one depicted in Figure 6 of Kraft et al. 2005, clusters with the group I comprising the amino acid sequence of AtSCE1a rather than with any other group.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding SCE1 polypeptides may be derived from any natural or artificial source. The nucleic acid may be modified from its native form in composition and/or genomic environment through deliberate human manipulation. Preferably the SCE1 polypeptide-encoding nucleic acid is from a plant, further preferably from a dicotyledonous plant, more preferably from the family brasicaceae, most preferably the nucleic acid is from *Arabidopsis thaliana.*

Advantageously, the present invention provides hitherto unknown SCE1 nucleic acid and polypeptide sequences.

According to a further embodiment of the present invention, there is provided an isolated nucleic acid molecule comprising:

(i) a nucleic acid represented by SEQ ID NO: 201; SEQ ID NO: 203; SEQ ID NO: 205; SEQ ID NO: 207; SEQ ID NO: 209; SEQ ID NO: 211 and SEQ ID NO: 213;

(ii) a nucleic acid or fragment thereof that is complementary to any one of the SEQ ID NOs given in (i);

(iii) a nucleic acid encoding an SCE1 polypeptide having, in increasing order of preference, at least 70%, 75%, 80%, 85%, 90%, 95%, 96%, 97%, 98%, 99% or more sequence identity to any one of the amino acid sequences given in SEQ ID NO: 202; SEQ ID NO: 204; SEQ ID NO: 206; SEQ ID NO: 208; SEQ ID NO: 210; SEQ ID NO: 212 and SEQ ID NO: 214;

(iv) a nucleic acid capable of hybridizing under stringent conditions to any one of the nucleic acids given in (i), (ii) or (iii) above.

According to a further embodiment of the present invention, there is therefore provided an isolated polypeptide comprising:

(i) an amino acid sequence having, in increasing order of preference, at least 80%, 85%, 90%, 95%, 96%, 97%, 98%, 99% or 100% sequence identity to any one of the amino acid sequences given in SEQ ID NO: 202; SEQ ID NO: 204; SEQ ID NO: 206; SEQ ID NO: 208; SEQ ID NO: 210; SEQ ID NO: 212 and SEQ ID NO: 214;

(ii) derivatives of any of the amino acid sequences given in (i).

Performance of the methods of the invention gives plants having enhanced yield-related traits. In particular performance of the methods of the invention gives plants having increased yield, especially increased seed yield relative to control plants. The terms "yield" and "seed yield" are described in more detail in the "definitions" section herein.

Reference herein to enhanced yield-related traits is taken to mean an increase in biomass (weight) of one or more parts of a plant, which may include aboveground (harvestable) parts and/or (harvestable) parts below ground. In particular, such harvestable parts are seeds, and performance of the methods of the invention results in plants having increased seed yield relative to the seed yield of control plants. Furthermore the term "yield-related trait" as defined herein may encompass an alteration of the ratio of roots to shoots (root:shoot ratio).

Taking corn as an example, a yield increase may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per square meter, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

The present invention provides a method for increasing yield, especially seed yield of plants, relative to control plants, which method comprises modulating expression in a plant of a nucleic acid encoding an SCE1 polypeptide as defined herein.

Since the transgenic plants according to the present invention have increased yield, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating expression in a plant of a nucleic acid encoding an SCE1 polypeptide as defined herein.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi, nematodes and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises modulating expression in a plant of a nucleic acid encoding an SCE1 polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises modulating expression in a plant of a nucleic acid encoding an SCE1 polypeptide. Nutrient deficiency may result from a lack of nutrients such as nitrogen, phosphates and other phosphorous-containing compounds, potassium, calcium, cadmium, magnesium, manganese, iron and boron, amongst others.

The present invention encompasses plants or parts thereof (including seeds) obtainable by the methods according to the present invention. The plants or parts thereof comprise a nucleic acid transgene encoding an SCE1 polypeptide, or a homologue thereof as defined above.

The invention also provides genetic constructs and vectors to facilitate introduction and/or expression in plants of nucleic acids encoding SCE1 polypeptides, or homologues thereof. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding an SCE1 polypeptide, or a homologue thereof as defined above;

(b) one or more control sequences capable of driving expression of the nucleic acid sequence of (a); and optionally
(c) a transcription termination sequence.

Preferably, the nucleic acid encoding an SCE1 polypeptide is as defined above. The term "control sequence" and "termination sequence" are as defined herein.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence. A constitutive promoter is particularly useful in the methods. Preferably the constitutive promoter is also a ubiquitous promoter. See the "Definitions" section herein for definitions of the various promoter types.

It should be clear that the applicability of the present invention is not restricted to the SCE1 polypeptide-encoding nucleic acid represented by SEQ ID NO: 199, nor is the applicability of the invention restricted to expression of an SCE1 polypeptide-encoding nucleic acid when driven by a constitutive promoter.

The constitutive promoter is preferably a GOS2 promoter, preferably a GOS2 promoter from rice. Further preferably the constitutive promoter is represented by a nucleic acid sequence substantially similar to SEQ ID NO: 247 most preferably the constitutive promoter is as represented by SEQ ID NO: 247. See Table 2a in the "Definitions" section herein for further examples of constitutive promoters.

Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The invention also provides a method for the production of transgenic plants having enhanced yield-related traits relative to control plants, comprising introduction and expression in a plant of any nucleic acid encoding an SCE1 polypeptide as defined hereinabove.

More specifically, the present invention provides a method for the production of transgenic plants having increased enhanced yield-related traits, particularly increased yield or increased seed yield, which method comprises:

(i) introducing and expressing in a plant or plant cell a SCE1 polypeptide-encoding nucleic acid; and
(ii) cultivating the plant cell under conditions promoting plant growth and development.

The nucleic acid of (i) may be any of the nucleic acids capable of encoding an SCE1 polypeptide as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding an SCE1 polypeptide, or a homologue thereof as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, linseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum, emmer, spelt, secale, einkorn, teff, milo and oats.

The invention also extends to harvestable parts of a plant such as, but not limited to seeds, leaves, fruits, flowers, stems, roots, rhizomes, tubers and bulbs. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. Methods for increasing expression of nucleic acids or genes, or gene products, are well documented in the art and examples are provided in the definitions section.

As mentioned above, a preferred method for modulating expression of an SCE1 polypeptide, or a homologue thereof is by introducing and expressing in a plant a nucleic acid encoding an SCE1 polypeptide, or a homologue thereof; however the effects of performing the method, i.e.altering the root:shoot ratio in plants and/or enhancing yield-related traits may also be achieved using other well-known techniques, including but not limited to T-DNA activation tagging, TILLING, homologous recombination. A description of these techniques is provided in the definitions section.

Nucleic acids encoding an SCE1 polypeptide described herein, or the SCE1 polypeptides themselves, may find use in breeding programmes in which a DNA marker is identified which may be genetically linked to a PRE-like, an SCE1, a YEF1, or a subgroup III Grx polypeptide -encoding gene. The nucleic acids/genes, or the SCE1 polypeptides themselves may be used to define a molecular marker. This DNA or protein marker may then be used in breeding programmes to select plants having an altered root:shoot ratio and/or having enhanced yield-related traits as defined hereinabove in the methods of the invention.

Allelic variants of an SCE1 polypeptide-encoding nucleic acid/gene, or a Sister of FT-encoding may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give an altered root:shoot ratio and/or increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding SCE1 polypeptides or homologues thereof may also be used as probes for genetically and physically mapping the genes that they are a part of, and as markers for traits linked to those genes. Such information may be useful in plant breeding in order to develop lines with desired phenotypes. Such use of SCE1 polypeptide-encoding nucleic acids requires only a nucleic acid sequence of at least 15 nucleotides in length. The SCE1 polypeptide-encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the SCE1 polypeptide-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the SCE1 polypeptide-encoding nucleic acids in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features. Furthermore, the methods according to the present invention result in plants having an altered root:shoot ratio, as described hereinbefore. These traits may also be combined with other economically advantageous traits, such as further yield-enhancing traits, tolerance to other abiotic and biotic stresses, traits modifying various architectural features and/or biochemical and/or physiological features.

Items

1. A method for enhancing yield-related traits in plants relative to control plants, comprising modulating expression in a plant of a nucleic acid encoding an SCE1, SUMO Conjugating Enzyme 1, polypeptide and optionally selecting for plants having enhanced yield-related traits.
2. Method according to item 1, wherein said SCE1 polypeptide comprises a sequence having at least one of the following:

   (i) 50%, 55%, 60%, 65%, 70%, 75%, 80%, 85%, 90%, 95%, 96%, 97%, 98%, 99% or more sequence identity to the amino acid sequence of any of the polypeptides of Table A;

(ii) 50%, 55%, 60%, 65%, 70%, 75%, 80%, 85%, 90%, 95%, 96%, 97%, 98%, 99% or more sequence identity to the amino acid sequence of any of the UBC domains as set forth in Table C of Example 4.

3. Method according to item 1 or 2, wherein said modulated expression is effected by introducing and expressing in a plant a nucleic acid encoding an SCE1 polypeptide.
4. Method according to any one of items 1 to 3, wherein said nucleic acid encoding an SCE1 polypeptide encodes any one of the proteins listed in Table A or is a portion of such a nucleic acid, or a nucleic acid capable of hybridising with such a nucleic acid.
5. Method according to any one of items 1 to 4, wherein said nucleic acid sequence encodes an orthologue or paralogue of any of the proteins given in Table A.
6. Method according to any one of items 1 to 5, wherein said enhanced yield-related traits comprise increased biomass, preferably shoot and/or root biomass relative to control plants.
7. Method according to any one of items 1 to 6, wherein said enhanced yield-related traits are obtained under conditions of nitrogen deficiency.
8. Method according to any one of items 3 to 7, wherein said nucleic acid is operably linked to a constitutive promoter, preferably to a GOS2 promoter, most preferably to a GOS2 promoter from rice.
9. Method according to any preceding item, wherein said nucleic acid encoding an SCE1 polypeptide is of plant origin, preferably from a dicotyledonous plant, further preferably from the family Brasicaceae, most preferably from Arabidopsis thaliana.
10. Plant or part thereof, including seeds, obtainable by a method according to any preceeding item, wherein said plant or part thereof comprises a recombinant nucleic acid encoding an SCE1 polypeptide.
11. An isolated nucleic acid molecule comprising any one of the following:

(i) a nucleic acid represented by SEQ ID NO: 201; SEQ ID NO: 203; SEQ ID NO: 205; SEQ ID NO: 207; SEQ ID NO: 209; SEQ ID NO: 211 and SEQ ID NO: 213;
(ii) a nucleic acid or fragment thereof that is complementary to any one of the SEQ ID NOs given in (i);
(iii) a nucleic acid encoding an SCE1 polypeptide having, in increasing order of preference, at least 70%, 75%, 80%, 85%, 90%, 95%, 96%, 97%, 98%, 99% or more sequence identity to any one of the amino acid sequences given in SEQ ID NO: 202; SEQ ID NO: 204; SEQ ID NO: 206; SEQ ID NO: 208; SEQ ID NO: 210; SEQ ID NO: 212 and SEQ ID NO: 214;
(iv) a nucleic acid capable of hybridizing under stringent conditions to any one of the nucleic acids given in (i), (ii) or (iii) above.

12. An isolated polypeptide comprising:

a. an amino acid sequence having, in increasing order of preference, at least 80%, 85%, 90%, 95%, 96%, 97%, 98%, 99% or 100% sequence identity to any one of the amino acid sequences given in SEQ ID NO: 202; SEQ ID NO: 204; SEQ ID NO: 206; SEQ ID NO: 208; SEQ ID NO: 210; SEQ ID NO: 212 and SEQ ID NO: 214;
b. a nucleic acid capable of hybridizing under derivatives of any of the amino acid sequences given in (i).

13. Construct comprising:

(i) nucleic acid encoding an SCE1 polypeptide as defined in items 1, 2 or 3, or a nucleic acid according to item 11;
(ii) one or more control sequences capable of driving expression of the nucleic acid sequence of (i); and optionally
(iii) a transcription termination sequence.

14. Construct according to item 13, wherein one of said control sequences is a constitutive promoter, preferably a GOS2 promoter, most preferably a GOS2 promoter from rice.
15. Use of a construct according to item 13 or 14 in a method for making plants having increased yield, particularly increased biomass relative to control plants.
16. Plant, plant part or plant cell transformed with a construct according to item 13 or 14.
17. Method for the production of a transgenic plant having increased yield, preferably increased seed yield relative to control plants, comprising:

(i) introducing and expressing in a plant a nucleic acid encoding an SCE1 polypeptide as defined in item 1, 2 or 12, or a nucleic acid according to item 11; and
(ii) cultivating the plant cell under conditions promoting plant growth and development; and optionally
(iii) selecting for plants having enhanced yield-related traits

18. Transgenic plant having increased yield, particularly increased biomass, relative to control plants, resulting from modulated expression of a nucleic acid encoding an SCE1 polypeptide as defined in item 1, 2 or 12 or a transgenic plant cell derived from said transgenic plant.
19. Transgenic plant according to item 10, 16 or 18, or a transgenic plant cell derived thereof, wherein said plant is a crop plant or a monocot or a cereal, such as rice, maize, wheat, barley, millet, rye, triticale, sorghum and oats.
20. Harvestable parts of a plant according to item 19, wherein said harvestable parts are preferably shoot biomass and/or seeds.
21. Products derived from a plant according to item 19 and/or from harvestable parts of a plant according to item 20.
22. Use of a nucleic acid encoding an SCE1 polypeptide in increasing yield, particularly in increasing shoot and/or biomass in plants, relative to control plants.

**Description of figures**

The present invention will now be described with reference to the following figures in which:

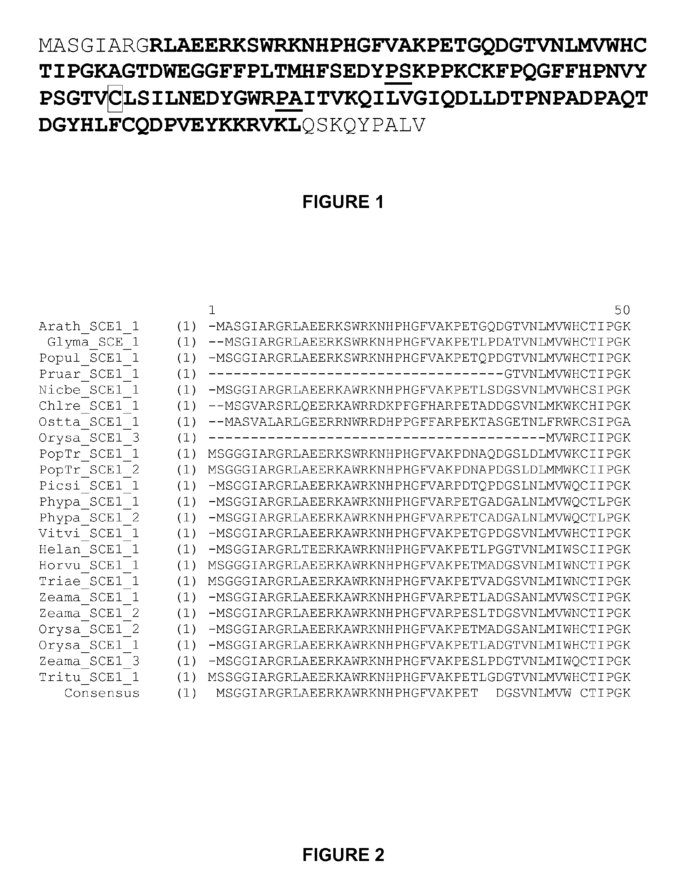

**Figure 1** represents the sequence ofArath_SCE1-1, SEQ ID NO: 200, with conserved UBC domain indicated in bold and the active-site Cysteine amino acid residue boxed. Amino acid residues proposed to interact with the E3 ligase are underlined.

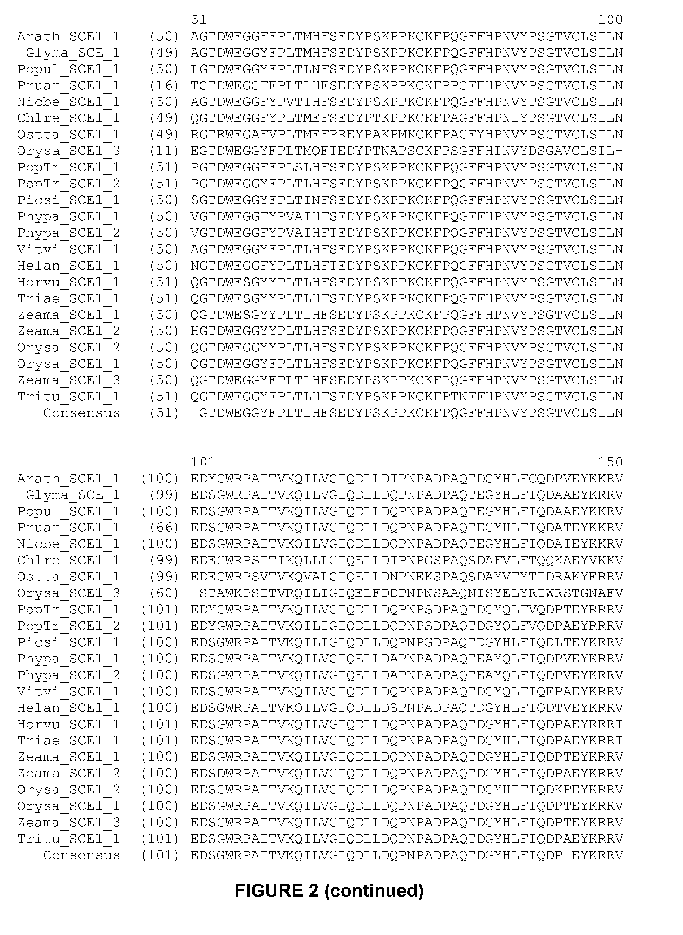

**Figure 2** represents a multiple alignment of the SCE1 polypeptides given in Table A. A consensus sequence is also given. Highly conserved residues are indicated in the consensus sequence.
**Figure 3** represents the binary vector for increased expression in Oryza sativa of an SCE1-encoding nucleic acid under the control of a rice GOS2 promoter (pGOS2).

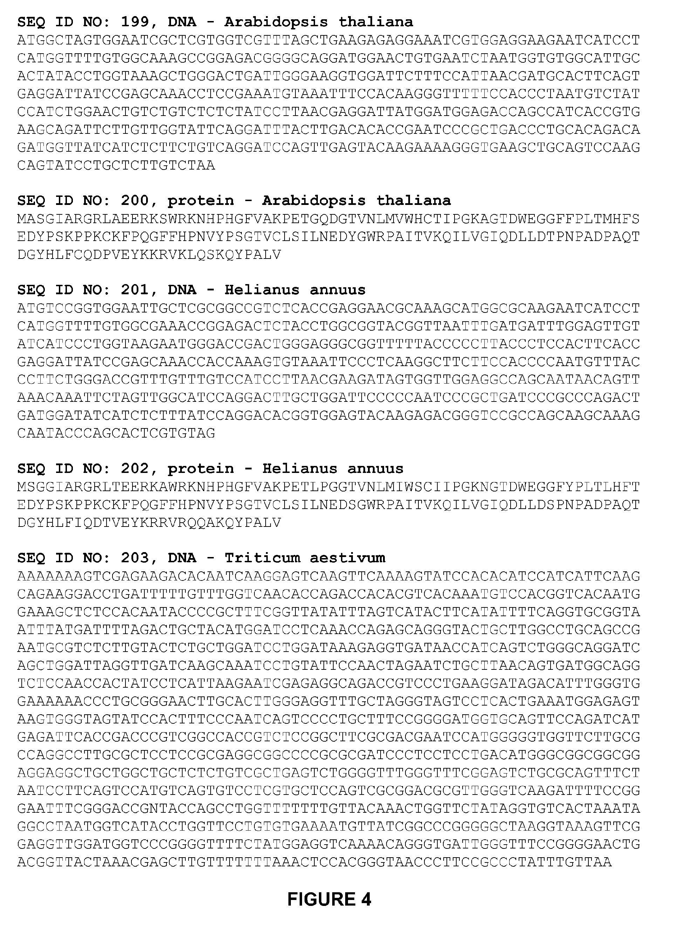

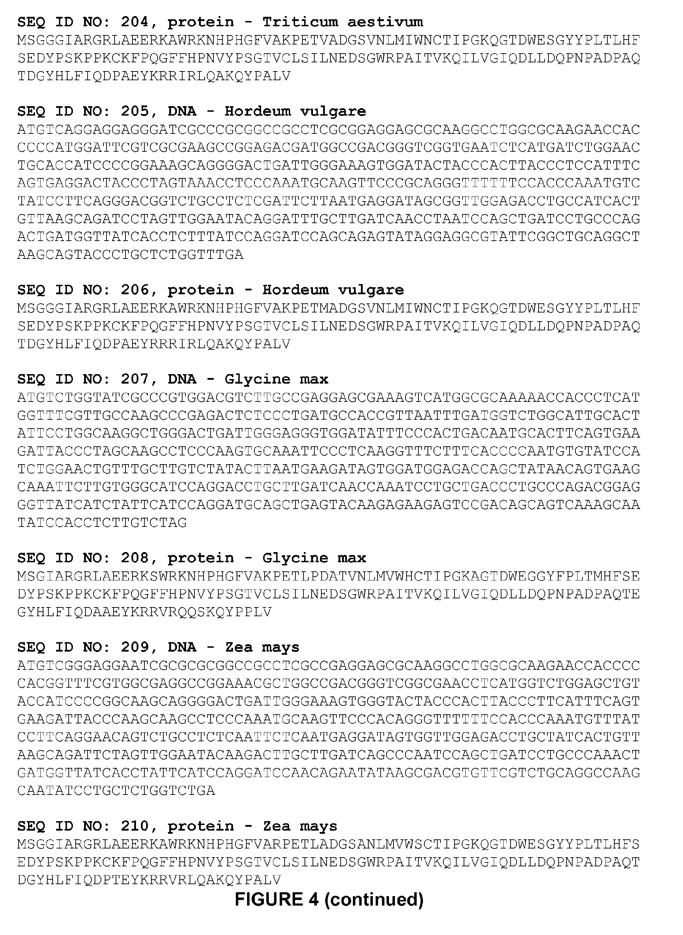

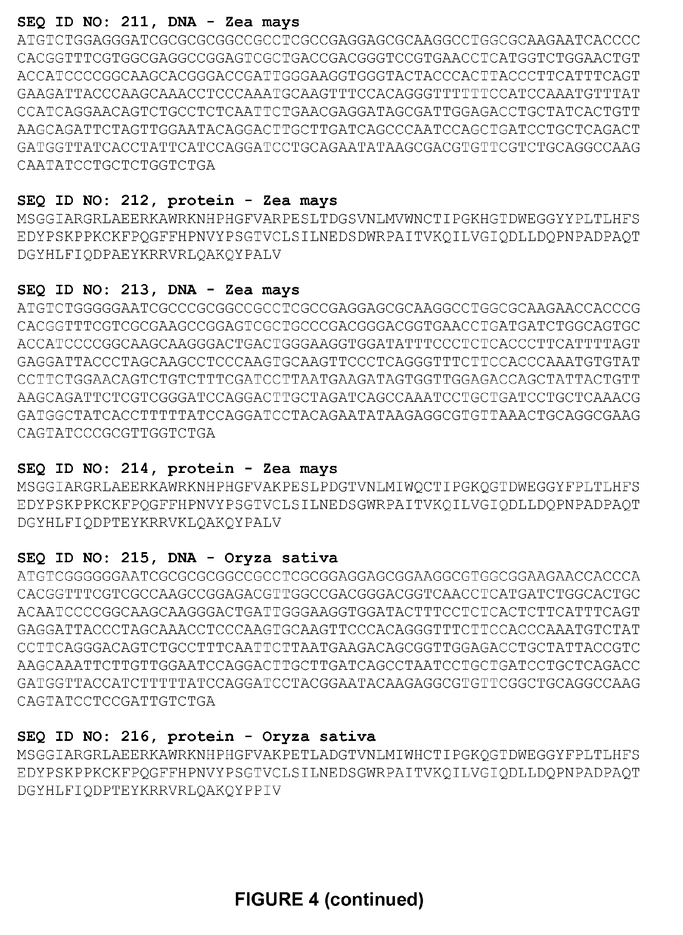

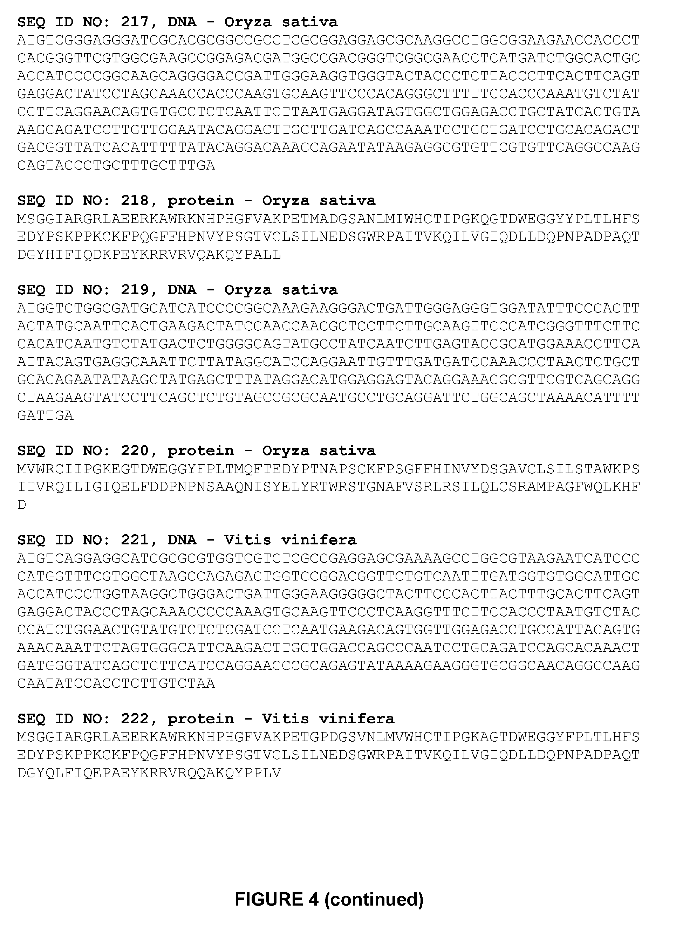

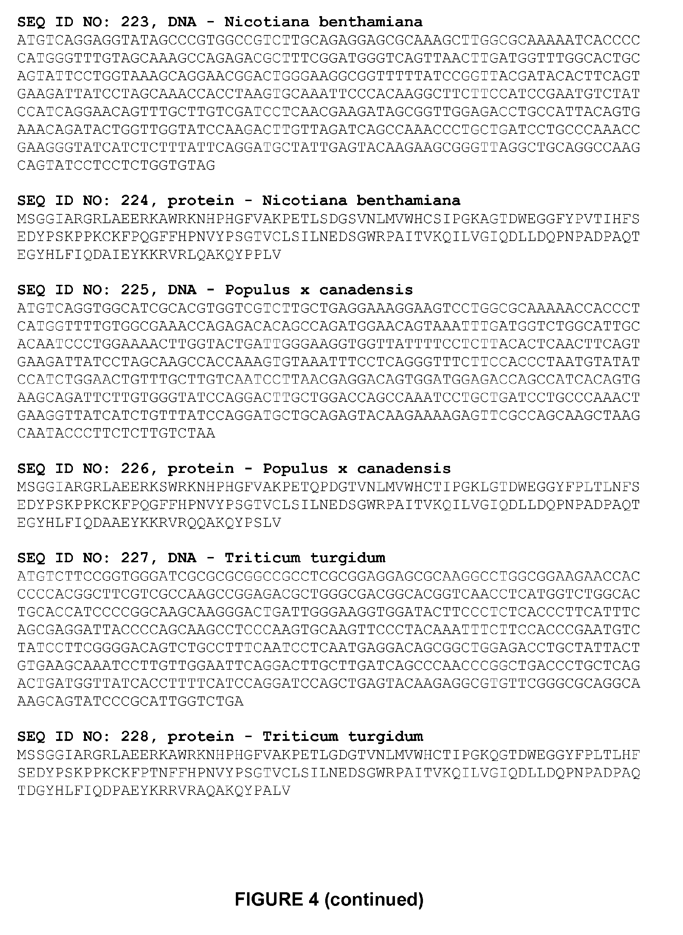

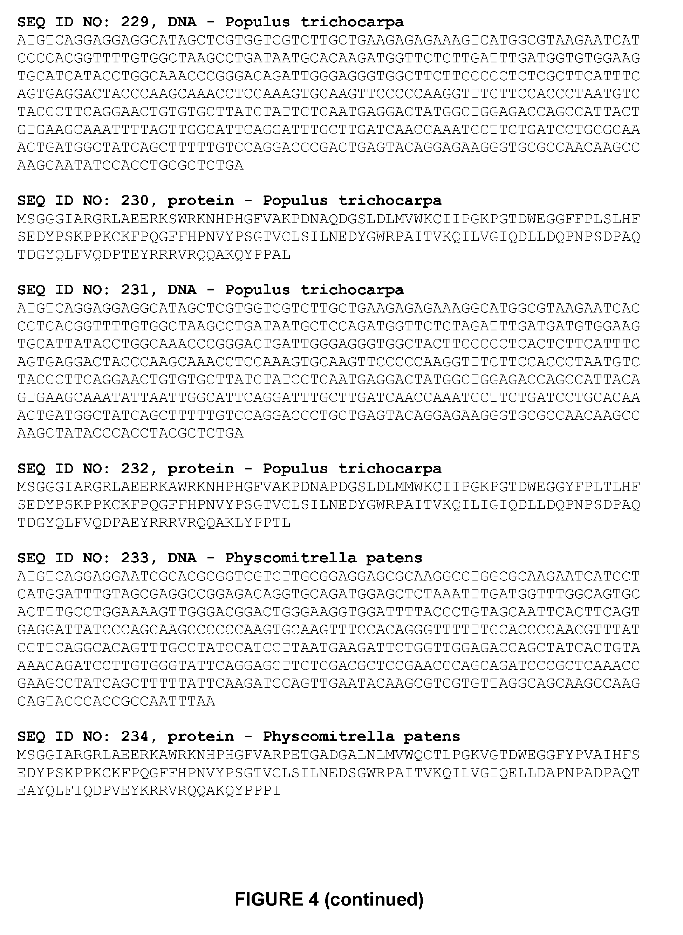

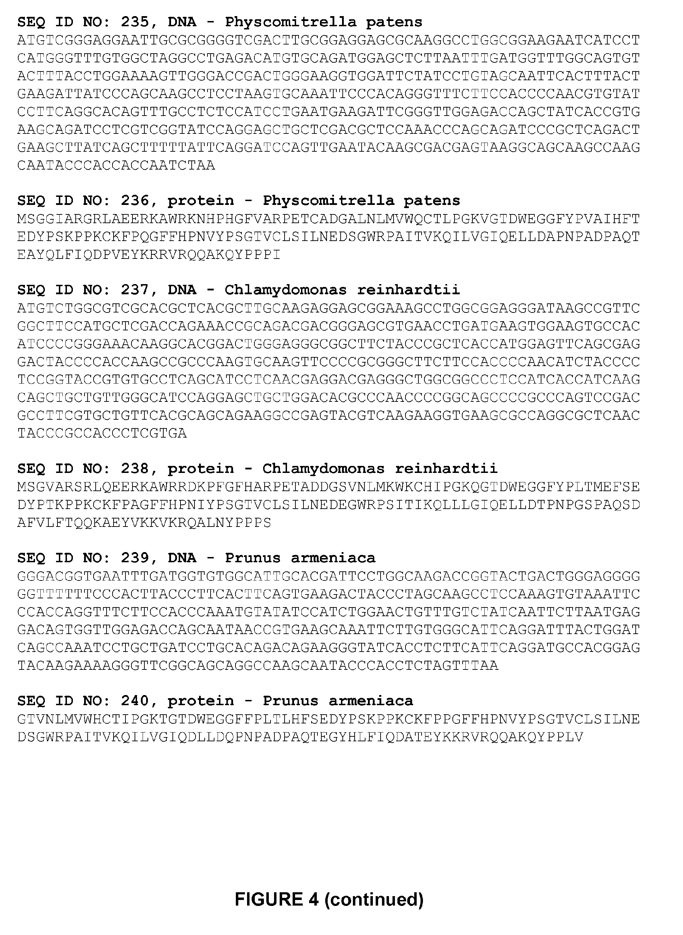

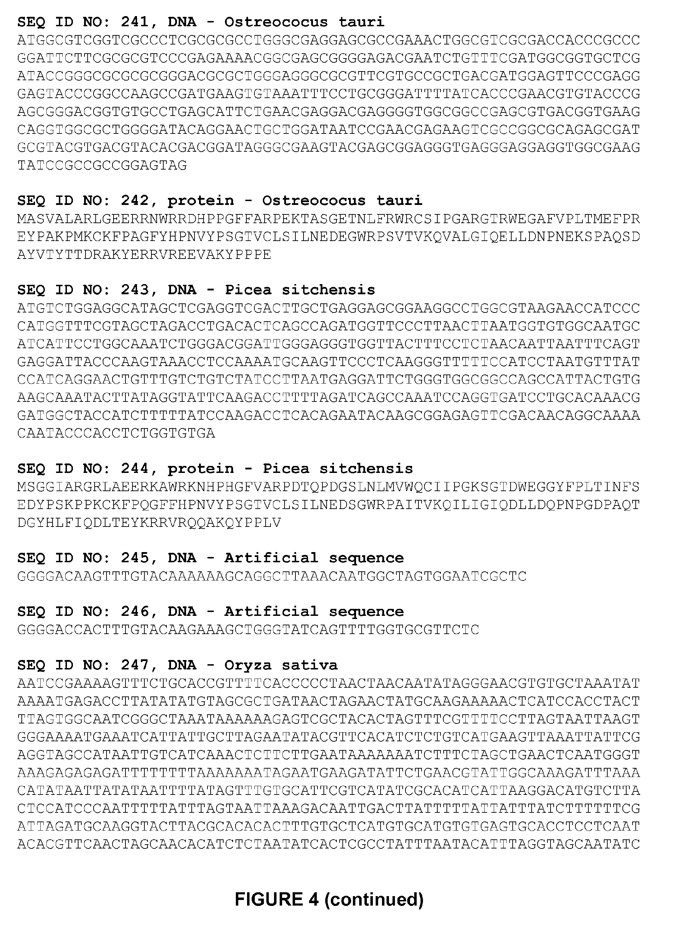

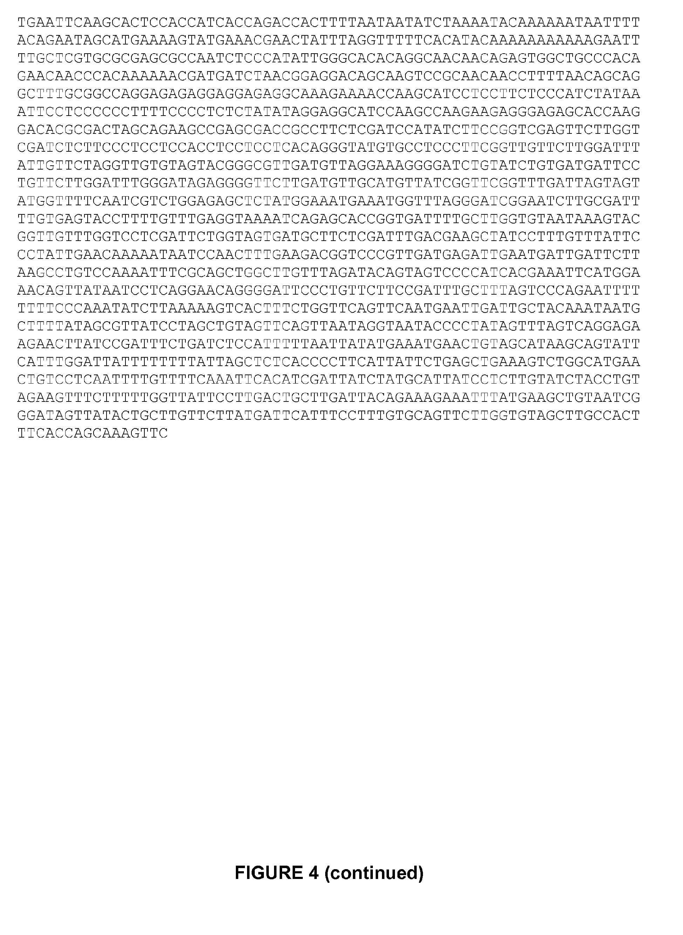

**Figure 4** details examples of SCE1 sequences useful in performing the methods according to the present invention.

**Examples**

The present invention will now be described with reference to the following examples, which are by way of illustration alone. The following examples are not intended to completely define or otherwise limit the scope of the invention.
DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

<u>Example 1: Identification of sequences related to the nucleic acid sequences and the polypeptide sequences used in the methods of the invention</u>

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid used in the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.
Table A provides a list of nucleic acid sequences related to the nucleic acid sequence used in the methods of the present invention.

**Table A:** Examples of SCE1 nucleic acids and polypeptides:

| **Plant Source** | **Origin species** | **Nucleic acid SEQ ID NO:** | **Protein SEQ ID NO:** |
|---|---|---|---|
| Arath_SCE1_1 | *Arabidopsis thaliana* | 199 | 200 |
| Helan_SCE1_1 | *Helianus annuus* | 201 | 202 |
| Triae_SCE1_1 | *Triticum aestivum* | 203 | 204 |
| Horvu_SCE1_1 | *Hordeum vulgare* | 205 | 206 |
| Glyma_SCE_1 | *Glycine max* | 207 | 208 |
| Zeama_SCE1_1 | *Zea mays* | 209 | 210 |
| Zeama_SCE1_2 | *Zea mays* | 211 | 212 |
| Zeama_SCE1_3 | *Zea mays* | 213 | 214 |
| Orysa_SCE1_1 | *Oryza sativa* | 215 | 216 |
| Orysa_SCE1_2 | *Oryza sativa* | 217 | 218 |
| Orysa_SCE1_3 | *Oryza sativa* | 219 | 220 |
| Vitvi_SCE1_1 | *Vitis vinifera* | 221 | 222 |
| Nicbe_SCE1_1 | *Nicotiana benthamiana* | 223 | 224 |
| Popul_SCE1_1 | *Populus x canadensis* | 225 | 226 |
| Tritu_SCE1_1 | *Triticum turgidum* | 227 | 228 |
| PopTr_SCE1_1 | *Populus trichocarpa* | 229 | 230 |
| PopTr_SCE1_2 | *Populus trichocarpa* | 231 | 232 |
| Phypa_SCE1_1 | *Physcomitrlla patens* | 233 | 234 |
| Phypa_SCE1_2 | *Vitis vinifera* | 235 | 236 |
| Chlre_SCE1_1 | *Chlamydomonas reinhardtii* | 237 | 238 |
| Pruar_SCE1_1 | *Prunus armeniaca* | 239 | 240 |
| Ostta_SCE1_1 | *Ostreococus tauri* | 241 | 242 |
| Picsi_SCE1_1 | *Picea sitchensis* | 243 | 244 |

Example 2: Alignment of polypeptide sequences

Alignment of polypeptide sequences was performed using the AlignX programme from the Vector NTI (Invitrogen) which is based on the popular Clustal W algorithm of progressive alignment (Thompson et al. (1997) Nucleic Acids Res 25:4876-4882; Chenna et al. (2003). Nucleic Acids Res 31:3497-3500). Default values are for the gap open penalty of 10, for the gap extension penalty of 0,1 and the selected weight matrix is Blosum 62 (if polypeptides are aligned). Sequence conservation among SCE1 polypeptides shown is highest in the region comprising the UBC domain of the polypeptides. The SCE1 polypeptides are aligned in Figure 7.

Example 3: Calculation of global percentage identity between polypeptide sequences useful in performing the methods of the invention

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

| | |
|---|---|
| Scoring matrix: | Blosum62 |
| First Gap: | 12 |
| Extending gap: | 2 |

Results of the software analysis are shown in Table B for the global similarity and identity over the full length of the polypeptide sequences.

Results of the MatGAT software analysis are shown in Table B for the global similarity and identity over the full length of the polypeptide sequences. Percentage identity is given below the diagonal and percentage similarity is given above the diagonal (normal face).

The percentage identity between the SCE1 polypeptide sequences useful in performing the methods of the invention can be as low as 57.5 % amino acid identity compared to SEQ ID NO: 200.

Table B: MatGAT results for global similarity and identity over the full length of the polypeptide sequences.

| | 1. | 2. | 3. | 4. | 5. | 6. | 7. | 8. | 9. | 10. | 11. | 12. | 13. | 14. | 15. | 16. | 17. | 18. | 19. | 20. | 21. | 22. | 23. |
|---|---|---|---|---|---|---|---|---|---|---|---|---|---|---|---|---|---|---|---|---|---|---|---|
| 1.Glyma SCE1_1 | | 95.6 | 96.9 | 76.7 | 73.6 | 97.5 | 82.4 | 93.8 | 95.6 | 94.4 | 54.1 | 96.9 | 94.4 | 93.1 | 95 | 95.6 | 94.4 | 93.1 | 93.1 | 92.5 | 91.9 | 92.5 | 92.5 |
| 2.Picsi SCE1_1 | 88.1 | | 96.2 | 76.2 | 72.5 | 96.9 | 83.1 | 92.5 | 95.6 | 93.8 | 53.8 | 95.6 | 92.5 | 93.1 | 93.1 | 95.6 | 92.5 | 94.4 | 92.5 | 93.8 | 94.4 | 93.1 | 93.1 |
| 4.Popul SCE1_1 | 94.4 | 90 | | 76.2 | 73.1 | 97.5 | 81.9 | 95 | 95.6 | 95.6 | 53.8 | 96.2 | 95.7 | 94.4 | 96.2 | 96.9 | 95.7 | 94.4 | 93.8 | 93.2 | 92.5 | 93.8 | 93.8 |
| 5.Pruar SCE1_1 | 74.2 | 70 | 75 | | 59.1 | 76.2 | 66 | 73.9 | 76.2 | 74.4 | 63.9 | 76.2 | 73.9 | 73.8 | 74.4 | 75.6 | 73.9 | 73.8 | 73.1 | 72.7 | 73.9 | 73.1 | 73.1 |
| 6.Ostta SCE1_1 | 57.9 | 57.5 | 56.2 | 47.2 | | 73.1 | 77.4 | 72 | 73.1 | 73.1 | 51.6 | 72.5 | 72 | 71.9 | 71.2 | 72.5 | 71.4 | 71.9 | 72.5 | 71.4 | 72 | 74.4 | 74.4 |
| 5.Vitvi SCE1_1 | 93.1 | 90.6 | 93.1 | 72.5 | 57.5 | | 83.1 | 95 | 96.9 | 95 | 54.4 | 96.9 | 95.7 | 94.4 | 96.2 | 96.9 | 95.7 | 93.8 | 95 | 94.4 | 93.8 | 95.6 | 95 |
| 7.Chlre SCE1_1 | 67.9 | 69.4 | 68.1 | 56 | 64.8 | 70 | | 80.7 | 81.9 | 81.2 | 58.5 | 81.2 | 80.7 | 80 | 81.2 | 81.9 | 80.7 | 81.2 | 81.2 | 82.6 | 82 | 83.1 | 83.1 |
| 8.Tritu SCE1_1 | 91.3 | 87.6 | 91.9 | 72 | 57.1 | 93.2 | 68.3 | | 95.7 | 95 | 55.3 | 94.4 | 96.3 | 94.4 | 96.3 | 96.3 | 96.3 | 91.9 | 93.2 | 91.3 | 91.3 | 91.3 | 91.3 |
| 9.Orysa SCE1_1 | 91.9 | 89.4 | 91.9 | 73.1 | 58.1 | 93.8 | 69.4 | 94.4 | | 97.5 | 55 | 98.1 | 97.5 | 97.5 | 97.5 | 98.8 | 97.5 | 93.1 | 94.4 | 92.5 | 93.8 | 94.4 | 94.4 |
| 10.Orysa SCE1_2 | 88.1 | 87.5 | 89.4 | 69.4 | 57.5 | 91.2 | 69.4 | 91.3 | 93.1 | | 56.9 | 96.9 | 96.9 | 97.5 | 96.9 | 97.5 | 96.9 | 93.1 | 93.8 | 90.7 | 91.3 | 93.1 | 93.1 |
| 11.Orysa SCE1_3 | 40.5 | 40.2 | 39.1 | 47.9 | 35.3 | 39.7 | 38.2 | 38.9 | 39.7 | 38.5 | | 55 | 55.3 | 55.6 | 55.6 | 56.9 | 55.3 | 56.2 | 54.4 | 55.3 | 55.3 | 53.8 | 53.8 |
| 12.Nicbe SCE1_1 | 91.2 | 88.1 | 91.2 | 73.1 | 56.9 | 91.2 | 70 | 89.4 | 91.2 | 89.4 | 38.5 | | 96.3 | 95.6 | 96.9 | 96.9 | 96.3 | 93.8 | 95 | 91.9 | 92.5 | 95 | 95 |
| 13.Triae SCE1_1 | 88.2 | 87 | 89.4 | 68.9 | 56.5 | 91.9 | 68.9 | 92.5 | 93.8 | 93.2 | 38.9 | 89.4 | | 98.1 | 97.5 | 96.9 | 100 | 93.2 | 93.8 | 92.5 | 92.5 | 92.5 | 92.5 |
| 14.Zeama | 88.8 | 90 | 89.4 | 70.6 | 57.5 | 91.9 | 69.4 | 92.5 | 94.4 | 93.8 | 39.7 | 90.6 | 95 | | 96.9 | 97.5 | 98.1 | 93.1 | 93.1 | 90.7 | 91.9 | 92.5 | 92.5 |

(continued)

| | 1. | 2. | 3. | 4. | 5. | 6. | 7. | 8. | 9. | 10. | 11. | 12. | 13. | 14. | 15. | 16. | 17. | 18. | 19. | 20. | 21. | 22. | 23. |
|---|---|---|---|---|---|---|---|---|---|---|---|---|---|---|---|---|---|---|---|---|---|---|---|
| SCE1_1 | | | | | | | | | | | | | | | | | | | | | | | |
| 15.Zeama SCE1_1 | 89.4 | 88.8 | 90 | 70.6 | 57.5 | 92.5 | 69.4 | 92.5 | 92.5 | 91.2 | 40.2 | 90.6 | 93.8 | 95 | | 97.5 | 97.5 | 94.4 | 94.4 | 92.5 | 92.5 | 92.5 | 92.5 |
| 16.Zeama SCE1_1 | 90.6 | 89.4 | 91.2 | 71.9 | 56.2 | 92.5 | 68.8 | 93.8 | 96.2 | 91.9 | 39.7 | 89.4 | 93.2 | 93.8 | 93.8 | | 96.9 | 94.4 | 95 | 93.8 | 94.4 | 93.1 | 93.1 |
| 17.Horvu SCE1_1 | 87.6 | 86.3 | 88.8 | 68.3 | 56.5 | 91.3 | 68.9 | 91.9 | 93.2 | 93.2 | 38.9 | 88.8 | 98.8 | 94.4 | 93.2 | 92.5 | | 93.2 | 93.8 | 92.5 | 92.5 | 92.5 | 92.5 |
| 18.Helan SCE1_1 | 88.1 | 86.9 | 88.8 | 70.6 | 57.5 | 89.4 | 68.1 | 89.4 | 90.6 | 89.4 | 40.2 | 88.8 | 89.4 | 90 | 89.4 | 91.2 | 88.8 | | 92.5 | 91.3 | 91.3 | 91.9 | 91.9 |
| 19.Arath SCE1_1 | 90.6 | 83.1 | 88.8 | 70.6 | 58.1 | 88.8 | 70 | 88.8 | 88.8 | 85.6 | 40.8 | 88.1 | 86.3 | 86.9 | 86.9 | 89.4 | 85.7 | 86.2 | | 90.7 | 91.9 | 92.5 | 91.9 |
| 20.PopTr SCE1_1 | 83.2 | 87.6 | 83.9 | 64.6 | 56.5 | 88.2 | 68.3 | 84.5 | 85.7 | 84.5 | 40 | 82 | 85.1 | 83.9 | 84.5 | 85.1 | 85.7 | 82.6 | 80.1 | | 97.5 | 91.9 | 91.9 |
| 21.PopTr SCE1_2 | 83.2 | 86.3 | 83.9 | 67.1 | 55.9 | 87 | 67.1 | 83.9 | 85.7 | 83.9 | 39.4 | 83.2 | 83.9 | 84.5 | 83.9 | 84.5 | 84.5 | 82.6 | 83.2 | 94.4 | | 93.2 | 93.2 |
| 22.Phypa SCE1_1 | 84.4 | 85.6 | 84.4 | 66.9 | 58.8 | 87.5 | 69.4 | 83.9 | 86.2 | 85 | 37.4 | 87.5 | 84.5 | 86.9 | 85.6 | 84.4 | 83.9 | 84.4 | 82.5 | 80.7 | 82.6 | | 99.4 |
| 23.Phypa SCE1_2 | 83.8 | 85 | 83.8 | 66.2 | 58.8 | 86.2 | 68.8 | 83.2 | 85.6 | 84.4 | 37.9 | 86.9 | 83.9 | 86.2 | 85 | 83.8 | 83.2 | 85 | 81.2 | 80.1 | 82 | 98.8 | |

Example 4: Identification of domains comprised in polypeptide sequences useful in performing the methods of the invention

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. InterPro is hosted at the European Bioinformatics Institute in the United Kingdom.

The results of the InterPro scan of the SCE1 polypeptides sequence as represented by SEQ ID NO: 200 by SEQ ID NO: 216 are presented in Table C.

Table C: InterPro scan results (major accession numbers) of the polypeptide sequence represented by SEQ ID NO: 200.

| query sequence | Intepro accession | Accession | Description | Description Alias | Short name | e (E) value | Start | End | Method |
|---|---|---|---|---|---|---|---|---|---|
| Arath SCE1_1 | IPR000608 | PD000461 | Ubiquitin-conjugating enzyme, E2 | UBC | UBQ_conjugat | 7,00E-92 | 5 | 156 | BlastProDom |
| Arath SCE1_1 | IPR000608 | PF00179 | Ubiquitin-conjugating enzyme, E2 | UBC | UQ_con | 3.3E-70 | 9 | 153 | HMMPfam |
| Arath SCE1_1 | IPR000608 | SM00212 | Ubiquitin-conjugating enzyme, E2 | UBC | UBCc | 1,00E-67 | 8 | 158 | HMMSmart |
| Arath SCE1_1 | IPR000608 | PS00183 | Ubiquitin-conjugating enzyme, E2 | UBC | UBIQUITIN_ CONJUGAT_1 | 0 | 83 | 97 | ProfileScan |
| Arath SCE1_1 | IPR000608 | PS50127 | Ubiquitin-conjugating enzyme, E2 | UBC | UBIQUITIN_ CONJUGAT_2 | 35.839 | 8 | 147 | ProfileScan |
| Orysa SCE1_1 | IPR000608 | PD000461 | Ubiquitin-conjugating enzyme, E2 | UBC | UBQ_conjugat | 8,00E-91 | 5 | 156 | BlastProDom |
| Orysa SCE1_1 | IPR000608 | PF00179 | Ubiquitin-conjugating enzyme, E2 | UBC | UQ_con | 9.3E-68 | 9 | 153 | HMMPfam |
| Orysa SCE1_1 | IPR000608 | SM00212 | Ubiquitin-conjugating enzyme, E2 | UBC | UBCc | 4.7E-66 | 8 | 158 | HMMSmart |
| Orysa SCE1_1 | IPR000608 | PS00183 | Ubiquitin-conjugating enzyme, E2 | UBC | UBIQUITIN_ CONJUGAT_1 | 0 | 83 | 97 | ProfileScan |
| Orysa SCE1_1 | IPR000608 | PS50127 | Ubiquitin-conjugating enzyme, E2 | UBC | UBIQUITIN _CONJUGAT_2 | 35.707 | 8 | 147 | ProfileScan |
| Orysa SCE1_2 | IPR000608 | PD000461 | Ubiquitin-conjugating enzyme, E2 | UBC | UBQ_conjugat | 6,00E-91 | 5 | 156 | BlastProDom |
| Orysa SCE1_2 | IPR000608 | PF00179 | Ubiquitin-conjugating enzyme, E2 | UBC | UQ_con | 1.1E-65 | 9 | 151 | HMMPfam |
| Orysa SCE1_2 | IPR000608 | SM00212 | Ubiquitin-conjugating enzyme, E2 | UBC | UBCc | 2.7E-64 | 8 | 158 | HMMSmart |
| Orysa SCE1_2 | IPR000608 | PS00183 | Ubiquitin-conjugating enzyme, E2 | UBC | UBIQUITIN_ CONJUGAT_1 | 0 | 83 | 97 | ProfileScan |

(continued)

| query sequence | Intepro accession | Accession | Description | Description Alias | Short name | e (E) value | Start | End | Method |
|---|---|---|---|---|---|---|---|---|---|
| Orysa SCE1_2 | IPR000608 | PS50127 | Ubiquitin-conjugating enzyme, E2 | UBC | UBIQUITIN_ CONJUGAT_2 | 35.76 | 8 | 147 | ProfileScan |
| Orysa SCE1_3 | IPR000608 | PD000461 | Ubiquitin-conjugating enzyme, E2 | UBC | Q8H8G9_EEEEE_ Q8H8G9; | 2,00E-36 | 1 | 97 | BlastProDom |
| Orysa SCE1_3 | IPR000608 | PF00179.15 | Ubiquitin-conjugating enzyme, E2 | UBC | Ubiquitin-conjugating enzyme | 2,00E-29 | 1 | 115 | HMMPfam |
| Orysa SCE1_3 | IPR000608 | SM00212 | Ubiquitin-conjugating enzyme, E2 | UBC | no description | 2.8E-24 | 1 | 120 | HMMSmart |
| Orysa SCE1_3 | IPR000608 | PS50127 | Ubiquitin-conjugating enzyme, E2 | UBC | UBIQUITIN_ CONJUGAT_2 | 26.416 | 1 | 106 | ProfileScan |

Example 5: Topology prediction of the polypeptide sequences useful in performing the methods of the invention

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no). The "plant" organism group is selected, no cutoffs defined, and the predicted length of the transit peptide requested.

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark

Example 6: Functional assays for the relevant sequences

Activity of SCE1 nucleic acids and SCE1 polypeptide is assayed by methods well known in the art (Castillo et al. 2004; Bernier-Villamor et al. (2002); Lois et al 2003).

*In vivo* functional activity of a Arath_SCE1_1 nucleic acid is analysed by complementation of the S. cerevisiae ubc9-2 mutant (YW098) essentially as described by Castillo et al. 2004. Briefly transformants of the temperature sensitive mutant (YWO98) harboring the SCE1 nucleic acid are streaked on selective plates and are incubated at 25 and 37°C in the absence or presence of doxycycline (10 _g/ml). Proliferation of yeast in the plates is recorded after at 3-10 days incubation.

*in vitro* the activity of Arath_SCE1_1 polypeptide is assayed essentially as described by Lois et al. 2003. SUMO conjugation is assayed with RanGAP1 peptide (amino acids 420 to 589) as described by Bernier-Villamor et al. (2002). Briefly, reactions mixtures are prepare to contain 2 μM glutathione S-transferase (GST)-RanGAP1, 0.3 μM human E1, 0.3 μM HsUBC9 or 3 μM AtSCE1a, and 8 μM HsSUMO1 in the reaction buffer (1 mM ATP, 50 mM NaCl, 20 mM Hepes, pH 7.5, 0.1% Tween 20, 5 mM $MgCl_2$, and 0.1 mM DTT). After incubation at 37°C for 4 h, reactions are stopped by the addition of protein-loading buffer and the mixture is boiled for 5 min. Three microliters of each reaction mixture is resolved by SDS-PAGE and transferred to polyvinylidene difluoride membranes (Immobilon-P; Millipore, Bedford, MA), and SUMO conjugation to GST-RanGAP is examined by protein gel blot analysis using anti-HsSUMO1 polyclonal antibody (diluted 1:1000; Alexis, San Diego, CA).

Example 7: Cloning of the nucleic acid sequence used in the methods of the invention

The nucleic acid sequence used in the methods of the invention was amplified by PCR using as template a custom-made Arabidopsis thaliana seedlings cDNA library (in pCMV Sport 6.0; Invitrogen, Paisley, UK). PCR was performed using Hifi Taq DNA polymerase in standard conditions, using 200 ng of template in a 50 μl PCR mix. The primers used were: 5'-ggggacaagtttgtacaaaaaagcaggcttaaacaatggctagtggaatcgctc-3' (SEQ ID NO: 245); and 5'-ggggaccactttgtacaagaaagctgggtatcagttttggtgcgttctc-3' (SEQ ID NO: 246) which include the AttB sites for Gateway recombination. The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone", pArath_SCE1_1. Plasmid pDONR201 was purchased from Invitrogen, as part of the Gateway® technology.

The entry clone comprising SEQ ID NO: 199 was then used in an LR reaction with a destination vector used for *Oryza sativa* transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 247) for constitutive specific expression was located upstream of this Gateway cassette.

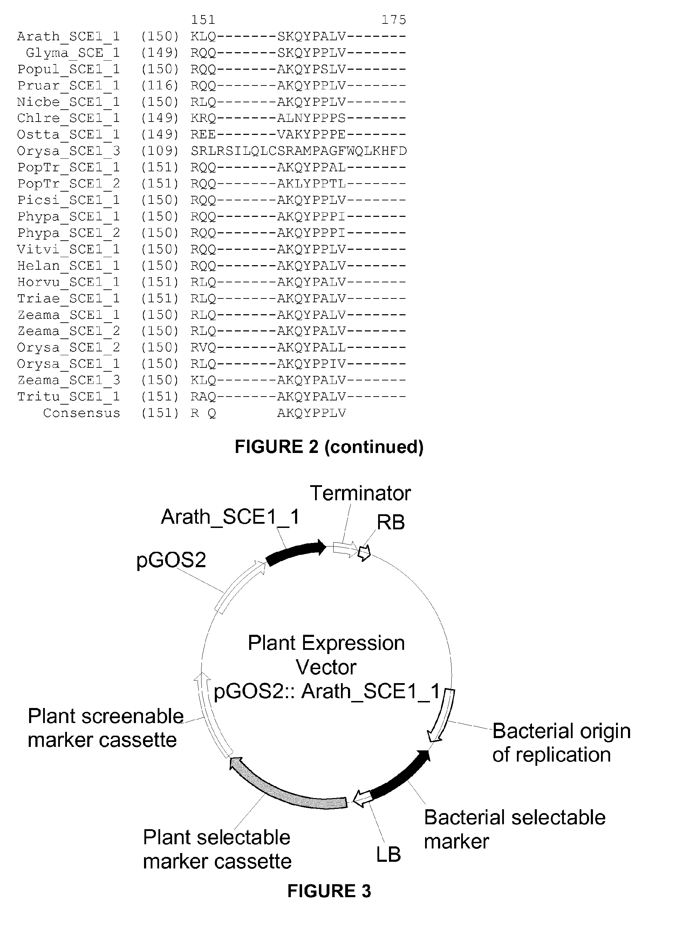

After the LR recombination step, the resulting expression vector pGOS2::Arath_SCE1_1 (Figure 3) was transformed into *Agrobacterium* strain LBA4044 according to methods well known in the art.

Example 8: Plant transformation

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2% $HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

*Corn transformation*

Transformation of maize *(Zea mays)* is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa *(Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

*Cotton transformation*

Cotton is transformed using *Agrobacterium tumefaciens* according to the method described in US 5,159,135. Cotton seeds are surface sterilised in 3% sodium hypochlorite solution during 20 minutes and washed in distilled water with 500 μg/ml cefotaxime. The seeds are then transferred to SH-medium with 50μg/ml benomyl for germination. Hypocotyls of 4 to 6 days old seedlings are removed, cut into 0.5 cm pieces and are placed on 0.8% agar. An *Agrobacterium* suspension (approx. 108 cells per ml, diluted from an overnight culture transformed with the gene of interest and suitable selection markers) is used for inoculation of the hypocotyl explants. After 3 days at room temperature and lighting, the tissues are transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 μg/ml MgCL2, and with 50 to 100 μg/ml cefotaxime and 400-500 μg/ml carbenicillin to kill residual bacteria. Individual cell lines are isolated after two to three months (with subcultures every four to six weeks) and are further cultivated on selective medium for tissue amplification (30°C, 16 hr photoperiod). Transformed tissues are subsequently further cultivated on non-selective medium during 2 to 3 months to give rise to somatic embryos. Healthy looking embryos of at least 4 mm length are transferred to tubes with SH medium in fine vermiculite, supplemented with 0.1 mg/l indole acetic acid, 6 furfurylaminopurine and gibberellic acid. The embryos are cultivated at 30°C with a photoperiod of 16 hrs, and plantlets at the 2 to 3 leaf stage are transferred to pots with vermiculite and nutrients. The plants are hardened and subsequently moved to the greenhouse for further cultivation.

Example 9: Phenotypic evaluation procedure

9.1 Evaluation setup

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions are watered at regular intervals to ensure that water and nutrients are not limiting to satisfy plant needs to complete growth and development.

Four T1 events were further evaluated in the T2 generation following the same evaluation procedure as for the T1 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

*Drought screen*

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC is below certain thresholds, the plants are automatically re-watered continuously until a normal level is reached again. The plants are then retransferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds were grown in potting soil under normal conditions except for the nutrient solution. The pots were watered from transplantation to maturation with a specific nutrient solution containing reduced N nitrogen (N) content, usually between 7 to 8 times less. The rest of the cultivation (plant maturation, seed harvest) was the same as for plants not grown under abiotic stress. Growth and yield parameters are recorded as detailed for growth under normal conditions.

*Salt stress screen*

Plants are grown on a substrate made of coco fibers and argex (3 to 1 ratio). A normal nutrient solution is used during the first two weeks after transplanting the plantlets in the greenhouse. After the first two weeks, 25 mM of salt (NaCl) is added to the nutrient solution, until the plants are harvested. Seed-related parameters are then measured.

9.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

Because two experiments with overlapping events were carried out, a combined analysis was performed. This is useful to check consistency of the effects over the two experiments, and if this is the case, to accumulate evidence from both experiments in order to increase confidence in the conclusion. The method used was a mixed-model approach that takes into account the multilevel structure of the data (i.e. experiment - event - segregants). P values were obtained by comparing likelihood ratio test to chi square distributions.

9.3. Parameters measured

*Biomass-related parameter measurement*

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

Early vigour was determined by counting the total number of pixels from aboveground plant parts discriminated from the background. This value was averaged for the pictures taken on the same time point from different angles and was converted to a physical surface value expressed in square mm by calibration. The results described below are for plants three weeks post-germination.

*Seed-related parameter measurements*

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

Example 10: Results of the phenotypic evaluation of the transgenic plants

Example 10.1: SCE1 sequences

The results of the evaluation of transgenic rice plants expressing an Arath_SCE1_1 nucleic acid under the non-stress conditions screen (YS: yield screen) and under nitrogen use deficiency screen (NUE) are presented below. In the YS screen, an increase of at least 5 % was observed for aboveground biomass (AreaMax), and root biomass ( RootMax) in the transgenic plants with respect of their corresponding nullyzygous control plants (Table D1). In the NUE screen an increase of at least 5 % was observed for aboveground biomass (AreaMax), early vigour (EmerVigor), number of first panicles (firstpan) and total number of seeds per plant (nrtotalseed), in the transgenic plants with respect of their corresponding nullyzygous control plants (Table D2).

**Table D1:** Results evaluation in YS: yield screen.

| Parameter | % increase in transgenic plants versus the nullizygous |
|---|---|
| AreaMax | 13.3 |
| RootMax | 8 |

**Table D2:** Results evaluation in NUE screen.

| Parameter | % increase in transgenic plants versus the nullizygous |
|---|---|
| AreaMax | 17.8 |
| EmerVigor | 22.8 |
| firstpan | 7.5 |
| nrtotalseed | 16 |

## Claims

1. A method for enhancing yield-related traits in plants relative to control plants, comprising modulating expression in a plant of a nucleic acid encoding an SCE1 (SUMO Conjugating Enzyme 1) polypeptide and optionally selecting for plants having enhanced yield-related traits.

**2.** Method according to claim 2, wherein said SCE1 polypeptide comprises a sequence having at least one of the following:

(i) 50%, 55%, 60%, 65%, 70%, 75%, 80%, 85%, 90%, 95%, 96%, 97%, 98%, 99% or more sequence identity to the amino acid sequence of any of the polypeptides of Table A;
(ii) 50%, 55%, 60%, 65%, 70%, 75%, 80%, 85%, 90%, 95%, 96%, 97%, 98%, 99% or more sequence identity to the amino acid sequence of any of the UBC domains as set forth in Table C of Example 4.

**3.** Method according to claim 1 or 2, wherein said modulated expression is effected by introducing and expressing in a plant a nucleic acid encoding an SCE1 polypeptide.

**4.** Method according to any one of claims 1 to 3, wherein said nucleic acid encoding a SCE1 polypeptide encodes any one of the proteins selected from the group consisting of: SEQ ID NO: 200, 216, 202, 204, 206, 208, 210, 212, 214, 218, 220, 222, 224, 226, 228, 230, 232, 234, 236, 238, 240, 242 and 244 or is a portion of such a nucleic acid, or a nucleic acid capable of hybridising with such a nucleic acid, or encodes an orthologue or paralogue of any of the said proteins.

**5.** Method according to any one of claims 1 to 4, wherein said enhanced yield-related traits comprise increased biomass, preferably shoot and/or root biomass relative to control plants.

**6.** Method according to any one of claims 1 to 5, wherein said enhanced yield-related traits are obtained under conditions of nitrogen deficiency.

**7.** Method according to any one of claims 3 to 6, wherein said nucleic acid is operably linked to a constitutive promoter, preferably to a GOS2 promoter, most preferably to a GOS2 promoter from rice.

**8.** Plant or part thereof, including seeds, obtainable by a method according to any preceding claim, wherein said plant or part thereof comprises a recombinant nucleic acid encoding an SCE1 polypeptide.

**9.** An isolated nucleic acid molecule comprising any one of the following:

(i) a nucleic acid represented by SEQ ID NO: 201; SEQ ID NO: 203; SEQ ID NO: 205; SEQ ID NO: 207; SEQ ID NO: 209; SEQ ID NO: 211 and SEQ ID NO: 213;
(ii) a nucleic acid or fragment thereof that is complementary to any one of the SEQ ID NOs given in (i);
(iii) a nucleic acid encoding an SCE1 polypeptide having, in increasing order of preference, at least 70%, 75%, 80%, 85%, 90%, 95%, 96%, 97%, 98%, 99% or more sequence identity to any one of the amino acid sequences given in SEQ ID NO: 202; SEQ ID NO: 204; SEQ ID NO: 206; SEQ ID NO: 208; SEQ ID NO: 210; SEQ ID NO: 212 and SEQ ID NO: 214;
(iv) a nucleic acid capable of hybridizing under stringent conditions to any one of the nucleic acids given in (i), (ii) or (iii) above.

**10.** An isolated polypeptide comprising:

(i) an amino acid sequence having, in increasing order of preference, at least 80%, 85%, 90%, 95%, 96%, 97%, 98%, 99% or 100% sequence identity to any one of the amino acid sequences given in SEQ ID NO: 202; SEQ ID NO: 204; SEQ ID NO: 206; SEQ ID NO: 208; SEQ ID NO: 210; SEQ ID NO: 212 and SEQ ID NO: 214;
(ii) a nucleic acid capable of hybridizing under derivatives of any of the amino acid sequences given in (i).

**11.** Construct comprising:

(i) nucleic acid encoding an SCE1 polypeptide as defined in claims 1 to 4, or a nucleic acid according to claim 9;
(ii) one or more control sequences capable of driving expression of the nucleic acid sequence of (a); and optionally
(iii) a transcription termination sequence;

wherein optionally one of said control sequences is a constitutive promoter, preferably a GOS2 promoter, most preferably a GOS2 promoter from rice.

**12.** Use of a construct according to claim 11 in a method for making plants having increased yield, particularly increased biomass relative to control plants.

**13.** Plant, plant part or plant cell transformed with a construct according to claim 11.

**14.** Method for the production of a transgenic plant having increased yield, preferably increased seed yield relative to control plants, comprising:

(i) introducing and expressing in a plant a nucleic acid encoding an SCE1 polypeptide as defined in claims 1 to 4 or 10, or a nucleic acid according to claim 9; and
(ii) cultivating the plant cell under conditions promoting plant growth and development; and optionally
(iii) selecting for plants having enhanced yield-related traits.

**15.** Transgenic plant having increased yield, particularly increased biomass, relative to control plants, resulting from modulated expression of a nucleic acid encoding an SCE1 polypeptide as defined in claims 1 to 4 or 10 or a transgenic plant cell derived from said transgenic plant.

**16.** Transgenic plant according to claims 8, 13 or 15, or a transgenic plant cell derived thereof, wherein said plant is a crop plant or a monocot or a cereal, such as rice, maize, wheat, barley, millet, rye, triticale, sorghum and oats.

**17.** Harvestable parts of a plant according to claim 16, wherein said harvestable parts are preferably shoot biomass and/or seeds.

**18.** Products derived from a plant according to claim 16 and/or from harvestable parts of a plant according to claim 17.

**19.** Use of a nucleic acid encoding an SCE1 polypeptide in increasing yield, particularly in increasing shoot and/or biomass in plants, relative to control plants.

**20.** Use of a nucleic acid sequence encoding a SCE1-like polypeptide, or use of a SCE1-like polypeptide as a molecular marker.

MASGIARG**RLAEERKSWRKNHPHGFVAKPETGQDGTVNLMVWHC**
**TIPGKAGTDWEGGFFPLTMHFSEDYPSKPPKCKFPQGFFHPNVY**
**PSGTVCLSILNEDYGWRPAITVKQILVGIQDLLDTPNPADPAQT**
**DGYHLFCQDPVEYKKRVKL**QSKQYPALV

FIGURE 1

```
                             1                                                50
Arath_SCE1_1         (1)  -MASGIARGRLAEERKSWRKNHPHGFVAKPETGQDGTVNLMVWHCTIPGK
 Glyma_SCE_1         (1)  --MSGIARGRLAEERKSWRKNHPHGFVAKPETLPDATVNLMVWHCTIPGK
Popul_SCE1_1         (1)  -MSGGIARGRLAEERKSWRKNHPHGFVAKPETQPDGTVNLMVWHCTIPGK
Pruar_SCE1_1         (1)  -----------------------------------GTVNLMVWHCTIPGK
Nicbe_SCE1_1         (1)  -MSGGIARGRLAEERKAWRKNHPHGFVAKPETLSDGSVNLMVWHCSIPGK
Chlre_SCE1_1         (1)  --MSGVARSRLQEERKAWRRDKPFGFHARPETADDGSVNLMKWKCHIPGK
Ostta_SCE1_1         (1)  --MASVALARLGEERRNWRRDHPPGFFARPEKTASGETNLFRWRCSIPGA
Orysa_SCE1_3         (1)  ----------------------------------------MVWRCIIPGK
PopTr_SCE1_1         (1)  MSGGGIARGRLAEERKSWRKNHPHGFVAKPDNAQDGSLDLMVWKCIIPGK
PopTr_SCE1_2         (1)  MSGGGIARGRLAEERKAWRKNHPHGFVAKPDNAPDGSLDLMMWKCIIPGK
Picsi_SCE1_1         (1)  -MSGGIARGRLAEERKAWRKNHPHGFVARPDTQPDGSLNLMVWQCIIPGK
Phypa_SCE1_1         (1)  -MSGGIARGRLAEERKAWRKNHPHGFVARPETGADGALNLMVWQCTLPGK
Phypa_SCE1_2         (1)  -MSGGIARGRLAEERKAWRKNHPHGFVARPETCADGALNLMVWQCTLPGK
Vitvi_SCE1_1         (1)  -MSGGIARGRLAEERKAWRKNHPHGFVAKPETGPDGSVNLMVWHCTIPGK
Helan_SCE1_1         (1)  -MSGGIARGRLTEERKAWRKNHPHGFVAKPETLPGGTVNLMIWSCIIPGK
Horvu_SCE1_1         (1)  MSGGGIARGRLAEERKAWRKNHPHGFVAKPETMADGSVNLMIWNCTIPGK
Triae_SCE1_1         (1)  MSGGGIARGRLAEERKAWRKNHPHGFVAKPETVADGSVNLMIWNCTIPGK
Zeama_SCE1_1         (1)  -MSGGIARGRLAEERKAWRKNHPHGFVARPETLADGSANLMVWSCTIPGK
Zeama_SCE1_2         (1)  -MSGGIARGRLAEERKAWRKNHPHGFVARPESLTDGSVNLMVWNCTIPGK
Orysa_SCE1_2         (1)  -MSGGIARGRLAEERKAWRKNHPHGFVAKPETMADGSANLMIWHCTIPGK
Orysa_SCE1_1         (1)  -MSGGIARGRLAEERKAWRKNHPHGFVAKPETLADGTVNLMIWHCTIPGK
Zeama_SCE1_3         (1)  -MSGGIARGRLAEERKAWRKNHPHGFVAKPESLPDGTVNLMIWQCTIPGK
Tritu_SCE1_1         (1)  MSSGGIARGRLAEERKAWRKNHPHGFVAKPETLGDGTVNLMVWHCTIPGK
   Consensus         (1)   MSGGIARGRLAEERKAWRKNHPHGFVAKPET  DGSVNLMVW CTIPGK
```

FIGURE 2

```
                        51                                                100
Arath_SCE1_1    (50)   AGTDWEGGFFPLTMHFSEDYPSKPPKCKFPQGFFHPNVYPSGTVCLSILN
 Glyma_SCE_1    (49)   AGTDWEGGYFPLTMHFSEDYPSKPPKCKFPQGFFHPNVYPSGTVCLSILN
Popul_SCE1_1    (50)   LGTDWEGGYFPLTLNFSEDYPSKPPKCKFPQGFFHPNVYPSGTVCLSILN
Pruar_SCE1_1    (16)   TGTDWEGGFFPLTLHFSEDYPSKPPKCKFPPGFFHPNVYPSGTVCLSILN
Nicbe_SCE1_1    (50)   AGTDWEGGFYPVTIHFSEDYPSKPPKCKFPQGFFHPNVYPSGTVCLSILN
Chlre_SCE1_1    (49)   QGTDWEGGFYPLTMEFSEDYPTKPPKCKFPAGFFHPNIYPSGTVCLSILN
Ostta_SCE1_1    (49)   RGTRWEGAFVPLTMEFPREYPAKPMKCKFPAGFYHPNVYPSGTVCLSILN
Orysa_SCE1_3    (11)   EGTDWEGGYFPLTMQFTEDYPTNAPSCKFPSGFFHINVYDSGAVCLSIL-
PopTr_SCE1_1    (51)   PGTDWEGGFFPLSLHFSEDYPSKPPKCKFPQGFFHPNVYPSGTVCLSILN
PopTr_SCE1_2    (51)   PGTDWEGGYFPLTLHFSEDYPSKPPKCKFPQGFFHPNVYPSGTVCLSILN
Picsi_SCE1_1    (50)   SGTDWEGGYFPLTINFSEDYPSKPPKCKFPQGFFHPNVYPSGTVCLSILN
Phypa_SCE1_1    (50)   VGTDWEGGFYPVAIHFSEDYPSKPPKCKFPQGFFHPNVYPSGTVCLSILN
Phypa_SCE1_2    (50)   VGTDWEGGFYPVAIHFTEDYPSKPPKCKFPQGFFHPNVYPSGTVCLSILN
Vitvi_SCE1_1    (50)   AGTDWEGGYFPLTLHFSEDYPSKPPKCKFPQGFFHPNVYPSGTVCLSILN
Helan_SCE1_1    (50)   NGTDWEGGFYPLTLHFTEDYPSKPPKCKFPQGFFHPNVYPSGTVCLSILN
Horvu_SCE1_1    (51)   QGTDWESGYYPLTLHFSEDYPSKPPKCKFPQGFFHPNVYPSGTVCLSILN
Triae_SCE1_1    (51)   QGTDWESGYYPLTLHFSEDYPSKPPKCKFPQGFFHPNVYPSGTVCLSILN
Zeama_SCE1_1    (50)   QGTDWESGYYPLTLHFSEDYPSKPPKCKFPQGFFHPNVYPSGTVCLSILN
Zeama_SCE1_2    (50)   HGTDWEGGYYPLTLHFSEDYPSKPPKCKFPQGFFHPNVYPSGTVCLSILN
Orysa_SCE1_2    (50)   QGTDWEGGYYPLTLHFSEDYPSKPPKCKFPQGFFHPNVYPSGTVCLSILN
Orysa_SCE1_1    (50)   QGTDWEGGYFPLTLHFSEDYPSKPPKCKFPQGFFHPNVYPSGTVCLSILN
Zeama_SCE1_3    (50)   QGTDWEGGYFPLTLHFSEDYPSKPPKCKFPQGFFHPNVYPSGTVCLSILN
Tritu_SCE1_1    (51)   QGTDWEGGYFPLTLHFSEDYPSKPPKCKFPTNFFHPNVYPSGTVCLSILN
   Consensus    (51)    GTDWEGGYFPLTLHFSEDYPSKPPKCKFPQGFFHPNVYPSGTVCLSILN


                        101                                               150
Arath_SCE1_1   (100)   EDYGWRPAITVKQILVGIQDLLDTPNPADPAQTDGYHLFCQDPVEYKKRV
 Glyma_SCE_1    (99)   EDSGWRPAITVKQILVGIQDLLDQPNPADPAQTEGYHLFIQDAAEYKRRV
Popul_SCE1_1   (100)   EDSGWRPAITVKQILVGIQDLLDQPNPADPAQTEGYHLFIQDAAEYKKRV
Pruar_SCE1_1    (66)   EDSGWRPAITVKQILVGIQDLLDQPNPADPAQTEGYHLFIQDATEYKKRV
Nicbe_SCE1_1   (100)   EDSGWRPAITVKQILVGIQDLLDQPNPADPAQTEGYHLFIQDAIEYKKRV
Chlre_SCE1_1    (99)   EDEGWRPSITIKQLLLGIQELLDTPNPGSPAQSDAFVLFTQQKAEYVKKV
Ostta_SCE1_1    (99)   EDEGWRPSVTVKQVALGIQELLDNPNEKSPAQSDAYVTYTTDRAKYERRV
Orysa_SCE1_3    (60)   -STAWKPSITVRQILIGIQELFDDPNPNSAAQNISYELYRTWRSTGNAFV
PopTr_SCE1_1   (101)   EDYGWRPAITVKQILVGIQDLLDQPNPSDPAQTDGYQLFVQDPTEYRRRV
PopTr_SCE1_2   (101)   EDYGWRPAITVKQILIGIQDLLDQPNPSDPAQTDGYQLFVQDPAEYRRRV
Picsi_SCE1_1   (100)   EDSGWRPAITVKQILIGIQDLLDQPNPGDPAQTDGYHLFIQDLTEYKRRV
Phypa_SCE1_1   (100)   EDSGWRPAITVKQILVGIQELLDAPNPADPAQTEAYQLFIQDPVEYKRRV
Phypa_SCE1_2   (100)   EDSGWRPAITVKQILVGIQELLDAPNPADPAQTEAYQLFIQDPVEYKRRV
Vitvi_SCE1_1   (100)   EDSGWRPAITVKQILVGIQDLLDQPNPADPAQTDGYQLFIQEPAEYKRRV
Helan_SCE1_1   (100)   EDSGWRPAITVKQILVGIQDLLDSPNPADPAQTDGYHLFIQDTVEYKRRV
Horvu_SCE1_1   (101)   EDSGWRPAITVKQILVGIQDLLDQPNPADPAQTDGYHLFIQDPAEYRRRI
Triae_SCE1_1   (101)   EDSGWRPAITVKQILVGIQDLLDQPNPADPAQTDGYHLFIQDPAEYKRRI
Zeama_SCE1_1   (100)   EDSGWRPAITVKQILVGIQDLLDQPNPADPAQTDGYHLFIQDPTEYKRRV
Zeama_SCE1_2   (100)   EDSDWRPAITVKQILVGIQDLLDQPNPADPAQTDGYHLFIQDPAEYKRRV
Orysa_SCE1_2   (100)   EDSGWRPAITVKQILVGIQDLLDQPNPADPAQTDGYHIFIQDKPEYKRRV
Orysa_SCE1_1   (100)   EDSGWRPAITVKQILVGIQDLLDQPNPADPAQTDGYHLFIQDPTEYKRRV
Zeama_SCE1_3   (100)   EDSGWRPAITVKQILVGIQDLLDQPNPADPAQTDGYHLFIQDPTEYKRRV
Tritu_SCE1_1   (101)   EDSGWRPAITVKQILVGIQDLLDQPNPADPAQTDGYHLFIQDPAEYKRRV
   Consensus   (101)   EDSGWRPAITVKQILVGIQDLLDQPNPADPAQTDGYHLFIQDP EYKRRV
```

**FIGURE 2 (continued)**

```
                          151                    175
Arath_SCE1_1  (150) KLQ-------SKQYPALV-------
 Glyma_SCE_1  (149) RQQ-------SKQYPPLV-------
Popul_SCE1_1  (150) RQQ-------AKQYPSLV-------
Pruar_SCE1_1  (116) RQQ-------AKQYPPLV-------
Nicbe_SCE1_1  (150) RLQ-------AKQYPPLV-------
Chlre_SCE1_1  (149) KRQ-------ALNYPPPS-------
Ostta_SCE1_1  (149) REE-------VAKYPPPE-------
Orysa_SCE1_3  (109) SRLRSILQLCSRAMPAGFWQLKHFD
PopTr_SCE1_1  (151) RQQ-------AKQYPPAL-------
PopTr_SCE1_2  (151) RQQ-------AKLYPPTL-------
Picsi_SCE1_1  (150) RQQ-------AKQYPPLV-------
Phypa_SCE1_1  (150) RQQ-------AKQYPPPI-------
Phypa_SCE1_2  (150) RQQ-------AKQYPPPI-------
Vitvi_SCE1_1  (150) RQQ-------AKQYPPLV-------
Helan_SCE1_1  (150) RQQ-------AKQYPALV-------
Horvu_SCE1_1  (151) RLQ-------AKQYPALV-------
Triae_SCE1_1  (151) RLQ-------AKQYPALV-------
Zeama_SCE1_1  (150) RLQ-------AKQYPALV-------
Zeama_SCE1_2  (150) RLQ-------AKQYPALV-------
Orysa_SCE1_2  (150) RVQ-------AKQYPALL-------
Orysa_SCE1_1  (150) RLQ-------AKQYPPIV-------
Zeama_SCE1_3  (150) KLQ-------AKQYPALV-------
Tritu_SCE1_1  (151) RAQ-------AKQYPALV-------
   Consensus  (151) R Q       AKQYPPLV
```

**FIGURE 2 (continued)**

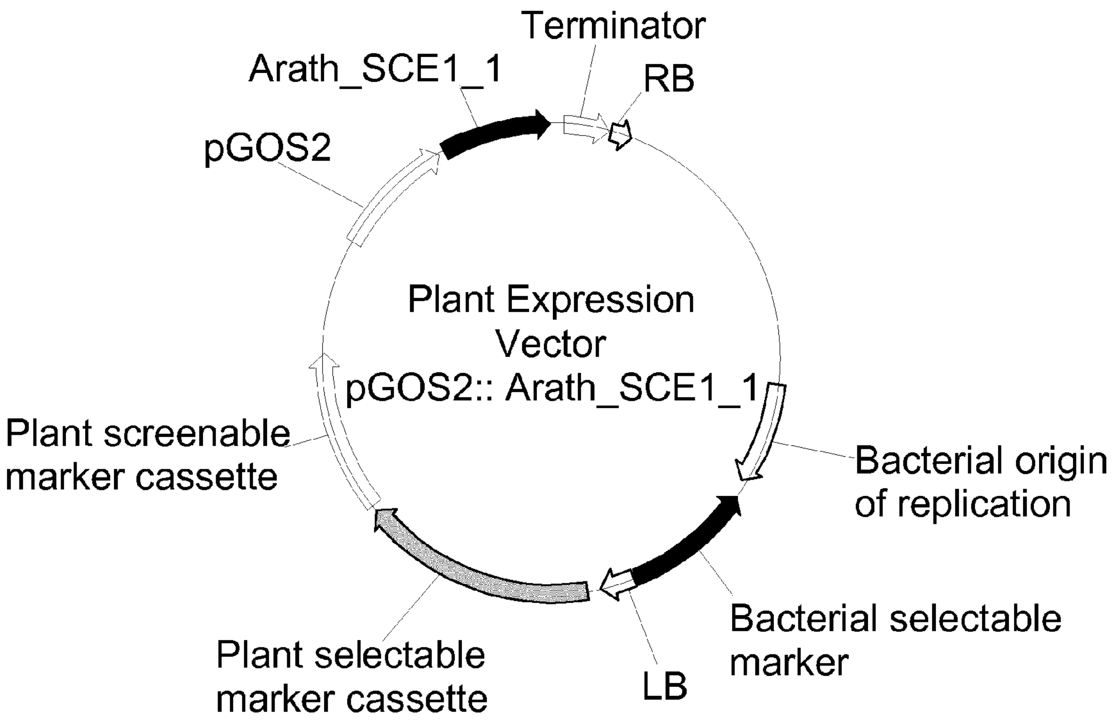


**FIGURE 3**

**SEQ ID NO: 199, DNA - Arabidopsis thaliana**
ATGGCTAGTGGAATCGCTCGTGGTCGTTTAGCTGAAGAGAGGAAATCGTGGAGGAAGAATCATCCT
CATGGTTTTGTGGCAAAGCCGGAGACGGGGCAGGATGGAACTGTGAATCTAATGGTGTGGCATTGC
ACTATACCTGGTAAAGCTGGGACTGATTGGGAAGGTGGATTCTTTCCATTAACGATGCACTTCAGT
GAGGATTATCCGAGCAAACCTCCGAAATGTAAATTTCCACAAGGGTTTTTCCACCCTAATGTCTAT
CCATCTGGAACTGTCTGTCTCTCTATCCTTAACGAGGATTATGGATGGAGACCAGCCATCACCGTG
AAGCAGATTCTTGTTGGTATTCAGGATTTACTTGACACACCGAATCCCGCTGACCCTGCACAGACA
GATGGTTATCATCTCTTCTGTCAGGATCCAGTTGAGTACAAGAAAAGGGTGAAGCTGCAGTCCAAG
CAGTATCCTGCTCTTGTCTAA

**SEQ ID NO: 200, protein - Arabidopsis thaliana**
MASGIARGRLAEERKSWRKNHPHGFVAKPETGQDGTVNLMVWHCTIPGKAGTDWEGGFFPLTMHFS
EDYPSKPPKCKFPQGFFHPNVYPSGTVCLSILNEDYGWRPAITVKQILVGIQDLLDTPNPADPAQT
DGYHLFCQDPVEYKKRVKLQSKQYPALV

**SEQ ID NO: 201, DNA - Helianus annuus**
ATGTCCGGTGGAATTGCTCGCGGCCGTCTCACCGAGGAACGCAAAGCATGGCGCAAGAATCATCCT
CATGGTTTTGTGGCGAAACCGGAGACTCTACCTGGCGGTACGGTTAATTTGATGATTTGGAGTTGT
ATCATCCCTGGTAAGAATGGGACCGACTGGGAGGGCGGTTTTTACCCCCTTACCCTCCACTTCACC
GAGGATTATCCGAGCAAACCACCAAAGTGTAAATTCCCTCAAGGCTTCTTCCACCCCAATGTTTAC
CCTTCTGGGACCGTTTGTTTGTCCATCCTTAACGAAGATAGTGGTTGGAGGCCAGCAATAACAGTT
AAACAAATTCTAGTTGGCATCCAGGACTTGCTGGATTCCCCCAATCCCGCTGATCCCGCCCAGACT
GATGGATATCATCTCTTTATCCAGGACACGGTGGAGTACAAGAGACGGGTCCGCCAGCAAGCAAAG
CAATACCCAGCACTCGTGTAG

**SEQ ID NO: 202, protein - Helianus annuus**
MSGGIARGRLTEERKAWRKNHPHGFVAKPETLPGGTVNLMIWSCIIPGKNGTDWEGGFYPLTLHFT
EDYPSKPPKCKFPQGFFHPNVYPSGTVCLSILNEDSGWRPAITVKQILVGIQDLLDSPNPADPAQT
DGYHLFIQDTVEYKRRVRQQAKQYPALV

**SEQ ID NO: 203, DNA - Triticum aestivum**
AAAAAAAGTCGAGAAGACACAATCAAGGAGTCAAGTTCAAAAGTATCCACACATCCATCATTCAAG
CAGAAGGACCTGATTTTTGTTTGGTCAACACCAGACCACACGTCACAAATGTCCACGGTCACAATG
GAAAGCTCTCCACAATACCCCGCTTTCGGTTATATTTAGTCATACTTCATATTTTCAGGTGCGGTA
ATTTATGATTTTAGACTGCTACATGGATCCTCAAACCAGAGCAGGGTACTGCTTGGCCTGCAGCCG
AATGCGTCTCTTGTACTCTGCTGGATCCTGGATAAAGAGGTGATAACCATCAGTCTGGGCAGGATC
AGCTGGATTAGGTTGATCAAGCAAATCCTGTATTCCAACTAGAATCTGCTTAACAGTGATGGCAGG
TCTCCAACCACTATCCTCATTAAGAATCGAGAGGCAGACCGTCCCTGAAGGATAGACATTTGGGTG
GAAAAAACCCTGCGGGAACTTGCACTTGGGAGGTTTGCTAGGGTAGTCCTCACTGAAATGGAGAGT
AAGTGGGTAGTATCCACTTTCCCAATCAGTCCCCTGCTTTCCGGGGATGGTGCAGTTCCAGATCAT
GAGATTCACCGACCCGTCGGCCACCGTCTCCGGCTTCGCGACGAATCCATGGGGGTGGTTCTTGCG
CCAGGCCTTGCGCTCCTCCGCGAGGCGGCCCCGCGCGATCCCTCCTCCTGACATGGGCGGCGGCGG
AGGAGGCTGCTGGCTGCTCTCTGTCGCTGAGTCTGGGGTTTGGGTTTCGGAGTCTGCGCAGTTTCT
AATCCTTCAGTCCATGTCAGTGTCCTCGTGCTCCAGTCGCGGACGCGTTGGGTCAAGATTTTCCGG
GAATTTCGGGACCGNTACCAGCCTGGTTTTTTTGTTACAAACTGGTTCTATAGGTGTCACTAAATA
GGCCTAATGGTCATACCTGGTTCCTGTGTGAAAATGTTATCGGCCCGGGGGCTAAGGTAAAGTTCG
GAGGTTGGATGGTCCCGGGGTTTTCTATGGAGGTCAAAACAGGGTGATTGGGTTTCCGGGGAACTG
ACGGTTACTAAACGAGCTTGTTTTTTTAAACTCCACGGGTAACCCTTCCGCCCTATTTGTTAA

# FIGURE 4

**SEQ ID NO: 204, protein - Triticum aestivum**
MSGGGIARGRLAEERKAWRKNHPHGFVAKPETVADGSVNLMIWNCTIPGKQGTDWESGYYPLTLHF
SEDYPSKPPKCKFPQGFFHPNVYPSGTVCLSILNEDSGWRPAITVKQILVGIQDLLDQPNPADPAQ
TDGYHLFIQDPAEYKRRIRLQAKQYPALV

**SEQ ID NO: 205, DNA - Hordeum vulgare**
ATGTCAGGAGGAGGGATCGCCCGCGGCCGCCTCGCGGAGGAGCGCAAGGCCTGGCGCAAGAACCAC
CCCCATGGATTCGTCGCGAAGCCGGAGACGATGGCCGACGGGTCGGTGAATCTCATGATCTGGAAC
TGCACCATCCCCGGAAAGCAGGGGACTGATTGGGAAAGTGGATACTACCCACTTACCCTCCATTTC
AGTGAGGACTACCCTAGTAAACCTCCCAAATGCAAGTTCCCGCAGGGTTTTTTCCACCCAAATGTC
TATCCTTCAGGGACGGTCTGCCTCTCGATTCTTAATGAGGATAGCGGTTGGAGACCTGCCATCACT
GTTAAGCAGATCCTAGTTGGAATACAGGATTTGCTTGATCAACCTAATCCAGCTGATCCTGCCCAG
ACTGATGGTTATCACCTCTTTATCCAGGATCCAGCAGAGTATAGGAGGCGTATTCGGCTGCAGGCT
AAGCAGTACCCTGCTCTGGTTTGA

**SEQ ID NO: 206, protein - Hordeum vulgare**
MSGGGIARGRLAEERKAWRKNHPHGFVAKPETMADGSVNLMIWNCTIPGKQGTDWESGYYPLTLHF
SEDYPSKPPKCKFPQGFFHPNVYPSGTVCLSILNEDSGWRPAITVKQILVGIQDLLDQPNPADPAQ
TDGYHLFIQDPAEYRRRIRLQAKQYPALV

**SEQ ID NO: 207, DNA - Glycine max**
ATGTCTGGTATCGCCCGTGGACGTCTTGCCGAGGAGCGAAAGTCATGGCGCAAAAACCACCCTCAT
GGTTTCGTTGCCAAGCCCGAGACTCTCCCTGATGCCACCGTTAATTTGATGGTCTGGCATTGCACT
ATTCCTGGCAAGGCTGGGACTGATTGGGAGGGTGGATATTTCCCACTGACAATGCACTTCAGTGAA
GATTACCCTAGCAAGCCTCCCAAGTGCAAATTCCCTCAAGGTTTCTTTCACCCCAATGTGTATCCA
TCTGGAACTGTTTGCTTGTCTATACTTAATGAAGATAGTGGATGGAGACCAGCTATAACAGTGAAG
CAAATTCTTGTGGGCATCCAGGACCTGCTTGATCAACCAAATCCTGCTGACCCTGCCCAGACGGAG
GGTTATCATCTATTCATCCAGGATGCAGCTGAGTACAAGAGAAGAGTCCGACAGCAGTCAAAGCAA
TATCCACCTCTTGTCTAG

**SEQ ID NO: 208, protein - Glycine max**
MSGIARGRLAEERKSWRKNHPHGFVAKPETLPDATVNLMVWHCTIPGKAGTDWEGGYFPLTMHFSE
DYPSKPPKCKFPQGFFHPNVYPSGTVCLSILNEDSGWRPAITVKQILVGIQDLLDQPNPADPAQTE
GYHLFIQDAAEYKRRVRQQSKQYPPLV

**SEQ ID NO: 209, DNA - Zea mays**
ATGTCGGGAGGAATCGCGCGCGGCCGCCTCGCCGAGGAGCGCAAGGCCTGGCGCAAGAACCACCCC
CACGGTTTCGTGGCGAGGCCGGAAACGCTGGCCGACGGGTCGGCGAACCTCATGGTCTGGAGCTGT
ACCATCCCCGGCAAGCAGGGGACTGATTGGGAAAGTGGGTACTACCCACTTACCCTTCATTTCAGT
GAAGATTACCCAAGCAAGCCTCCCAAATGCAAGTTCCCACAGGGTTTTTTCCACCCAAATGTTTAT
CCTTCAGGAACAGTCTGCCTCTCAATTCTCAATGAGGATAGTGGTTGGAGACCTGCTATCACTGTT
AAGCAGATTCTAGTTGGAATACAAGACTTGCTTGATCAGCCCAATCCAGCTGATCCTGCCCAAACT
GATGGTTATCACCTATTCATCCAGGATCCAACAGAATATAAGCGACGTGTTCGTCTGCAGGCCAAG
CAATATCCTGCTCTGGTCTGA

**SEQ ID NO: 210, protein - Zea mays**
MSGGIARGRLAEERKAWRKNHPHGFVARPETLADGSANLMVWSCTIPGKQGTDWESGYYPLTLHFS
EDYPSKPPKCKFPQGFFHPNVYPSGTVCLSILNEDSGWRPAITVKQILVGIQDLLDQPNPADPAQT
DGYHLFIQDPTEYKRRVRLQAKQYPALV

**FIGURE 4 (continued)**

**SEQ ID NO: 211, DNA - Zea mays**
ATGTCTGGAGGGATCGCGCGCGGCCGCCTCGCCGAGGAGCGCAAGGCCTGGCGCAAGAATCACCCC
CACGGTTTCGTGGCGAGGCCGGAGTCGCTGACCGACGGGTCCGTGAACCTCATGGTCTGGAACTGT
ACCATCCCCGGCAAGCACGGGACCGATTGGGAAGGTGGGTACTACCCACTTACCCTTCATTTCAGT
GAAGATTACCCAAGCAAACCTCCCAAATGCAAGTTTCCACAGGGTTTTTTCCATCCAAATGTTTAT
CCATCAGGAACAGTCTGCCTCTCAATTCTGAACGAGGATAGCGATTGGAGACCTGCTATCACTGTT
AAGCAGATTCTAGTTGGAATACAGGACTTGCTTGATCAGCCCAATCCAGCTGATCCTGCTCAGACT
GATGGTTATCACCTATTCATCCAGGATCCTGCAGAATATAAGCGACGTGTTCGTCTGCAGGCCAAG
CAATATCCTGCTCTGGTCTGA

**SEQ ID NO: 212, protein - Zea mays**
MSGGIARGRLAEERKAWRKNHPHGFVARPESLTDGSVNLMVWNCTIPGKHGTDWEGGYYPLTLHFS
EDYPSKPPKCKFPQGFFHPNVYPSGTVCLSILNEDSDWRPAITVKQILVGIQDLLDQPNPADPAQT
DGYHLFIQDPAEYKRRVRLQAKQYPALV

**SEQ ID NO: 213, DNA - Zea mays**
ATGTCTGGGGGAATCGCCCGCGGCCGCCTCGCCGAGGAGCGCAAGGCCTGGCGCAAGAACCACCCG
CACGGTTTCGTCGCGAAGCCGGAGTCGCTGCCCGACGGGACGGTGAACCTGATGATCTGGCAGTGC
ACCATCCCCGGCAAGCAAGGGACTGACTGGGAAGGTGGATATTTCCCTCTCACCCTTCATTTTAGT
GAGGATTACCCTAGCAAGCCTCCCAAGTGCAAGTTCCCTCAGGGTTTCTTCCACCCAAATGTGTAT
CCTTCTGGAACAGTCTGTCTTTCGATCCTTAATGAAGATAGTGGTTGGAGACCAGCTATTACTGTT
AAGCAGATTCTCGTCGGGATCCAGGACTTGCTAGATCAGCCAAATCCTGCTGATCCTGCTCAAACG
GATGGCTATCACCTTTTTATCCAGGATCCTACAGAATATAAGAGGCGTGTTAAACTGCAGGCGAAG
CAGTATCCCGCGTTGGTCTGA

**SEQ ID NO: 214, protein - Zea mays**
MSGGIARGRLAEERKAWRKNHPHGFVAKPESLPDGTVNLMIWQCTIPGKQGTDWEGGYFPLTLHFS
EDYPSKPPKCKFPQGFFHPNVYPSGTVCLSILNEDSGWRPAITVKQILVGIQDLLDQPNPADPAQT
DGYHLFIQDPTEYKRRVKLQAKQYPALV

**SEQ ID NO: 215, DNA - Oryza sativa**
ATGTCGGGGGGAATCGCGCGCGGCCGCCTCGCGGAGGAGCGGAAGGCGTGGCGGAAGAACCACCCA
CACGGTTTCGTCGCCAAGCCGGAGACGTTGGCCGACGGGACGGTCAACCTCATGATCTGGCACTGC
ACAATCCCCGGCAAGCAAGGGACTGATTGGGAAGGTGGATACTTTCCTCTCACTCTTCATTTCAGT
GAGGATTACCCTAGCAAACCTCCCAAGTGCAAGTTCCCACAGGGTTTCTTCCACCCAAATGTCTAT
CCTTCAGGGACAGTCTGCCTTTCAATTCTTAATGAAGACAGCGGTTGGAGACCTGCTATTACCGTC
AAGCAAATTCTTGTTGGAATCCAGGACTTGCTTGATCAGCCTAATCCTGCTGATCCTGCTCAGACC
GATGGTTACCATCTTTTTATCCAGGATCCTACGGAATACAAGAGGCGTGTTCGGCTGCAGGCCAAG
CAGTATCCTCCGATTGTCTGA

**SEQ ID NO: 216, protein - Oryza sativa**
MSGGIARGRLAEERKAWRKNHPHGFVAKPETLADGTVNLMIWHCTIPGKQGTDWEGGYFPLTLHFS
EDYPSKPPKCKFPQGFFHPNVYPSGTVCLSILNEDSGWRPAITVKQILVGIQDLLDQPNPADPAQT
DGYHLFIQDPTEYKRRVRLQAKQYPPIV

## FIGURE 4 (continued)

**SEQ ID NO: 217, DNA - Oryza sativa**
ATGTCGGGAGGGATCGCACGCGGCCGCCTCGCGGAGGAGCGCAAGGCCTGGCGGAAGAACCACCCT
CACGGGTTCGTGGCGAAGCCGGAGACGATGGCCGACGGGTCGGCGAACCTCATGATCTGGCACTGC
ACCATCCCCGGCAAGCAGGGGACCGATTGGGAAGGTGGGTACTACCCTCTTACCCTTCACTTCAGT
GAGGACTATCCTAGCAAACCACCCAAGTGCAAGTTCCCACAGGGCTTTTTCCACCCAAATGTCTAT
CCTTCAGGAACAGTGTGCCTCTCAATTCTTAATGAGGATAGTGGCTGGAGACCTGCTATCACTGTA
AAGCAGATCCTTGTTGGAATACAGGACTTGCTTGATCAGCCAAATCCTGCTGATCCTGCACAGACT
GACGGTTATCACATTTTTATACAGGACAAACCAGAATATAAGAGGCGTGTTCGTGTTCAGGCCAAG
CAGTACCCTGCTTTGCTTTGA

**SEQ ID NO: 218, protein - Oryza sativa**
MSGGIARGRLAEERKAWRKNHPHGFVAKPETMADGSANLMIWHCTIPGKQGTDWEGGYYPLTLHFS
EDYPSKPPKCKFPQGFFHPNVYPSGTVCLSILNEDSGWRPAITVKQILVGIQDLLDQPNPADPAQT
DGYHIFIQDKPEYKRRVRVQAKQYPALL

**SEQ ID NO: 219, DNA - Oryza sativa**
ATGGTCTGGCGATGCATCATCCCCGGCAAAGAAGGGACTGATTGGGAGGGTGGATATTTCCCACTT
ACTATGCAATTCACTGAAGACTATCCAACCAACGCTCCTTCTTGCAAGTTCCCATCGGGTTTCTTC
CACATCAATGTCTATGACTCTGGGGCAGTATGCCTATCAATCTTGAGTACCGCATGGAAACCTTCA
ATTACAGTGAGGCAAATTCTTATAGGCATCCAGGAATTGTTTGATGATCCAAACCCTAACTCTGCT
GCACAGAATATAAGCTATGAGCTTTATAGGACATGGAGGAGTACAGGAAACGCGTTCGTCAGCAGG
CTAAGAAGTATCCTTCAGCTCTGTAGCCGCGCAATGCCTGCAGGATTCTGGCAGCTAAAACATTTT
GATTGA

**SEQ ID NO: 220, protein - Oryza sativa**
MVWRCIIPGKEGTDWEGGYFPLTMQFTEDYPTNAPSCKFPSGFFHINVYDSGAVCLSILSTAWKPS
ITVRQILIGIQELFDDPNPNSAAQNISYELYRTWRSTGNAFVSRLRSILQLCSRAMPAGFWQLKHF
D

**SEQ ID NO: 221, DNA - Vitis vinifera**
ATGTCAGGAGGCATCGCGCGTGGTCGTCTCGCCGAGGAGCGAAAAGCCTGGCGTAAGAATCATCCC
CATGGTTTCGTGGCTAAGCCAGAGACTGGTCCGGACGGTTCTGTCAATTTGATGGTGTGGCATTGC
ACCATCCCTGGTAAGGCTGGGACTGATTGGGAAGGGGGCTACTTCCCACTTACTTTGCACTTCAGT
GAGGACTACCCTAGCAAACCCCCAAAGTGCAAGTTCCCTCAAGGTTTCTTCCACCCTAATGTCTAC
CCATCTGGAACTGTATGTCTCTCGATCCTCAATGAAGACAGTGGTTGGAGACCTGCCATTACAGTG
AAACAAATTCTAGTGGGCATTCAAGACTTGCTGGACCAGCCCAATCCTGCAGATCCAGCACAAACT
GATGGGTATCAGCTCTTCATCCAGGAACCCGCAGAGTATAAAAGAAGGGTGCGGCAACAGGCCAAG
CAATATCCACCTCTTGTCTAA

**SEQ ID NO: 222, protein - Vitis vinifera**
MSGGIARGRLAEERKAWRKNHPHGFVAKPETGPDGSVNLMVWHCTIPGKAGTDWEGGYFPLTLHFS
EDYPSKPPKCKFPQGFFHPNVYPSGTVCLSILNEDSGWRPAITVKQILVGIQDLLDQPNPADPAQT
DGYQLFIQEPAEYKRRVRQQAKQYPPLV

## FIGURE 4 (continued)

**SEQ ID NO: 223, DNA - Nicotiana benthamiana**
ATGTCAGGAGGTATAGCCCGTGGCCGTCTTGCAGAGGAGCGCAAAGCTTGGCGCAAAAATCACCCC
CATGGGTTTGTAGCAAAGCCAGAGACGCTTTCGGATGGGTCAGTTAACTTGATGGTTTGGCACTGC
AGTATTCCTGGTAAAGCAGGAACGGACTGGGAAGGCGGTTTTTATCCGGTTACGATACACTTCAGT
GAAGATTATCCTAGCAAACCACCTAAGTGCAAATTCCCACAAGGCTTCTTCCATCCGAATGTCTAT
CCATCAGGAACAGTTTGCTTGTCGATCCTCAACGAAGATAGCGGTTGGAGACCTGCCATTACAGTG
AAACAGATACTGGTTGGTATCCAAGACTTGTTAGATCAGCCAAACCCTGCTGATCCTGCCCAAACC
GAAGGGTATCATCTCTTTATTCAGGATGCTATTGAGTACAAGAAGCGGGTTAGGCTGCAGGCCAAG
CAGTATCCTCCTCTGGTGTAG

**SEQ ID NO: 224, protein - Nicotiana benthamiana**
MSGGIARGRLAEERKAWRKNHPHGFVAKPETLSDGSVNLMVWHCSIPGKAGTDWEGGFYPVTIHFS
EDYPSKPPKCKFPQGFFHPNVYPSGTVCLSILNEDSGWRPAITVKQILVGIQDLLDQPNPADPAQT
EGYHLFIQDAIEYKKRVRLQAKQYPPLV

**SEQ ID NO: 225, DNA - Populus x canadensis**
ATGTCAGGTGGCATCGCACGTGGTCGTCTTGCTGAGGAAAGGAAGTCCTGGCGCAAAAACCACCCT
CATGGTTTTGTGGCGAAACCAGAGACACAGCCAGATGGAACAGTAAATTTGATGGTCTGGCATTGC
ACAATCCCTGGAAAACTTGGTACTGATTGGGAAGGTGGTTATTTTCCTCTTACACTCAACTTCAGT
GAAGATTATCCTAGCAAGCCACCAAAGTGTAAATTTCCTCAGGGTTTCTTCCACCCTAATGTATAT
CCATCTGGAACTGTTTGCTTGTCAATCCTTAACGAGGACAGTGGATGGAGACCAGCCATCACAGTG
AAGCAGATTCTTGTGGGTATCCAGGACTTGCTGGACCAGCCAAATCCTGCTGATCCTGCCCAAACT
GAAGGTTATCATCTGTTTATCCAGGATGCTGCAGAGTACAAGAAAAGAGTTCGCCAGCAAGCTAAG
CAATACCCTTCTCTTGTCTAA

**SEQ ID NO: 226, protein - Populus x canadensis**
MSGGIARGRLAEERKSWRKNHPHGFVAKPETQPDGTVNLMVWHCTIPGKLGTDWEGGYFPLTLNFS
EDYPSKPPKCKFPQGFFHPNVYPSGTVCLSILNEDSGWRPAITVKQILVGIQDLLDQPNPADPAQT
EGYHLFIQDAAEYKKRVRQQAKQYPSLV

**SEQ ID NO: 227, DNA - Triticum turgidum**
ATGTCTTCCGGTGGGATCGCGCGCGGCCGCCTCGCGGAGGAGCGCAAGGCCTGGCGGAAGAACCAC
CCCCACGGCTTCGTCGCCAAGCCGGAGACGCTGGGCGACGGCACGGTCAACCTCATGGTCTGGCAC
TGCACCATCCCCGGCAAGCAAGGGACTGATTGGGAAGGTGGATACTTCCCTCTCACCCTTCATTTC
AGCGAGGATTACCCCAGCAAGCCTCCCAAGTGCAAGTTCCCTACAAATTTCTTCCACCCGAATGTC
TATCCTTCGGGGACAGTCTGCCTTTCAATCCTCAATGAGGACAGCGGCTGGAGACCTGCTATTACT
GTGAAGCAAATCCTTGTTGGAATTCAGGACTTGCTTGATCAGCCCAACCCGGCTGACCCTGCTCAG
ACTGATGGTTATCACCTTTTCATCCAGGATCCAGCTGAGTACAAGAGGCGTGTTCGGGCGCAGGCA
AAGCAGTATCCCGCATTGGTCTGA

**SEQ ID NO: 228, protein - Triticum turgidum**
MSSGGIARGRLAEERKAWRKNHPHGFVAKPETLGDGTVNLMVWHCTIPGKQGTDWEGGYFPLTLHF
SEDYPSKPPKCKFPTNFFHPNVYPSGTVCLSILNEDSGWRPAITVKQILVGIQDLLDQPNPADPAQ
TDGYHLFIQDPAEYKRRVRAQAKQYPALV

## FIGURE 4 (continued)

**SEQ ID NO: 229, DNA - Populus trichocarpa**
ATGTCAGGAGGAGGCATAGCTCGTGGTCGTCTTGCTGAAGAGAGAAAGTCATGGCGTAAGAATCAT
CCCCACGGTTTTGTGGCTAAGCCTGATAATGCACAAGATGGTTCTCTTGATTTGATGGTGTGGAAG
TGCATCATACCTGGCAAACCCGGGACAGATTGGGAGGGTGGCTTCTTCCCCCTCTCGCTTCATTTC
AGTGAGGACTACCCAAGCAAACCTCCAAAGTGCAAGTTCCCCCAAGGTTTCTTCCACCCTAATGTC
TACCCTTCAGGAACTGTGTGCTTATCTATTCTCAATGAGGACTATGGCTGGAGACCAGCCATTACT
GTGAAGCAAATTTTAGTTGGCATTCAGGATTTGCTTGATCAACCAAATCCTTCTGATCCTGCGCAA
ACTGATGGCTATCAGCTTTTTGTCCAGGACCCGACTGAGTACAGGAGAAGGGTGCGCCAACAAGCC
AAGCAATATCCACCTGCGCTCTGA

**SEQ ID NO: 230, protein - Populus trichocarpa**
MSGGGIARGRLAEERKSWRKNHPHGFVAKPDNAQDGSLDLMVWKCIIPGKPGTDWEGGFFPLSLHF
SEDYPSKPPKCKFPQGFFHPNVYPSGTVCLSILNEDYGWRPAITVKQILVGIQDLLDQPNPSDPAQ
TDGYQLFVQDPTEYRRRVRQQAKQYPPAL

**SEQ ID NO: 231, DNA - Populus trichocarpa**
ATGTCAGGAGGAGGCATAGCTCGTGGTCGTCTTGCTGAAGAGAGAAAGGCATGGCGTAAGAATCAC
CCTCACGGTTTTGTGGCTAAGCCTGATAATGCTCCAGATGGTTCTCTAGATTTGATGATGTGGAAG
TGCATTATACCTGGCAAACCCGGGACTGATTGGGAGGGTGGCTACTTCCCCCTCACTCTTCATTTC
AGTGAGGACTACCCAAGCAAACCTCCAAAGTGCAAGTTCCCCCAAGGTTTCTTCCACCCTAATGTC
TACCCTTCAGGAACTGTGTGCTTATCTATCCTCAATGAGGACTATGGCTGGAGACCAGCCATTACA
GTGAAGCAAATATTAATTGGCATTCAGGATTTGCTTGATCAACCAAATCCTTCTGATCCTGCACAA
ACTGATGGCTATCAGCTTTTTGTCCAGGACCCTGCTGAGTACAGGAGAAGGGTGCGCCAACAAGCC
AAGCTATACCCACCTACGCTCTGA

**SEQ ID NO: 232, protein - Populus trichocarpa**
MSGGGIARGRLAEERKAWRKNHPHGFVAKPDNAPDGSLDLMMWKCIIPGKPGTDWEGGYFPLTLHF
SEDYPSKPPKCKFPQGFFHPNVYPSGTVCLSILNEDYGWRPAITVKQILIGIQDLLDQPNPSDPAQ
TDGYQLFVQDPAEYRRRVRQQAKLYPPTL

**SEQ ID NO: 233, DNA - Physcomitrella patens**
ATGTCAGGAGGAATCGCACGCGGTCGTCTTGCGGAGGAGCGCAAGGCCTGGCGCAAGAATCATCCT
CATGGATTTGTAGCGAGGCCGGAGACAGGTGCAGATGGAGCTCTAAATTTGATGGTTTGGCAGTGC
ACTTTGCCTGGAAAAGTTGGGACGGACTGGGAAGGTGGATTTTACCCTGTAGCAATTCACTTCAGT
GAGGATTATCCCAGCAAGCCCCCCAAGTGCAAGTTTCCACAGGGTTTTTTCCACCCCAACGTTTAT
CCTTCAGGCACAGTTTGCCTATCCATCCTTAATGAAGATTCTGGTTGGAGACCAGCTATCACTGTA
AAACAGATCCTTGTGGGTATTCAGGAGCTTCTCGACGCTCCGAACCCAGCAGATCCCGCTCAAACC
GAAGCCTATCAGCTTTTTATTCAAGATCCAGTTGAATACAAGCGTCGTGTTAGGCAGCAAGCCAAG
CAGTACCCACCGCCAATTTAA

**SEQ ID NO: 234, protein - Physcomitrella patens**
MSGGIARGRLAEERKAWRKNHPHGFVARPETGADGALNLMVWQCTLPGKVGTDWEGGFYPVAIHFS
EDYPSKPPKCKFPQGFFHPNVYPSGTVCLSILNEDSGWRPAITVKQILVGIQELLDAPNPADPAQT
EAYQLFIQDPVEYKRRVRQQAKQYPPPI

## FIGURE 4 (continued)

**SEQ ID NO: 235, DNA - Physcomitrella patens**
ATGTCGGGAGGAATTGCGCGGGGTCGACTTGCGGAGGAGCGCAAGGCCTGGCGGAAGAATCATCCT
CATGGGTTTGTGGCTAGGCCTGAGACATGTGCAGATGGAGCTCTTAATTTGATGGTTTGGCAGTGT
ACTTTACCTGGAAAAGTTGGGACCGACTGGGAAGGTGGATTCTATCCTGTAGCAATTCACTTTACT
GAAGATTATCCCAGCAAGCCTCCTAAGTGCAAATTCCCACAGGGTTTCTTCCACCCCAACGTGTAT
CCTTCAGGCACAGTTTGCCTCTCCATCCTGAATGAAGATTCGGGTTGGAGACCAGCTATCACCGTG
AAGCAGATCCTCGTCGGTATCCAGGAGCTGCTCGACGCTCCAAACCCAGCAGATCCCGCTCAGACT
GAAGCTTATCAGCTTTTTATTCAGGATCCAGTTGAATACAAGCGACGAGTAAGGCAGCAAGCCAAG
CAATACCCACCACCAATCTAA

**SEQ ID NO: 236, protein - Physcomitrella patens**
MSGGIARGRLAEERKAWRKNHPHGFVARPETCADGALNLMVWQCTLPGKVGTDWEGGFYPVAIHFT
EDYPSKPPKCKFPQGFFHPNVYPSGTVCLSILNEDSGWRPAITVKQILVGIQELLDAPNPADPAQT
EAYQLFIQDPVEYKRRVRQQAKQYPPPI

**SEQ ID NO: 237, DNA - Chlamydomonas reinhardtii**
ATGTCTGGCGTCGCACGCTCACGCTTGCAAGAGGAGCGGAAAGCCTGGCGGAGGGATAAGCCGTTC
GGCTTCCATGCTCGACCAGAAACCGCAGACGACGGGAGCGTGAACCTGATGAAGTGGAAGTGCCAC
ATCCCCGGGAAACAAGGCACGGACTGGGAGGGCGGCTTCTACCCGCTCACCATGGAGTTCAGCGAG
GACTACCCCACCAAGCCGCCCAAGTGCAAGTTCCCCGCGGGCTTCTTCCACCCCAACATCTACCCC
TCCGGTACCGTGTGCCTCAGCATCCTCAACGAGGACGAGGGCTGGCGGCCCTCCATCACCATCAAG
CAGCTGCTGTTGGGCATCCAGGAGCTGCTGGACACGCCCAACCCCGGCAGCCCCGCCCAGTCCGAC
GCCTTCGTGCTGTTCACGCAGCAGAAGGCCGAGTACGTCAAGAAGGTGAAGCGCCAGGCGCTCAAC
TACCCGCCACCCTCGTGA

**SEQ ID NO: 238, protein - Chlamydomonas reinhardtii**
MSGVARSRLQEERKAWRRDKPFGFHARPETADDGSVNLMKWKCHIPGKQGTDWEGGFYPLTMEFSE
DYPTKPPKCKFPAGFFHPNIYPSGTVCLSILNEDEGWRPSITIKQLLLGIQELLDTPNPGSPAQSD
AFVLFTQQKAEYVKKVKRQALNYPPPS

**SEQ ID NO: 239, DNA - Prunus armeniaca**
GGGACGGTGAATTTGATGGTGTGGCATTGCACGATTCCTGGCAAGACCGGTACTGACTGGGAGGGG
GGTTTTTTCCCACTTACCCTTCACTTCAGTGAAGACTACCCTAGCAAGCCTCCAAAGTGTAAATTC
CCACCAGGTTTCTTCCACCCAAATGTATATCCATCTGGAACTGTTTGTCTATCAATTCTTAATGAG
GACAGTGGTTGGAGACCAGCAATAACCGTGAAGCAAATTCTTGTGGGCATTCAGGATTTACTGGAT
CAGCCAAATCCTGCTGATCCTGCACAGACAGAAGGGTATCACCTCTTCATTCAGGATGCCACGGAG
TACAAGAAAAGGGTTCGGCAGCAGGCCAAGCAATACCCACCTCTAGTTTAA

**SEQ ID NO: 240, protein - Prunus armeniaca**
GTVNLMVWHCTIPGKTGTDWEGGFFPLTLHFSEDYPSKPPKCKFPPGFFHPNVYPSGTVCLSILNE
DSGWRPAITVKQILVGIQDLLDQPNPADPAQTEGYHLFIQDATEYKKRVRQQAKQYPPLV

## FIGURE 4 (continued)

**SEQ ID NO: 241, DNA - Ostreococus tauri**
ATGGCGTCGGTCGCCCTCGCGCGCCTGGGCGAGGAGCGCCGAAACTGGCGTCGCGACCACCCGCCC
GGATTCTTCGCGCGTCCCGAGAAAACGGCGAGCGGGGAGACGAATCTGTTTCGATGGCGGTGCTCG
ATACCGGGCGCGCGCGGGACGCGCTGGGAGGGCGCGTTCGTGCCGCTGACGATGGAGTTCCCGAGG
GAGTACCCGGCCAAGCCGATGAAGTGTAAATTTCCTGCGGGATTTTATCACCCGAACGTGTACCCG
AGCGGGACGGTGTGCCTGAGCATTCTGAACGAGGACGAGGGGTGGCGGCCGAGCGTGACGGTGAAG
CAGGTGGCGCTGGGGATACAGGAACTGCTGGATAATCCGAACGAGAAGTCGCCGGCGCAGAGCGAT
GCGTACGTGACGTACACGACGGATAGGGCGAAGTACGAGCGGAGGGTGAGGGAGGAGGTGGCGAAG
TATCCGCCGCCGGAGTAG

**SEQ ID NO: 242, protein - Ostreococus tauri**
MASVALARLGEERRNWRRDHPPGFFARPEKTASGETNLFRWRCSIPGARGTRWEGAFVPLTMEFPR
EYPAKPMKCKFPAGFYHPNVYPSGTVCLSILNEDEGWRPSVTVKQVALGIQELLDNPNEKSPAQSD
AYVTYTTDRAKYERRVREEVAKYPPPE

**SEQ ID NO: 243, DNA - Picea sitchensis**
ATGTCTGGAGGCATAGCTCGAGGTCGACTTGCTGAGGAGCGGAAGGCCTGGCGTAAGAACCATCCC
CATGGTTTCGTAGCTAGACCTGACACTCAGCCAGATGGTTCCCTTAACTTAATGGTGTGGCAATGC
ATCATTCCTGGCAAATCTGGGACGGATTGGGAGGGTGGTTACTTTCCTCTAACAATTAATTTCAGT
GAGGATTACCCAAGTAAACCTCCAAAATGCAAGTTCCCTCAAGGGTTTTTCCATCCTAATGTTTAT
CCATCAGGAACTGTTTGTCTGTCTATCCTTAATGAGGATTCTGGGTGGCGGCCAGCCATTACTGTG
AAGCAAATACTTATAGGTATTCAAGACCTTTTAGATCAGCCAAATCCAGGTGATCCTGCACAAACG
GATGGCTACCATCTTTTTATCCAAGACCTCACAGAATACAAGCGGAGAGTTCGACAACAGGCAAAA
CAATACCCACCTCTGGTGTGA

**SEQ ID NO: 244, protein - Picea sitchensis**
MSGGIARGRLAEERKAWRKNHPHGFVARPDTQPDGSLNLMVWQCIIPGKSGTDWEGGYFPLTINFS
EDYPSKPPKCKFPQGFFHPNVYPSGTVCLSILNEDSGWRPAITVKQILIGIQDLLDQPNPGDPAQT
DGYHLFIQDLTEYKRRVRQQAKQYPPLV

**SEQ ID NO: 245, DNA - Artificial sequence**
GGGGACAAGTTTGTACAAAAAAGCAGGCTTAAACAATGGCTAGTGGAATCGCTC

**SEQ ID NO: 246, DNA - Artificial sequence**
GGGGACCACTTTGTACAAGAAAGCTGGGTATCAGTTTTGGTGCGTTCTC

**SEQ ID NO: 247, DNA - Oryza sativa**
AATCCGAAAAGTTTCTGCACCGTTTTCACCCCCTAACTAACAATATAGGGAACGTGTGCTAAATAT
AAAATGAGACCTTATATATGTAGCGCTGATAACTAGAACTATGCAAGAAAAACTCATCCACCTACT
TTAGTGGCAATCGGGCTAAATAAAAAAGAGTCGCTACACTAGTTTCGTTTTCCTTAGTAATTAAGT
GGGAAAATGAAATCATTATTGCTTAGAATATACGTTCACATCTCTGTCATGAAGTTAAATTATTCG
AGGTAGCCATAATTGTCATCAAACTCTTCTTGAATAAAAAAATCTTTCTAGCTGAACTCAATGGGT
AAAGAGAGAGATTTTTTTTAAAAAAATAGAATGAAGATATTCTGAACGTATTGGCAAAGATTTAAA
CATATAATTATATAATTTTATAGTTTGTGCATTCGTCATATCGCACATCATTAAGGACATGTCTTA
CTCCATCCCAATTTTTATTTAGTAATTAAAGACAATTGACTTATTTTTATTATTTATCTTTTTTCG
ATTAGATGCAAGGTACTTACGCACACACTTTGTGCTCATGTGCATGTGTGAGTGCACCTCCTCAAT
ACACGTTCAACTAGCAACACATCTCTAATATCACTCGCCTATTTAATACATTTAGGTAGCAATATC

## FIGURE 4 (continued)

TGAATTCAAGCACTCCACCATCACCAGACCACTTTTAATAATATCTAAAATACAAAAAATAATTTT
ACAGAATAGCATGAAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACA
GAACAACCCACAAAAAACGATGATCTAACGGAGGACAGCAAGTCCGCAACAACCTTTTAACAGCAG
GCTTTGCGGCCAGGAGAGAGGAGGAGAGGCAAAGAAAACCAAGCATCCTCCTTCTCCCATCTATAA
ATTCCTCCCCCCTTTTCCCCTCTCTATATAGGAGGCATCCAAGCCAAGAAGAGGGAGAGCACCAAG
GACACGCGACTAGCAGAAGCCGAGCGACCGCCTTCTCGATCCATATCTTCCGGTCGAGTTCTTGGT
CGATCTCTTCCCTCCTCCACCTCCTCCTCACAGGGTATGTGCCTCCCTTCGGTTGTTCTTGGATTT
ATTGTTCTAGGTTGTGTAGTACGGGCGTTGATGTTAGGAAAGGGGATCTGTATCTGTGATGATTCC
TGTTCTTGGATTTGGGATAGAGGGGTTCTTGATGTTGCATGTTATCGGTTCGGTTTGATTAGTAGT
ATGGTTTTCAATCGTCTGGAGAGCTCTATGGAAATGAAATGGTTTAGGGATCGGAATCTTGCGATT
TTGTGAGTACCTTTTGTTTGAGGTAAAATCAGAGCACCGGTGATTTTGCTTGGTGTAATAAAGTAC
GGTTGTTTGGTCCTCGATTCTGGTAGTGATGCTTCTCGATTTGACGAAGCTATCCTTTGTTTATTC
CCTATTGAACAAAAATAATCCAACTTTGAAGACGGTCCCGTTGATGAGATTGAATGATTGATTCTT
AAGCCTGTCCAAAATTTCGCAGCTGGCTTGTTTAGATACAGTAGTCCCCATCACGAAATTCATGGA
AACAGTTATAATCCTCAGGAACAGGGGATTCCCTGTTCTTCCGATTTGCTTTAGTCCCAGAATTTT
TTTTCCCAAATATCTTAAAAAGTCACTTTCTGGTTCAGTTCAATGAATTGATTGCTACAAATAATG
CTTTTATAGCGTTATCCTAGCTGTAGTTCAGTTAATAGGTAATACCCCTATAGTTTAGTCAGGAGA
AGAACTTATCCGATTTCTGATCTCCATTTTTAATTATATGAAATGAACTGTAGCATAAGCAGTATT
CATTTGGATTATTTTTTTTATTAGCTCTCACCCCTTCATTATTCTGAGCTGAAAGTCTGGCATGAA
CTGTCCTCAATTTTGTTTTCAAATTCACATCGATTATCTATGCATTATCCTCTTGTATCTACCTGT
AGAAGTTTCTTTTTGGTTATTCCTTGACTGCTTGATTACAGAAAGAAATTTATGAAGCTGTAATCG
GGATAGTTATACTGCTTGTTCTTATGATTCATTTCCTTTGTGCAGTTCTTGGTGTAGCTTGCCACT
TTCACCAGCAAAGTTC

## FIGURE 4 (continued)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5811238 A
- US 6395547 A
- WO 2004070039 A
- WO 2004065596 A
- US 4962028 A
- WO 0114572 A
- WO 9514098 A
- WO 9412015 A
- US 5401836 A
- US 20050044585 A
- EP 99106056 A
- US 5565350 A, Kmiec
- WO 9322443 A, Zarling
- WO 9853083 A, Grierson
- WO 9953050 A, Waterhouse
- US 4987071 A, Cech
- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- WO 0015815 A
- EP 1198985 A1
- US 5164310 A
- US 5159135 A


### Non-patent literature cited in the description


- **WANG et al.** *Planta,* 2003, vol. 218, 1-14
- **MIURA et al.** *Current Opinion in Plant Biol.,* 2007, vol. 10, 495-502
- **LOIS et al.** *The Plant Cell,* 2003, vol. 15, 1347-1359
- **KRAFT et al.** *Plant Phys,* 2005, 1597-1611
- **CASTILO et al.** *J. virology,* 2004, vol. 78, 2758-2769
- **CREIGHTON.** Proteins. W.H. Freeman and Company, 1984
- **TERPE.** *Appl. Microbiol. Biotechnol.,* 2003, vol. 60, 523-533
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989
- **FOISSAC ; SCHIEX.** *BMC Bioinformatics,* 2005, vol. 6, 25
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Science,* 1999, vol. 39 (6), 1696
- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- *Plant Mol Biol.,* January 1995, vol. 27 (2), 237-48
- **MUDGE et al.** *Plant J.,* 2002, vol. 31, 341
- **NITZ et al.** *Plant Sci,* 2001, vol. 161 (2), 337-346
- **TINGEY et al.** *EMBO J.,* 1987, vol. 6, 1
- **VAN DER ZAAL et al.** *Plant Mol. Biol.,* 1991, vol. 16, 983
- **OPPENHEIMER et al.** *Gene,* 1988, vol. 63, 87

- **CONKLING et al.** *Plant Physiol.,* 1990, vol. 93, 1203
- **SUZUKI et al.** *Plant Mol. Biol.,* 1993, vol. 21, 109-119
- **BAUMBERGER et al.** *Genes & Dev.,* 2001, vol. 15, 1128
- **LAUTER et al.** *PNAS,* 1996, vol. 3, 8139
- **LIU et al.** *Plant Mol. Biol.,* 1991, vol. 153, 386-395
- **DOWNEY et al.** *J. Biol. Chem.,* 2000, vol. 275, 39420
- **W SONG.** *PhD Thesis,* 1997
- **WANG et al.** *Plant Sci.,* 2002, vol. 163, 273
- **DIENER et al.** *Plant Cell,* 2001, vol. 13, 1625
- **QUESADA et al.** *Plant Mol. Biol.,* 1997, vol. 34, 265
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1994, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* 1986, 15-22
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **WAGNER ; KOHORN.** *Plant Cell,* 2001, vol. 13 (2), 303-318
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418

- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **TRIBBLE et al.** *J. Biol. Chem.,* 2000, vol. 275, 22255-22267
- **VELMURUGAN et al.** *J. Cell Biol.,* 2000, vol. 149, 553-566
- **KRENS, F.A. et al.** *Nature,* 1982, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, vol. 1, 128-143
- **POTRYKUS.** *Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants. **F.F. WHITE.** Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- **FELDMANN K.** Methods in Arabidopsis Research. Word Scientific, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **MILLER et al.** *Nature Biotechnol.,* 2007, vol. 25, 778-785
- **MULDER et al.** *Nucl. Acids. Res.,* 2003, vol. 31, 315-318
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- A generalized profile syntax for biomolecular sequences motifs and its function in automatic sequence interpretation. **BUCHER ; BAIROCH.** ISMB-94; Proceedings 2nd International Conference on Intelligent Systems for Molecular Biology. AAAI Press, 1994, 53-61
- **HULO et al.** *Nucl. Acids. Res.,* 2004, vol. 32, D134-D137
- **BATEMAN et al.** *Nucleic Acids Research,* 2002, vol. 30 (1), 276-280
- **GASTEIGER et al.** ExPASy: the proteomics server for in-depth protein knowledge and analysis. *Nucleic Acids Res.,* 2003, vol. 31, 3784-3788
- **NEEDLEMAN ; WUNSCH.** *J Mol Biol,* 1970, vol. 48, 443-453
- **ALTSCHUL et al.** *J Mol Biol,* 1990, vol. 215, 403-410
- **CAMPANELLA et al.** MatGAT: an application that generates similarity/identity matrices using protein or DNA sequences. *BMC Bioinformatics,* 10 July 2003, vol. 4, 29

- **SMITH TF ; WATERMAN MS.** *J. Mol. Biol,* 1981, vol. 147 (1), 195-197
- Current Protocols in Molecular Biology. Wiley
- **RABBANI et al.** *Plant Physiol,* 2003, vol. 133, 1755-1767
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* 1989, vol. 17, 6795-6807
- **SAMBROOK.** Molecular Cloning: a laboratory manual. Cold Spring Harbor Laboratory Press, 2001
- **AUSUBEL et al.** Current Protocols in Molecular Biology. Current Protocols, 1994, vol. 1, 2
- **R.D.D. CROY.** Plant Molecular Biology Labfax. BIOS Scientific Publications Ltd (UK, 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **THOMPSON et al.** *Nucleic Acids Res,* 1997, vol. 25, 4876-4882
- **CHENNA et al.** *Nucleic Acids Res,* 2003, vol. 31, 3497-3500
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659
- **GAMBORG et al.** *Exp. Cell Res.,* 1968, vol. 50, 151-158